(19) Europäisches Patentamt
European Patent Office
Office européen des brevets

(11) EP 1 373 233 B1

(12) FASCICULE DE BREVET EUROPEEN

(45) Date de publication et mention de la délivrance du brevet:
**05.09.2007 Bulletin 2007/36**

(21) Numéro de dépôt: **02724383.1**

(22) Date de dépôt: **27.03.2002**

(51) Int Cl.:
***C07D 295/18*** (2006.01) ***C07D 211/34*** (2006.01)
***C07C 237/30*** (2006.01) ***C07C 311/19*** (2006.01)
***C07D 317/60*** (2006.01) ***C07D 319/18*** (2006.01)
***C07D 213/40*** (2006.01) ***C07D 215/36*** (2006.01)
***C07D 205/04*** (2006.01) ***C07D 317/66*** (2006.01)
***C07D 405/12*** (2006.01) ***C07D 271/12*** (2006.01)
***C07D 333/62*** (2006.01) ***C07D 307/52*** (2006.01)
***C07D 307/54*** (2006.01) ***C07D 277/80*** (2006.01)
***C07D 233/54*** (2006.01) ***C07D 213/76*** (2006.01)
***C07D 409/12*** (2006.01) ***C07D 333/24*** (2006.01)
***C07D 317/46*** (2006.01)

(86) Numéro de dépôt international:
**PCT/FR2002/001059**

(87) Numéro de publication internationale:
**WO 2002/076964 (03.10.2002 Gazette 2002/40)**

# (54) DERIVES DE N-(ARYLSULFONYL)BETA-AMINOACIDES COMPORTANT UN GROUPE AMINOETHYLE SUBSTITUE, LEUR PROCEDE DE PREPARATION ET LES COMPOSITIONS PHARMACEUTIQUES EN CONTENANT

AMINOMETHYLGRUPPEN ENTHALTENDE N-(ARYLSULFONYL)BETA-AMINOSÄURE-DERIVATE, VERFAHREN ZU DEREN HERSTELLUNG SOWIE DEREN PHARMAZEUTISCHE ZUBEREITUNGEN

DERIVATIVES OF N-(ARYLSULFONYL)BETA-AMINOACIDS COMPRISING A SUBSTITUTED AMINOMETHYL GROUP, THE PREPARATION METHOD THEREOF AND THE PHARMACEUTICAL COMPOSITIONS CONTAINING SAME

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **28.03.2001 FR 0104315**

(43) Date de publication de la demande:
**02.01.2004 Bulletin 2004/01**

(73) Titulaire: **Sanofi-Aventis**
**75013 Paris (FR)**

(72) Inventeurs:
- **FERRARI, Bernard**
**F-34270 Les Matelles (FR)**
- **GOUGAT, Jean**
**F-34790 Grabels (FR)**
- **MUNEAUX, Yvette**
**F-34270 Les Matelles (FR)**
- **PERREAUT, Pierre**
**F-34980 Saint Clément de Rivière (FR)**
- **SARRAN, Lionel**
**FR-34270 Saint Jean de Cuculles (FR)**

(74) Mandataire: **Varady, Peter et al**
**Sanofi-Aventis**
**174 avenue de France**
**75013 Paris (FR)**

(56) Documents cités:
**EP-A- 0 614 911** **WO-A-96/40639**
**WO-A-97/25315**

- **DATABASE CHEMCATS [en ligne] CHEMICAL ABSTRACT SERVICE, COLUMBUS, OHIO, US; retrieved from STN Database accession no. 2002: 642428 XP002206094 & "ChemDiv, Inc. Product Library" 26 avril 2001 (2001-04-26) , CHEMDIV, INC. , SAN DIEGO, CA, 92121 USA**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention a pour objet des nouveaux dérivés de N-(arylsulfonyl)béta-aminoacides comportant un groupe aminométhyle substitué, leur préparation, les compositions pharmaceutiques en contenant.

Ces composés présentent une affinité pour les récepteurs de la bradykinine (BK). La bradykinine est un nonapeptide appartenant comme le décapeptide kallidine à la classe des kinines et qui montre une activité physiologique dans le domaine cardiovasculaire et comme médiateur dans l'inflammation et la douleur. On distingue plusieurs récepteurs de la bradykinine : les récepteurs $B_1$ et $B_2$ (D. Regoli et al., Pharmacol. Rev., 1980, 32, 1-46). Plus précisément, les récepteurs $B_2$ sont les récepteurs de la bradykinine et de la kallidine : ils sont prédominants et on les trouve normalement dans la plupart des tissus ; les récepteurs $B_1$ sont les récepteurs spécifiques de la [des-$Arg^9$] bradykinine et de la [des-$Arg^{10}$] kallidine : ils sont induits au cours des processus inflammatoires.

Les récepteurs de la bradykinine ont été clonés pour différentes espèces, notamment pour l'espèce humaine : récepteur $B_1$ : J.G. Menke et al., J. Biol. Chem., 1994, 269 (34), 21583-21586 ; récepteur $B_2$ : J.F. Hess, Biochem. Biophys. Res. Commun., 1992, 184, 260-268.

La demande de brevet WO 97-25315 décrit des composés de formule :

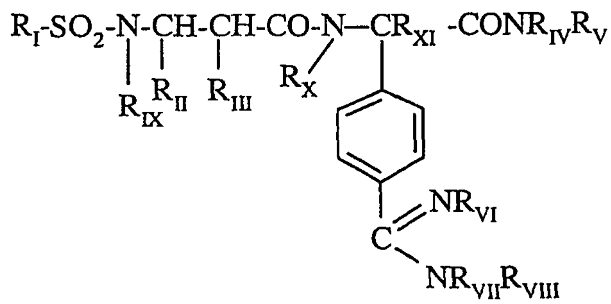

(A)

dans laquelle :

- $R_I$, $R_{II}$, $R_{III}$, $R_{IV}$, $R_V$, $R_{VI}$, $R_{VII}$, $R_{VIII}$, $R_{IX}$, $R_X$ et $R_{XI}$ ont différentes valeurs. Ces composés présentent une affinité pour les récepteurs de la bradykinine.

On a maintenant trouvé de nouveaux composés qui présentent une affinité pour les récepteurs de la bradykinine avec une sélectivité pour les récepteurs $B_1$ de la bradykinine, qui sont des antagonistes des récepteurs $B_1$ de la bradykinine et qui présentent des avantages quant à leur absorption.

Ces composés peuvent être utilisés pour la préparation de médicaments utiles pour le traitement ou la prévention de toutes pathologies où la bradykinine et les récepteurs $B_1$ sont impliqués, notamment les pathologies de l'inflammation et les maladies inflammatoires persistantes ou chroniques.

Ainsi, selon un de ses aspects, la présente invention a pour objet des composés de formule :

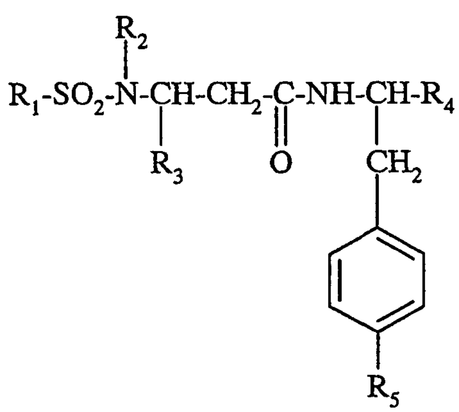

(I)

dans laquelle :

- $R_1$ représente un groupe phénylvinyle ; phényle non substitué ou substitué une ou plusieurs fois par $R_6$, identiques ou différents ; naphtyle non substitué ou substitué une ou plusieurs fois par $R_6$, identiques ou différents ; tétrahydronaphtyle-; naphto[2,3-d][1,3]dioxol-6-yle ; un radical hétérocyclique choisi parmi quinolyle, isoquinolyle, 1-benzofuran-2-yle, 2,1,3-benzoxadiazol-4-yle, 2,1,3-benzothiadiazol-4-yle, 1,3-benzothiazol-2-yle, 1-benzothiophèn-2-yle, 1*H*-pyrazol-4-yle, thièn-2-yle, 5-isoxazolthièn-2-yle, benzothièn-2-yle, thiéno[3,2-c]pyridin-2-yle ; lesdits radicaux hétérocycliques étant non substitués ou substitués une ou plusieurs fois par $R_6$ identiques ou différents ;
- $R_2$ représente l'hydrogène ou un groupe $(C_1-C_4)$alkyle et $R_3$ représente un groupe phényle non substitué ou substitué une ou plusieurs fois par $R_7$, identiques ou différents ; un radical hétérocyclique choisi parmi benzo[1,3]dioxol-5-yle, 2,1,3-benzothiadiazol-5-yle, 2,1,3-benzoxadiazol-5-yle, benzothiophèn-5-yle, 2,3-dihydro-benzo[1,4]dioxin-6-yle, benzofuranyle, dihydrobenzofuranyle, 1,3-thiazol-2-yle, furyle, thiènyle, ledit radical hétérocyclique étant non substitué ou substitué une ou plusieurs fois par un atome d'halogène ou par un groupe $(C_1-C_4)$alkyle ;
- ou bien $R_2$ représente un groupe phényle non substitué ou substitué une ou plusieurs fois par $R_6$, identiques ou différents ; un radical hétérocyclique choisi parmi benzo[1,3]dioxol-5-yle, pyridyle, indanyle, et $R_3$ représente l'hydrogène ;
- $R_4$ représente un groupe $-CONR_8R_9$ ; un groupe $-CSNR_8R_9$ ; un groupe $-COR_{13}$ ; un groupe phényle non substitué ou substitué une ou plusieurs fois par $R_{10}$ ; un radical hétérocyclique choisi parmi pyridyle, imidazolyle, furyle, benzimidazolyle, benzothiazol-2-yle, benzo[1,3]dioxol-5-yle, lesdits radicaux étant non substitués ou substitués par un ou plusieurs groupes méthyle ou atomes d'halogène ;
- $R_5$ représente un groupe $-CH_2NR_{11}R_{12}$ ou $-CH_2N(O)R_{11}R_{12}$ ;
- $R_6$ représente un atome d'halogène ; un groupe $(C_1-C_4)$alkyle ; trifluorométhyle ; $(C_1-C_4)$ alcoxy ; 2-fluoroéthoxy ; trifluorométhoxy, méthylènedioxy, difluorométhylènedioxy ;
- $R_7$ représente un atome d'halogène ; un groupe $(C_1-C_4)$alkyle ; phényle ; trifluorométhyle ; $(C_1-C_4)$alcoxy ; benzyloxy ; trifluorométhoxy ;
- $R_8$ et $R_9$ représentent chacun indépendamment l'hydrogène ; un groupe $(C_1-C_4)$alkyle ; $(C_3-C_7)$cycloalkyle ; $(C_3-C_7)$cycloalkyle$(C_1-C_4)$alkyle ; ω-$(C_1-C_4)$ dialkylamino$(C_2-C_4)$alkyle ;
- ou bien $R_8$ et $R_9$ ensemble avec l'atome d'azote auquel ils sont liés constituent un radical hétérocyclique choisi parmi pyrrolidinyle, morpholin-4-yle, thiomorpholin-4-yle, azépin-1-yle, pipéridinyle non substitué ou substitué par un ou plusieurs atomes d'halogène ou un ou plusieurs groupes $(C_1-C_4)$alkyle ou $(C_1-C_4)$alcoxy ou trifluorométhyle, 3,4-dihydropipéridin-1-yle, cyclohexylspiro-4-pipéridin-1-yle, pipérazinyle non substitué ou substitué par un ou plusieurs groupes $(C_1-C_4)$alkyle ;
- $R_{10}$ représente un atome halogène ; un groupe $(C_1-C_4)$alkyle ; hydroxy ; $(C_1-C_6)$alcoxy ; $R_{10}$ peut de plus représenter un groupe $-CH_2NR_{11}R_{12}$ lorsque $R_5$ représente un groupe-$CH_2NR_{11}R_{12}$, lesdits groupes étant alors identiques ;
- $R_{11}$ et $R_{12}$ représentent chacun indépendamment l'hydrogène ; un groupe $(C_1-C_6)$alkyle ; $(C_2-C_4)$alcènyle ; $(C_3-C_7)$cycloalkyle ; $(C_3-C_7)$cycloalkyle$(C_1-C_4)$alkyle ; ω-hydroxy$(C_2-C_4)$alkylène; ω-méthoxy$(C_2-C_4)$alkylène ; (ω-trifluorométhyl$(C_2-C_4)$alkylène ; ω-halogéno$(C_2-C_4)$alkylène,
- - ou bien $R_{11}$ et $R_{12}$ ensemble avec l'atome d'azote auquel ils sont liés constituent un radical hétérocyclique, mono ou bicyclique, choisi parmi azétidinyle, pyrrolidinyle, morpholin-4-yle, thiomorpholin-4-yle, pipéridin-1-yle, pipérazin-1-yle, 1,2,3,6-tétrahydropyrid-1-yle, 2,3,4,5-tétrahydropyridinium, decahydroquinolyle, decahydroisoquinolyle, tétrahydroisoquinolyle, octahydro-1*H*-isoindolyle, $(C_4-C_6)$cycloalkylspiropipéridinyle, 3-azabicyclo[3.1.0]hexyle, 7-azabicyclo[2.2.1]heptan-7-yle, non substitués ou substitués une ou plusieurs fois par un atome d'halogène, un groupe $(C_1-C_4)$alkyle, hydroxy, $(C_1-C_4)$alcoxy, trifluorométhyle, difluorométhylène, phényle ;
- $R_{13}$ représente un groupe phényle, thiazol-2-yle ou pyridyle ;

ainsi que leurs sels avec des acides minéraux ou organiques et/ou leurs solvats ou leurs hydrates.

Par halogène, on entend un atome de fluor, de chlore, de brome ou d'iode.

Par alkyle, alkylène ou respectivement alcoxy, on entend un radical alkyle, un radical alkylène ou respectivement un radical alcoxy linéaire ou ramifié.

Les composés de formule (I) comprennent au moins un atome de carbone asymétrique et les énantiomères ou diastéréoisomères purs ainsi que leur mélange en proportions quelconques sont objets de l'invention.

Préférentiellement, la présente invention a pour objet des composés de formule :

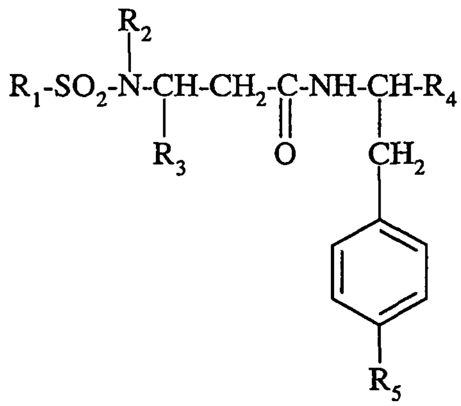

(I)

dans laquelle :

- $R_1$ représente un groupe phénylvinyle ; phényle non substitué ou substitué une ou plusieurs fois par $R_6$, identiques ou différents ; naphtyle non substitué ou substitué une ou plusieurs fois par $R_6$, identiques ou différents ; tétrahydronaphtyle ; un radical hétérocyclique choisi parmi quinolyle, 1-benzofuran-2-yle, 2,1,3-benzoxadiazol-4-yle, 2,1,3-benzothiadiazol-4-yle, 1,3-benzothiazol-2-yle, 1-benzothiophèn-2-yle, 1*H*-pyrazol-4-yle, thièn-2-yle, S-isoxazolthièn-2-yle, benzothièn-2-yle, thiéno[3,2-c]pyridin-2-yle ; naphto[2,3-d][1,3]dioxol-6-yle ; lesdits radicaux hétérocycliques étant non substitués ou substitués une ou plusieurs fois par $R_6$ identiques ou différents ;
- $R_2$ représente l'hydrogène ou un groupe $(C_1-C_4)$alkyle et $R_3$ représente un groupe phényle non substitué ou substitué une ou plusieurs fois par $R_7$, identiques ou différents ; un radical hétérocyclique choisi parmi benzo[1,3]dioxol-5-yle non substitué ou substitué en -2 par deux atomes de fluor; 2,1,3-benzothiadiâzol-5-yle ; 2,3-dihydro-benzo[1,4]dioxin-6-yle ; 1,3-thiazol-2-yle ; 1-benzofuran-2-yle ; 1-benzofuran-5-yle ; furyle ; thièn-2-yle ; thièn-3-yle ;
- ou bien $R_2$ représente un groupe phényle non substitué ou substitué une ou plusieurs fois par $R_6$, identiques ou différents ; un radical hétérocyclique choisi parmi benzo[1,3]dioxol-5-yle ; pyridyle ; indanyle ; et $R_3$ représente l'hydrogène ;
- $R_4$ représente un groupe $-CONR_8R_9$ ; un groupe phényle non substitué ou substitué une ou plusieurs fois par $R_{10}$ ; un radical hétérocyclique choisi parmi pyridyle, imidazolyle, furyle, benzimidazolyle, benzothiazol-2-yle, benzo[1,3]dioxol-5-yle, lesdits radicaux étant non substitués ou substitués par un méthyle ;
- $R_5$ représente un groupe $-CH_2NR_{11}R_{12}$;
- $R_6$ représente un atome d'halogène ; un groupe $(C_1-C_4)$alkyle ; trifluorométhyle ; $(C_1-C_4)$ alcoxy ; 2-fluoroéthoxy ; trifluorométhoxy, méthylènedioxy, difluorométhylènedioxy ;
- $R_7$ représente un atome d'halogène ; un groupe $(C_1-C_4)$alkyle ; trifluorométhyle ; $(C_1-C_4)$alcoxy ; benzyloxy ; trifluorométhoxy ;
- $R_8$ et $R_9$ représentent chacun indépendamment l'hydrogène ; un groupe $(C_1-C_4)$alkyle ; $(C_3-C_7)$cycloalkyle ; $(C_3-C_7)$cycloalkyle$(C_1-C_4)$alkyle ; ω-$(C_1-C_4)$ dialkylamino$(C_2-C_4)$alkyle ;
- ou bien $R_8$ et $R_9$ ensemble avec l'atome d'azote auquel ils sont liés constituent un radical hétérocyclique choisi parmi pyrrolidinyle, pipéridinyle, morpholin-4-yle, thiomorpholin-4-yle, pipérazin-1-yle, 4-méthylpipérazin-1-yle, 4-méthylpipéridin-1-yle, 2-méthylpipéridin-1-yle, 4,4-diméthylpipéridin-1-yle, 4,4-difluoropipéridin-1-yle, 4-trifluorométhylpipéridin-1-yle, 3,4-dihydropipéridin-1-yle, azépin-1-yle, cyclohexylspiro-4-pipéridin-1-yle ;
- $R_{10}$ représente un atome halogène ; un groupe $(C_1-C_4)$alkyle ; hydroxy ; $(C_1-C_6)$alcoxy ; $R_{10}$ peut de plus représenter un groupe $-CH_2NR_{11}R_{12}$ lorsque $R_5$ représente un groupe-$CH_2NR_{11}R_{12}$, lesdits groupes étant alors identiques ;
- $R_{11}$ et $R_{12}$ représentent chacun indépendamment l'hydrogène ; un groupe $(C_1-C_6)$alkyle ; $(C_2-C_4)$alcènyle ; $(C_3-C_7)$cycloalkyle ; $(C_3-C_7)$cycloalkyle$(C_1-C_4)$alkyle ; ω-hydroxy$(C_2-C_4)$alkylène ; ω-méthoxy$(C_2-C_4)$alkylène ; (ω-trifluorométhyl$(C_2-C_4)$alkylène ; ω-halogéno$(C_2-C_4)$alkylène ;
- ou bien $R_{11}$ et $R_{12}$ ensemble avec l'atome d'azote auquel ils sont liés constituent un radical hétérocyclique, mono ou bicyclique, choisi parmi azétidinyle, pyrrolidinyle, morpholin-4-yle, thiomorpholin-4-yle, pipéridin-1-yle, pipérazin-1-yle, 1,2,3,6-tétrahydropyrid-1-yle, decahydroquinolyle, decahydroisoquinolyle, octahydro-1*H*-isoindolyle, $(C_4-C_6)$cycloalkylspiropipéridinyle, 3-azabicyclo[3.1.0]hexyle, non substitués ou substitués une ou plusieurs fois par un atome d'halogène, un groupe $(C_1-C_4)$alkyle, hydroxy, $(C_1-C_4)$alcoxy, trifluorométhyle, difluorométhylène, phényle ;

ainsi que leurs sels avec des acides minéraux ou organiques et/ou leurs solvats ou leurs hydrates.

De plus, certaines valeurs des substituants sont préférées. Ainsi on préfère les composés de formule (I) dans laquelle au moins un des substituants a une valeur spécifiée ci-après :

a - $R_1$ représente un groupe 2,4-dichloro-3-méthylphényle, naphtyle, 6-méthoxynapht-2-yle, 3-méthylbenzothiophèn-2-yle, 3-méthyl-5-chloro-benzothiophèn-2-yle, 3-méthyl-5-méthoxybenzothiophèn-2-yle, 3-méthyl-6-méthoxybenzothiophèn-2-yle ou 3-méthyl-1-benzofuran-2-yle ; $R_2$, $R_3$, $R_4$ et $R_5$ sont tels que définis pour un composé de formule (I) ;
b - $R_2$ représente l'hydrogène et préférentiellement $R_3$ représente un groupe benzo[1,3]dioxol-5-yle ou phényle non substitué ou substitué par un halogène ; $R_1$, $R_4$ et $R_5$ sont tels que définis pour un composé de formule (I) ;
c - $R_4$ représente un groupe $-CONR_8R_9$ et préférentiellement $-NR_8R_9$ représente un radical di($C_1$-$C_4$)alkylamino tout particulièrement un radical N-méthylisopropyle ; pyrrolidinyle ou pipéridinyle non substitué ou substitué une ou deux fois par un méthyle ou un halogène ; $R_1$, $R_2$, $R_3$ et $R_5$ sont tels que définis pour un composé de formule (I) ;
d - $R_5$ représente un groupe $-CH_2NR_{11}R_{12}$ dans lequel $-NR_{11}R_{12}$ représente un radical éthylisobutylamino, éthylisopropylamino, éthyl*tert*butylamino, diisopropylamino, cyclopentylméthylamino, cyclopentyléthylamino ou un radical pipéridinyle non substitué ou substitué une ou plusieurs fois par un méthyle ou un halogène ; $R_1$, $R_2$, $R_3$ et $R_4$ sont tels que définis pour un composé de formule (I).

Ainsi de façon préférentielle, la présente invention a pour objet des composés de formule :

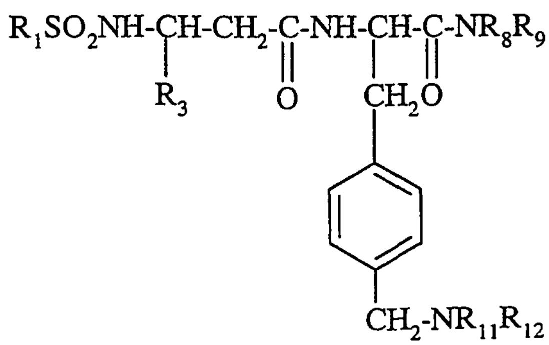

(Ia)

dans laquelle:

- $R_1$ représente un groupe 2,4-dichloro-3-méthylphényle, naphtyle, 6-méthoxynapht-2-yle, 3-méthylbenzothiophèn-2-yle, 3-méthyl-5-chlorobenzothiophèn-2-yle, 3-méthyl-5-méthoxybenzothiophèn-2-yle, 3-méthyl-6-méthoxybenzothiophèn-2-yle ou 3-méthyl-1-benzofuran-2-yle ;
- $R_3$ représente un groupe benzo[1,3]dioxol-5-yle ou phényle non substitué ou substitué par un halogène ;
- $R_8$ et $R_9$ ensemble avec l'atome d'azote auxquels ils sont liés, constituent un radical di($C_1$-$C_4$)alkylamino, pyrrolidinyle ou pipéridinyle non substitué ou substitué une ou deux fois par un méthyle ou un halogène ;
- $R_{11}$ et $R_{12}$ ensemble avec l'atome d'azote auxquels ils sont liés, constituent un radical éthylisobutylamino, éthylisopropylamino, éthyl*tert*butylamino, diisopropylamino, cyclopentylméthylamino, cyclopentyléthylamino ou un radical pipéridinyle non substitué ou substitué une ou plusieurs fois par un méthyle ou un halogène ;

ainsi que leurs sels avec des acides minéraux ou organiques et/ou leurs solvats ou leurs hydrates.
Les composés de formule (Ia) ayant la configuration (R,R) sont particulièrement préférés.
Tout particulièrement, la présente invention a pour objet un composé parmi :

- (R,R) 2-((3-(1,3-benzodioxol-5-yl)-3-(((6-méthoxy-2-naphtyl)sulfonyl)amino) propanoyl)amino)-3-(4-((2,6-cis-diméthyl-1-pipéridinyl)méthyl)phényl)-N-isopropyl-N-méthylpropanamide ;
- (R,R) 2-((3-(1,3-benzodioxol-5-yl)-3-((2-naphtylsulfonyl)amino)propanoyl) amino)-3-(4-((cyclopentyl(éthyl)amino) méthyl)phényl)-N-isopropyl-N-méthylpropanamide ;
- (R,R) 3-(4-((2,6-cis-diméthyl-1-pipéridinyl)méthyl)phényl)-2-((3-(((6-méthoxy-2-naphtyl)sulfonyl)amino)-3-phénylpropanoyl)amino)-N-isopropyl-N-méthyl propanamide ;
- (R,R) 2-((3-(1,3-benzodioxol-5-yl)-3-(((6-méthoxy-2-naphtyl)sulfonyl)amino) propanoyl)amino)-3-(4-((*tert*-butyl (éthyl)amino)méthyl)phényl)-N-isopropyl-N-méthylpropanamide ;
- (R,R) 2-((3-(1,3-benzodioxol-5-yl)-3-(((3-méthyl-1-benzothiophèn-2-yl)sulfonyl)amino)propanoyl)amino)-3-(4-((2,6-cis-diméthyl-1-pipéridinyl)méthyl) phényl)N-isopropyl-N-méthylpropanamide ;
- (R,R) 3-(4-((2,6-cis-diméthyl-1-pipéridinyl)méthyl)phényl)-2-((3-(((5-méthoxy-3-méthyl-1-benzothiophèn-2-yl)sulfonyl)amino)-3-phénylpropanoyl)amino)-N-isopropyl-N-méthylpropanamide ;

- (R,R) N-(4-((2,6-cis-diméthyl-1-pipéridinyl) méthyl)benzyl)-3-(((6-méthoxy-2-naphtyl)sulfonyl)amino)-3-phényl-N-(1-pipéridinylcarbonyl)propanamide ;
- (R,R) 2-((3-(4-chlorophényl)-3-(((6-méthoxy-2-naphtyl)sulfonyl) amino)propanoyl)amino)-3-(4-((2,6-cis-diméthyl-1-pipéridinyl)méthyl)phényl)-N-isopropyl-N-méthylpropanamide ;
- (R,R) 3-(4-((2,6-cis-diméthyl-1-pipéridinyl)méthyl)phényl)-2-((3-(((6-méthoxy-2-naphtyl)sulfonyl)amino)-3-phénylpropanoyl)amino)-N,N-diéthylpropanamide ;
- (R,R) 2-((3-(3-fluorophényl)-3-(((6-méthoxy-2-naphtyl)sulfonyl)amino) propanoyl)amino)-3-(4-((2,6-cis-diméthyl-1-pipéridinyl)méthyl)phényl)-N-isopropyl-N-méthylpropanamide ;
- (R,R) 2-((3-(1,3-benzodioxol-5-yl)-3-(((3-méthyl-1-benzofuran-2-yl)sulfonyl) amino)propanoyl)amino)-3-(4-((2,6-cis-diméthyl-1-pipéridinyl)méthyl)phényl)-N-isopropyl-N-méthylpropanamide ;
- (R,R) 2-((3-(1,3-benzodioxol-5-yl)-3-((2-naphtylsulfonyl)amino)propanoyl) amino)-3-(3-((*tert*-butyl(éthyl)amino)méthyl)phényl)-N-isopropyl-N-méthyl propanamide ;
- (R,R) 3-(4-(7-azabicyclo[2.2.1]hept-7-ylméthyl)phényl)-2-((3-(1,3-benzodioxol-5-yl)-3-(((6-méthoxynaphtyl)sulfonyl)amino)propanoyl)amino)-N-isopropyl-N-méthyl propanamide ;

ainsi que leurs sels avec des acides minéraux ou organiques et/ou leurs solvats ou leurs hydrates.

Selon un autre de ses aspects, la présente invention concerne un procédé pour la préparation des composés de formule (I), de leurs sels et/ou de leurs solvats ou hydrates, caractérisé en ce que :

on fait réagir un acide ou un dérivé fonctionnel de cet acide de formule :

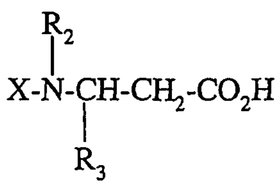

(II)

dans laquelle $R_2$ et $R_3$ sont tels que définis pour un composé de formule (I), X représente soit l'hydrogène, soit un groupe $R_1$-$SO_2$- dans lequel $R_1$ est tel que défini pour un composé de formule (I), soit un groupe N-protecteur, avec un composé de formule :

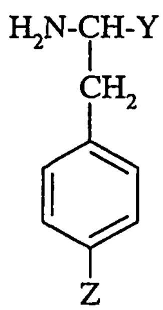

(III)

dans laquelle Y représente soit $R_4$ tel que défini pour un composé de formule (I), soit un ($C_1$-$C_4$)alcoxycarbonyle, et Z représente soit $R_5$ tel que défini pour un composé de formule (I), soit un groupe -CN, à la condition que lorsque Y représente $R_4$ qui représente un phényle substitué par un groupe $-CH_2NR_{11}R_{12}$, Z représente $R_5$ qui représente un groupe $-CH_2NR_{11}R_{12}$, $R_{11}$ et $R_{12}$ étant tels que définis pour un composé de formule (I) ; et,

- lorsque X = $R_1SO_2$-, Y = $R_4$ et Z = $R_5$, on obtient le composé de formule (I) attendu ;
- ou lorsque X ≠ $R_1SO_2$ et/ou Y ≠ $R_4$ et/ou Z ≠ $R_5$, c'est à dire lorsqu'au moins un des groupes X, Y, Z représente respectivement X = H ou groupe N-protecteur, Y =($C_1$-$C_4$)alcoxycarbonyle, Z = -CN, on soumet le composé ainsi obtenu de formule :

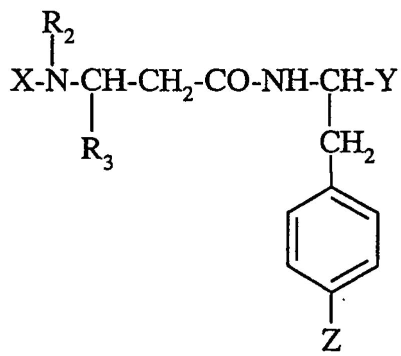

(IV)

à une ou plusieurs des étapes suivantes :

- lorsque X représente un groupe N-protecteur, on élimine ce groupe et on fait réagir le composé ainsi obtenu de formule :

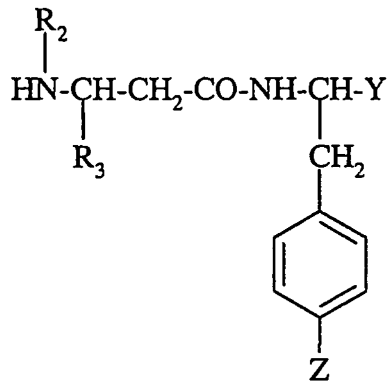

(V)

avec un halogénure de sulfonyle de formule :

$R_1SO_2$-Hal (VI)

dans laquelle Hal représente un halogène ;

- lorsque Y représente un ($C_1$-$C_4$)alcoxycarbonyle, on l'hydrolyse et on fait réagir l'acide ainsi obtenu ou un dérivé fonctionnel de cet acide de formule :

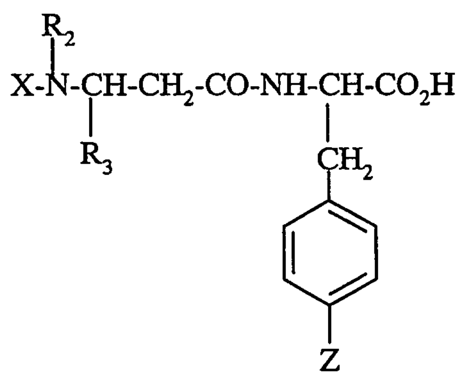

(VII)

avec un composé de formule :

$HNR_8R_9$ (VIII) ;

dans laquelle $R_8$ et $R_9$ sont tels que définis pour un composé de formule (I) ;

- lorsque Z représente un groupe -CN, on transforme ce groupe en $R_5$.

Eventuellement, on transforme le composé ainsi obtenu en l'un de ses sels avec des acides minéraux ou organiques.

Au cours de l'une quelconque des étapes du procédé de préparation des composés de formule (I) ou de leurs composés intermédiaires de formule (II), (III), (IV), (V) ou (VII) il peut être nécessaire et/ou souhaitable de protéger les groupes fonctionnels réactifs ou sensibles, tels que les groupes amine, hydroxyle ou carboxyle, présents sur l'une quelconque des molécules concernées. Cette protection peut s'effectuer en utilisant les groupes protecteurs conventionnels, tels que ceux décrits dans Protective Groups in Organic Chemistry, J.F.W. McOmie, Ed. Plenum Press, 1973 et dans Protective Groups in Organic Synthesis, T.W. Greene et P.G.M. Wutts, Ed. John Wiley et Sons, 1991. L'élimination des groupes protecteurs peut s'effectuer à une étape ultérieure opportune en utilisant les méthodes connues de l'homme de l'art et qui n'affectent pas le reste de la molécule concernée.

Les groupes N-protecteurs éventuellement utilisés sont les groupes N-protecteurs classiques bien connus de l'homme de l'art tels que par exemple le groupe *tert*-butoxycarbonyle, fluorénylméthoxycarbonyle, benzyle, trityle ou benzyloxycarbonyle.

Les composés de formule (I) ou les composés de formule (IV) peuvent être préparés sous forme de mélange d'isomères ou sous forme optiquement pure. Pour obtenir des composés optiquement purs, on peut soit effectuer la séparation des isomères par des méthodes connues de la chimie organique, soit utiliser comme produit de départ des composés de formule (II) et (III) optiquement purs et procéder ensuite, le cas échéant, par des méthodes de synthèse non racémisantes connues en elles-mêmes.

A l'étape a) du procédé, les composés de formule (I) ou les composés de formule (IV) sont préparés en utilisant les méthodes classiques de la chimie des peptides, par exemple celles décrites dans The Peptides Ed. E. Gross et J. Meienhofer, Academic Press, 1979, 1, 65-104. Des méthodes connues permettent d'effectuer des couplages peptidiques sans racémisation des atomes de carbone de chaque aminoacide constitutif; de plus, les β-alanines β-substituées pour lesquelles le carbone chiral n'est pas adjacent au groupe carboxyle sont réputées ne pas subir de racémisation (Ann. Rev. Biochem., 1986, 55, 855-878). Par ailleurs, la demande de brevet EP 236 163 décrit des procédés permettant de conserver la chiralité de chaque aminoacide.

Ainsi, dans l'étape a) du procédé selon l'invention, comme dérivé fonctionnel de l'acide (II) on peut utiliser un dérivé fonctionnel qui réagit avec les amines tel qu'un anhydride, un anhydride mixte, un chlorure d'acide ou un ester activé comme l'ester de 2,5-dioxopyrrolidin-1-yle, l'ester de p-nitrophényle ou l'ester de benzotriazol-1-yle.

Lorsqu'on utilise l'ester de 2,5-dioxopyrrolidin-1-yle de l'acide (II), la réaction avec l'amine (III) s'effectue dans un solvant tel que le N,N-diméthylformamide, en présence d'une base telle que la triéthylamine ou la N-N-diisopropyléthylamine, à une température comprise entre 0°C et la température ambiante.

Lorsqu'on utilise un chlorure d'acide, la réaction s'effectue dans un solvant tel que le dichlorométhane en présence d'une base telle que la triéthylamine, la N-méthylmorpholine ou la N,N-diisopropyléthylamine, à une température comprise entre -60°C et la température ambiante.

Lorsqu'on utilise un anhydride mixte de l'acide (II), on génère celui-ci *in situ* par réaction d'un chloroformiate de ($C_1$-$C_4$)alkyle avec l'acide de formule (II) dans un solvant tel que le dichlorométhane, en présence d'une base telle que la triéthylamine, à une température comprise entre -70°C et 50°C, et on le fait réagir avec l'amine de formule (III), dans un solvant tel que le dichlorométhane, en présence d'une base telle que la triéthylamine et à une température comprise entre 0°C et la température ambiante.

Lorsqu'on met en oeuvre l'acide de formule (II) lui-même, on opère en présence d'un agent de couplage utilisé en chimie peptidique tel que le 1,3-dicyclohexylcarbodiimide ou bien l'hexafluorophosphate de benzotriazol-1-yl-oxytris (diméthylamino)phosphonium ou le benzotriazol-1-yl-N,N,N',N'-tétraméthyluronium tétrafluoroborate en présence d'une base telle que la triéthylamine, la N,N-diisopropyléthylamine ou la N-éthylmorpholine, dans un solvant tel que le dichlorométhane, l'acétonitrile ou le N,N-diméthylformamide, ou un mélange de ces solvants, à une température comprise entre 0°C et la température ambiante.

On obtient ainsi soit directement un composé de formule (I), soit un composé de formule (IV) dans laquelle au moins un des groupes X, Y et Z représente respectivement : X = groupe N-protecteur, Y = ($C_1$-$C_4$)alcoxycarbonyle, Z = -CN et que l'on transforme en une ou plusieurs réactions, par des méthodes classiques bien connues de l'homme de l'art, en un composé de formule (I). Il est entendu que lorsque plusieurs réactions sont nécessaires pour transformer un composé de formule (IV) en un composé de formule (I), l'ordre de ces réactions s'effectue de manière à ne pas affecter les autres substituants de la molécule concernée.

Lorsque dans le composé de formule (TV), X représente un groupe N-protecteur, on élimine ce groupe selon les méthodes connues de l'homme de l'art. Par exemple, lorsque X représente un groupe *tert*-butoxycarbonyle, on

l'élimine par action d'un acide tel que l'acide chlorhydrique ou l'acide trifluoroacétique par exemple dans un solvant tel que le méthanol, l'éther éthylique, le dioxane, le tétrahydrofurane ou le dichlorométhane à une température comprise entre 0°C et la température ambiante. Puis, l'amine de formule (V) ainsi obtenue est mise en réaction avec un halogénure de sulfonyle de formule (VI) (de préférence un chlorure), en présence d'une base telle qu'un hydroxyde de métal alcalin (hydroxyde de sodium ou hydroxyde de potassium par exemple) ou d'une base organique (triéthylamine, diisopropyléthylamine ou N-méthylmorpholine par exemple), dans un solvant tel que le dioxane, le dichlorométhane ou l'acétonitrile, en présence ou non d'un activateur tel que la diméthylaminopyridine (DMAP) ; la réaction peut également être effectuée dans la pyridine en présence ou non de DMAP. La réaction est effectuée à une température comprise entre 0°C et la température ambiante.

On obtient soit un composé de formule (I), soit un composé de formule (IV) dans laquelle $X=R_1SO_2$-.

Lorsque dans le composé de formule (IV), Y représente un $(C_1-C_4)$alcoxycarbonyle, on l'hydrolyse en milieu acide ou basique selon les méthodes connues de l'homme de l'art. Puis l'acide de formule (VII) ainsi obtenu est mis en réaction avec un composé de formule (VIII) selon les conditions précédemment décrites pour la mise en oeuvre d'un composé de formule (II). On obtient soit un composé de formule (I), soit un composé de formule (IV) dans laquelle $R_4$ = $-CONR_8R_9$.

Lorsque dans le composé de formule (IV), Z représente un groupe cyano, la transformation de ce groupe en un groupe $R_5$ s'effectue selon les méthodes classiques bien connues de l'homme de l'art.

Par exemple, par réduction du groupe -CN on obtient soit un composé de formule (I), soit un composé de formule (IV), dans lesquelles $R_5$ = $-CH_2NH_2$. Cette réduction peut s'effectuer au moyen d'hydrogène, en présence d'un catalyseur tel que le nickel de Raney®, dans un solvant tel que le méthanol, le toluène, le dioxane ou un mélange de ces solvants en mélange avec l'ammoniaque à une température comprise entre la température ambiante et 50°C.

On peut transformer le groupe -CN en un groupe $R_5$ = $-CH_2NR_{11}R_{12}$ selon le Schéma 1 ci-après.

SCHEMA 1

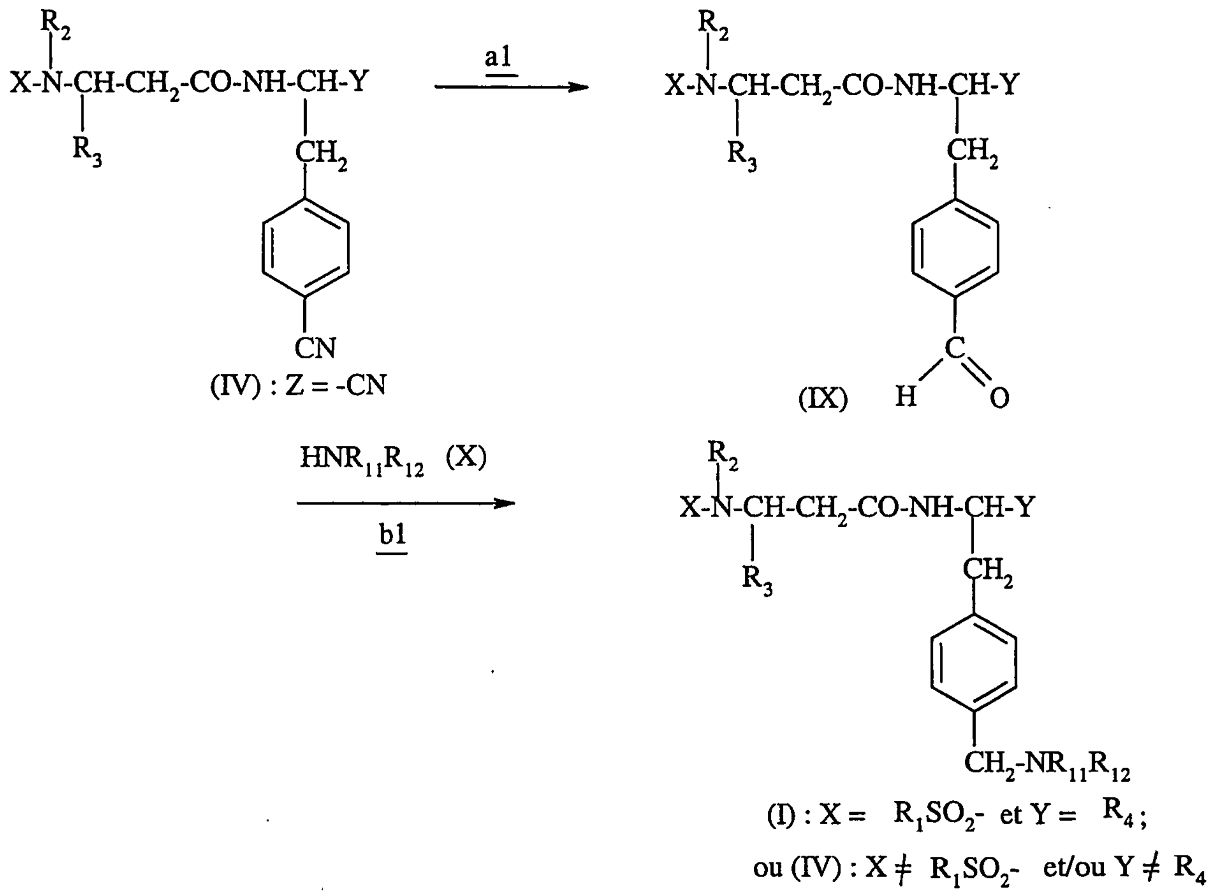


(IV) : Z = -CN

(IX)

(I) : X = $R_1SO_2$- et Y = $R_4$;

ou (IV) : X ≠ $R_1SO_2$- et/ou Y ≠ $R_4$

A l'étape a1 du Schéma 1, la réduction du dérivé nitrile de formule (IV) en un aldéhyde de formule (IX) s'effectue selon les méthodes connues de l'homme de l'art telle que par exemple celle décrite dans Synth. Commun., 1990, 20(3), 459-467. On peut également utiliser la méthode décrite dans Farmaco, Ed. Sci., 1988, 43(7/8), 597-612, à la condition que dans le composé de formule (IV), X soit différent d'un groupe N-protecteur labile en milieu acide.

Puis à l'étape b1 on fait réagir un composé de formule (X) avec l'aldéhyde de formule (IX) en présence ou non d'un acide tel que l'acide acétique, dans un solvant tel que le méthanol, le dichlorométhane ou le 1,2-dichloroéthane, pour former *in situ* une imine intermédiaire qui est réduite chimiquement en utilisant par exemple le cyanoborohydrure de sodium, le triacétoxyborohydrure de sodium ou le borohydrure de sodium.

On peut également transformer le groupe -CN en un groupe $R_5$ selon le Schéma 2 ci-après.

SCHEMA 2

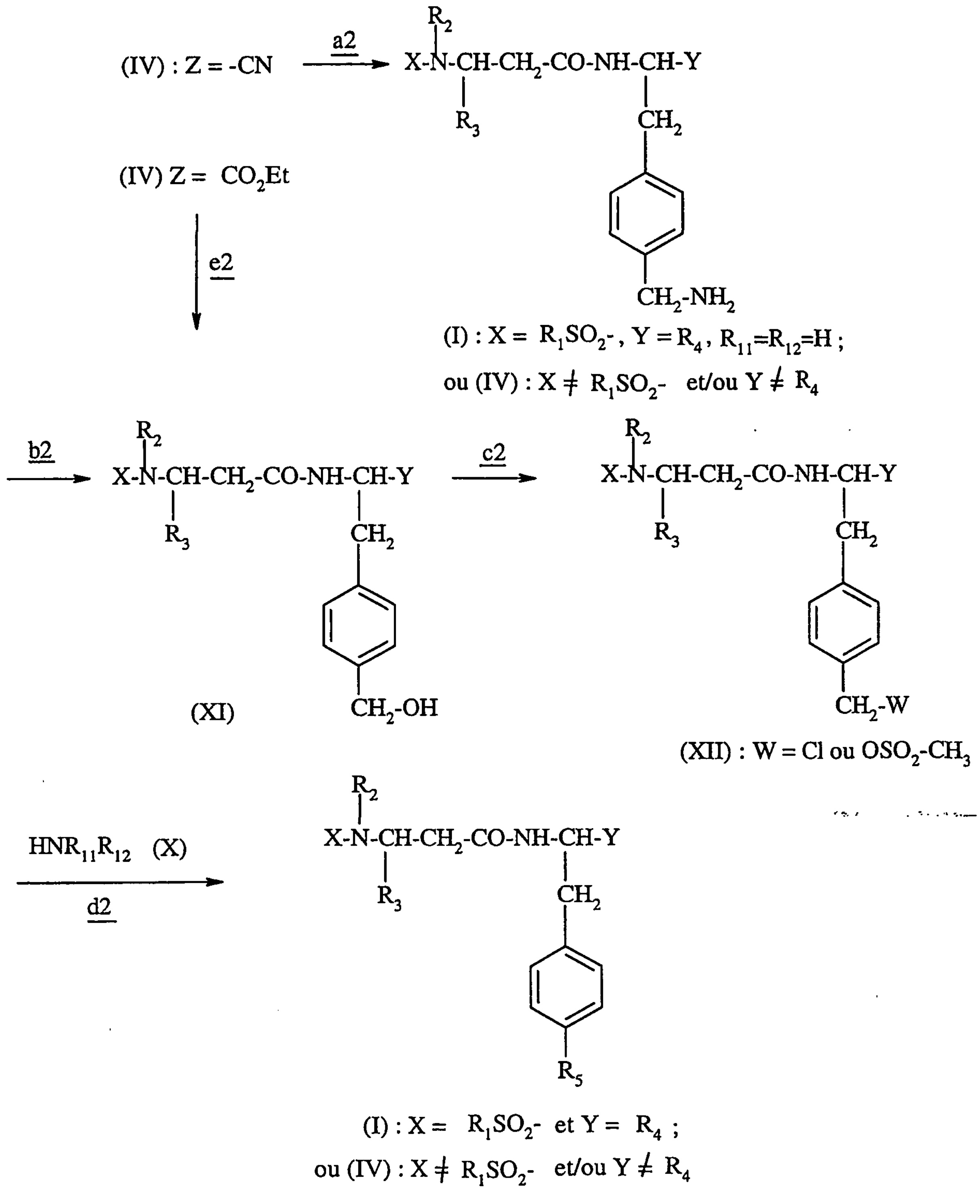


A l'étape <u>a2</u> du Schéma 2, on effectue la réduction du groupe cyano selon les méthodes précédemment décrites.

Puis à l'étape <u>b2,</u> on transforme l'amine ainsi obtenue en un alcool de formule (XI) selon les méthodes connues de l'homme de l'art, par exemple par action du nitrite de sodium en milieu aqueux, dans un solvant tel que le dioxane et à une température comprise entre la température ambiante et 110°C.

A l'étape <u>c2,</u> on traite l'alcool de formule (XI) par le chlorure de méthanesulfonyle, en présence d'une base telle que la triéthylamine, dans un solvant tel que le dichlorométhane et à une température comprise entre 0°C et la

température de reflux du solvant. On obtient ainsi un composé de formule (XII) dans laquelle W = Cl ou O-$SO_2CH_3$ selon les conditions opératoires utilisées.

A l'étape d2, lorsqu'on utilise un composé de formule (XII) dans laquelle W = Cl, on effectue la réaction avec le composé de formule (X) en présence d'une base telle que l'hydrure de sodium, dans un solvant tel que le tétrahydrofurane, le N,N-diméthylformamide, l'acétonitrile, le toluène ou le propan-2-ol et à une température comprise entre la température ambiante et 100°C. Lorsqu'on utilise un composé de formule (XII) dans laquelle W = O-$SO_2CH_3$, on effectue la réaction avec un composé de formule (X) en présence ou non d'une base telle que la triéthylamine, dans un solvant tel que l'éthanol, le N,N-diméthylformamide, l'acétonitrile, ou le dichlorométhane et a une température comprise entre 0°C et 100°C.

A l'étape e2, on effectue une réduction de l'ester par un agent réducteur tel que $NaBH_4$ ou $LiAlH_4$ par exemple.

Un composé selon l'invention de formule (I) dans laquelle $R_5$ représente un groupe -$CH_2$-N(O)-$R_{11}$ $R_{12}$ est obtenu à partir du composé de formule (I) analogue dans laquelle $R_5$ représente un groupe -$CH_2$-$NR_{11}R_{12}$, les autres substituants étant identiques, la réaction est effectuée par action d'un agent oxydant tel que l'acide métachloroperbenzoïque.

On obtient finalement les composés de formule (I) selon l'invention.

Les composés de formule (I) ainsi obtenus sont isolés sous forme de base libre ou de sel, selon les techniques classiques.

Lorsque les composés de formule (I) sont obtenus sous forme de base libre, la salification est effectuée par traitement avec l'acide choisi dans un solvant organique. Par traitement de la base libre, dissoute par exemple dans un éther tel que l'éther diéthylique ou dans un alcool tel que le propan-2-ol ou le méthanol ou dans l'acétone ou dans le dichlorométhane, ou dans l'acétate d'éthyle, ou dans l'acétonitrile avec une solution de l'acide choisi dans un des solvants précités, on obtient le sel correspondant qui est isolé selon les techniques classiques.

Ainsi, on prépare par exemple le chlorhydrate, le bromhydrate, le sulfate, l'hydrogénosulfate, le dihydrogénophosphate, le méthanesulfonate, l'oxalate, le maléate, le succinate, le fumarate, le naphtalène-2-sulfonate, le benzènesulfonate, le para-toluènesulfonate.

A la fin de la réaction, les composés de formule (I) peuvent être isolés sous forme d'un de leurs sels, par exemple le chlorhydrate ; dans ce cas, s'il est nécessaire, la base libre peut être préparée par neutralisation dudit sel avec une base minérale ou organique, telle que l'hydroxyde de sodium ou la triéthylamine ou avec un carbonate ou bicarbonate alcalin, tel que le carbonate ou bicarbonate de sodium ou de potassium.

Les composés de formule (VI) sont connus ou préparés selon des méthodes connues. Par exemple, on prépare le chlorure de 2,4-dichloro-3-méthylbenzènesulfonyle selon le procédé décrit dans J. Am. Chem. Soc., 1940, 62, 511-512. On prépare le chlorure de quinoléine-2-sulfonyle et le chlorure de 5,6,7,8-tétrahydronaphtalène-2-sulfonyle selon le procédé décrit dans WO 97/25315. On prépare le chlorure de 6-méthoxynaphtalène-2-sulfonyle selon J. Org. Chem., 1992, 57, 2631-2641. On prépare le chlorure de 3-méthylbenzothiènyle-2-sulfonyle selon J. Het. Chem., 1988, 25, 639-641.

Les composés de formule (VIII) sont connus ou préparés selon des méthodes connues.

Les composés de formule (X) sont connus ou préparés selon des méthodes connues.

Les composés de formule (II) dans laquelle $R_2$ représente l'hydrogène ou un ($C_1$-$C_4$)alkyle sous forme racémique ou sous forme d'énantiomères purs sont connus (tableau 1) ou préparés selon des méthodes connues telles que celles décrites dans WO 97/25315.

TABLEAU I

| 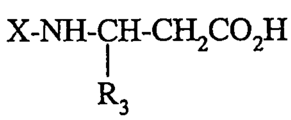 (II) | | |
|---|---|---|
| X | $R_3$ | Références |
| H | 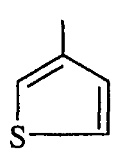 | Eur. J. Med. Chem. Chim. Therap.,1992, 27 (9), 961-965 |

(suite)

| X | $R_3$ | Références |
|---|---|---|
| Boc | 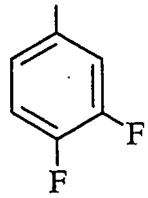 | J. Med. Chem.,1997, 40 (26), 4308-4318 |
| H | 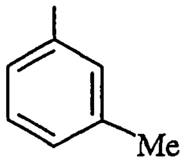 | Bull. Soc. Chim. Fr.,1987, 6, 1079-1083 |
| H | 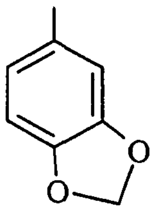 | Biorg. Med. Chem.1994, 2 (9), 881 |
| H | 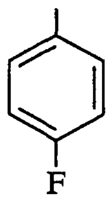 | Eur. J. Med. Chem. Chim. Therap.,1992, 27 (9), 961-965 |
| | 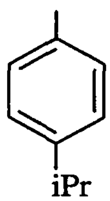 | Bull. Soc. Chim. Fr.,1987, 6, 1079-1083 |
| H | 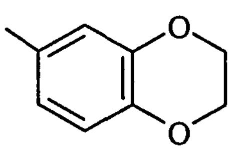 | Tetrahedron Lett.,1991, 32 (44), 6327-6328 |
| H | 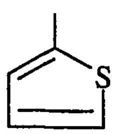 | Zh. Obshch. Khim.,1958, 28, 213-215 |
| H | 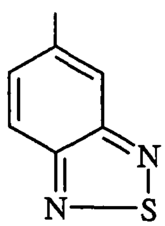 | Chem. Heterocycl. Compd., (Engl. Transl.)1969, 5, 453-456 |

(suite)

| X | $R_3$ | Références |
|---|---|---|
| H | 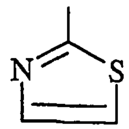 | J. American. Chem. Soc.,1957, 79, 4524-4527 |
| H | 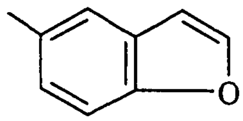 | Tetrahedron Lett.,2000, 41 (31), 5803-5806 |
| H | 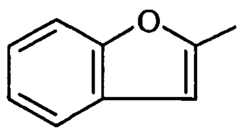 | WO 97/08145 |
| H | 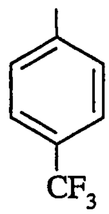 | WO 98/40055 |
| H | 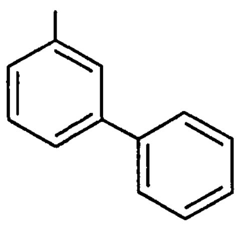 | WO 97/08145 |
| H | 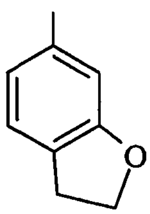 | Tetrahedron Lett.2000, 41, 5803-5806 |

Les composés de formule (II) dans laquelle $R_3$ représente l'hydrogène peuvent être préparés par des méthodes connues. On peut, par exemple, utiliser le procédé décrit dans le Schéma 3 ci-après dans lequel R' représente un ($C_1$-$C_4$)alkyle et Pr représente un groupe N-protecteur, par exemple un groupe *tert*-butoxycarbonyle ou fluorénylméthoxycarbonyle.

SCHEMA 3

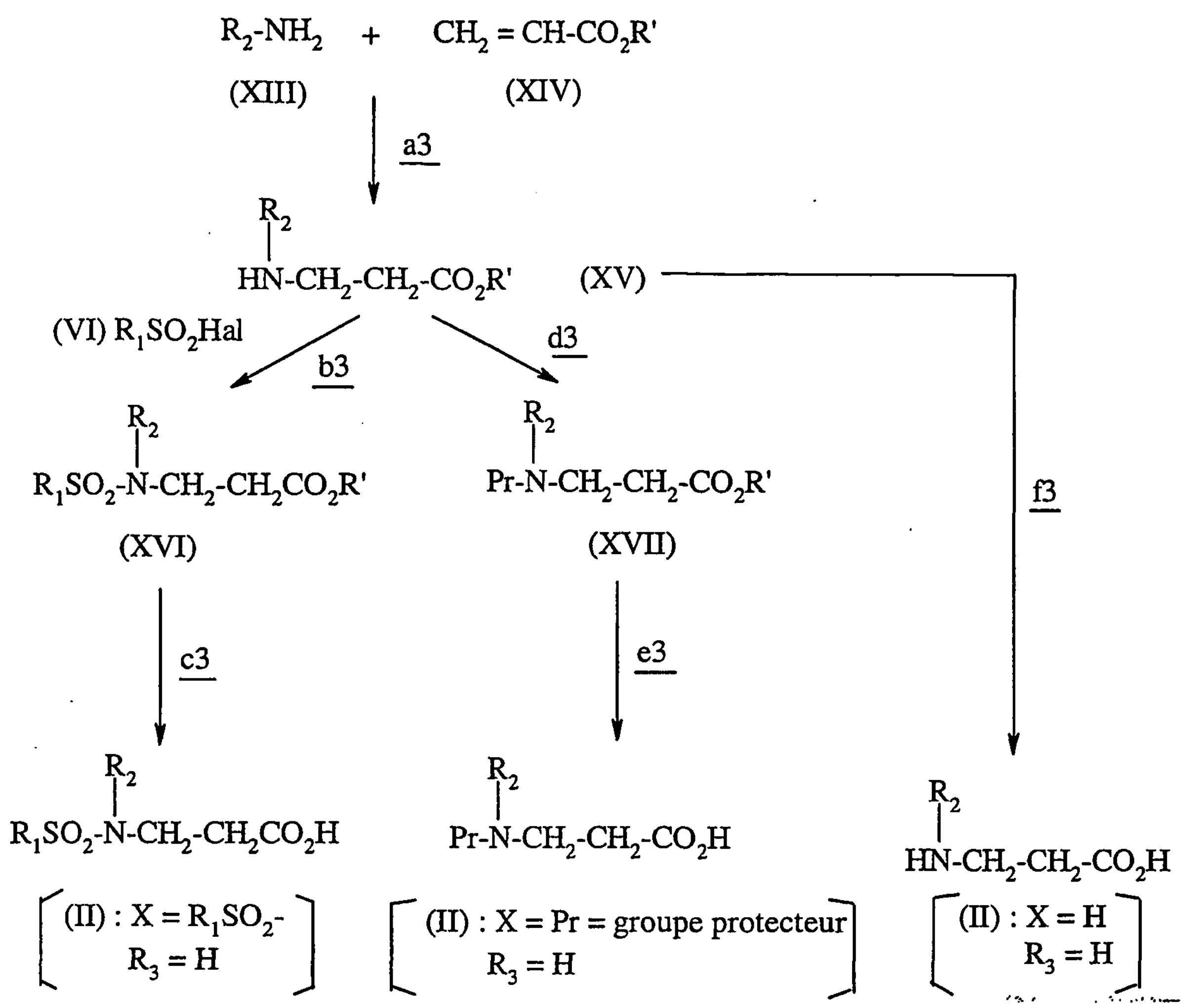


A l'étape a3 du Schéma 3, on fait réagir un composé de formule (XIII) avec un ester de l'acide acrylique de formule (XIV) pour obtenir un composé de formule (XV). La réaction s'effectue en présence d'un acide tel que l'acide acétique et à la température de reflux du mélange réactionnel.

A l'étape b3, on fait réagir le composé (XV) avec un halogénure de sulfonyle de formule (VI), en présence d'une base telle que l'hydroxyde de sodium, l'hydroxyde de potassium ou la 4-diméthylaminopyridine, dans un solvant tel que le dioxane ou la pyridine, et à une température comprise entre la température ambiante et 60°C.

A l'étape c3, on hydrolyse l'ester de formule (XVI) ainsi obtenu, selon les méthodes connues de l'homme de l'art et obtient le composé de formule (II) attendu dans laquelle X = $R_1SO_2$-.

Alternativement, à l'étape d3, on protège l'amino-ester de formule (XV) selon les méthodes connues de l'homme de l'art, par exemple par un groupe *tert*-butoxycarbonyle ou fluorénylméthoxycarbonyle.

A l'étape e3, on hydrolyse l'ester de formule (XVII) ainsi obtenu selon les méthodes connues, et obtient le composé de formule (II) dans laquelle X représente un groupe protecteur.

Alternativement, selon l'étape f3, on hydrolyse l'ester de formule (XV) en milieu acide ou basique pour obtenir un composé de formule (II).

Les composés de formule (III) dans laquelle Y représente un ($C_1$-$C_4$)alcoxycarbonyle ou un groupe $R_4$ = $CONR_8R_9$ et Z = -CN sont connus ou préparés selon des méthodes connues telles que celles décrites dans WO 97/25315, EP 0614911 ou EP 0 236 164.

Les autres composés de formule (III) dans laquelle Y représente $R_4$ et Z = -CN se préparent selon le Schéma 4 ci-après.

SCHEMA 4

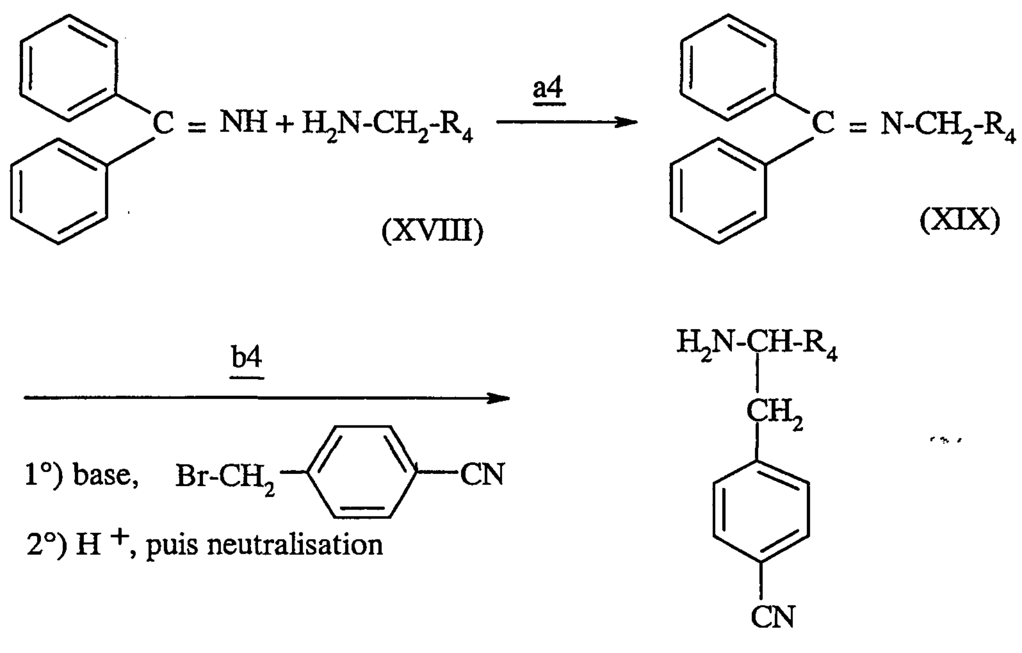


(XVIII)

(XIX)

(III)

A l'étape a4 du Schéma 4, on fait réagir la benzophénone imine avec une amine de formule (XVIII) pour obtenir un composé de formule (XIX). La réaction s'effectue dans un solvant tel que le dichlorométhane, le chloroforme ou l'acétate d'éthyle, à une température comprise entre la température ambiante et 50°C et en présence ou non d'une base telle que la triéthylamine.

A l'étape b4, le composé de formule (XIX) est traité par une base forte telle que le butyllithium, le *tert*-butylate de potassium ou le diisopropylamidure de lithium pour fournir un carbanion qui est mis en réaction avec le 4-(bromométhyl) benzonitrile.

La réaction s'effectue dans un solvant tel que le tétrahydrofurane, à une température comprise entre -78°C et la température ambiante. Par traitement en milieu acide, on élimine le groupe N-protecteur benzhydrylidène et obtient un composé de formule (III) attendu.

On peut également préparer des composés de formule (XIX) selon les méthodes décrites dans Bull. Soc. Chim. Fr., 1973, 2985, 2987, 2988, J. Am. Chem. Soc., 1982, 104 (3), 730 ou Tetrahedron Lett., 1996, 1137.

Les composés de formule (XVIII) sont connus ou préparés selon des méthodes connues. Par exemple, on peut préparer des composés de formule (XVIII) selon le Schéma 5 ci-après.

SCHEMA 5

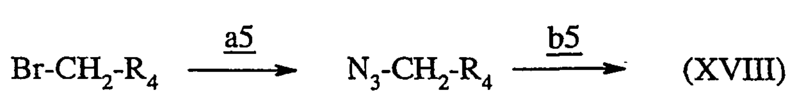


(XX)

(XXI)

A l'étape a5 du Schéma 5, on fait réagir un composé de formule (XX) avec l'azidure de sodium pour obtenir un composé de formule (XXI). La réaction s'effectue dans un solvant tel que le diméthylsulfoxyde, à une température comprise entre 0°C et la température ambiante.

A l'étape b5, la réduction du composé de formule (XXI) en un composé de formule (XVIII) s'effectue selon les méthodes connues de l'homme de l'art.

Les composés de formule (XVIII) peuvent également être préparés par réduction d'un nitrile de formule $R_4CN$, par exemple par action de $LiAlH_4$.

Les composés de formule (III) dans laquelle Y représente un ($C_1$-$C_4$)alcoxycarbonyle et Z représente $R_5$ tel que défini pour un composé de formule (I) se préparent selon le Schéma 6 ci-après dans lequel R' représente un ($C_1$-$C_4$) alkyle.

SCHEMA 6

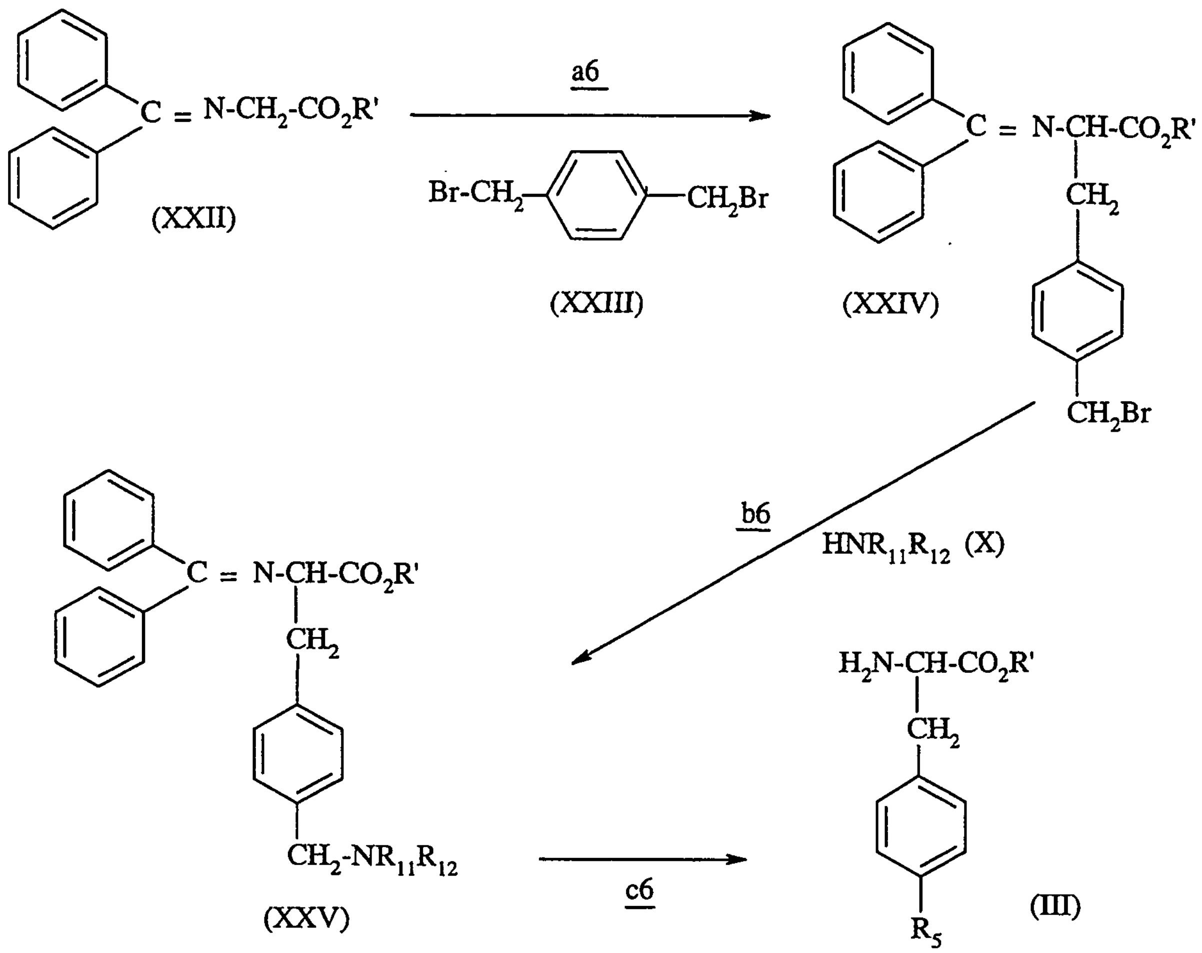


A l'étape a6 du Schéma 6, on fait réagir un composé de formule (XXII) avec l'α,α'-dibromo-p-xylène (XXIII) selon la méthode décrite dans Tetrahedron Asymmetry, 1992, 3 (5), 637-650.

A l'étape b6, le composé de formule (XXIV) ainsi obtenu est mis en réaction avec un composé de formule (X) pour obtenir un composé de formule (XXV). La réaction s'effectue en présence d'une base telle qu'un carbonate ou bicarbonate de métal alcalin (carbonate de potassium, carbonate de sodium ou bicarbonate de sodium), dans un solvant tel que le N,N-diméthylformamide, l'acétonitrile, le dichlorométhane ou le toluène et à une température comprise entre la température ambiante et 100°C.

A l'étape c6, on élimine le groupe N-protecteur par action d'un acide tel que par exemple l'acide chlorhydrique.

Les composés de formule (III) dans laquelle Y représente $R_4$ = $CONR_8R_9$, se préparent aussi selon le Schéma 7 ci-après dans lequel R' représente un ($C_1$-$C_4$)alkyle.

SCHEMA 7

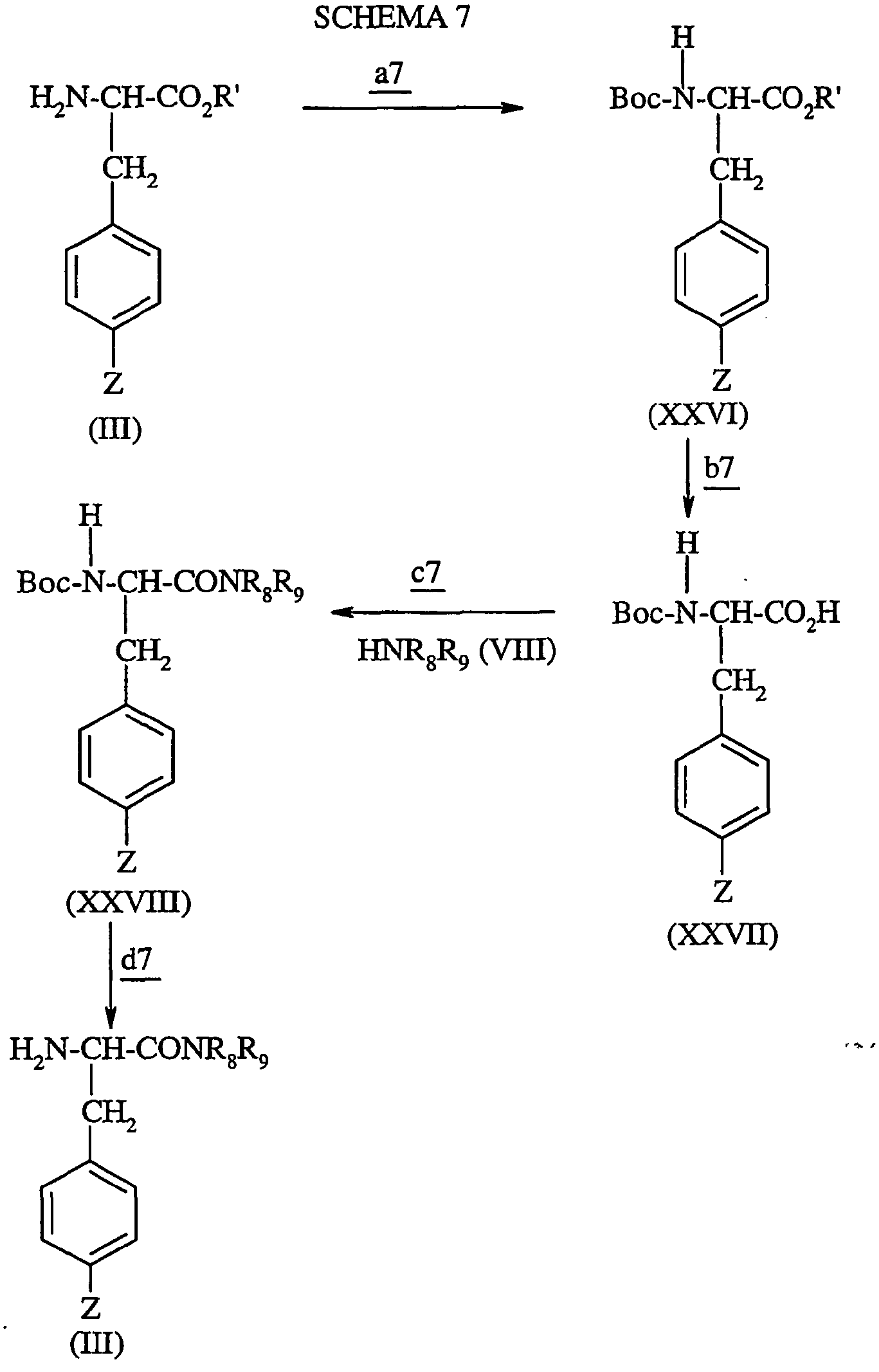


(III) (XXVI) (XXVII) (XXVIII) (III)

A l'étape a7, on protège l'amine d'un composé de formule (III) par un groupe *tert*-butoxycarbonyle, puis on hydrolyse l'ester ainsi obtenu selon les méthodes classiques (étape b7) ; on fait ensuite réagir l'acide de formule (XXVII) ainsi obtenu avec un composé de formule (VIII) selon les méthodes précédemment décrites (étape c7) et déprotège le composé (XXVIII) obtenu en milieu acide (étape d7).

Les composés de formule (III) dans laquelle Y représente $R_4$ et Z représente $R_5$ tel que défini pour un composé de formule (I) se préparent selon le Schéma 8 ci-après.

SCHEMA 8

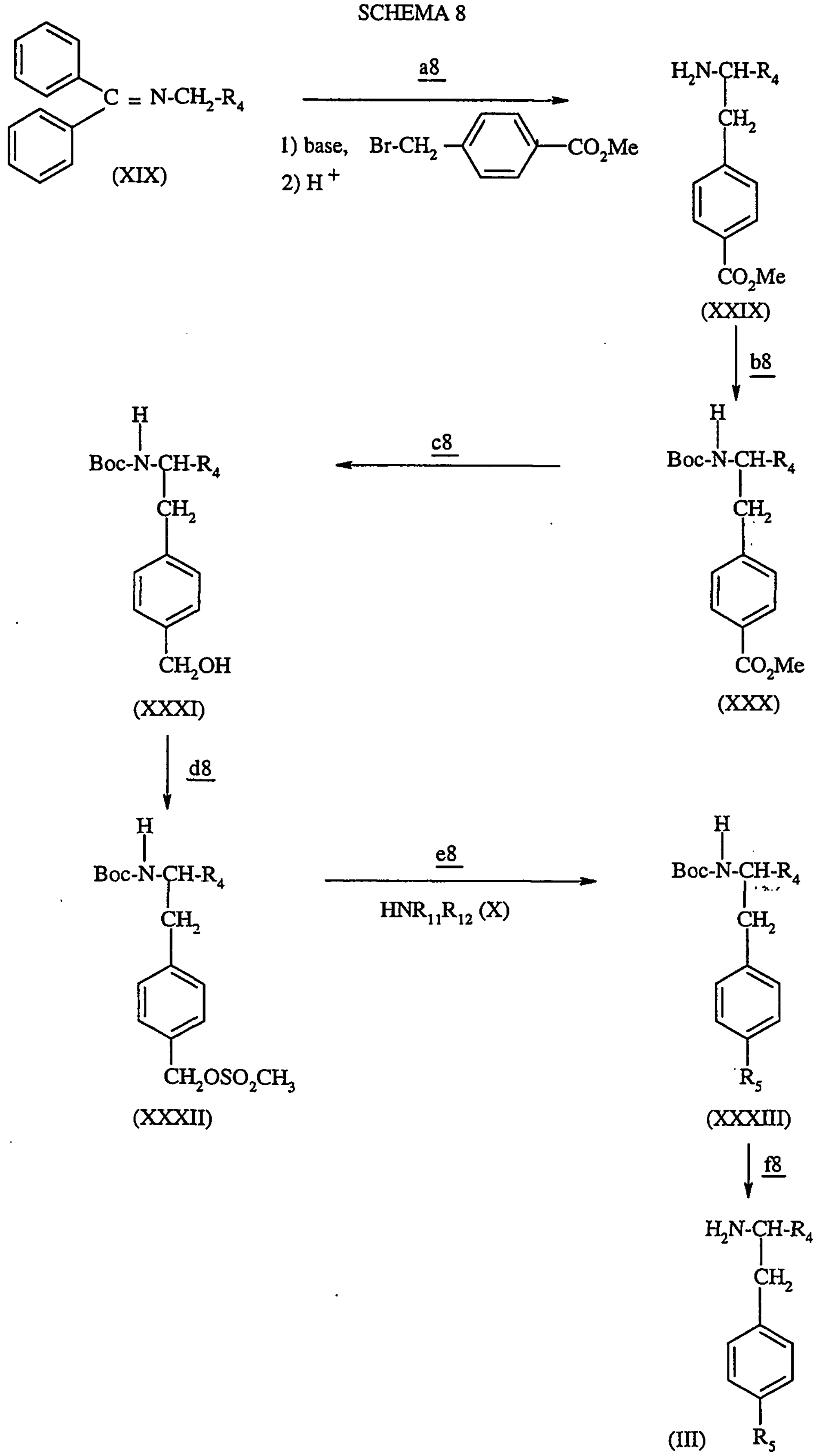
(XIX)
a8
1) base, Br-CH2 —CO2Me
2) H+
(XXIX)
b8
(XXX)
c8
(XXXI)
d8
(XXXII)
e8
HNR11R12 (X)
(XXXIII)
f8
(III)

A l'étape a8 du Schéma 8, la réaction du composé de formule (XIX) avec le 4-(bromométhyl)benzoate de méthyle s'effectue en présence d'une base telle que le *tert*-butylate de potassium ou le lithium diisopropylamide, dans un solvant tel que le tétrahydrofurane à une température comprise entre -78°C et la température ambiante. Par traitement en milieu acide, on élimine le groupe protecteur benzhydrylidène.

A l'étape b8, on protège l'amine de formule (XXIX) par réaction avec du di-*tert*-butyldicarbonate.

A l'étape c8, l'ester de formule (XXX) ainsi obtenu est réduit en un alcool de formule (XXXI). La réduction s'effectue en présence d'un agent réducteur tel que l'hydrure d'aluminium lithium, le borohydrure de sodium ou l'hydrure de diisobutylaluminium, dans un solvant tel que le tétrahydrofurane ou le toluène, à une température comprise entre -78°C et la température ambiante.

L'alcool de formule (XXXI) est mis en réaction à l'étape d8 avec le chlorure de méthanesulfonyle, en présence d'une base telle que la triéthylamine, dans un solvant tel que le dichlorométhane ou le tétrahydrofurane et à une température comprise entre 0°C et la température de reflux du solvant.

A l'étape e8, on fait réagir le composé de formule (XXXII) ainsi obtenu avec un composé de formule (X), en présence ou non d'une base telle que la triéthylamine, dans un solvant tel que l'éthanol, le N,N-diméthylformamide, l'acétonitrile, le toluène ou le dichlorométhane et à une température comprise entre 0°C et 100°C.

A l'étape f8, on élimine le groupe N-protecteur du composé de formule (XXXIII) ainsi obtenu par traitement en milieu acide.

Les composés de formule (III) dans laquelle Y représente soit un $(C_1\text{-}C_4)$alcoxycarbonyle, soit $R_4$ qui représente un groupe $-CONR_8R_9$, un radical hétéroxyclique ou un phényle non substitué ou substitué par un groupe autre qu'un groupe $-CH_2NR_{11}R_{12}$, et Z représente $R_5$ = $-CH_2NR_{11}R_{12}$, peuvent également se préparer selon le Schéma 9 ci-après :

SCHEMA 9

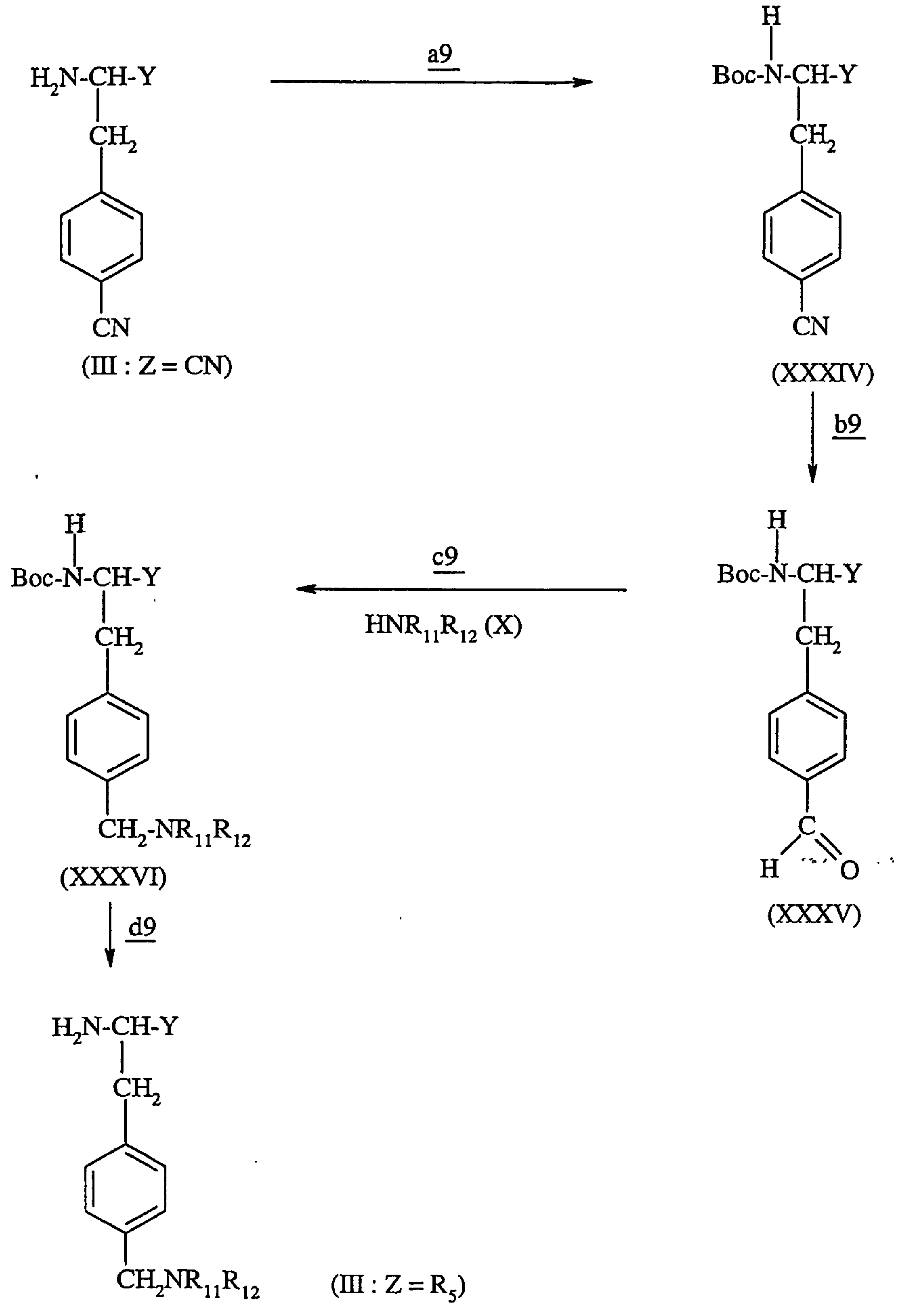


(III : Z = CN)

(XXXIV)

(XXXV)

(XXXVI)

(III : Z = $R_5$)

A l'étape a9 du Schéma 9, on protège l'amine d'un composé de formule (III) selon les méthodes conventionnelles.

A l'étape b9, on réduit le nitrile de formule (XXXIV) en un aldéhyde de formule (XXXV) selon la méthode décrite dans Synth. Commun., 1990, 20 (3), 459-467.

Puis, à l'étape c9, on fait réagir un composé de formule (X) avec l'aldéhyde de formule (XXXV) en présence

ou non d'un acide tel que l'acide acétique, dans un solvant tel que le méthanol, le dichlorométhane ou le 1,2-dichloroéthane pour former *in situ* une imine intermédiaire qui est réduite chimiquement en utilisant, par exemple, le cyanoborohydrure de sodium, le triacétoxyborohydrure de sodium ou le borohydrure de sodium.

Enfin à l'étape d9, on élimine le groupe N-protecteur du composé de formule (XXXVI) par traitement en milieu acide.

Les amines de formule (X) sont connues ou préparées selon des méthodes connues indiquées ci-après :

TABLEAU II

| $HNR_{11}R_{12}$ | Références |
|---|---|
| 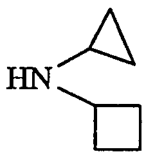 | J. Pharm. Sci., 1963, 52, 1191 |
| 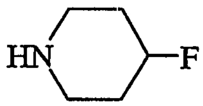 | J. Org. Chem., 1979, 44 (5), 771-7 |
| 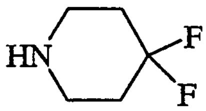 | Chem. Pharm. Bull., 1993, 41 (11), 1971-1986 |
| 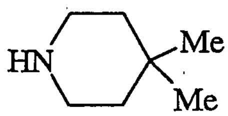 | Chem. Pharm. Bull., 1993, 41 (11), 1971-1986 |
| 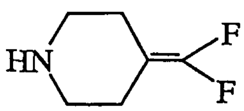 | WO 98/22443 |
| 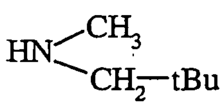 | J. Org. Chem., 1970, 35 (11), 3663-3666 |
| 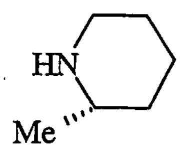 | Synth. Commun., 1999, 29 (10), 1747-1756 |
| 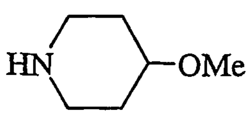 | J. Chem. Soc. Perkin Trans. 2, 1984, 737-744 |
| 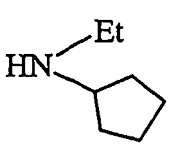 | Brevet US 2424063 |
| 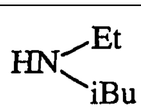 | J. Org. Chem. USSR (Engl. Trans 1), 1992, 28 (3.1), 374-380 |

(suite)

| $HNR_{11}R_{12}$ | Références |
|---|---|
| 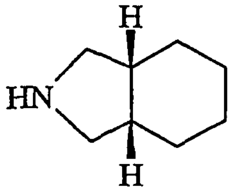 | Tetrahedron, 1999, 55 (31), 9439-9454 |
| 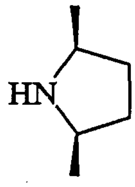 | J. Amer. Chem. Soc., 1955, 77, 4100-4102 |
| 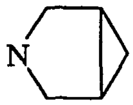 | WO 95/22547 |
| 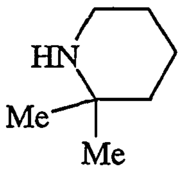 | WO 94/03437 |
| 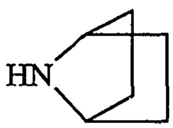 | Chem. Ber., 1991, 791-801 |

On peut également transformer un composé de formule (XXXV) en un composé de formule (XXXVI) selon le Schéma réactionnel suivant :

SCHEMA 9 (bis)

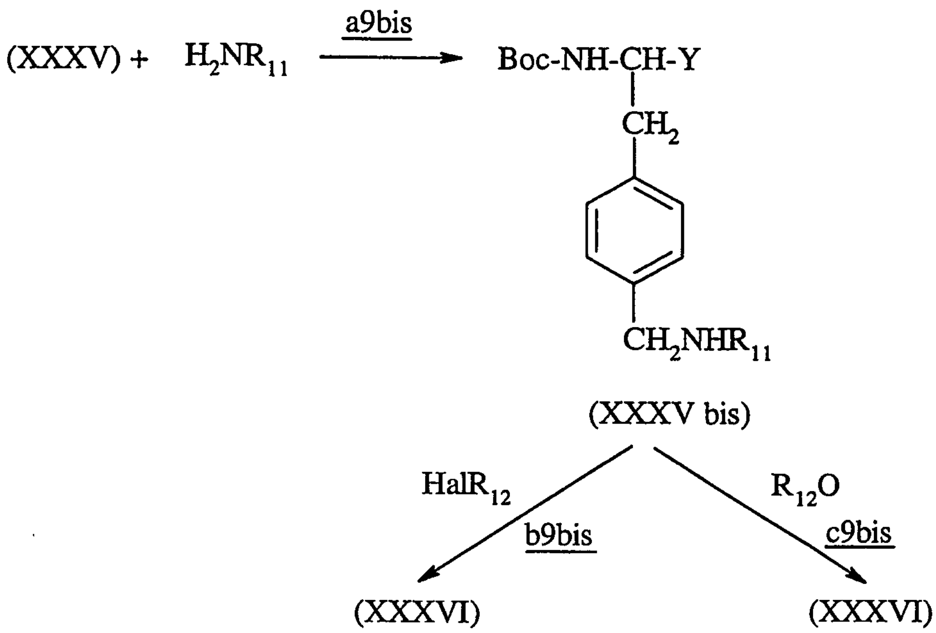


A l'étape a9bis, on effectue une amination réductrice en présence d'un agent réducteur tel que $NaHB(OAc)_3$. Ensuite, soit l'on procède à une alkylation par un halogénure d'alkyle, par exemple un iodure (étape b9bis), soit l'on procède à une nouvelle amination réductrice en faisant agir un dérivé carbonylé de formule $R_{12}O$ (étape c9bis), $R_{12}O$ représentant une dialkylcétone ou un alkylaldehyde. Ainsi par exemple, en traitant par l'acétone un composé (XXXVbis) dans lequel $R_{11}$ est cyclopropyle, on obtient un composé (XXXVI) dans lequel $R_{11}$ est cyclopropyle et $R_{12}$ est isopropyle.

Dans le cas où Y représente un groupe alcoxycarbonyle, on peut préparer un composé (III) dans lequel Y représente un groupe $CONR_8R_9$ selon le Schéma 10 ci-après :

SCHEMA 10

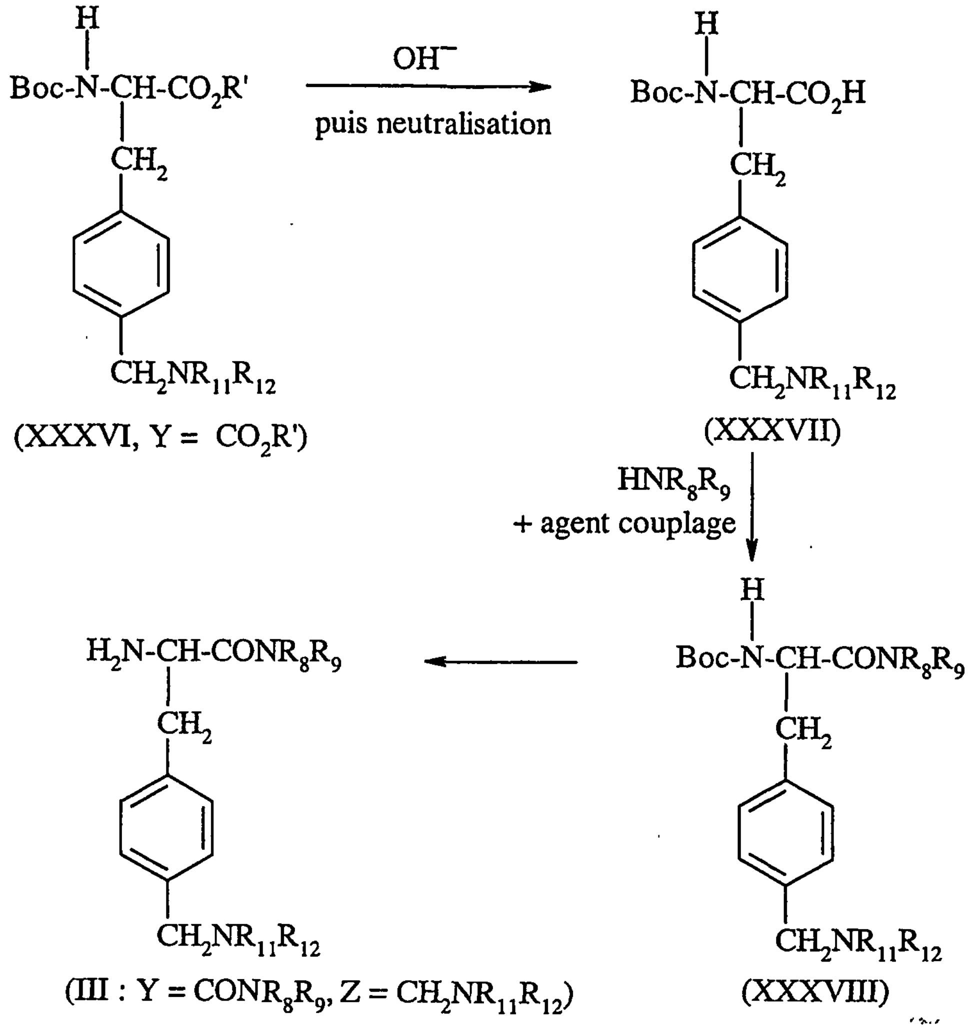


(XXXVI, Y = $CO_2R'$)

(XXXVII)

(XXXVIII)

(III : Y = $CONR_8R_9$, Z = $CH_2NR_{11}R_{12}$)

Les composés de formule (III) dans laquelle Y représente un phényle substitué par un groupe $-CH_2NR_{11}R_{12}$ et Z représente $R_5 = -CH_2NR_{11}R_{12}$, se préparent selon le Schéma 11 ci-après :

SCHEMA 11

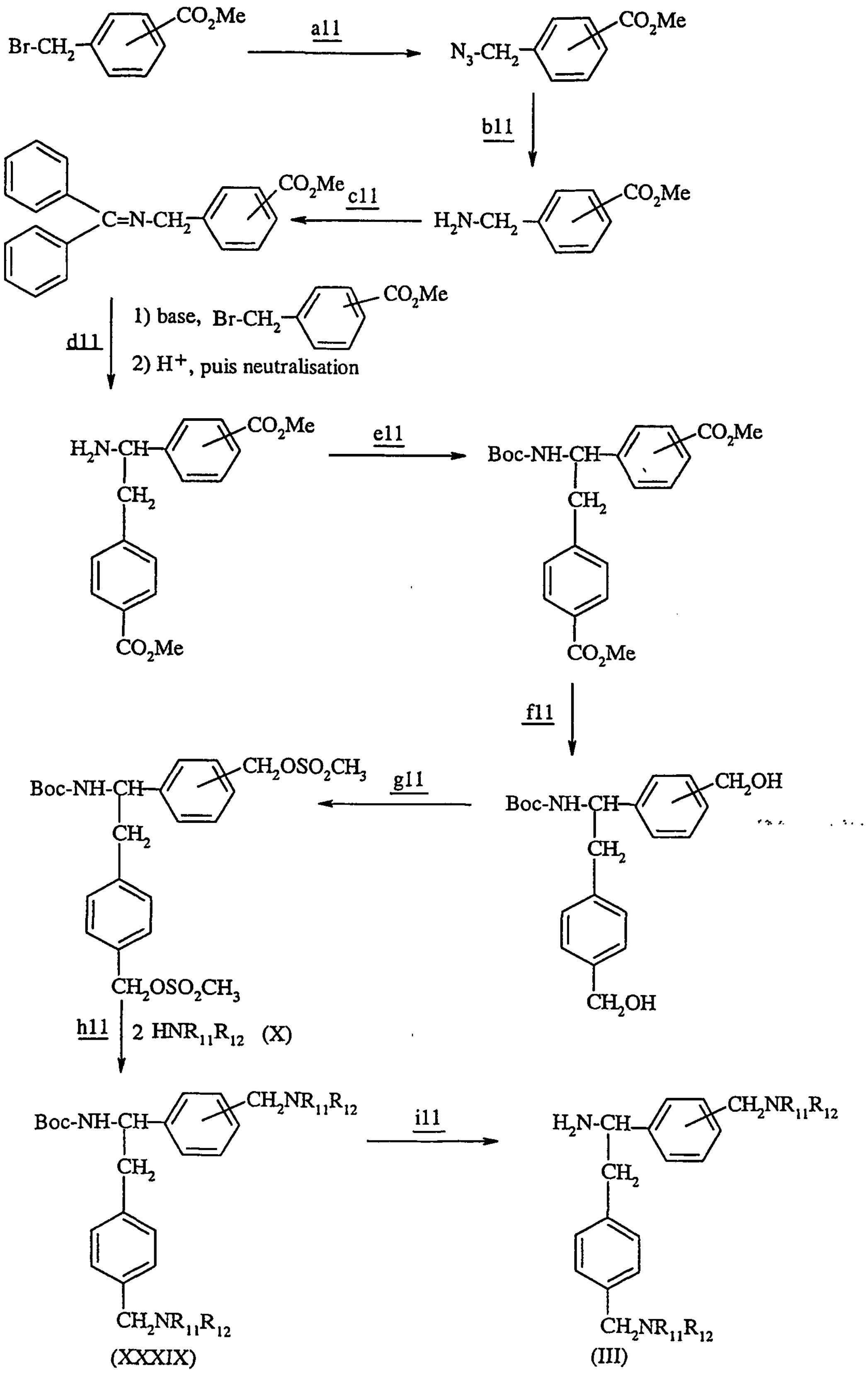


(XXXIX) (III)

Les étapes a11 et b11 s'effectuent selon les méthodes décrites au Schéma 5.
L'étape c11 s'effectue selon le procédé décrit à l'étape a4 du Schéma 4.
Les étapes d11 à i11 s'effectuent selon les procédés décrits aux étapes a8 à f8 du Schéma 8.
Les composés de formule (III) dans laquelle $R_4$ représente un groupe $COR_{13}$ peuvent être préparés selon le schéma ci-après.

SCHEMA 12

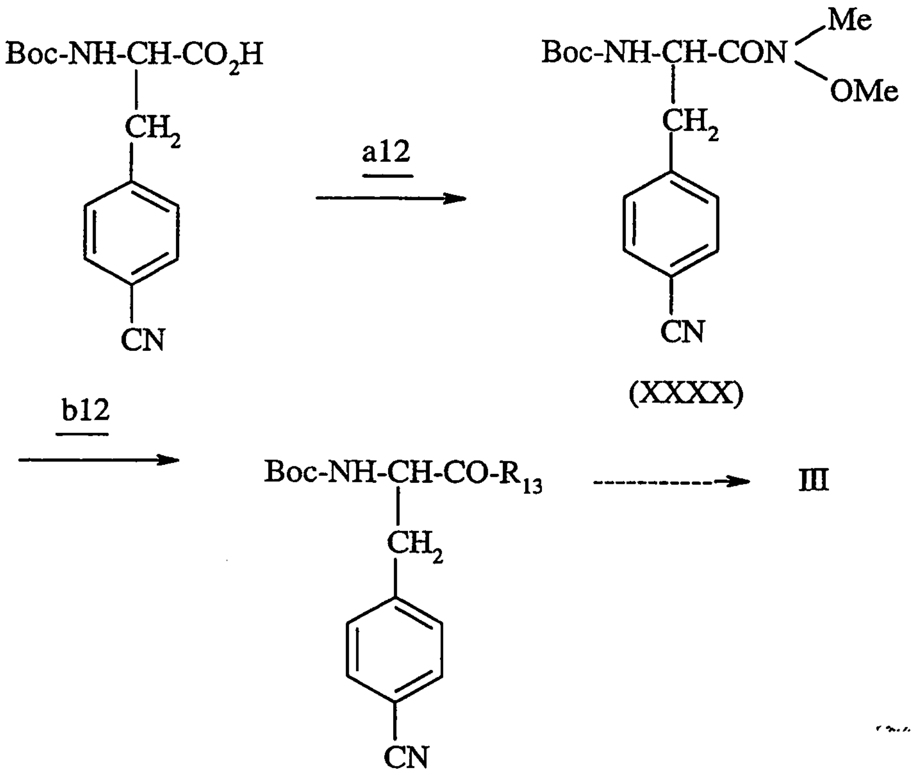


(XXXX)

III

(XXXXI)

A l'étape a12, on fait agir le chlorhydrate de N,O-diméthylhydroxylamine en présence d'un agent de couplage et à l'étape b12 on ajoute le dérivé lithié du composé $R_{13}H$ préparé par action du butyllithium sur le composé $R_{13}H$. On procède ensuite selon les étapes b9, c9 et d9 du schéma 9 pour obtenir un composé de formule (III) à partir du composé de formule (XXXXI).
Les composés de formule (III) dans laquelle $R_4$ représente un groupe $CSNR_8R_9$ sont préparés par action du réactif de Lawesson sur un composé analogue de formule (III) dans laquelle $R_4$ représente un groupe $CONR_8R_9$ et les autres substituants sont identiques (Tetrahedron, 1985, 41 (22), 5061-5087).
Les composés de formule (III) optiquement purs peuvent être préparés en dédoublant les racémiques par des méthodes classiques telles que celle utilisant des agents optiquement actifs ou des enzymes. Par exemple, lorsque dans un composé de formule (III), Y représente un groupe $-CONR_8R_9$ ou un $(C_1\text{-}C_4)$alcoxycarbonyle et Z = CN, on peut utiliser les méthodes décrites dans WO 97/25315 ; lorsque dans un composé de formule (III), Y représente un pyridyle et Z = CN, on peut utiliser les méthodes décrites dans Tetrahedron, 1995, 51/46, 12731-12744 ou dans Synthesis, 1996, N° 8, 991-996.
Parmi les composés de formule (III), certains sont nouveaux et constituent un aspect ultérieur de l'invention.
Les composés de formule :

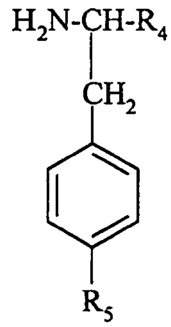

(III')

dans laquelle :

- $R_4$ représente un groupe $-CONR_8R_9$ ; un groupe $CSNR_8R_9$ ; un groupe $COR_{13}$ ; un phényle non substitué ou substitué une ou plusieurs fois par $R_{10}$ ; un radical hétérocyclique choisi parmi pyridyle, imidazolyle, furyle, benzimidazolyle, benzothiazol-2-yle, benzo[1,3]dioxol-5-yle, lesdits radicaux étant non substitués ou substitués par un méthyle ;
- $R_5$ représente un groupe $-CH_2NR_{11}R_{12}$ ou $-CH_2N(O)R_{11}R_{12}$ ;
- $R_8$ et $R_9$ représentent chacun indépendamment l'hydrogène ; un groupe $(C_1\text{-}C_4)$alkyle ; $(C_3\text{-}C_7)$cycloalkyle ; $(C_3\text{-}C_7)$cylcoalkyle$(C_1\text{-}C_4)$alkyle ; ω-$(C_1\text{-}C_4)$dialkylamino$(C_2\text{-}C_4)$alkyle ;
- ou bien $R_8$ et $R_9$ ensemble avec l'atome d'azote auquel ils sont liés constituent un radical hétérocyclique choisi parmi pyrrolidinyle, pipéridinyle, morpholin-4-yle, thiomorpholin-4-yle, pipérazin-1-yle, 4-méthylpipérazin-1-yle, 4-méthylpipéridin-1-yle, 2-méthylpipéridin-1-yle, 4,4-diméthylpipéridin-1-yle, 4,4-difluoropipéridin-1-yle, 4-trifluorométhylpipéridin-1-yle, 4-méthoxypipéridin-1-yle, 3,4-dihydropipéridin-1-yle, azépin-1-yle, cyclohexylspiro-4-pipéridin-1-yle ;
- $R_{10}$ représente un atome d'halogène ; un groupe $(C_1\text{-}C_4)$alkyle ; hydroxy ; $(C_1\text{-}C_6)$ alcoxy ; $R_{10}$ peut de plus représenter un groupe $-CH_2NR_{11}R_{12}$ lorsque $R_5$ représente un groupe-$CH_2NR_{11}R_{12}$, lesdits groupes étant alors identiques ;
- $R_{11}$ et $R_{12}$ représentent chacun indépendamment l'hydrogène; un groupe $(C_1\text{-}C_6)$alkyle; $(C_2\text{-}C_4)$alcènyle ; $(C_3\text{-}C_7)$cycloalkyle ; $(C_3\text{-}C_7)$cycloalkyle$(C_1\text{-}C_4)$alkyle ; ω-hydroxy$(C_2\text{-}C_4)$alkylène ; ω-méthoxy$(C_2\text{-}C_4)$alkylène ; ω-trifluorométhyl$(C_2\text{-}C_4)$alkylène ; ω-halogéno$(C_2\text{-}C_4)$alkylène ;
- ou bien $R_{11}$ et $R_{12}$ ensemble avec l'atome d'azote auquel ils sont liés constituent un radical hétérocyclique, mono ou bicyclique, choisi parmi azétidinyle, pyrrolidinyle, morpholin-4-yle, thiomorpholin-4-yle, pipéridin-1-yle, pipérazin-1-yle, 1,2,3,6-tétrahydropyrid-1-yle, 2,3,4,5-tétrahydropyridinium, decahydroquinolyle, decahydroisoquinolyle, tétrahydroisoquinolyle, octahydro-1*H*-isoindolyle, $(C_4\text{-}C_6)$cycloalkylspiropipéridinyle, 3-azabicyclo[3.1.0]hexyle, 7-azabicyclo[2.2.1]heptan-7-yle, non substitué ou substitué une ou plusieurs fois par un atome d'halogène, un groupe $(C_1\text{-}C_4)$alkyle, hydroxy, $(C_1\text{-}C_4)$alcoxy, trifluorométhyle, difluorométhylène ;
- $R_{13}$ représente un groupe phényle, thiazol-2-yle ou pyridyle ;

ainsi que leurs sels avec des acides minéraux ou organiques, sous forme de racémiques ou d'énantiomères purs, sont nouveaux et font partie de l'invention.

L'affinité des composés selon l'invention pour les récepteurs $B_1$ de la bradykinine a été mesurée sur des suspensions de membranes de cellules MRC5 en utilisant une technique proche de celle décrite par K.H. Schneck et al., dans Eur. J. Pharmacol., 1994, 266, 277-282. Dans ce test, l'affinité de la [des-$Arg^9$] bradykinine est comprise entre $10^{-6}$M et $10^{-7}$M, celle de la [des-$Arg^{10}$]kallidine est $2.10^{-9}$M ; et les composés de l'invention présentent une affinité allant jusqu'à $10^{-9}$M.

L'affinité est exprimée en termes de $CI_{50}$, la $CI_{50}$ étant la concentration qui inhibe à 50 % la liaison spécifique du ligand tritié [des-$Arg^{10}$]kallidine aux récepteurs des cellules MRC5.

L'affinité des composés selon l'invention pour les récepteurs $B_2$ de la bradykinine a été mesurée sur des suspensions de membranes de cellules MRC5 selon une technique proche de celle décrite par D.G. Sawutz et al., dans Eur. J. Pharmacol., 1992, 227, 309-315. Dans ce test, l'affinité de la bradykinine exprimée en termes de $CI_{50}$ est voisine de $10^{-9}$M alors que les composés de l'invention ne présentent pas d'affinité pour les récepteurs $B_2$ de la bradykinine à la concentration de $10^{-6}$M.

L'effet antagoniste des composés selon l'invention a été mesuré *in vitro* par l'inhibition de la contraction de l'aorte thoracique de lapin provoquée par l'administration de la [des-$Arg^9$]bradykinine, après une préincubation du tissu pendant 20 heures dans du tampon de Krebs saturé en mélange $CO_2/O_2$, suivant une adaptation de la technique décrite par L. Levesque et al. Br. J. Pharmacol., 1993, 109, 1254-1262.

L'effet antagoniste des composés selon l'invention a été également mesuré sur la libération de [$^3$H]inositol phosphate par des fibroblastes MRC5 en culture : après incorporation de [$^3$H]myoinositol pendant 48 heures, suivant la technique décrite par F. Oury Donat et al., J. Neurochem., 1994, 62, (4), 1399-1407. L'effet antagoniste est exprimé par la pourcentage d'inhibition de la libération de [$^3$H]inositol phosphate provoquée par la [des-Arg$^{10}$]kallidine à $10^{-8}$ M, sur des fibroblastes MRC5 préincubés pendant 4 heures en présence d'$IL_1\beta$ à 0.5 $\mu$g/ml.

L'absorption intestinale des composés selon l'invention a été étudiée *in vitro* sur le modèle de monocouche de cellules CACO-2, suivant une adaptation de la technique décrite par T. Lindmark et al., J. Pharmacol. Exp. Therap., 1995, 275 (2), 958-964.

Par ailleurs, plusieurs composés selon l'invention ont été étudiés *in-vivo* sur des modèles animaux.

L'effet antinociceptif a été vérifié sur un modèle de douleur neuropathique chez le rat après administration d'un composé selon l'invention à la dose de 30 mg/kg per os (selon le protocole décrit dans Pain, 2000, 86, 265-271).

On a observé qu'un composé selon l'invention, à la dose de 1 à 30 mg/kg per os, inhibe la phase tardive de la nociception induite par la formaline chez la souris (Pain, 1987, 203-114) ; ceci est le signe d'une action sur la douleur inflammatoire.

Un modèle d'hyperalgie thermique induite par irradiation UV chez le rat (Brit. Med. J., 1993, 110, 1441-1444) a permis de mettre en évidence les effets anti-hyperalgiques d'un composé selon l'invention à la dose de 1 à 3 mg/kg per os.

Les composés selon l'invention pourront être utiles pour le traitement ou la prévention de nombreuses pathologies, en particulier les pathologies de l'inflammation, les maladies inflammatoires persistantes ou chroniques (Drug News and Perspectives, 1994, 10 (7), 603-611), l'inflammation neurogénique, la douleur (Brit. J. Pharmacol., 1993, 110, 193-198), les douleurs chroniques, les neuropathies, le choc septique, les brûlures (Pain, 1993, 53, 191-197), les plaies, les maladies des voies respiratoires, l'asthme, le syndrome de réponse inflammatoire systémique, l'oedème (Brit. J. Pharmacol., 1995, 114, 1005-1013), l'oedème cérébral, l'angiogénèse (Brit. J. Pharmacol., 1993, 109, 14-17), le diabète infectieux de type I (Abst. 14th Intern. Symp. on Kinins, C49, Denver Colorado, 10-15 Sept. 1995), la vasculopathie diabétique, l'hypertrophie ventriculaire, la fibrose pulmonaire et la sclérose progressive systémique.

A titre d'exemple on peut citer :

- l'inflammation, les douleurs osseuses, les douleurs musculaires, les douleurs articulaires, les douleurs faciales, les fibromyalgies, les hyperalgies, les douleurs liées au cancer, les douleurs périopératoires, les douleurs menstruelles, les maux de tête, les maux de dents, les douleurs gynécologiques, les migraines ;
- l'hyperactivité des voies respiratoires dans l'asthme, l'asthme atopique ou non-atopique, l'asthme allergique ou non-allergique, la bronchite, la pneumoconiose, les maladies chroniques obstructives des voies respiratoires, la pleurésie, les maladies chroniques obstructives pulmonaires, les rhinites d'origine virale ou allergique ;
- la résistance post-capillaire, le diabète, la vasculopathie diabétique, les symptomes diabétiques associés à l'insulite (par exemple : hyperglycémie, diurèse, protéinurie) ;
- le choc septique ;
- la maladie d'Alzheimer, les traumatismes crâniens ;
- l'arthrite, l'arthrite rhumatoide, les maladies inflammatoires des articulations, l'athérosclérose, la sclérose en plaques ou sclérose multiple ;
- les maladies des voies digestives, les maladies des voies urinaires, la cystite, la pancréatite, la néphrite, l'entérocolite, la colite ulcérative, le syndrome du colon irritable, la maladie de Crohn, les maladies du foie;
- les pathologies du système oculaire, l'uveite, la rétinite, le glaucome ;
- les maladies de la peau telle que les maladies atopiques, l'eczéma, le psoriasis, les dermatites, les démangeaisons ;
- la perte des cheveux.

Par ailleurs, les composés de l'invention sont utiles pour leur effet antiprolifératif des cellules cancéreuses ; leur action sur les maladies neurodégénératives, la dégénérescence de la myeline, les maladies dégénératives d'origine virale ; leur effet cardioprotecteur.

De plus, les composés de l'invention sont utiles comme relaxants musculaires, relaxant des muscles lisses, des spasmes du tractus gastrointestinal et le l'utérus.

Plus généralement les composés selon l'invention peuvent être utiles pour le traitement ou la prévention de toutes les pathologies où la bradykinine joue un rôle déterminant désignées ci-après les pathologies bradykinine-dépendantes.

Les composés de la présente invention sont notamment des principes actifs de compositions pharmaceutiques, dont la toxicité est compatible avec leur utilisation en tant que médicaments.

Les composés de formule (I) ci-dessus peuvent être utilisés à des doses journalières de 0,01 à 100 mg par kilo de poids corporel du mammifère à traiter, de préférence à des doses journalières de 0,1 à 50 mg/kg. Chez l'être humain, la dose peut varier de préférence de 0,5 à 4000 mg par jour, plus particulièrement de 2,5 à 1000 mg selon l'âge

du sujet à traiter ou le type de traitement : prophylactique ou curatif.

Pour leur utilisation comme médicaments, les composés de formule (I) sont généralement administrés en unités de dosage. Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (I) ou l'un de ses sels et /ou solvats ou hydrates pharmaceutiquement acceptables.

Dans les compositions pharmaceutiques de la présente invention pour l'administration par voie orale, sublinguale, inhalée, sous-cutanée, intramusculaire, intraveineuse, transdermique, locale ou rectale, les principes actifs peuvent être administrés sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les aérosols, les formes d'administration topique, les implants, les formes d'administration sous-cutanée, intramusculaire, intraveineuse, intranasale ou intraoculaire et les formes d'administration rectale.

Lorsque l'on prépare une composition solide sous forme de comprimés, on ajoute au principe actif, micronisé ou non, un véhicule pharmaceutique qui peut être composé de diluants comme par exemple le lactose, la cellulose microcristalline, l'amidon et des adjuvants de formulation comme des liants (polyvinylpyrrolidone, hydroxypropylméthylcellulose, etc...), des agents d'écoulement comme la silice, des lubrifiants comme le stéarate de magnésium, l'acide stéarique, le tribéhénate de glycérol, le stéarylfumarate de sodium.

Des agents mouillants ou tensioactifs tels que le laurylsulfate de sodium peuvent être ajoutés à la formulation.

Les comprimés peuvent être réalisés par différentes techniques, compression directe, granulation sèche, granulation humide, fusion à chaud.

Les comprimés peuvent être nus ou dragéifiés (par du saccharose par exemple) ou enrobés avec divers polymères ou autres matières appropriés.

Les comprimés peuvent avoir une libération flash, retardée ou prolongée en réalisant des matrices polymériques ou en utilisant des polymères spécifiques au niveau du pelliculage.

On obtient une préparation en gélule par simple mélange du principe actif avec des véhicules pharmaceutiques secs (simple mélange ou granulation sèche, humide, fusion à chaud), liquides ou semi-solides.

Les gélules peuvent être molles ou dures, pelliculées ou non de manière à avoir une activité flash, prolongée ou retardée (par exemple par une forme entérique).

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion, des agents mouillants ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents solubilisants pharmacologiquement compatibles, par exemple le propylèneglycol ou le butylèneglycol.

Ainsi, pour préparer une solution aqueuse injectable par voie intraveineuse on peut utiliser un cosolvant comme par exemple un alcool tel que l'éthanol ou un glycol tel que le polyéthylèneglycol ou le propylèneglycol, et un tensioactif hydrophile tel que le Tween® 80. Pour préparer une solution huileuse injectable par voie intramusculaire, on peut solubiliser le principe actif par un triglycéride ou un ester de glycérol.

Pour l'administration locale on peut utiliser des crèmes, des pommades, des gels, des collyres.

Pour l'administration transdermique, on peut utiliser des patches sous forme multilaminée ou à réservoir dans lequel le principe actif peuvent être en solution alcoolique.

Pour une administration par inhalation on utilise un aérosol contenant par exemple du trioléate de sorbitane ou de l'acide oléique ainsi que du trichlorofluorométhane, du dichlorofluorométhane, du dichlorotétrafluoroéthane ou tout autre gaz propulseur biologiquement compatible ; on peut également utiliser un système contenant le principe actif seul ou associé à un excipient, sous forme de poudre.

Le principe actif peut être également présenté sous forme de complexe avec une cyclodextrine, par exemple, α, β, γ-cyclodextrine, 2-hydroxypropyl-β-cyclodextrine, méthyl-β-cyclodextrine.

Le principe actif peut être formulé également sous forme de microcapsules ou microsphères, éventuellement avec un ou plusieurs supports ou additifs.

Parmi les formes à libération prolongée utiles dans le cas de traitements chroniques, on peut utiliser les implants.

Ceux-ci peuvent être préparés sous forme de suspension huileuse ou sous forme de suspension de microsphères dans un milieu isotonique.

Dans chaque unité de dosage le principe actif de formule (I) est présent dans les quantités adaptées aux doses journalières envisagées. En général chaque unité de dosage est convenablement ajustée selon le dosage et le type d'administration prévu, par exemple comprimés, gélules et similaires, sachets, ampoules, sirops et similaires, gouttes de façon à ce qu'une telle unité de dosage contienne de 0,5 à 1000 mg de principe actif, de préférence de 2,5 à 250 mg devant être administrés une à quatre fois par jour.

Les compositions de la présente invention peuvent contenir, à côté des composés de formule (I) ci-dessus ou d'un de leurs sels et/ou solvats ou hydrates pharmaceutiquement acceptables, d'autres principes actifs qui peuvent être utiles dans le traitement des troubles ou maladies indiquées ci-dessus. Par exemple, une composition pharmaceutique selon la présente invention peut contenir un antagoniste des récepteurs $B_2$ de la bradykinine associé à un composé selon la présente invention.

Selon un autre de ses aspects, la présente invention concerne l'utilisation des composés de formule (I), ou l'un de leurs sels et/ou solvats ou hydrates pharmaceutiquement acceptables pour la préparation de médicaments destinés à traiter toute pathologie où la bradykinine et les récepteurs $B_1$ sont impliquées.

Selon un autre de ses aspects, la présente invention concerne l'utilisation des composés de formule (I) ou de l'un de leurs sels et/ou solvats ou hydrates pharmaceutiquement acceptables pour la préparation de médicaments destinés à traiter les pathologies de l'inflammation, les maladies inflammatoires persistantes ou chroniques.

Dans les Préparations et dans les EXEMPLES, on utilise les abréviations suivantes :

éther : éther diéthylique
éther iso : éther diisopropylique
DCM : dichlorométhane
DMF : N,N-diméthylformamide
DMSO : diméthylsulfoxyde
AcOEt : acétate d'éthyle
THF : tétrahydrofurane
AcOH : acide acétique
TFA : acide trifluoroacétique
TEA : triéthylamine
DIPEA : diisopropyléthylamine
DMAP : 4-diméthylaminopyridine
DCE : dichloroéthane
DCC : 1,3-dicyclohexylcarbodiimide
DCU : dicyclohexylurée
TBTU : O-(1H-benzotriazol-1-yl)-N,N,N',N'-tétraméthyluronium tétrafluoroborate
Boc : *tert*-butoxycarbonyle
$(Boc)_2O$ : di-*tert*-butyledicarbonate
Penicilline amidase commercialisée par SIGMA
Alcalase commercialisée par NOVO
Séphadex® LH 20 : commercialisée par PHARMACIA
BOP : hexafluorophosphate de benzotriazol-1-yloxytris(diméthylamino) phosphonium
TBTU : benzotriazol-1-yl-N,N,N',N'-tétraméthyluronium tétrafluoroborate
tampon $KHSO_4/K_2SO_4$ : solution de 16,66 g de $KHSO_4$ et 32,32 g de $K_2SO_4$ dans 1 litre d'eau
éther chlorhydrique : solution saturée d'HCl gaz dans l'éther diéthylique
F : point de fusion
TA : température ambiante

Sauf indication contraire, les spectres de résonance magnétique nucléaire (RMN) du proton sont enregistrés à 200 MHz dans DMSO-$d_6$ contenant éventuellement du TFA, la référence est placée sur le DMSO qui se situe à 2,50 ppm du tétraméthylsilane. Les déplacement chimiques $\delta$ sont indiqués en ppm.
s : singulet ; se : singulet élargi; sd : singulet dédoublé ; d : doublet ; de : doublet élargi; dd : doublet dédoublé : t : triplet ; qd : quadruplet ; qt : quintuplet ; mt : multiplet ; m : massif ; sp septuplet.

Lorsque seule l'indication RMN apparaît dans la présente description, cela signifie que le spectre RMN enregistré dans les conditions ci-dessus est conforme à la structure attendue.

Les spectres de masse indiquent la valeur $MH^+$.

PREPARATIONS

Préparation 1.1

Acide 3-(naphtalène-2-sulfonylamino)-3-phénylpropionique.

(II) : X = 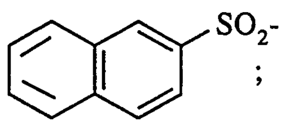 ; $R_2$ = H ; $R_3$ = 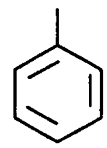 ;

On dissout 4,13 g d'acide 3-amino-3-phénylpropionique dans un mélange de 100 ml de dioxane et 25 ml de NaOH 1N, ajoute par portions 5,6 g de chlorure de naphtalène-2-sulfonyle en maintenant le pH = 10,5-10,8 par addition de NaOH 1N et laisse 2 heures sous agitation à TA. On dilue le mélange réactionnel par addition de 400 ml d'eau, acidifie à pH = 2 par ajout d'HCl 2N, extrait à l'AcOEt, lave la phase organique par une solution tampon $KHSO_4$/$K_2SO_4$, sèche sur $Na_2SO_4$ et évapore sous vide le solvant. On obtient 7,33 g du produit attendu après trituration dans l'heptane et séchage sous vide, F = 126 - 129°C.
RMN : δ (ppm) : 2,55 - 2,70 : mt : 2H ; 4,70 : t : 1H ; 6,85 - 8,15 : m : 12H.

Préparation 1.2

Acide 3-[méthyl-(naphtalène-2-sulfonyl)amino]-3-phénylpropionique.

(II) : X = 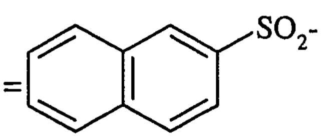 ; $R_2$=Me ; $R_3$= 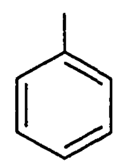

A) Acide 3-(*tert*-butoxycarbonylamino)-3-phénylpropionique.

A un mélange de 8,3 g d'acide 3-amino-3-phénylpropionique dans 35 ml d'eau et 5 ml de dioxane, on ajoute 8,3 ml de triéthylamine puis, en 30 minutes, une solution de 12,8 g de di-*tert*-butyldicarbonate dans 25 ml de dioxane et laisse une nuit sous agitation à TA. On dilue le mélange réactionnel à l'eau, lave la phase aqueuse à l'éther, acidifie la phase aqueuse à pH = 2,5 par ajout d'HCl 2N, extrait à l'AcOEt, lave la phase organique à l'eau, par une solution saturée de NaCl, sèche sur $Na_2SO_4$ et évapore sous vide le solvant. On obtient 12,4 g du produit attendu.

B) Acide-3-(N-*tert*-butoxycarbonyl-méthylamino)-3-phénylpropionique.

A un mélange de 0,795 g du composé obtenu à l'étape précédente et de 0,62 ml d'iodure de méthyle dans 10 ml de THF, on ajoute par portions 0,27 g d'hydrure de sodium à 80 % dans l'huile et laisse une nuit sous agitation à TA. On dilue le mélange réactionnel à l'AcOEt, ajoute de l'eau et acidifie à pH = 2 par ajout d'HCl 1N. Après décantation, on lave la phase organique à l'eau, par une solution saturée de NaCl, sèche sur $Na_2SO_4$ et évapore sous vide le solvant. On obtient 0,82 g du produit attendu.

C) Acide 3-(méthylamino)-3-phénylpropionique, trifluoroacétate.

On laisse 55 minutes sous agitation à TA, un mélange de 0,81 g du composé obtenu à l'étape précédente et 12 ml de TFA dans 10 ml de DCM. On concentre sous vide le mélange réactionnel, reprend le résidu au DCM et évapore sous vide le solvant. On dissout le résidu dans l'éther, ajoute de l'heptane jusqu'à précipitation, décante le solvant et sèche la gomme obtenue. On obtient 0,86 g du produit attendu sous forme de mousses.

D) Acide 3-[méthyl-(naphtalène-2-sulfonyl)amino]-3-phénylpropionique

A un mélange de 0,85 g du composé obtenu à l'étape précédente dans 5 ml de dioxane, on ajoute 5 ml de NaOH 1N, puis par portions 0,698 g de chlorure de naphtalène-2-sulfonyle en maintenant le pH = 10-11 par ajout de NaOH 1N et laisse 2 heures sous agitation à TA. On dilue le mélange réactionnel avec de l'eau, lave la phase aqueuse à l'éther, acidifie la phase aqueuse à pH = 2 par ajout d'HCl 1N, extrait à l'AcOEt, lave la phase organique à l'eau, par une solution saturée de NaCl, sèche sur $Na_2SO_4$ et évapore sous vide le solvant. On obtient 0,65 g du produit attendu sous forme de cire.

Préparation 1.3

Acide 3-(3-méthylphényl)-3-(naphtalène-2-sulfonylamino)propionique

(II) : X = 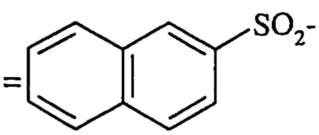 ; $R_2$=H ; $R_3$ = 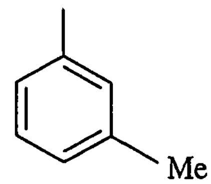

A) Acide 3-amino-3-(3-méthylphényl)propionique

A un mélange de 10,4 g d'acide malonique et 15,4 g d'acétate d'ammonium dans 150 ml de 2-méthoxyéthanol, on ajoute 11,8 ml de 3-méthylbenzaldéhyde et chauffe à 80° C pendant une nuit. Après refroidissement à TA, on essore le précipité formé, le lave à l'éther et le sèche. On obtient 6,8 g du produit attendu.
RMN : δ (ppm) : 2,25 : s : 3H ; 2,80 à 3,05 : mt :2H ; 4,55 : t :1H ; 7,10 à 7,30: m :4H.

B) Acide 3-(3-méthylphényl)-3-(naphtalène-2-sulfonylamino)propionique

A une suspension de 1,79 g du composé obtenu à l'étape précédente dans 25 ml de dioxane, on ajoute 10 ml de NaOH 1N puis, par petites fractions, 2,26 g de chlorure de naphtalène-2-sulfonyle en maintenant le pH = 10,5-12 par ajout de NaOH 1N et laisse 2 heures sous agitation à TA. On dilue le mélange réactionnel à l'eau, lave la phase aqueuse à l'AcOEt, acidifie la phase aqueuse à pH = 1 par ajout d'HCl 6N, extrait à l'AcOEt, lave la phase organique par une solution saturée de NaCl, sèche sur $Na_2SO_4$ et évapore sous vide le solvant. On obtient 3,37 g du produit attendu après cristallisation dans l'heptane.

Préparation 1.4

Acide-3-(3,4-diméthylphényl)-3-(naphtalène-2-sulfonylamino)propionique.

(II) : X = 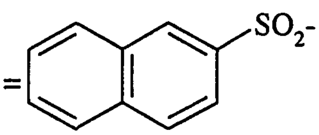 ; $R_2$=H ; $R_3$ = 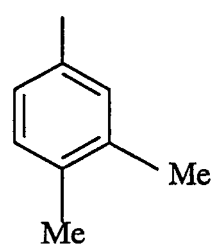

A) Acide 3-amino-3-(3,4-diméthylphényl)propionique,chlorhydrate.

On chauffe à reflux pendant 5 heures un mélange de 5 g de 3,4-diméthylbenzaldéhyde, 3,88 g d'acide malonique

et 5,74 g d'acétate d'ammonium dans 50 ml d'EtOH. Après refroidissement à TA, on essore le précipité formé et le lave à l'EtOH. On reprend le précipité dans un mélange DCM/HCl 1N, filtre un insoluble, décante le filtrat et concentre sous vide la phase aqueuse acide jusqu'à début de précipitation, refroidit à 0° C et essore le précipité formé. On reprend le précipité dans un mélange DCM/MeOH (6/4 ;v/v), sèche sur $Na_2SO_4$ et évapore sous vide les solvants. On reprend le résidu dans l'éther et essore le précipité formé après trituration. On obtient 3,01 g du produit attendu, F = 192° C (déc.).

B) Acide-3-(3,4-diméthylphényl)-3-(naphtalène-2-sulfonylamino)propionique.

A un mélange de 2 g du composé obtenu à l'étape précédente et 17,4 ml de NaOH 1N dans 20 ml de dioxane, on ajoute goutte à goutte une solution de 1,98 g de chlorure de naphtalène-2-sulfonyle dans 15 ml de dioxane et laisse une nuit sous agitation à TA. On neutralise le mélange réactionnel à pH = 7 par ajout d'HCl 1N et concentre sous vide. On reprend le résidu dans un mélange AcOEt/$NaHCO_3$ saturée, essore le précipité formé, le reprend dans un mélange DCM/HCl 1N, sèche la phase organique sur $Na_2SO_4$ après décantation et évapore sous vide le solvant. On obtient 1,66 g du produit attendu, F = 122° C (déc.).

Préparation 1.5

Acide 3-(3,5-diméthoxyphényl)-3-(naphtalène-2-sulfonylamino)propionique.

(II) : X = 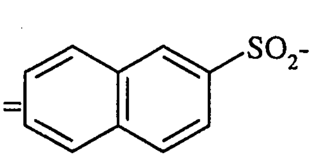 ; $R_2$=H ; $R_3$ = 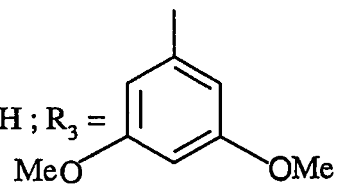

A) Acide 3-amino-3-(3,5-diméthoxyphényl)propionique.

On chauffe à reflux pendant 5 heures un mélange de 5 g de 3,5-diméthoxybenzaldéhyde, 3,13 g d'acide malonique et 4,64 g d'acétate d'ammonium dans 50 ml d'EtOH. On concentre sous vide le mélange réactionnel, reprend le résidu à l'eau, filtre un insoluble, lave le filtrat au DCM et concentre sous vide la phase aqueuse. On reprend le résidu à l'eau et concentre sous vide à nouveau. On reprend le résidu à l'EtOH et évapore sous vide le solvant. On obtient 2,96 g du produit attendu.

B) Acide 3-(3,5-diméthoxyphényl)-3-(naphtalène-2-sulfonylamino)propionique.

A un mélange de 1 g du composé obtenu à l'étape précédente et 4,5 ml de NaOH 1N dans 15 ml de dioxane, on ajoute par portions 1,01 g de chlorure de naphtalène-2-sulfonyle et laisse une nuit sous agitation à TA. On dilue le mélange réactionnel à l'eau, lave à l'AcOEt, acidifie la phase aqueuse à pH = 1 par ajout de HCl concentré, extrait à l'éther, lave la phase organique par HCl 1N, sèche sur $Na_2SO_4$ et évapore sous vide le solvant. On obtient 1,41 g du produit attendu, F = 190° C.

Préparation 1.6

Acide 3-(3,4-diméthoxyphényl)-3-(naphtalène-2-sulfonylamino)propionique.

(II) : X = 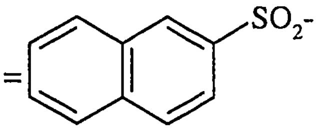 ; $R_2$=H ; $R_3$ = 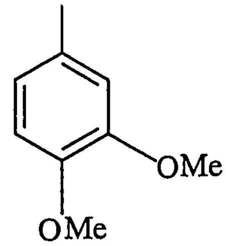

A) Acide 3-amino-3-(3,4-diméthoxyphényl)propionique,chlorhydrate.

On chauffe à reflux pendant 5 heures un mélange de 5 g de 3,4-diméthoxybenzaldéhyde, 3,131 g d'acide malonique et 4,64 g d'acétate d'ammonium dans 50 ml d'EtOH. Après refroidissement à TA, on essore le précipité formé. On reprend le précipité à l'eau, acidifie à pH = 2 par ajout de HCl 1N, lave la phase aqueuse au DCM et la concentre sous vide. On reprend le résidu à l'eau et concentre à nouveau sous vide. On reprend le résidu à l'EtOH et évapore sous vide. On obtient 2,99 g du produit attendu.

B) Acide 3-(3,4-diméthoxyphényl)-3-(naphtalène-2-sulfonylamino)propionique.

On prépare ce composé selon le mode opératoire décrit à l'étape B) de la préparation 1.5 à partir de 1 g du composé obtenu à l'étape précédente et 7,65 ml de NaOH 1N dans 15 ml de dioxane. On obtient 1,33 g du produit attendu.

Préparation 1.7

Acide 3-(benzo[1,3]dioxol-5-yl)-3-(naphtalène-2-sulfonylamino)propionique.

(II) : X = 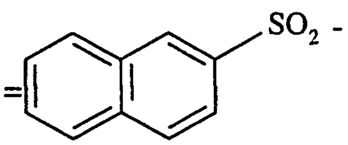 ; $R_2$ =H ; $R_3$ = 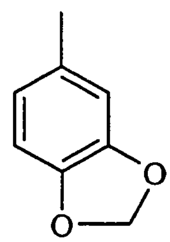

A) Acide 3-amino-3-(benzo[1,3]dioxol-5-yl)propionique.

On prépare ce composé selon le mode opératoire décrit dans Biorg. Med. Chem., 1994, 2 (9), 881.

B) Acide 3-(benzo[1,3]dioxol-5-yl)-3-(naphtalène-2-sulfonylamino)propionique.

A une suspension de 2,16 g du composé obtenu à l'étape précédente dans 40 ml de dioxane, on ajoute NaOH 1N jusqu'à pH = 11,8 puis, par petites fractions, 2,33 g de chlorure de naphtalène-2-sulfonyle et laisse 2 heures sous agitation à TA en maintenant le pH = 10,5 - 11,5 par ajout de NaOH 1N. On dilue le mélange réactionnel par un égal volume d'eau, lave à l'AcOET, acidifie la phase aqueuse à pH = 1,5 par ajout d'HCl 6N, extrait à l'AcOET, lave la phase organique par un tampon $KHSO_4/K_2SO_4$, par une solution saturée de NaCl, sèche sur $Na_2SO_4$ et évapore sous vide le solvant. On obtient 3,9 g du produit attendu après cristallisation dans l'heptane, F = 173 - 175° C.
RMN : δ (ppm) : 2,45-2,60 : mt : 2H ; 4,65 : qd : 1H; 5,40-5,70 : mt : 2H ; 6,45-6,65 : mt : 3H ; 7,55-7,75 : mt : 3H ; 7,90-8,10 : mt : 4H ; 8,35 : de : 1H.

Préparation 1.8

Acide 3-(2,3-dihydro-benzo[1,4]dioxin-6-yl)-3-(naphtalène-2-sulfonylamino) propionique.

(II) : X = 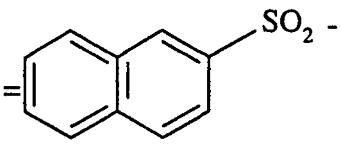 ; $R_2$ = H ; $R_3$ = 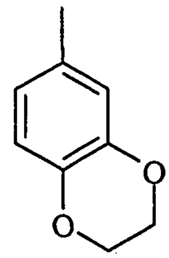

A) Acide 3-amino-3-(2,3-dihydrobenzo[1,4]dioxin-6-yl)propionique.

On chauffe à reflux pendant 5 heures un mélange de 5 g de 2,3-dihydro-benzo[1,4]dioxine-6-carbaldéhyde, 3,17 g d'acide malonique et 4,69 g d'acétate d'ammonium dans 50 ml de EtOH. On laisse le mélange réactionnel revenir à TA, essore le précipité formé, le lave à l'EtOH puis à l'eau. On obtient 1,965 g du produit attendu après séchage sous vide.

B) Acide 3-(2,3-dihydro-benzo[1,4]dioxin-6-yl)-3-(naphtalène-2-sulfonylamino) propionique.

On prépare ce composé selon le mode opératoire décrit à l'étape B de la préparation 1.5 à partir de 1 g du composé obtenu à l'étape précédente, 4,5 ml de NaOH 1N, 15 ml de dioxane et 1,02 g de chlorure de naphtalène-2-sulfonyle. On obtient 0,876 g du produit attendu après cristallisation dans l'hexane.

Préparation 1.9

Acide 3-(2,3-dihydrobenzo[1,4]dioxin-6-yl)-3-(5,6,7,8-tétrahydronaphtalène-2-sulfonylamino)propionique.

(II) : X = 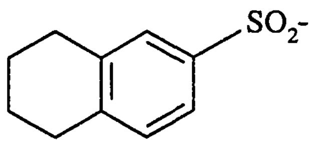 ; $R_2$ = H ; $R_3$ = 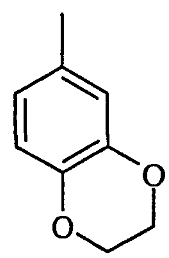

A un mélange de 0,5 g du composé obtenu à l'étape A de la préparation 1.8 et 4,5 ml de NaOH 1N dans 20 ml de dioxane, on ajoute goutte à goutte une solution de 0,570 g de chlorure de 5,6,7,8-tétrahydro-naphtalène-2-sulfonyle dans 15 ml de dioxane et laisse 4 heures sous agitation à TA. On concentre sous vide le mélange réactionnel, reprend le résidu par NaOH 1N, lave au DCM, acidifie la phase aqueuse à pH = 1 par ajout d'HCl concentrée, extrait au DCM, lave la phase organique par une solution saturée de NaCl, sèche sur $Na_2SO_4$ et évapore sous vide le solvant. On obtient 0,5 g du produit attendu.

Préparation 1.10

Acide 3-[(2,4-dichloro-3-méthylbenzènesulfonyl)-phénylamino]propionique.

(II) : X = 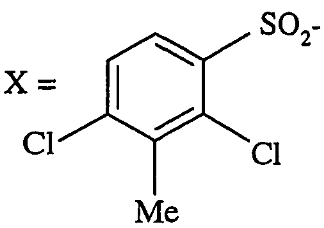 ; $R_2$ = 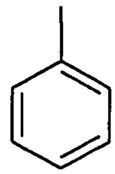 ; $R_3$ = H

A) Chlorure de 2,4-dichloro-3-méthylbenzènesulfonyle.

On prépare ce composé selon le mode opératoire décrit dans J. Am. Chem. Soc., 1940, 62, 511-512.

B) Ester méthylique de l'acide 3-(phénylamino)propionique.

On chauffe à reflux pendant 8 heures un mélange de 20 ml d'aniline, 22 ml d'ester méthylique de l'acide acrylique et 2 ml d'acide acétique. Après concentration sous vide du mélange réactionnel, on distille sous pression réduite l'huile résultante (Eb = 132° C sous 333,3 Pa puis Eb = 110° C sous 6,66 Pa). On reprend le produit obtenu dans l'hexane et essore le précipité formé. On obtient 25 g du produit attendu.

C) Ester méthylique de l'acide 3-[(2,4-dichloro-3-méthylbenzènesulfonyl) phénylamino]propionique.

On laisse 1 heure sous agitation à TA un mélange de 1,5 g du composé obtenu à l'étape A, 1,03 g du composé obtenu à l'étape B et 0,08 g de DMAP dans 20 ml de pyridine. On concentre sous vide le mélange réactionnel, extrait le résidu à l'AcOEt, lave la phase organique par une solution tampon pH = 2, par une solution saturée de NaCl, sèche sur $Na_2SO_4$ et évapore sous vide le solvant. On obtient 2,32 g du produit attendu.

D) Acide 3-[(2,4-dichloro-3-méthylbenzènesulfonyl)-phénylamino]propionique.

A une solution de 2,32 g du composé obtenu à l'étape précédente dans 10 ml d'EtOH et 10 ml de dioxane, on ajoute 8,7 ml de KOH 1N et on laisse une nuit sous agitation à TA. On concentre sous vide le mélange réactionnel, reprend le résidu par une solution saturée de $NaHCO_3$, lave la phase aqueuse à l'éther, acidifie la phase aqueuse à pH = 1 par ajout d'HCl 1N, extrait au DCM, lave la phase organique à l'eau, sèche sur $Na_2SO_4$ et évapore sous vide le solvant. On obtient 0,912 g du produit attendu.
RMN : δ (ppm) : 2,35 : t : 2H ; 2,5 : s : 3H ; 4,0 : t : 2H ; 7,15 à 7,4 : m : 5H ; 7,45 à 7,65 : qd : 2H ; 12,2 à 12,5 : se : 1H.

Préparation 1.11

Ester 2,5-dioxopyrrolidin-1-yle de l'acide 3-(naphtalène-2-sulfonylamino)-3-phénylpropionique.

A un mélange de 1,78 g du composé obtenu à la Préparation 1.1 et 0,578 g de N-hydroxysuccinimide dans 15 ml de DMF, on ajoute 1,13 g de 1,3-dicyclohexylcarbodiimide et laisse une nuit sous agitation à TA. Après essorage de la 1,3-dicyclohexylurée formée, on dilue le filtrat à l'eau, extrait à l'AcOEt, lave la phase organique par une solution saturée de $NaHCO_3$, par une solution saturée de NaCl, sèche sur $Na_2SO_4$ et évapore sous vide le solvant. On obtient 1,81 g du produit attendu.

Préparation 1.12

Acide 3-phényl-3-(quinoléine-2-sulfonylamino)propionique.

(II) : X = 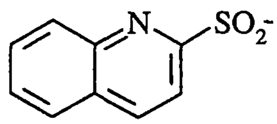 ; $R_2 = H$ ; $R_3$ = 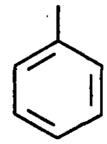

On prépare ce composé selon le mode opératoire décrit à la Préparation 3.13 de la demande internationale WO 97/25315.

Préparation 1.13

Acide (R) 3-(N-Boc)amino-3-(benzo[1.3]dioxol-5-yl)propionique.

(II) (R) : X = H ; $R_2 = H$ ; $R_3$ = 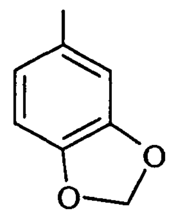

A) Acide (R) 3-(phénylacétyl)amino-3-(benzo[1,3]dioxol-5-yl)propionique.

On place 20 g de chlorhydrate d'acide 3-amino-3-(benzo[1,3]dioxol-5-yl) propionique dans 10 ml d'acétone, 30 ml d'eau et 38 ml de TEA à -5°C et on ajoute goutte à goutte 14 ml de chlorure de phénylacétyle dans 20 ml d'acétone puis on agite 2 heures à -5°C. On concentre l'acétone. La phase aqueuse est lavée par $Et_2O$ et l'insoluble est éliminé par filtration. On décante à nouveau, la phase aqueuse est lavée par $Et_2O$ puis acidifiée par HCl 1N jusqu'à pH = 2. On jette dans DCM et il se forme un précipité.On essore le précipité et rince au DCM. On obtient 24 g du composé attendu.

B) Acide (R) 3-amino-3-(benzo[1,3]dioxol-5-yl)propionique, HCl.

4 g du composé de l'étape précédente sont placés dans 150 ml d'eau, on ajoute 12,23 ml de KOH 1N et on laisse sous agitation 15 minutes à TA. Le pH de la solution est environ 11 ; on ajoute AcOH et on ajuste à pH = 7,5 ± 0,1. On ajoute 250 μl de pénicilline amidase et on laisse une nuit sous agitation à TA, en maintenant à pH = 7,5 ± 0,1. On acidifie à pH = 2 par addition d'HCl 1N, puis on lave par AcOEt. La phase aqueuse est chauffée à 65°C avec du charbon végétal actif pendant 5 minutes. On filtre sur Célite®, lave la phase aqueuse par $Et_2O$ puis concentre à sec et évapore. On reprend par un mélange MeOH/DCM (6/4 ; v/v) puis on élimine l'insoluble (minéral), sèche sur sulfate de sodium et concentre. On obtient 1,75 g du composé attendu.
RMN : δ (ppm) : 2,7-3,1 : mt : 2H ; 4,4 : m : 1H ; 6,0 : s : 2H ; 6,9 : qd : 2H; 7,1 : s : 1H.

C) Acide (R) 3-(N-Boc)amino-3-(benzo[1.3]dioxol-5-yl)propionique.

On place 1,30 g du composé obtenu à l'étape précédente dans 20 ml de dioxane et 20 ml d'eau ; on ajoute 1,8 ml de TEA puis 1,54 g de $(Boc)_2O$ et on laisse une nuit sous agitation à TA. On dilue par de l'eau, lave par $Et_2O$ puis acidifie à pH = 2 par addition de $KHS_4/K_2SO_4$. On extrait par AcOEt puis lave par une solution saturée de NaCl. On obtient 0,850 g du composé attendu.

$$\alpha_D^{25} = +54° \text{ (c = 0,5 ; MeOH)}$$

En opérant selon les méthodes décrites ci-dessus, on prépare les composés de formule (II) décrits dans le tableau I ci-après :

TABLEAU III

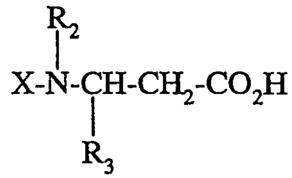 (II)

| Préparation | X | $R_2$ | $R_3$ | F°C |
|---|---|---|---|---|
| 1.14 | 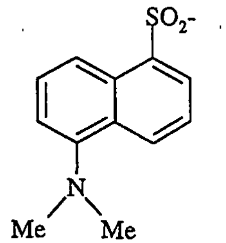 | H | 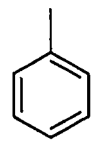 | |
| 1.15 | 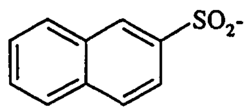 | H | 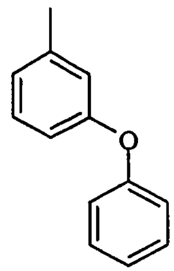 | |
| 1.16 | 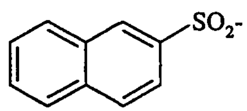 | H | 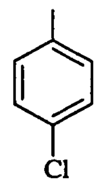 | |

(suite)

| Préparation | X | $R_2$ | $R_3$ | F°C |
|---|---|---|---|---|
| 1.17 | 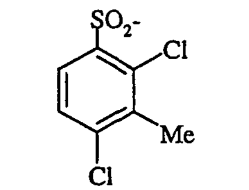 | 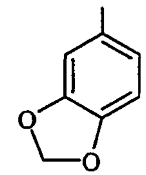 | H | 174°C |
| 1.18 | 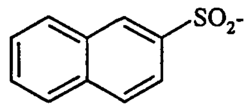 | H | 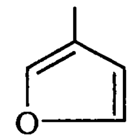 | 139-142°C |
| 1.19 | 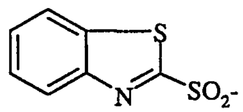 | H | 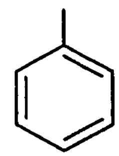 | 148°C |
| 1.20 | 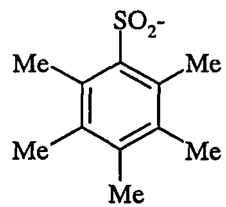 | 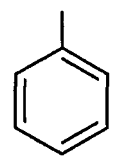 | H | 120°C |
| 1.21 | 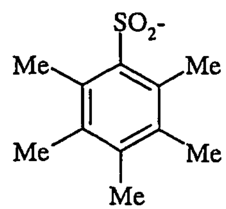 | H | 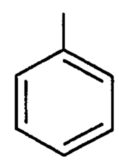 | 185°C |

(suite)

| Préparation | X | $R_2$ | $R_3$ | F°C |
| --- | --- | --- | --- | --- |
| 1.22 | 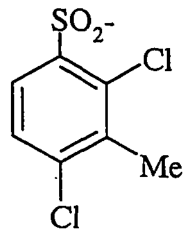 | 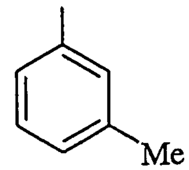 | H | 159°C |
| 1.23 | 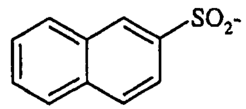 | H | 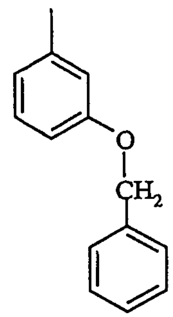 | 68°C |
| 1.24 | 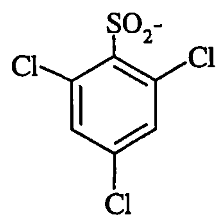 | 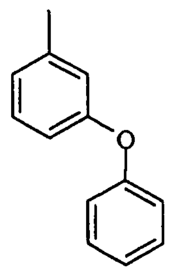 | H | huile |
| 1.25<br>TFA | 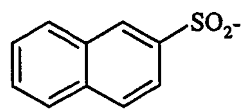 | 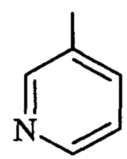 | H | 137°C |

(suite)

| Préparation | X | $R_2$ | $R_3$ | F°C |
|---|---|---|---|---|
| 1.26 TFA | 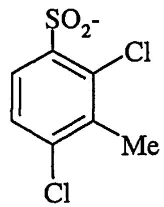 | 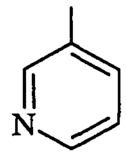 | H | 184°C |
| 1.27 TFA | 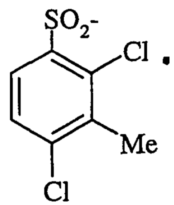 | 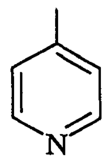 | H | 163°C |
| 1.28 | 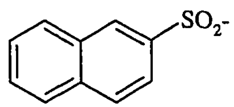 | 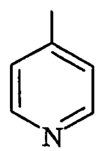 | H | 180°C |
| 1.29 TFA | 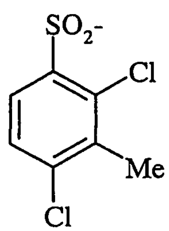 | 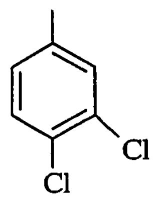 | H | 154°C |
| 1.30 | 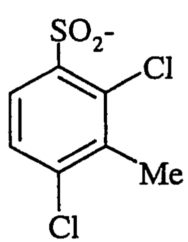 | 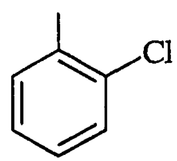 | H | 160°C |

(suite)

| Préparation | X | $R_2$ | $R_3$ | F°C |
|---|---|---|---|---|
| 1.31 | 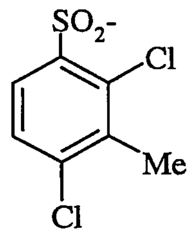 | 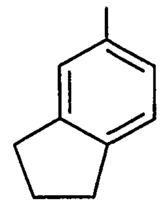 | H | 193°C |

Préparation 1.14 : RMN : 2,2 ppm : mt : 2H ; 2,8 ppm : s : 6H ; 4,5 ppm : t : 1H ; 6,9 ppm : mt : 6H ; 7,2 ppm : d : 1H ; 7,4 ppm : t : 1H ; 7,55 ppm : t : 1H ; 7,9 ppm : d : 1H ; 8,3 ppm : t : 1H.
Préparation 1.15 : RMN ; 2,55 ppm : mt : 2H ; 4,65 ppm : mt : 1H ; 6,4-8,0 ppm : mt : 15 H ; 8,1 ppm : s : 1H ; 8,4 ppm : d : 1H.
Préparation 1.16 : RMN ; 2,5-2,7 ppm : mt : 2H ; 4,7 ppm : qd : 1H ; 7,0-8,1 ppm : mt : 11H ; 8,5 ppm : d : 1H.
Préparation 1.17 : RMN (DMSO + TFA) : 2,4 ppm : t : 2H ; 2,55 ppm : s : 3H ; 4,0 ppm : t : 2H ; 6,05 ppm : s : 2H ; 6,6 ppm : dd : 1H ; 6,8 ppm : mt : 2H ; 7,55 ppm : d : 1H ; 7,65 ppm : d : 1H.
Préparation 1.18 : RMN (DMSO + TFA) : 2,40-2,65 ppm : mt : 2H ; 4,60-4,75 ppm : t : 1H ; 6,20 ppm : s : 1H ; 7,10-8,40 ppm : m : 9H.
Préparation 1.25 : RMN (250 MHz) : 2,35-2,45 ppm : t : 2H ; 3,80-3,90 ppm : t : 2H ; 7,30-8,60 ppm : mt : 11H.
Préparation 1.26 : RMN (250 MHz) : 2,35-2,45 ppm : t : 2H ; 2,50 ppm : s : 3H ; 3,95-4,05 ppm : t : 2H ; 7,35-8,50 ppm : mt : 6H.
Préparation 1.27 : RMN (250 MHz) : 2,40 ppm : s : 3H ; 2,50-2,60 ppm : t : 2H ; 4,15-4,25 ppm : t : 2H ; 7,55-8,65 ppm : mt : 6H.
Préparation 1.28 : RMN: 2,55-2,75 ppm : t : 2H ; 4,15-4,45 ppm : t : 2H ; 7,55-8,75 ppm : mt : 11H.
Préparation 1.29 : RMN (DMSO + TFA) : 2,4 ppm : t : 2H ; 2,5 ppm : mt : 5H ; 4,0 ppm : t : 2H ; 7,25 ppm : dd : 1H ; 7,7 ppm : mt : 4H.
Préparation 1.30 : RMN : 2,4 ppm : t : 2H ; 2,5 ppm : mt : 5H ; 4,0 ppm : m : 2H ; 7,3-7,6 ppm : mt : 6H.
Préparation 1.31 : RMN (DMSO + TFA) : 1,9 ppm : qt : 2H ; 2,25 ppm : t : 2H ; 2,4 ppm : mt : 4H ; 2,85 ppm : t : 4H ; 3,85 ppm : t : 2H ; 6,8 : dd : 1H ; 7,0 ppm : m : 2H ; 7,5 ppm : qd : 2H.
Préparation 1.32

(II) : X = 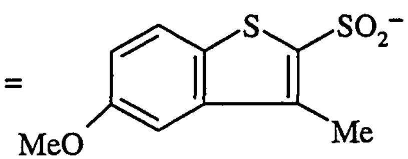 ; $R_2$ = H ; $R_3$ = 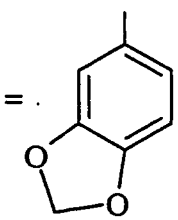

A) S-(4-méthoxy)phényléthanethioate.

On dissout 15 g de 4-méthoxybenzènethiol dans 20 ml d'eau contenant 4,3 g de NaOH et on laisse sous agitation 20 minutes à TA. On ajoute goutte à goutte 8,5 ml de 1-chloroacétone et on laisse 1 heure sous agitation à TA. On extrait à l'éther (2 fois), sèche et évapore pour obtenir 20 g du composé attendu.

B) 5-méthoxy-3-méthylbenzothiophène.

On mélange 10 g du composé de l'étape précédente et 20 ml d'acide polyphosphorique dans 400 ml de chlorobenzène et on laisse sous agitation 1 heure à TA puis on chauffe pendant 18 heures à 120°C. On laisse refroidir puis on décante. On reprend l'huile résiduelle par DCM (2 fois) puis on décante à nouveau.

Les phases surnageantes sont rassemblées puis évaporées. On reprend par DCM, puis on lave à l'eau puis par une solution de $NaHCO_3$. Après évaporation du solvant, on chromatographie sur silice en éluant par un mélange hexane/AcOEt (98/2 ; v/v) pour obtenir 5,2 g du composé attendu.

C) ((5-méthoxy-3-méthyl-1-benzothiophèn-2-yl)sulfinate de lithium.

On dissout 1,69 g du composé de l'étape précédente dans 10 ml de THF et on refroidit à -20°C. On ajoute 6,6 ml de butyllithium (1,8M dans hexane) en 15 minutes puis, lentement, $SO_2$ en quantité suffisante pour saturer le milieu. Après 20 minutes sous agitation à -20°C, on laisse revenir à TA. On ajoute 50 ml d'$Et_2O$ puis on essore et sèche pour obtenir 2,4 g du composé attendu.

D) Chlorure de 5-méthoxy-3-méthyl-1-benzothiophèn-2-sulfonyle.

On met en suspension 2,4 g du composé de l'étape précédente dans 20 ml de $CH_2Cl_2$. On refroidit à 5°C puis on ajoute peu à peu 1,26 g de N-chlorosuccinimide et on laisse une heure sous agitation à 5°C. On lave à l'eau et on reextrait l'eau par $CH_2Cl_2$. On joint les phases organiques, lave par une solution de NaCl, sèche et évapore pour obtenir 1,8 g du composé attendu.
RMN : 2,50 ppm : s : 3H ; 3,85 ppm : s : 3H ; 7,0-7,80 ppm : mt : 3H.

E) On procède ensuite selon les méthodes habituelles pour obtenir le composé attendu.

Préparation 1.33

(II) : X = 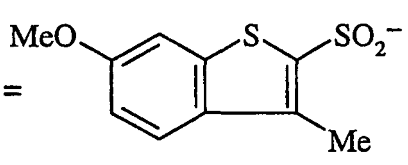 ; $R_2$ = H ; $R_3$ = 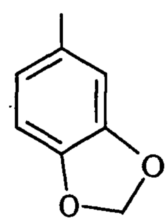

A) Chlorure de 6-méthoxy-3-méthyl-1-benzothiophène-2-sulfonyle.

Ce composé est préparé selon le procédé décrit à la préparation ci-dessus.
RMN : 2,50 ppm : s : 3H ; 3,85 ppm : s : 3H ; 7,0-7,70 ppm : mt : 3H. B)

On procédé ensuite selon les méthodes habituelles pour obtenir le composé attendu.

D'autres composés de formule (II) préparés selon les méthodes connues de la littérature ou décrites ci-dessus ont été préparés sous forme racémique ou sous forme d'isomères purs.

TABLEAU IV

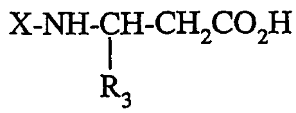 (II)

| Préparation (isomère) | X | R3 | F°C ou RMN |
|---|---|---|---|
| 1.34 | Boc | 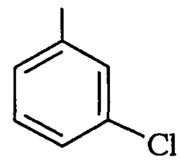 | 114°C |
| 1.35 | 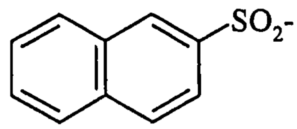 | 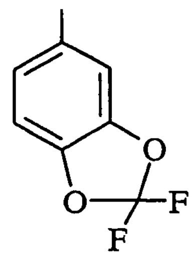 | 159°C |
| 1.36 | 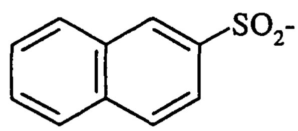 | 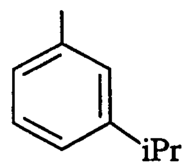 | RMN |
| 1.37 | H 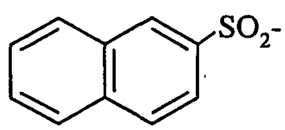 | 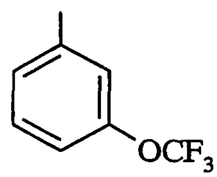 | 248°C<br>145°C |
| 1.38 (R) | Boc | 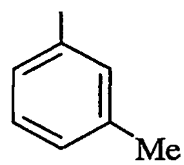 | 120°C |
| 1.39 | H<br>Boc | 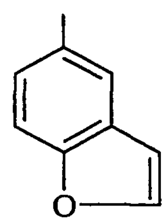 | RMN<br>167°C |
| 1.40 (R) | H | 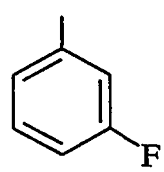 | RMN |

(suite)

| Préparation (isomère) | X | $R_3$ | F°C ou RMN |
|---|---|---|---|
| 1.41 | H<br>Boc | 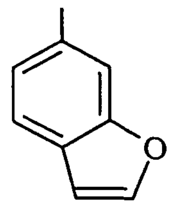 | 242°C<br>190°C |
| 1.42 | H<br>Boc | 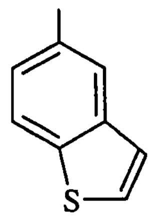 | 260°C<br>198°C |
| 1.43 | H<br>Boc | 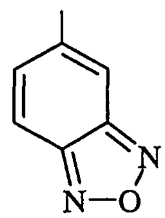 | 228°C<br>RMN |
| 1.44 | H<br>Boc | 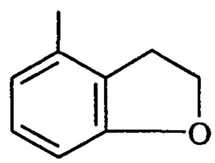 | 204°C<br>125°C |
| 1.45<br>(a) | H<br>Boc | 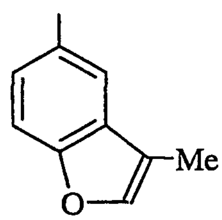 | RMN<br>RMN |
| (a) ce composé est préparé à partir du 1-benzofurancarbonitrile décrit dans la demande de brevet européenne 540 041. | | | |

Préparation 2.1

Trifluoroacétate de 2-amino-3-(4-cyanophényl)-1-(pyrrolidin-1-yl)propan-1-one.

(III), TFA : Y = $R_4$ = $CONR_8R_9$ = -CO-N 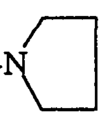 ; Z = -CN.

A un mélange de 4,06 g d'acide 2-(*tert*-butoxycarbonylamino)-3-(4-cyanophényl) propionique et 1,15 ml de pyrrolidine dans 20 ml de DMF, on ajoute 7,5 g de BOP et laisse 2 heures sous agitation à TA, en maintenant le pH = 7 par ajout de N-éthylmorpholine. On concentre sous vide le mélange réactionnel et chromatographie le résidu sur gel

de silice en éluant par le mélange chloroforme/MeOH (95/5 ; v/v). On reprend le produit obtenu par 20 ml de TFA dans 20 ml de DCM, laisse 30 minutes sous agitation à TA et concentre sous vide le mélange réactionnel. On reprend le résidu à l'éther et essore le précipité formé. On obtient 3,95 g du produit attendu, après séchage.

Préparation 2.2

Ditrifluoroacétate de 2-amino-3-[4-(*tert*-butylaminométhyl)phényl]-1-(pyrrolidin-1-yl) propan-1-one.

(III), 2TFA : Y = -CO- 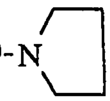 ; Z = $R_5$ = $-CH_2-NR_{11}R_{12}$ = $-CH_2-NH-tBu$

A) Ester éthylique de l'acide 2-(benzhydrylidèneamino)-3-(4-bromométhyl phényl)propionique.

On prépare ce composé selon le mode opératoire décrit dans Tetrahedron : Asymmetry, 1992, 3 (5), 637-650.

B) Ester éthylique de l'acide 2-(benzhydrylidèneamino)-3-[4-(*tert*-butylamino méthyl)phényl]propionique.

A une solution de 0,58 ml de *tert*-butylamine dans 8 ml de DMF, on ajoute 0,76 g de $K_2CO_3$ et laisse 20 minutes sous agitation à TA. Puis on ajoute en 3 minutes une solution de 2,15 g du composé obtenu à l'étape précédente dans 3 ml de DMF et laisse 3 heures 30 minutes sous agitation à TA. On extrait le mélange réactionnel à l'AcOEt, lave la phase organique à l'eau, par une solution saturée de NaCl, sèche sur $Na_2SO_4$ et évapore sous vide le solvant. On obtient 2,67 g du produit attendu sous forme d'huile.

C) Ester éthylique de l'acide 2-amino-3-[4-(*tert*-butylaminométhyl)phényl] propionique.

On laisse une nuit sous agitation à TA un mélange de 2,66 g du composé obtenu à l'étape précédente et 30 ml d'une solution aqueuse d'HCl 1N dans 30 ml d'éther. Après décantation, on ajoute de l'AcOEt à la phase aqueuse acide et alcalinise à pH= 11 par ajout de NaOH 10N. Après décantation, on lave la phase organique à l'eau par une solution saturée de NaCl, sèche sur $Na_2SO_4$ et évapore sous vide le solvant. On obtient 0,88 g du produit attendu sous forme d'huile.

D) Ester éthylique de l'acide 2-(*tert*-butoxycarbonylamino)-3-[4-(*tert*-butylamino méthyl)phényl]propionique.

A une solution de 0,88 g du composé obtenu à l'étape précédente dans 12 ml de dioxane, on ajoute par portions 0,7 g de di-*tert*-butyldicarbonate et laisse 1 heure 30 minutes sous agitation à TA. On extrait le mélange réactionnel à l'AcOEt, lave la phase organique à l'eau, par une solution saturée de NaCl, sèche sur $Na_2SO_4$ et évapore sous vide le solvant. On obtient 1,34 g du produit attendu sous forme d'huile.

E) Acide 2-(*tert*-butoxycarbonylamino)-3-(4-(*tert*-butylaminométhyl)phényl] propionique.

On laisse 2 heures sous agitation à TA un mélange de 1,33 g du composé obtenu à l'étape précédente et 0,75 ml de KOH 8,3N dans 8 ml de MeOH. On ajoute au mélange réactionnel un mélange chloroforme/eau et acidifie à pH = 4 par ajout d'HCl 10N. Après décantation, on lave la phase organique à l'eau, par une solution saturée de NaCl, sèche sur $Na_2SO_4$ et évapore sous vide le solvant pour obtenir un premier jet de 0,54 g du produit attendu. On concentre sous vide les phases aqueuses et les eaux de lavage, reprend le résidu dans du propan-2-ol, filtre l'insoluble et concentre sous vide le filtrat pour obtenir un deuxième jet de 0,51 g du produit attendu.

F) 2-(*tert*-Butoxycarbonylamino)-3-[4-(*tert*-butylaminométhyl)phényl]-1-(pyrrolidin-1-yl)propan-1-one.

A un mélange de 1,03 g du composé obtenu à l'étape précédente dans 15 ml de DMF, on ajoute 0,6 ml de DIPEA, puis 0,28 ml de pyrrolidine et 1,46 g de BOP et laisse 4 heures sous agitation à TA en maintenant le pH = 6 par ajout de DIPEA. On extrait le mélange réactionnel à l'AcOEt, lave la phase organique à l'eau, par NaOH 0,2N, à l'eau, par une solution saturée de NaCl, sèche sur $Na_2SO_4$ et évapore sous vide le solvant. On obtient 1,3 g du produit attendu sous forme d'huile.

G) Ditrifluoroacétate de 2-amino-3-[4-(*tert*-butylaminométhyl) phényl]-1-(pyrrolidin-1-yl)propan-1-one.

On laisse 50 minutes sous agitation à TA un mélange de 1,29 g du composé obtenu à l'étape précédente et 17 ml de TFA dans 15 ml de DCM. Après concentration sous vide du mélange réactionnel, on reprend le résidu au DCM et évapore sous vide le solvant. On reprend le résidu à l'éther, puis décante le solvant. On obtient 1,5 g du produit attendu sous forme de mousses après séchage que l'on utilise tel quel à l'exemple 2.

Préparation 2.3

Dichlorhydrate de 2-amino-3-[4-(N-propyl-N-méthylaminométhyl)phényl]-1-(pyrrolidin-1-yl)propan-1-one.

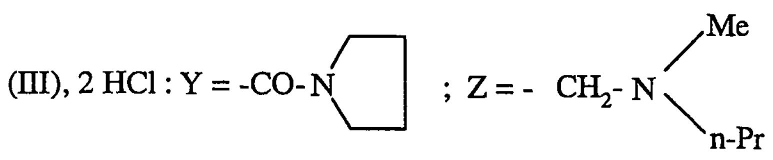

Ce composé est préparé selon le Schéma 9.

A) 2-(*tert*-Butoxycarbonylamino)-3-(4-cyanophényl)-1-(pyrrolidin-1-yl)propan-1-one.

A un mélange de 4,06 g d'acide 2-(*tert*-Butoxycarbonylamino)-3-(4-cyanophényl)propionique et 1,15 ml de pyrrolidine dans 20 ml de DMF, on ajoute 7,5 g de BOP et laisse 2 heures sous agitation à TA, en maintenant le pH = 7 par ajout de N-éthylmorpholine. On concentre sous vide le mélange réactionnel et chromatographie le résidu sur gel de silice en éluant par le mélange chloroforme/MeOH (95/5 ; v/v). On obtient 3,8 g du produit attendu.

B) 2-(*tert*-Butoxycarbonylamino)-3-(4-formylphényl)-1-(pyrrolidin-1-yl)propan-1-one.

On refroidit à 0° C une solution de 2 g du composé obtenu à l'étape précédente dans 150 ml d'un mélange pyridine/AcOH/$H_2O$ (2/1/1 ; v/v/v), ajoute sous atmosphère d'argon 10,57 g d'hypophosphite de sodium hydrate puis 1,8 g de nickel de Raney® dans l'eau et laisse 10 minutes sous agitation à TA. On chauffe à 50° C pendant 3 heures le mélange réactionnel. Après refroidissement à TA, on filtre sur Célite®, lave à l'EtOH puis au DCM et concentre sous vide le filtrat. On extrait le résidu à l'AcOEt, lave la phase organique à l'eau, par une solution à 5 % de $KHSO_4$, par une solution saturée de NaCl, par une solution saturée de $NaHCO_3$, par une solution saturée de NaCl, sèche sur $Na_2SO_4$ et évapore sous vide le solvant. On chromatographie le résidu sur gel de silice en éluant à l'AcOEt. On obtient 1,7 g du produit attendu, F = 134°C.

C) 2-(*tert*-Butoxycarbonylamino)-3-[4-(N-propyl-N-méthylamino méthyl)phényl]-1-(pyrrolidin-1-yl)propan-1-one.

A un mélange de 0,77 g du composé obtenu à l'étape précédente et 0,34 ml de N-méthylpropylamine dans 10 ml de 1,2-dichloroéthane, on ajoute 0,42 ml d'AcOH puis 0,707 g de triacétoxyborohydrure de sodium et laisse une nuit sous agitation à TA. On dilue le mélange réactionnel au DCM, lave la phase organique par une solution saturée de $NaHCO_3$, par une solution saturée de NaCl, sèche sur $Na_2SO_4$ et évapore sous vide le solvant. On obtient 0,9 g du produit attendu.

D) Dichlorhydrate de 2-amino-3-[4-(N-propyl-N-méthylaminométhyl)phényl]-1-(pyrrolidin-1-yl)propan-1-one.

A un mélange de 0,88 g du composé obtenu à l'étape précédente dans 10 ml de MeOH et 5 ml de dioxane, on ajoute 5 ml d'HCl 10N et laisse 4 heures sous agitation à TA. On ajoute de l'EtOH et concentre sous vide le mélange réactionnel. On reprend le résidu à l'éther et essore le précipité formé après trituration. On obtient 0,83 g du produit attendu, F = 140° C (déc.).

Préparation 2.4

Ditrifluoroacétate de 2-amino-3-[4-[N,N-bis(2-hydroxyéthyl)aminométhyl] phényl]-1-(pyrrolidin-1-yl)propan-1-one.

(III), 2TFA : Y = -CO- 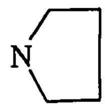 ; Z = $-CH_2-N(CH_2CH_2OH)_2$

A) Ester éthylique de l'acide 2-(benzhydrylidèneamino)-3-[4-[N,N-bis(2-hydroxy éthyl)aminométhyl]phényl]propionique.

On prépare ce composé selon le mode opératoire décrit à l'étape B de la préparation 2.2 à partir de 0,925 g de diéthanolamine dans 15 ml de DMF, 1,21 g de $K_2CO_3$ et 3,6 g du composé obtenu à l'étape A) de la préparation 2.2 dans 20 ml de DMF. On obtient 4,23 g du produit attendu sous forme d'huile.

B) Ester éthylique de l'acide 2-amino-3-[4-[N,N-bis(2-hydroxyéthyl)amino méthyl]phényl]propionique.

On prépare ce composé selon le mode opératoire décrit à l'étape C) de la préparation 2.2 à partir de 4,22 g du composé obtenu à l'étape précédente et 70 ml d'HCl 1N dans 70 ml d'éther. On obtient 1,87 g du produit attendu sous forme de cire.

C) Ester éthylique de l'acide 2-(*tert*-Butoxycarbonylamino)-3-[4-[N,N-bis(2-hydroxyéthyl)aminométhyl]phényl]propionique.

On prépare ce composé selon le mode opératoire décrit à l'étape D de la préparation 2.2 à partir de 1,87 g du composé obtenu à l'étape précédente dans 20 ml de dioxane et 1,4 g de di-*tert*-butyldicarbonate. On obtient 2,15 g du produit attendu.

D) Acide 2-(*tert*-butoxycarbonylamino)-3-[4-[N,N-bis(2-hydroxyéthyl)amino méthyl]phényl]propionique.

On laisse 3 heures sous agitation à TA un mélange de 2,13 g du composé obtenu à l'étape précédente et 1,3 ml de KOH 8,3 N dans 15 ml de MeOH. Puis on rajoute 0,3 ml de KOH 8,3 N et laisse 1 heure sous agitation à TA. On ajoute au mélange réactionnel un mélange AcOEt/$H_2O$ et acidifie à pH = 3,7 par ajout d'HCl 1N. Après décantation, on lave la phase organique à l'eau, par une solution saturée de NaCl, sèche sur $Na_2SO_4$ et évapore sous vide le solvant pour obtenir un premier jet du produit attendu. On concentre sous vide la phase aqueuse, reprend le résidu dans un mélange MeOH/propan-2-ol (1/1 ; v/v), filtre l'insoluble et concentre sous vide le filtrat pour obtenir un deuxième jet. On obtient 1,66 g du produit attendu sous forme d'un solide blanc.

E) 2-(*tert*-Butoxycarbonylamino)-3-[4-[N,N-bis(2-hydroxyéthyl)aminométhyl] phényl]-1-(pyrrolidin-1-yl)propan-1-one.

On prépare ce composé selon le mode opératoire décrit à l'étape F de la préparation 2.2 à partir de 0,95 g du composé obtenu à l'étape précédente dans 12 ml de DMF, 0,6 ml de DIPEA, 0,24 ml de pyrrolidine et 1,44 g de BOP. On obtient le produit attendu sous forme de cire que l'on utilise tel quel à l'étape suivante.

F) Ditrifluoroacétate de 2-amino-3-[4-[N,N-bis(2-hydroxyéthyl)aminométhyl] phényl]-1-(pyrrolidin-1-yl)propan-1-one.

On prépare ce composé selon le mode opératoire décrit à l'étape G) de la préparation 2.2 à partir du composé obtenu à l'étape précédente et 18 ml de TFA dans 15 ml de DCM. On obtient le produit attendu que l'on utilise tel quel à l'exemple 4.

Préparation 2.5

Tritrifluoroacétate de 1,2-bis[4-(diéthylaminométhyl)phényl]éthylamine.

(III), 3TFA : Y = $R_4$ = 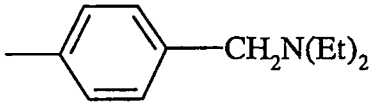 ; Z = $-CH_2N(Et)_2$

A) 4-(Azidométhyl)benzoate de méthyle.

A une solution de 5,73 g de 4-(bromométhyl)benzoate de méthyle dans 30 ml de DMSO, on ajoute en 5 minutes 8,13 g d'azidure de sodium et laisse 3 heures 30 minutes sous agitation à TA. On extrait le mélange réactionnel à l'éther, lave la phase organique à l'eau, par une solution saturée de NaCl, sèche sur $Na_2SO_4$ et évapore sous vide le solvant. On obtient 4,72 g du produit attendu sous forme d'huile incolore.

B) Chlorhydrate de 4-(aminométhyl)benzoate de méthyle.

On refroidit à 4° C une solution de 4,71 g du composé obtenu à l'étape précédente dans 30 ml de THF, ajoute en 30 minutes et par portions 6,57 g de triphénylphosphine et laisse 6 heures sous agitation en laissant remonter la température à TA. Puis on ajoute 0,68 ml d'eau et laisse 16 heures sous agitation à TA. On extrait le mélange réactionnel à l'AcOEt, lave la phase organique à l'eau, par une solution saturée de NaCl, sèche la phase organique sur $Na_2SO_4$ et évapore sous vide les solvants. On reprend le résidu à l'éther, filtre un insoluble, concentre sous vide le filtrat et chromatographie le résidu sur gel de silice en éluant par le mélange chloroforme/MeOH/$NH_4OH$ (90/10/0,2 ; v/v/v). On reprend le produit obtenu dans du MeOH, ajoute HCl 10N jusqu'à pH = 1 et concentre sous vide. On obtient 4,25 g du produit attendu.

C) 4-(Benzhydrylidèneaminométhyl)benzoate de méthyle.

On laisse une nuit sous agitation à TA un mélange de 3,03 g du composé obtenu à l'étape précédente, 2,07 ml de triéthylamine et 2,51 ml de benzophénone imine dans 50 ml de DCM. On extrait le mélange réactionnel au DCM, lave la phase organique à l'eau, par une solution saturée de NaCl, sèche sur $Na_2SO_4$ et évapore sous vide le solvant. On obtient 5,33 g du produit attendu sous forme d'huile

D) 4-[1-amino-2-(4-méthoxycarbonylphényl)éthyl]benzoate de méthyle.

On refroidit à -50° C une solution de 5,32 g du composé obtenu à l'étape précédente dans 40 ml de THF, ajoute en 35 minutes 10,3 ml d'une solution 1,6 M de n-butyllithium dans l'hexane et laisse 30 minutes sous agitation entre -50° C et -30° C. On refroidit à nouveau à -50° C le mélange réactionnel, ajoute en 30 minutes une solution de 3,78 g de 4-(bromométhyl)benzoate de méthyle dans 25 ml de THF et laisse 4 heures sous agitation après que la température soit revenue à TA. Puis on ajoute au mélange réactionnel 50 ml d'HCl 1N et laisse 16 heures sous agitation à TA. On décante le mélange réactionnel, lave la phase acide à l'éther, alcalinise la phase aqueuse acide à pH = 10 par ajout de NaOH 10N, extrait à l'AcOEt, lave la phase organique à l'eau, par une solution saturée de NaCl, sèche sur $Na_2SO_4$ et évapore sous vide le solvant. On obtient 3,88 g du produit attendu sous forme d'un solide blanc pâteux.

E) 4-[1-(*tert*-Butoxycarbonylamino)-2-(4-méthoxycarbonylphényl)éthyl]benzoate de méthyle.

A une solution de 3,88 g du composé obtenu à l'étape précédente dans 30 ml de dioxane, on ajoute en 10 minutes 2,94 g de di-*tert*-butyldicarbonate et laisse 3 heures sous agitation à TA. On extrait le mélange réactionnel à l'AcOEt, lave la phase organique à l'eau, par une solution tampon $KHSO_4/K_2SO_4$ à l'eau, par une solution saturée de NaCl, sèche sur $Na_2SO_4$ et évapore sous vide le solvant. On obtient 2,3 g du produit attendu après cristallisation dans l'éther.

F) N-*tert*-Butoxycarbonyl-1,2-bis[4-(hydroxyméthyl)phényl]éthylamine.

A une suspension de 0,456 g d'hydrure d'aluminium et de lithium dans 25 ml de THF, on ajoute en 40 minutes une solution de 1,24 g du composé obtenu à l'étape précédente dans 15 ml de THF et laisse 2 heures sous agitation à TA. On ajoute ensuite au mélange réactionnel un mélange AcOEt/glace, extrait à l'AcOEt, lave la phase organique à l'eau par une solution saturée de NaCl, sèche sur $Na_2SO_4$ et évapore sous vide le solvant. on obtient 0,95 g du produit attendu sous forme d'un solide blanc.

G) Méthanesulfonate de 4-[2-(*tert*-butoxycarbonylamino)-2-(4-méthanesulfonyl oxyméthylphényl)éthyl]benzyle.

On refroidit à 4° C une solution du composé obtenu à l'étape précédente dans 20 ml de DCM et 3 ml de THF, ajoute 0,82 ml de triéthylamine puis en 10 minutes une solution de 0,45 ml de chlorure de méthanesulfonyle dans 1 ml de DCM et laisse 3 heures 30 minutes sous agitation à TA. On extrait le mélange réactionnel au DCM, lave la phase organique à l'eau, par une solution saturée de NaCl, sèche sur $Na_2SO_4$ et évapore sous vide le solvant. On obtient 1,21 g du produit attendu.

H) N-(*tert*-Butoxycarbonyl)-1,2-bis[4-(diéthylaminométhyl)phényl]éthylamine.

On chauffe à 60° C pendant 4 heures un mélange de 1,2 g du composé obtenu à l'étape précédente et 1,05 ml de diéthylamine dans 20 ml d'EtOH. Après refroidissement à TA, on extrait le mélange réactionnel à l'AcOEt, lave la phase organique à l'eau, par une solution saturée de $NaHCO_3$, à l'eau, par une solution saturée de NaCl, sèche sur $Na_2SO_4$ et évapore sous vide le solvant. On chromatographie le résidu sur gel de silice en éluant par le mélange chloroforme/MeOH/$NH_4OH$ (85/15/0,2 ; v/v/v). On obtient 0,51 g du produit attendu.

I) Tritrifluoroacétate de 1,2-bis[4-(diéthylaminométhyl)phényl]éthylamine.

On laisse 45 minutes sous agitation à TA un mélange de 0,5 g du composé obtenu à l'étape précédente et 12 ml de TFA dans 10 ml de DCM. On concentre sous vide le mélange réactionnel, reprend le résidu au DCM et concentre à nouveau sous vide. On obtient 0,82 g du produit attendu sous forme d'huile.

Préparation 2.6

Dichlorhydrate de 2-amino-3-[4-(pipérid-1-ylméthyl)phényl]-1-(pyrrolidin-1-yl) propan-1-one

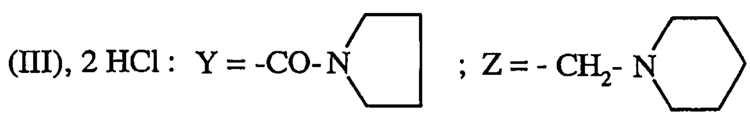

A) 2-(*tert*-Butoxycarbonylamino)-3-[4-(pipérid-1-ylméthyl)phényl]-1-(pyrrolidin-1-yl)propan-1-one

On prépare ce composé selon le mode opératoire décrit à l'étape C) de la préparation 2.3 à partir de 0,84 g du composé obtenu à l'étape B) de la préparation 2.3, 0,263 ml de pipéridine, 10 ml de 1,2-dichloroéthane, 0,452 ml d'AcOH et 0,771 g de triacétoxyborohydrure de sodium. On dilue le mélange réactionnel au DCM, lave la phase organique par une solution saturée de $NaHCO_3$, par une solution saturée de NaCl, sèche sur $Na_2SO_4$ et évapore sous vide le solvant On reprend le produit obtenu dans l'éther, filtre un insoluble et concentre sous vide le filtrat. On obtient 0,975 g du produit attendu sous forme d'huile.

B) Dichlorhydrate de 2-amino-3-[4-(pipérid-1-ylméthyl)phényl]-1-(pyrrolidin-1-yl)propan-1-one

On prépare ce composé selon le mode opératoire décrit à l'étape D) de la préparation 2.3 à partir de 0,97 g du composé obtenu à l'étape précédente, 10 ml de MeOH, 5 ml de dioxane et 5 ml d'HCl 10N. On obtient 0,91 g du produit attendu, F = 170° C (déc.)
RMN : 1,2-2,0 ppm : m : 10H ; 2,4-3,4 ppm : m : 10H ; 4,2 ppm : s.e : 3H ; 7,2 ppm : d : 2H ; 7,6 ppm : d : 2H ; 8,4 ppm : s : 3H ; 11,2 ppm: s : 1H.

Préparation 2.7

Dichlorhydrate de 2-amino-3-[4-(diéthylaminométhyl)phényl]-1-(pyrrolidin-1-yl) propan-1-one

(III), 2 HCl : Y = -CO-N 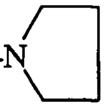 ; Z = -$CH_2$-N(Et)$_2$

Pour ses étapes de préparation D, E et F, ce composé est obtenu selon le Schéma 10.

A) Ester éthylique de l'acide 2-(benzhydrylidèneamino)-3-[4-(diéthylamino méthyl)phényl]propionique

On laisse 2 heures sous agitation à TA un mélange de 1 g du composé obtenu à l'étape A) de la préparation 2.2, 0,235 ml de diéthylamine et 0,307 g de $K_2CO_3$ dans 10 ml de DMF. On extrait le mélange réactionnel à l'AcOEt, lave la phase organique à l'eau, par une solution saturée de $NaHCO_3$, par une solution saturée de NaCl, sèche sur $Na_2SO_4$ et évapore sous vide le solvant. On obtient 0,927 g du produit attendu sous forme d'huile.

B) Ester éthylique de l'acide 2-amino-3-[4-(diéthylaminométhyl)phényl] propionique

On laisse 2 heures sous agitation à TA un mélange de 0,927 g du composé obtenu à l'étape précédente et 20 ml d'HCl 1N dans 30 ml d'éther. Après décantation, on lave la phase aqueuse acide à l'éther, alcalinise la phase aqueuse à pH = 11 par ajout de $NaHCO_3$ solide, extrait au DCM, lave la phase organique par une solution saturée de NaCl, sèche sur $Na_2SO_4$ et évapore sous vide le solvant. On obtient 0,395 g du produit attendu.

C) Ester éthylique de l'acide 2-(*tert*-butoxycarbonylamino)-3-[4-(diéthylamino méthyl)phényl]propionique

A une solution de 2 g du composé obtenu à l'étape précédente dans 20 ml de DCM, on ajoute 1,72 g de di-*tert*-butyldicarbonate puis 1,1 ml de triéthylamine et laisse 2 heures sous agitation à TA. On concentre sous vide le mélange réactionnel, extrait le résidu à l'AcOEt, lave la phase organique à l'eau, par une solution saturée de NaCl, sèche sur $Na_2SO_4$ et évapore sous vide le solvant. On obtient 2,6 g du produit attendu, F = 56° C.

D) Acide 2-(*tert*-butoxycarbonylamino)-3-[4-(diéthylaminométhyl)phényl] propionique

A une solution de 2,55 g du composé obtenu à l'étape précédente dans 60 ml d'EtOH et 20 ml de dioxane, on ajoute 10,1 ml de KOH 1N et chauffe 1 heure à 60° C. Après refroidissement à TA, on ajoute 10 ml d'HCl 1N et concentre sous vide le mélange réactionnel. On azéotrope le résidu par ajout du mélange EtOH/toluène puis concentration sous vide. On reprend le résidu par un mélange DCM/MeOH (9/1 ; v/v), filtre un insoluble et concentre sous vide le filtrat. On obtient 2,4 g du produit attendu.

E) 2-(*tert*-Butoxycarbonylamino)-3-[4-(diéthylaminométhyl)phényl]-1-(pyrrolidin-1-yl)propan-1-one

A un mélange de 1,53 g du composé obtenu à l'étape précédente et 0,365 ml de pyrrolidine dans 10 ml de DMF, on ajoute 0,61 ml de triéthylamine puis 2,12 g de BOP et laisse 2 heures sous agitation à TA. On verse le mélange réactionnel dans l'eau, extrait à l'AcOEt, lave la phase organique à l'eau, par une solution saturée de $NaHCO_3$, par une solution saturée de NaCl, sèche sur $Na_2SO_4$ et évapore sous vide le solvant. On obtient 1,5 g du produit attendu.

F) Dichlorhydrate de 2-amino-3-[4-(diéthylaminométhyl)phényl]-1-(pyrrolidin-1-yl)propan-1-one

A une solution de 1,5 g du composé obtenu à l'étape précédente dans 20 ml de dioxane et 10 ml de MeOH, on ajoute 6 ml d'HCl concentré et laisse une nuit sous agitation à TA. On concentre sous vide le mélange réactionnel, reprend le résidu à l'EtOH et concentre sous vide à nouveau. On reprend le résidu à l'éther et décante le solvant après trituration. On obtient 1,37 g du produit attendu sous forme de gomme.

Préparation 2.8

Ditrifluoroacétate de 2-amino-3-[4-(diéthylaminométhyl)phényl]-N-isopropyl-N-méthylpropionamide

(III), 2 TFA : Y = 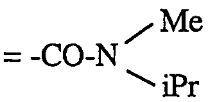 ; Z = $-CH_2-N(Et)_2$

A) 2-(*tert*-Butoxycarbonylamino)-3-[4-(diéthylaminométhyl)phényl]-N-isopropyl-N-méthylpropionamide

A un mélange de 0,7 g du composé obtenu à l'étape D) de la préparation 2.7 et 0,208 ml de N-méthylisopropylamine dans 10 ml de DMF, on ajoute 0,279 ml de triéthylamine puis 0,973 g de BOP et laisse 2 heures sous agitation à TA. On verse le mélange réactionnel dans l'eau, extrait à l'AcOEt, lave la phase organique à l'eau, par une solution saturée de $NaHCO_3$, par une solution saturée de NaCl, sèche sur $Na_2SO_4$ et évapore sous vide le solvant. On obtient 0,65 g du produit attendu.

B) Ditrifluoroacétate de 2-amino-3-[4-(diéthylaminométhyl)phényl]-N-isopropyl-N-méthylpropionamide

A une solution de 0,63 g du composé obtenu à l'étape précédente dans 5 ml de DCM, on ajoute 5 ml de TFA et laisse 2 heures sous agitation à TA. On concentre sous vide le mélange réactionnel, reprend le résidu dans l'éther iso et décante le solvant après trituration. On obtient le produit attendu après séchage sous vide.

Préparation 2.9

Dichlorhydrate de 2-amino-3-[4-(diéthylaminométhyl)phényl]-N,N-diisopropyl propionamide

(III), 2HCl : Y = $-CO-N(iPr)_2$ ; Z = $-CH_2-N(Et)_2$

A) 2-(*tert*-Butoxycarbonylamino)-3-(4-cyanophényl)-N,N-diisopropyl propionamide

On refroidit à 0° C un mélange de 1 g d'acide 2-(*tert*-butoxycarbonylamino)-3-(4-cyanophényl)propionique et 0,540 g de diisopropylamine dans 10 ml de DCM, ajoute 0,960 ml de triéthylamine puis 1,76 g d'hexafluorophosphate de bromotripyrrolidinophosphonium et laisse 2 heures sous agitation en laissant remonter la température à TA. On concentre sous vide le mélange réactionnel, extrait le résidu à l'AcOEt, lave la phase organique à l'eau, par une solution à 5 % de $KHSO_4$, à l'eau, par une solution saturée de $NaHCO_3$, par une solution saturée de NaCl, sèche sur $Na_2SO_4$ et évapore sous vide le solvant. On chromatographie le résidu sur gel de silice en éluant par le mélange heptane/AcOEt (80/20 ; v/v). On obtient 0,4 g du produit attendu, F = 172° C.

B)2-(*tert*-Butoxycarbonylamino)-3-(4-formylphényl)-N,N-diisopropyl propionamide

On prépare ce composé selon le mode opératoire décrit à l'étape B de la préparation 2.3 à partir de 0,380 g du composé obtenu à l'étape précédente, 25 ml du mélange pyridine/AcOH/$H_2O$ (2/1/1 ; v/v/v), 1,8 g d'hypophosphite de sodium hydrate et 0,31 g de nickel de Raney®. On obtient 0,35 g du produit attendu sans effectuer la chromatographie, F = 152° C.

C) 2-(*tert*-Butoxycarbonylamino)-3-[4-(diéthylaminométhyl)phényl)-N,N-diisopropylpropionamide

On prépare ce composé selon le mode opératoire décrit à l'étape C de la préparation 2.3 à partir de 0,333 g du composé obtenu à l'étape précédente, 0,135 ml de diéthylamine, 10 ml de 1,2-dichloroéthane, 0,08 ml d'AcOH et 0,281 g de triacétoxyborohydrure de sodium. On obtient 0,4 g du produit attendu.

D) Dichlorhydrate de 2-amino-3-[4-(diéthylaminométhyl)phényl]-N,N-diisopropylpropionamide

A un mélange de 0,4 g du composé obtenu à l'étape précédente dans 10 ml de MeOH et 5 ml de dioxane, on ajoute 5 ml d'HCl concentré et laisse une nuit sous agitation à TA. On concentre sous vide le mélange réactionnel, reprend le résidu à l'EtOH et évapore sous vide. On obtient 0,37 g du produit attendu.

Préparation 2.10

Tritrifluoroacétate de 2-[4-(diéthylaminométhyl)phényl]-1-(pyrid-3-yl)éthylamine.

(III), 3TFA : Y = 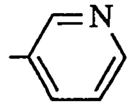 ; Z = $-CH_2N(Et)_2$

A) Benzhydrylidène-pyrid-3-ylméthylamine.

A une solution de 2,8 ml de benzophénone imine dans 50 ml de DCM, on ajoute 1,7 ml de 3-(aminométhyl) pyridine et laisse une nuit sous agitation à TA. On rajoute 0,28 ml de benzophénone imine et laisse 2 heures sous agitation à TA. On concentre sous vide le mélange réactionnel, extrait le résidu à l'éther, lave la phase organique à l'eau, par une solution saturée de NaCl, sèche sur $Na_2SO_4$ et évapore sous vide le solvant. On obtient 4,56 g du produit attendu.

B) 4-[2-amino-2-(pyrid-3-yl)éthyl]benzoate de méthyle.

On refroidit à -78°C, sous atmosphère d'argon, une solution de 3 g du composé obtenu à l'étape précédente dans 30 ml de THF, ajoute 8,2 ml d'une solution 1,5M de lithium diisopropylamide dans le cyclohexane et laisse 30 minutes sous agitation en laissant remonter la température à -20°C. On refroidit à nouveau à -78°C, ajoute goutte à goutte une solution de 2,82 g de 4-(bromométhyl)benzoate de méthyle dans 10 ml de THF et laisse 2 heures sous agitation en laissant remonter la température à TA. On refroidit le mélange réactionnel à 0°C, ajoute lentement 25 ml d'HCl 1N puis une solution d'HCl concentrée jusqu'à pH = 2-3, et laisse une nuit sous agitation à TA. On ajoute au mélange réactionnel, un mélange éther/eau (1/1 ; v/v), après décantation on extrait la phase organique par une solution d'HCl 1N, alcalinise les phases aqueuses jointes à pH = 8 par ajout de NaOH concentrée, extrait au DCM, lave la phase organique par une solution saturée de NaCl, sèche sur $Na_2SO_4$ et évapore sous vide le solvant. On obtient 2 g du produit attendu.

C) 4-[2-(*tert*-butoxycarbonylamino)-2-(pyrid-3-yl)éthyl]benzoate de méthyle.

A une solution de 1,95 g du composé obtenu à l'étape précédente dans 20 ml de DCM, on ajoute 1,83 g de di-*tert*-butyldicarbonate puis 1,16 ml de triéthylamine et laisse 2 heures sous agitation à TA. On concentre sous vide le mélange réactionnel, extrait le résidu à l'AcOEt, lave la phase organique à l'eau, par une solution saturée de NaCl, sèche sur $Na_2SO_4$ et évapore sous vide le solvant. On chromatographie le résidu sur gel de silice en éluant par le mélange heptane/AcOEt de (60/40 ; v/v) à (40/60 ; v/v). On obtient 1,3 g du produit attendu.

D) N-*tert*-Butoxycarbonyl-2-(4-hydroxyméthylphényl)-1-(pyrid-3-yl)éthylamine.

On refroidit à -78°C, sous atmosphère d'argon, une solution de 1,13 g du composé obtenu à l'étape précédente dans 30 ml de THF, ajoute goutte à goutte 7,2 ml d'une solution 1M d'hydrure de diisobutylaluminium dans le toluène et laisse 2 heures sous agitation en laissant remonter la température à 0°C. On refroidit à -40°C le mélange réactionnel, rajoute 7,2 ml d'une solution 1M d'hydrure de diisobutylaluminium dans le toluène et laisse 2 heures sous agitation en laissant remonter la température à 0°C. On ajoute 40 ml d'une solution saturée de $NH_4Cl$, extrait à l'AcOEt, lave la phase organique à l'eau, par une solution saturée de NaCl, sèche sur $Na_2SO_4$ et évapore sous vide le solvant. On chromatographie le résidu sur gel de silice en éluant par le mélange DCM/AcOEt (40/60; v/v). On obtient 0,62 g du produit attendu après cristallisation dans l'éther, F = 130°C.

E) N-(*tert*-butoxycarbonyl)-2-[4-(diéthylaminométhyl)phényl]-1-(pyrid-3-yl) éthylamine.

On refroidit à 0°C une solution de 0,61 g du composé obtenu à l'étape précédente dans 20 ml de DCM, ajoute 0,311 ml de triéthylamine puis 0,16 ml de chlorure de méthanesulfonyle et laisse 30 minutes sous agitation à 0°C. Puis à 0°C, on ajoute 0,58 ml de diéthylamine et laisse 3 heures sous agitation à TA. On verse le mélange réactionnel dans l'eau, extrait au DCM, lave la phase organique à l'eau, par une solution saturée de NaCl, sèche sur $Na_2SO_4$ et évapore sous vide le solvant. On obtient 0,45 g du produit attendu.

F) Tritrifluoroacétate de 2-[4-(diéthylaminométhyl)phényl]-1-(pyrid-3-yl) éthylamine.

On laisse 15 minutes sous agitation à TA un mélange de 0,45 g du composé obtenu à l'étape précédente et 10 ml de TFA dans 10 ml de DCM. On concentre sous vide le mélange réactionnel, reprend le résidu dans l'éther, décante le solvant et utilise tel quel le produit brut attendu.

Préparation 2.11

Ester éthylique de l'acide 2-amino-3-[4-(N-éthyl-N-méthylaminométhyl)phényl] propionique.

(III) : Y = $-CO_2Et$ ; Z = 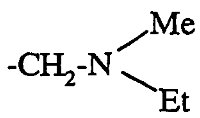

A) Ester éthylique de l'acide 2-(benzhydrylidèneamino)-3-[4-(N-éthyl-N-méthyl aminométhyl)phényl]propionique.

On refroidit à 0˚C un mélange de 1 g du composé obtenu à l'étape A de la Préparation 2.2 et 0,29 ml de N-méthyléthylamine dans 10 ml de DMF, ajoute 0,307 de $K_2CO_3$ et laisse 4 heures sous agitation à 0˚C. On verse le mélange réactionnel dans une solution saturée de NaCl, extrait à l'AcOEt, lave la phase organique par une solution saturée de NaCl, sèche sur $Na_2SO_4$ et évapore sous vide le solvant. On obtient le produit attendu que l'on utilise tel quel.

B) Ester éthylique de l'acide 2-amino-3-[4-(N-éthyl-N-méthylaminométhyl) phényl]propionique.

On laisse une nuit sous agitation à TA un mélange du composé obtenu à l'étape précédente et 15 ml d'HCl 1N dans 15 ml d'éther. Après décantation, on alcalinise la phase aqueuse à pH = 11 par ajout de $Na_2CO_3$, extrait au DCM, lave la phase organique par une solution saturée de NaCl, sèche sur $Na_2SO_4$ et évapore sous vide le solvant. On obtient 0,43 g du produit attendu que l'on utilise tel quel à l'EXEMPLE 13.

Préparation 2.12

Ester éthylique de l'acide 2-amino-3-[4-(dipropylaminométhyl)phényl] propionique.

(III) : Y = $-CO_2Et$ ; Z = $-CH_2N(nPr)_2$

A) Ester éthylique de l'acide 2-(benzhydrylidèneamino)-3-[4-(dipropylamino méthyl)phényl]propionique.

On prépare ce composé selon le mode opératoire décrit à l'étape A de la Préparation 2.11 à partir de 1 g du composé obtenu à l'étape A de la Préparation 2.2, 0,335 ml de dipropylamine et 0,307 g de $K_2CO_3$ dans 10 ml de DMF. On obtient le produit attendu que l'on utilise tel quel.

B) Ester éthylique de l'acide 2-amino-3-[4-(dipropylaminométhyl)phényl] propionique.

On prépare ce composé selon le mode opératoire décrit à l'étape B de la Préparation 2.11 à partir du composé obtenu à l'étape précédente et 15 ml d'HCl 1N dans 15 ml d'éther. On obtient le produit attendu que l'on utilise tel quel.

Préparation 2.13

Ester éthylique de l'acide 2-amino-3-[4-(diisopropylaminométhyl)phényl] propionique.

(III) : $Y = -CO_2Et$ ; $Z = -CH_2N(iPr)_2$

A) Ester éthylique de l'acide 2-(benzhydrylidèneamino)-3-[4-(diisopropylamine méthyl)phényl]propionique.

On prépare ce composé selon le mode opératoire décrit à l'étape A de la Préparation 2.11 à partir de 1 g du composé obtenu à l'étape A de la Préparation 2.2, 0,320 ml de diisopropylamine et 0,307 g de $K_2CO_3$ dans 10 ml de DMF. On obtient le produit attendu que l'on utilise tel quel.

B) Ester éthylique de l'acide 2-amino-3-[4-(düsopropylaminométhyl)phényl] propionique.

On prépare ce composé selon le mode opératoire décrit à l'étape B de la Préparation 2.11 à partir du composé obtenu à l'étape précédente et 30 ml d'HCl 1N dans 30 ml d'éther. On obtient 0,5 g du produit attendu.

Préparation 2.14

Ditrifluoroacétate de 2-amino-3-[4-(*tert*-butylméthylaminométhyl)phényl]-N-isopropyl-N-méthylpropionamide, isomère (R).

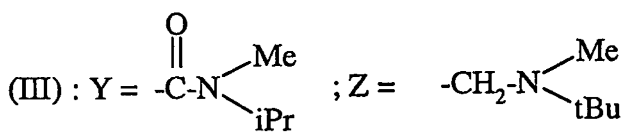

Ce composé est préparé selon le mode opératoire décrit à la Préparation 2.3, en utilisant comme produit de départ l'acide 2-(*tert*-butoxycarbonylamino)-3-[4-(*tert*-butylméthylaminométhyl)phényl]propionique, isomère (R) comme produit de départ.
RMN : 0,8-1,2 ppm : mt : 6H ; 1,4 ppm : s : 9H ;2,5 ppm : d : 6H ; 3,0 ppm : m : 2H ; 3,9 ppm : mt : 1H ; 4,5 ppm : mt : 2H ; 7,3-7,5 ppm : mt : 4H.

Préparation 2.15

Dichlorhydrate de 2-amino-3-[4-(pipérid-1-ylméthyl)phényl]N-isopropyl-N-méthylpropionamide.

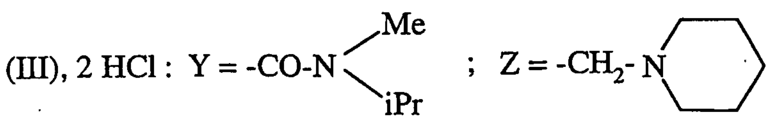

A) 2-(*tert*-Butoxycarbonylamino)-3-(4-cyanophényl)-N-isopropyl-N-méthyl propionamide.

On prépare ce composé selon le mode opératoire décrit à la Préparation 1.6 de la demande internationale WO 97/25315.

B) 2-(*tert*-Butoxycarbonylamino)-3-(4-formylphényl)-N-isopropyl-N-méthyl propionamide.

On refroidit à 0°C une solution de 9 g du composé obtenu à l'étape précédente dans 600 ml d'un mélange pyridine/AcOH/$H_2O$ (2/1/1 ; v/v/v), ajoute sous atmosphère d'argon 47 g d'hypophosphite de sodium hydrate puis 8 g de nickel de Raney® dans l'eau et laisse 10 minutes sous agitation à TA. On chauffe à 55°C pendant 3 heures le mélange réactionnel. Après refroidissement à TA, on filtre sur Célite®, lave à l'EtOH puis au DCM et concentre sous vide le filtrat. On extrait le résidu à l'AcOEt, lave la phase organique à l'eau, par une solution à 5 % de $KHSO_4$ par une solution saturée de NaCl, par une solution saturée de $NaHCO_3$, par une solution saturée de NaCl, sèche sur $Na_2SO_4$ et évapore sous

vide le solvant. On obtient 7,6 g du produit attendu, après cristallisation dans le mélange éther iso/pentane, F = 122˚C.

C) 2-(*tert*-Butoxycarbonylamino)-3-[4-(pipérid-1-ylméthyl)phényl]-N-isopropyl-N-méthylpropionamide

A un mélange de 0,55 g du composé obtenu à l'étape précédente et 0,172 ml de pipéridine dans 10 ml de 1,2-dichloroéthane, on ajoute 0,18 ml d'AcOH puis 0,5 g de triacétoxyborohydrure de sodium et laisse une nuit sous agitation à TA. On dilue le mélange réactionnel au DCM, lave la phase organique par une solution saturée de $NaHCO_3$, par une solution saturée de NaCl, sèche sur $Na_2SO_4$ et évapore sous vide le solvant. On obtient 0,65 g du produit attendu.

D) Dichlorhydrate de 2-amino-3-[4-(pipérid-1-ylméthyl)phényl]N-isopropyl-N-méthylpropionamide.

A un mélange de 0,65 g du composé obtenu à l'étape précédente dans 10 ml de MeOH et 5 ml de dioxane, on ajoute 3,5 ml d'HCl 10N et laisse une nuit sous agitation à TA. On concentre sous vide le mélange réactionnel, reprend le résidu à l'EtOH et évapore sous vide le solvant. On reprend le résidu à l'éther et essore le précipité formé après trituration. On obtient 0,64 g du produit attendu, F = 165˚C (déc).

Préparation 2.16

Ditrifluoroacétate de 2-amino-3-[4-(N-cyclopentyl-N-méthylaminométhyl) phényl]-N-isopropyl-N-méthylpropionamide.

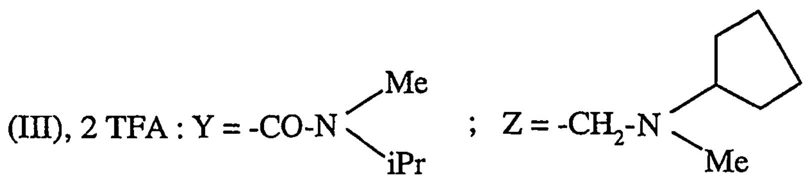

A) 2-(*tert*-Butoxycarbonylamino)-3-[4-(N-cyclopentyl-N-méthylaminométhyl) phényl]-N-isopropyl-N-méthylpropionamide.

A un mélange de 0,5 g du composé obtenu à l'étape B de la Préparation 2.15 et 0,214 g de N-méthylcyclopentylamine dans 10 ml de 1,2-dichloroéthane, on ajoute 0,164 ml d'AcOH puis 0,456 g de triacétoxyborohydrure de sodium et laisse une nuit sous agitation à TA. On dilue le mélange réactionnel au DCM, lave la phase organique par une solution saturée de $NaHCO_3$ et évapore sous vide le solvant. On reprend le résidu dans l'AcOEt, extrait au tampon pH = 4, lave la phase aqueuse acide à l'AcOEt, alcalinise la phase acide par ajout d'une solution saturée de $NaHCO_3$, extrait au DCM, lave la phase organique par une solution saturée de NaCl, sèche sur $Na_2SO_4$ et évapore sous vide le solvant. On obtient 0,31 g du produit attendu.

B) Ditrifluoroacétate de 2-amino-3-[4-(N-cyclopentyl-N-méthylaminométhyl) phényl]-N-isopropyl-N-méthylpropionamide.

On laisse 2 heures sous agitation à TA un mélange de 0,309 g du composé obtenu à l'étape précédente et 10 ml de TFA dans 10 ml de DCM. On concentre sous vide le mélange réactionnel. On obtient le produit obtenu que l'on utilise tel quel à l'EXEMPLE 36.

Préparation 2.17

Ditrifluoroacétate de 2-amino-3-[4-(4-hydroxypipérid-1-ylméthyl)phényl]-N-isopropyl-N-méthylpropionamide.

(III), 2 TFA : Y = 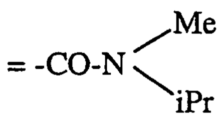 ; Z = 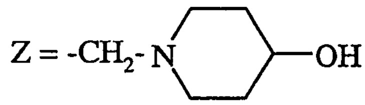

A) 2-(*tert*-Butoxycarbonylamino)-3-[4-(4-hydroxypipérid-1-ylméthyl) phényl]-N-isopropyl-N-méthylpropionamide.

A un mélange de 0,5 g du composé obtenu à l'étape B de la Préparation 2.15 et 0,146 g de 4-hydroxypipéridine dans 10 ml de 1,2-dichloroéthane, on ajoute 0,164 ml d'AcOH puis 0,456 g de triacétoxyborohydrure de sodium et laisse 24 heures sous agitation à TA. On dilue le mélange réactionnel au DCM, lave la phase organique par une solution saturée de $NaHCO_3$, par une solution saturée de NaCl, sèche sur $Na_2SO_4$ et évapore sous vide le solvant. On obtient 0,622 g du produit attendu.

B) Ditrifluoroacétate de 2-amino-3-[4-(4-hydroxypipérid-l-ylméthyl) phényl]-N-isopropyl-N-méthylpropionamide.

On laisse 1 heure sous agitation à TA un mélange de 0,622 g du composé obtenu à l'étape précédente et 10 ml de TFA dans 10 ml de DCM. On concentre sous vide le mélange réactionnel. On obtient le produit attendu que l'on utilise tel quel ultérieurement.

Préparation 2.18

Trifluoroacétate de (R) 1-[2-Amino-3-(4-cyanophényl)propionyl)pyrrolidine.

(III), TFA, (R) : Y = 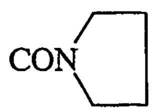 ; Z = CN

A) Ester éthylique de l'acide 2-(N-Boc)amino-3-(4-cyanophényl)propionique.

26 g de $Boc_2O$ en solution dans 100 ml de DCM sont ajoutés progressivement à une solution de 25,5 g du chlorhydrate d'ester éthylique de l'acide 2-amino-3-(4-cyanophényl) propionique et 13,9 ml de $NEt_3$ dans 400 ml de DCM. Après 6 heures d'agitation à TA le milieu réactionnel est lavé par une solution $KHSO_4/K_2SO_4$, par une solution saturée de $NaHCO_3$, par une solution saturée de NaCl. Après séchage sur $Na_2SO_4$ et évaporation du DCM, le résidu est trituré dans l'heptane pour donner 29 g de poudre blanche.

B) Ester éthylique de l'acide (R) 2-(N-Boc)amino-3-(4-cyanophényl)propionique.

Un mélange de 24 g du produit obtenu à l'étape précédente et 8,4 g de $NaHCO_3$ dans 900 ml d'AcOEt et 500 ml $H_2O$ est traité par 2 ml d'Alcalase® pendant 24 heures à TA. Les 2 phases sont décantées ; la phase AcOEt est relavée par 100 ml d'un solution à 10 % de $NaHCO_3$ qui est jointe à la première phase aqueuse et la phase aqueuse est relavée par 100 ml d'AcOEt jointes à la première phase AcOEt. La phase AcOEt ainsi obtenue est séchée sur $Na_2SO_4$ puis évaporée à sec ; on obtient 12,45 g de composé B

$$\alpha_D^{25} = +8{,}8° \ (c = 1 \ ; MeOH)$$

C) Acide (R) 2-(N-Boc)amino-3-(4-cyanophényl)propionique.

A 12,18 g du composé B en solution dans 180 ml de MeOH, on ajoute 43 ml de solution NaOH 1N et agite 1 heure à TA. Puis on additionne 43 ml de solution HCl 1N et évapore 150 ml de méthanol puis on reprend dans AcOEt, et on lave par de l'eau puis par une solution saturée de NaCl. On obtient 11 g de composé attendu, après cristallisation par un mélange $Et_2O$/heptane.

$\alpha_D^{25}$ = -9,5° (c = 1 ; MeOH)

D) Ester de N-hydroxysuccinimide de l'acide (R) 2-(N-Boc)amino-3-(4-cyanophényl)propionique.

A 10 g de l'acide obtenu ci-dessus dissous dans 10 ml de dioxane, on ajoute 4,2 g de NSuOH puis en 20 minutes on ajoute 8,62 g de DCC dissous dans 30 ml de dioxane. Après 1 nuit d'agitation à TA la DCU formée est filtrée, lavée par du dioxane. Le filtrat est évaporé à sec et le résidu trituré dans de l'éther pour donner un solide qui est filtré et séché. On obtient 12,09 g du composé attendu.

$\alpha_D^{25}$ = +27,1° (c = 1 ; MeOH)

E) (R) 1-[2-(N-Boc)amino-3-(4-cyanophényl)propionyl]pyrrolidine.

A 11,6 g du composé obtenu à l'étape ci-dessus dissous dans 150 ml d'acétonitrile plus 20 ml de DMF on ajoute en 10 minutes 2,6 ml de pyrrolidine dissoute dans 20 ml d'acétonitrile. Après 1 nuit d'agitation à TA, on élimine un peu d'insoluble et concentre le filtrat sous vide. Le résidu est repris dans de l'AcOEt et on lave par une solution $KHSO_4/K_2SO_4$, une solution saturée de $NaHCO_3$, une solution saturée par NaCl ; après séchage sur $Na_2SO_4$ l'AcOEt est évaporé sous vide et le résidu trituré dans l'éther, on obtient 9,3 g du composé attendu sous forme d'un solide blanc.

$\alpha_D^{25}$ = -29,2° (c = 1 ; MeOH)

F) Trifluoroacétate de (R) 1-[2-Amino-3-(4-cyanophényl)propionyl]pyrrolidine, trifluoroacétate.

8,7 g de produit obtenu à l'étape précédente sont agités 35 minutes dans un mélange de 50 ml de DCM et 50 ml de TFA. Après évaporation à sec, on reprend le résidu dans de l'isopropanol et on réévapore à sec, on obtient 8,67 g du composé attendu sous forme solide.

$\alpha_D^{25}$ = -46° (c = 1 ; MeOH)

Préparation 2.19

Trifluoroacétate de (R) 2-amino-3-(4-diisopropylaminométhylphényl)-N-isopropyl-N-méthylpropionamide.

(III), 2TFA, (R) : Y = 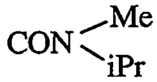 ; Z = 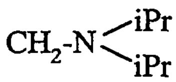

A) (R) 2-(N-Boc)amino-3-(4-cyanophényl)-N-isopropyl-N-méthylpropionamide.

On place 2 g de l'acide obtenu à la préparation 2.18, étape C dans 20 ml de DCM en présence de 750 µl de isopropylméthylamine, 1,26 ml de DIPEA et 3,2 g de BOP puis on laisse 4 heures sous agitation à TA. On concentre à sec puis on extrait par AcOEt puis on lave successivement par de l'eau, une solution tampon à pH = 2, une solution saturée de $NaHCO_3$, une solution saturée de NaCl. On obtient 2,40 g du composé attendu.

B) (R) 2-(N-Boc)amino-3-(4-formylphényl)-N-isopropyl-N-méthylpropionamide.

On place 2,4 g du composé de l'étape précédente dans 130 ml de solvant constitué d'un mélange pyridine/AcOH/eau (2/1/1 ; v/v/v) ; on ajoute 2,45 g de Nickel de Raney et 12,12 g de Na $H_2$ $PO_2$ puis on chauffe à 55°C pendant 3 heures. On filtre sur Célite et concentre le filtrat. On extrait par AcOEt, puis on lave successivement par de l'eau, une solution tampon à pH = 2, une solution saturée de $NaHCO_3$ une solution saturée de NaCl. Après concentration et séchage, le résidu est chromatographié sur silice en éluant par un mélange DCM/MeOH (100/1 ; v/v). On obtient 1,60

g du composé attendu.

C) (R) 2-(N-Boc)amino-3-(4-diisopropylaminométhyl)-N-isopropyl-N-méthyl propionamide.

On laisse sous agitation pendant 24 heures à TA, un mélange contenant 0,5 g du composé obtenu à l'étape précédente, 402 µl de diisopropylamine et 608 mg de $NaBH(OAc)_3$ dans 20 ml de DCE. On concentre à sec, reprend dans un mélange eau/AcOEt puis on extrait au DCM et lave par une solution saturée de NaCl. On obtient 0,296 g du composé attendu.

D) Trifluoroacétate de (R) 2-amino-3-(4-diisopropylaminométhylphényl)-N-isopropyl-N-méthylpropionamide.

On place 296 mg du composé de l'étape précédente dans 5 ml de TFA et 10 ml de DCM et on laisse sous agitation 3 heures à TA. On concentre à sec, triture dans le pentane, décante puis récupère l'huile formée. Le composé obtenu est utilisée brut à l'exemple 39.

En opérant selon les méthodes décrites ci-dessus, on prépare les composés de formule (III) décrits dans la tableau V ci-après :

TABLEAU V

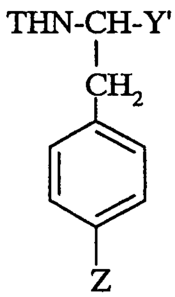

(III)' = (III) ou (XXXVII)

| Préparation | T | Y' | Z | RMN ou F°C |
|---|---|---|---|---|
| 2.20 | H | $CO_2H$ | 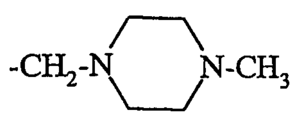 | RMN |
| 2.21 2HCl | H | 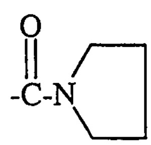 | 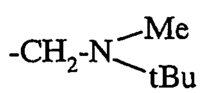 | RMN |
| 2.22 | Boc | 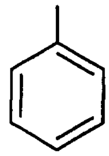 | 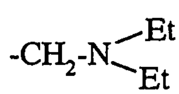 | 68-70°C RMN |
| 2.23 2HCl | H | 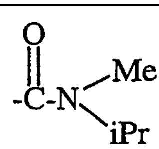 | 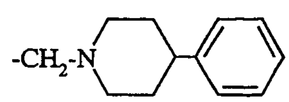 | 170°C |

(suite)

| Préparation | T | Y' | Z | RMN ou F°C |
|---|---|---|---|---|
| 2.24 | Boc | 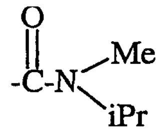 | 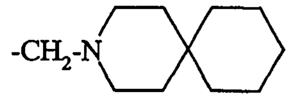 | RMN |
| 2.25 | Boc | 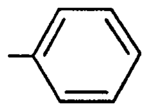 | 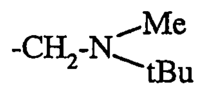 | 102°C RMN |
| 2.26 | Boc | 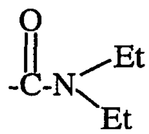 | 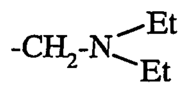 | 85°C |
| 2.27 | Boc | 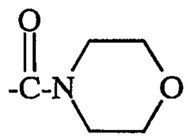 | 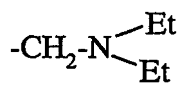 | 75°C |
| 2.28 2HCl | H | 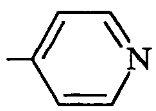 | -CN | 267°C |
| 2.29 | H | 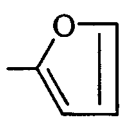 | -CN | 62°C RMN |
| 2.30 2HCl | H | 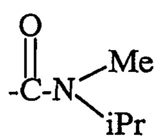 | 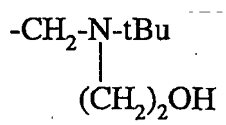 | |
| 2.31 | Boc | 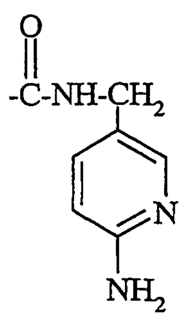 | 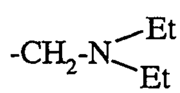 | RMN |
| 2.32 | Boc | 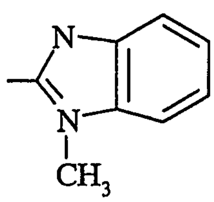 | 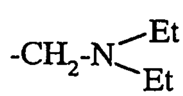 | 128-130°C RMN |

(suite)

| Préparation | T | Y' | Z | RMN ou F°C |
|---|---|---|---|---|
| 2.33 | Boc | 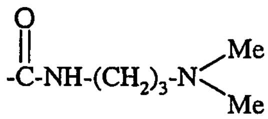 | 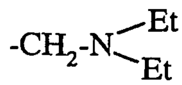 | RMN |
| 2.34 | Boc | 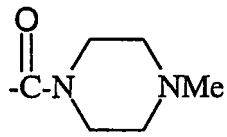 | 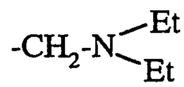 | RMN |
| 2.35 2HCl | H | 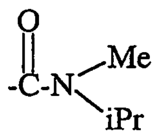 | 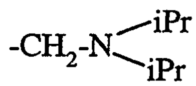 | 166°C |
| 2.36 | Boc | 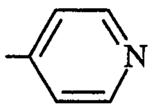 | 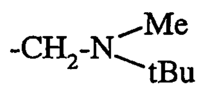 | 66-68°C RMN |
| 2.37 | Boc | 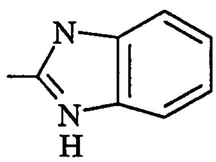 | 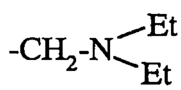 | 99-102°C RMN |
| 2.38 | Boc | 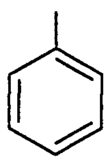 | 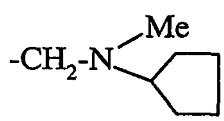 | RMN |
| 2.39 | H | 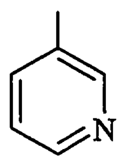 | -CN | 92°C |

(suite)

| Préparation | T | Y' | Z | RMN ou F°C |
|---|---|---|---|---|
| 2.40 | H | 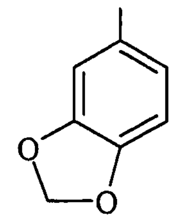 | -CN | |

Préparation 2.20 : RMN (DMSO + TFA) : 2,8 ppm : s : 3H ; 3,1 ppm : mt : 2H ; 3,5 ppm : m : 8H ; 4,2 ppm : t : 1H ; 4,4 ppm : s : 2H ; 7,35 ppm : d : 2H ; 7,6 ppm : d : 2H.
Préparation 2.21 : RMN : 1,25 ppm : s : 9H ; 1,40-1,65 ppm : m : 4H ; 2,40-4,60 ppm : mt : 10H ; 7,10-7,60 ppm : mt : 4H.
Préparation 2.22 : RMN : 0,85-1,00 ppm : t : 6H ; 1,20 ppm : s : 9H ; 2,30-4,30 ppm : m : 8H ; 4,70 ppm : qd : 1H ; 7,10-7,75 ppm : m : 10H.
Préparation 2.24 : RMN (DMSO + TFA) : 1,0 ppm : mt : 6H ; 1,2 ppm : s : 9H ; 1,8 ppm : m : 14H ; 2,6 ppm : d : 3H ; 3,0 ppm : m : 6H ; 4,1-4,7 ppm : m : 4H ; 7,3 ppm : q : 4H.
Préparation 2.25 : RMN : 1,0 ppm : s : 9H ; 1,2 ppm : s : 9H ; 1,9 ppm : s : 3H ; 2,8 ppm : d : 2H ; 3,4 ppm : s : 2H ; 4,6 ppm : mt : 1H ; 7,0-7,3 ppm : mt : 9H ; 7,4 ppm : d : 1H.
Préparation 2.29 : RMN: 2,9 ppm : mt : 2H ; 4,0 ppm : t : 1H ; 6,1 ppm : d : 1H ; 6,3 ppm : d : 1H ; 7,3 ppm : d : 2H ; 7,5 ppm : s : 1H ; 7,6 ppm : d : 2H.
Préparation 2.31 : RMN: 0,85 ppm : t : 6H ; 1,20 ppm : s : 9H ; 2,40-4,30 ppm : m : 11H ; 6,90-8,05 ppm : m : 9H.
Préparation 2.32 : RMN : 0,90 ppm : t : 6H ; 1,20 ppm : s : 9H ; 2,40 ppm : qd : 4H ; 3,20-3,70 ppm : mt : 7H ; 5,10 ppm : qd : 1H ; 7,10-7,70 ppm : mt : 9H.
Préparation 2.33 : RMN (DMSO + TFA) : 1,15 ppm : t : 6H ; 1,20 ppm : s : 9H ; 1,60-1,80 ppm : mt : 2H ; 2,70-4,40 ppm : m : 19H ; 7,25-7,45 ppm : mt : 4H.
Préparation 2.34 : RMN (DMSO + TFA) : 1,10 ppm : t : 6H ; 1,20 ppm : s : 9H ; 2,60-4,60 ppm : m : 20H ; 7,20-7,45 ppm : mt : 4H.
Préparation 2.36 : RMN : 1,00 ppm : s : 9H ; 1,15 ppm : s : 9H ; 2,70-4,60 ppm : m : 8H ; 7,00-8,40 ppm : m : 9H.
Préparation 237 : RMN : 0,90 ppm : t : 6H ; 1,20 ppm : s : 9H ; 2,35 ppm : qd : 4H ; 2,80-3,40 ppm : m : 4H ; 4,80-5,00 ppm : mt : 1H ; 7,00-7,50 ppm : m : 9H ; 12,1 ppm : se : 1H.
Préparation 2.38 : RMN : 1,2 ppm : s : 9H ; 1,2-1,8 ppm : m : 8H ; 1,9 ppm : s : 3H ; 2,6 ppm : mt : 1H ; 2,8 ppm : d : 2H ; 3,3 ppm : s : 2H ; 4,6 ppm : mt : 1H ; 7,0-7,2 ppm : mt : 9H ; 7,4 ppm : d : 1H.

Préparation 2.41

(R) 2-Amino-3-(4-((2,6-diméthyl-1-pipéridinyl)méthyl)phényl-N-isopropyl-N-méthylpropanamide, 2TFA.

(III), (R) : Y = 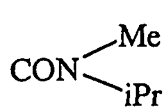 ; Z = 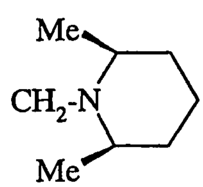

A)

On laisse sous agitation pendant 48 heures à TA un mélange contenant 500 mg du composé décrit à la préparation 2.19, étape B, 390 µl de (cis) 2,6-diméthylpipéridine et 608 mg de $NaBH(OAc)_3$. On concentre à sec le

milieu puis on dilue par 30 ml de AcOEt et on extrait par une solution tampon pH = 4. On extrait la phase aqueuse par DCM puis on lave par une solution de $NaHCO_3$ saturé puis une solution de NaCl saturée. On obtient 411 mg du composé attendu.

B)

On laisse sous agitation à TA pendant 4 heures 411 mg du composé obtenu à l'étape précédente et 10 ml de TFA dans 10 ml de DCM. On concentre à sec puis on triture dans un mélange pentane/éther. Après décantation et séchage, on obtient 0,480 g du composé attendu sous forme d'huile.

Préparation 2.42

(R) 2-(N-Boc)amino-3-(4-((3,3-diméthyl-1-pipéridinyl)méthylphényl)-N-isopropyl)-N-méthylpropanamide.

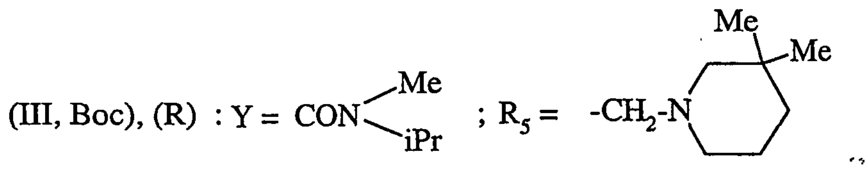

On place 497 mg du composé décrit à la préparation 2.19, étape B, dans 10 ml de DCE et on ajoute 165 µl d'AcOH et 455 mg de $NaBH(OAc)_3$. Après une nuit sous agitation à TA, on concentre le milieu réactionnel puis on reprend par $Et2O/H_2O$ et on extrait par un tampon pH = 4: Les phases aqueuses sont basifiées à pH = 8 par de la lessive de soude puis on extrait au DCM et lave par une solution saturée de NaCl. On obtient 0,168 g du composé attendu.

Préparation 2.43

(R) 2-(N,Boc)amino-3-(4-((4,4-difluoro-1-pipéridinyl)méthyl)phényl-N-isopropyl-N-méthylpropanamide.

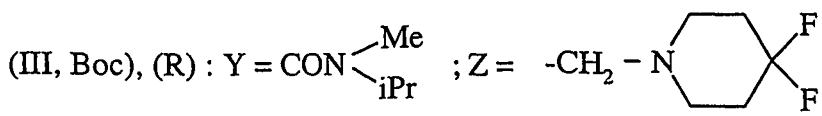

Ce composé est préparé comme décrit à la préparation 2.41 à partir du composé de la préparation 2.19, étape B et de la 4,4-difluoropipéridine.

Préparation 2.44

Acide 2-(R), 2-(N-Boc)amino-3-(4-((2,6-diméthyl-1-pipéridinyl)méthyl)phényl) propanoïque.

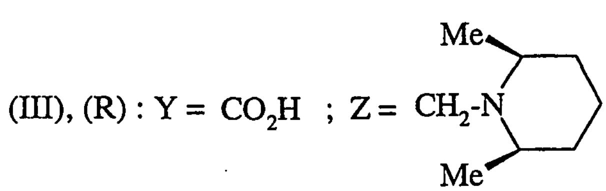

Ce composé est préparé en suivant les Schémas 9 et 10.

A) 2-(R), 2-(N-Boc)amino-3-(4-formylphényl)propanoate d'éthyle.

On place 4 g du composé de la Préparation 2.18, étape B sous argon dans 300 ml d'un mélange pyridine/$AcOH/H_2O$ (2/1/1 ; v/v/v). On traite par 5 g de Nickel de Raney et 30 g de $NaH_2PO_2$, $H_2O$ pendant 6 heures à 55°C. On filtre sur célite®, puis concentre. On reprend par AcOEt et on lave par $H_2O$, $KHSO_4$ à 5 %, $H_2O$, une solution saturée de $NaHCO_3$, un solution saturée de NaCl, puis on sèche et concentre. On obtient 3,91 g du produit attendu sous forme d'huile.
RMN : 1,2 : t : 3H ; 1,4 : s : 9H ; 2,9-3,2 : mt : 2H ; 4,0-4,4 : mt : 3H ; 7,4 : d : 1H ; 7,55 : d : 2H ; 7,9 : d : 2H ; 10,0 : s : 1H.

B) 2-(R), 2-(N-Boc)amino-3-(4-((2,6-diméthyl-1-pipéridinyl)méthyl)phényl propanoate d'éthyle.

On place 3,9 g du composé obtenu à l'étape B dans 30 ml de dichloroéthane et on traite par 6,7 g du $NaHB(OAc)_3$ et 4 ml de (cis)-2,6-diméthylpipéridine pendant 24 heures à TA. On concentre, reprend par de l'éther, puis extrait par $KHSO_4$ à 5 % puis de l'eau. Les phases aqueuses sont basifiées à pH = 8 par addition de $NaHCO_3$, on extrait au DCM, sèche et concentre et on obtient 3 g du produit attendu sous forme d'huile.
RMN: 0,9 : d : 6H ; 1,0 : t : 3H ; 1,05-1,8 : mt : 15H ; 2,3 : m : 2H ; 2,6-2,9 : mt : 2H ; 3,6 : s : 2H ; 2,9-4,1 : mt : 3H ; 7,0-7,2 : mt : 5H.

C) (R) Acide 2-(N-Boc)amino-3-(4-((2,6-diméthyl-1-pipéridinyl)méthyl)phényl) propanoïque.

On place 3 g du composé obtenu à l'étape précédente dans 20 ml de MeOH et 20 ml de dioxane, puis on ajoute goutte à goutte 7,5 ml de NaOH 1N et on laisse 1 heure sous agitation à TA. On ajoute 7,5 ml d'HCl 1N puis on concentre à sec. On effectue une distillation azéotropique par EtOH absolu et le toluène. On reprend par DCM, sèche et filtre pour obtenir 2,7 g du composé attendu sous forme d'une mousse sèche.
RMN : 0,9 : d : 6H ; 1,0-1,4 : mt : 15H ; 2,4 : m : 2H ; 2,6-3,0 : mt : 2H ; 3,65 : s : 2H ; 4,0 : m : 1H ; 7,0 : d : 1H ; 7,2 : d : 2H.

Préparation 2.45

2-(R), 2-(N-Boc)amino-3-(4-(((2-fluoroéthyl)-(éthyl)amino)méthyl)phényl)-N-isopropyl-N-méthylpropanamide.

(III), (R)

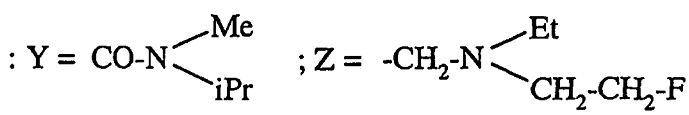

Ce composé est préparé en suivant le Schéma 9 bis (étape b) de la description ci-dessus.

A) 2-(R), 2-(N-Boc)amino-3-(4-(((éthylamino)méthyl)phényl)-N-isopropyl-N-méthyl)propanamide.

On place 520 mg de 2-(R), 2-(N-Boc)-3-(4-formylphényl)-N-isopropyl-N-méthyl propanamide dans 20 ml de DCE et on traite par 3 ml d'éthaneamine et 633 mg de $NaBB(OAc)_3$ plus, 1 ml de AcOH pendant une nuit à TA. On concentre puis on reprend par un mélange eau/éther. La phase aqueuse est basifiée à pH = 8 par $NaHCO_3$ puis on extrait au DCM. On obtient 350 mg du composé attendu sous forme d'huile. Le spectre de RMN est conforme avec la structure du composé.

B)

310 mg du composé de l'étape précédente sont dissous dans le DMF (10 ml) et traité par du 2-fluoro-1-iodoéthane (143 mg) en présence de $K_2CO_3$ (114 mg) pendant 48 heures à TA. La réaction est concentrée, reprise par AcOEt, extraite par $H_2O$ puis $KHSO_4$ à 5 %. La phase aqueuse est basifiée à pH = 8 par $NaHCO_3$ puis extraite par $CH_2Cl_2$. On obtient 115 mg du produit attendu sous forme d'huile.

RMN

Préparation 2.46

2-(R), 2-(N-Boc)amino-3-(4-(((cyclopropyl)(isopropyl)amino)méthyl)phényl)-N-isopropyl-N-méthyl)propanamide.

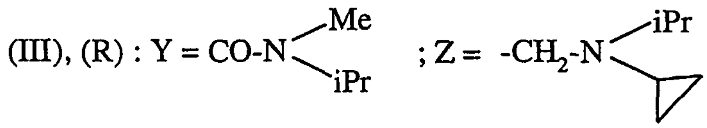

Ce composé est préparé en suivant le Schéma 9 bis (étape c).

A) 2-(R), 2-(N-Boc)amino-3-(4-((cyclopropylamino)méthyl)phényl)-N-isopropyl-N-méthyl)propanamide.

Ce composé est obtenu en faisant réagir le cyclopropylamine sur la 2-(R), 2-(N-Boc)-3-(4-formylphényl)-N-isopropyl-N-méthylpropanamide selon le mode opératoire décrit à l'étape A de la Préparation ci-dessus.

B) 2-(R), 2-(N-Boc)amino-3-(4-(((cyclopropyl)(isopropyl)amino)méthyl)phényl)-N-isopropyl-N-méthyl)propanamide.

On place 220 mg du composé de l'étape précédente et 125 µl d'acétone dans 10 ml de DCE et on traite par 180 mg de $NaHB(OAc)_3$ pendant 24 heures à TA. On concentre, reprend par de l'éther puis extrait par une solution de $KHSO_4$ à 5 % puis $H_2O$. Les phases aqueuses sont basifiées à pH = 8 par $NaHCO_3$ puis on extrait au DCM et sèche. On obtient 105 mg du composé attendu sous forme d'huile.
En opérant selon les préparations décrites ci-dessus, on prépare les composés intermédiaires décrits dans le tableau ci-après. (On indique dans la colonne de droite le procédé réactionnel utilisé pour la préparation).

TABLEAU VI

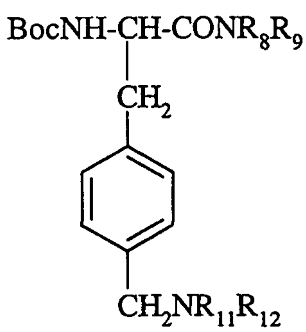

| Préparations (stéréochimie) | $CONR_8R_9$ | $-CH_2NR_{11}R_{12}$ | F°C ou RMN | Procédé |
|---|---|---|---|---|
| 2.47 (R) | 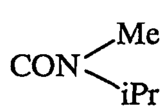 | 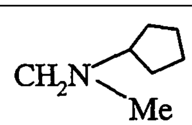 | RMN | a |
| 2.48 | 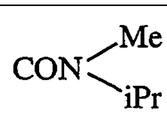 | 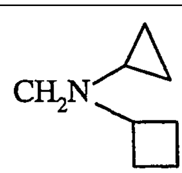 | RMN | a |

(suite)

| Préparations (stéréochimie) | $CONR_8R_9$ | $-CH_2NR_{11}R_{12}$ | F°C ou RMN | Procédé |
|---|---|---|---|---|
| 2.49 | 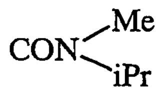 | 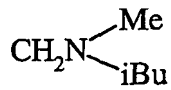 | RMN | a |
| 2.50 | 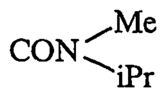 | 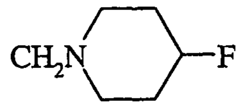 | RMN | a |
| 2.51 | 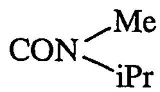 | 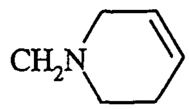 | RMN | a |
| 2.52 (R) | 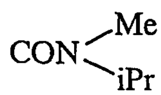 | 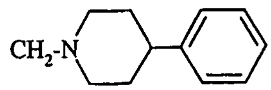 | RMN | a |
| 2.53 | 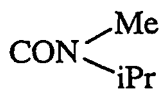 | 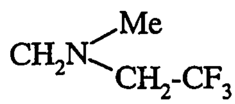 | RMN | c |
| 2.54 (R) | 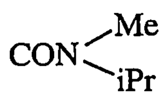 | 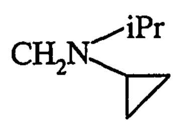 | utilisé brut | c |
| 2.55 (R) | 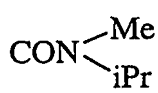 | 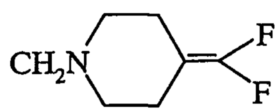 | RMN | a |
| 2.56 | 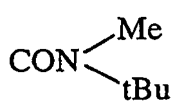 | 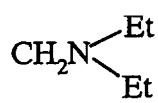 | utilisé brut | a |
| 2.57 | 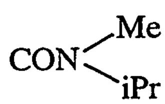 | 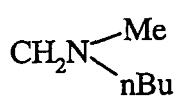 | RMN | a |
| 2.58 | 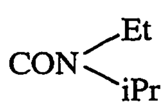 | 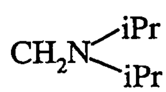 | RMN | d |
| 2.59 | 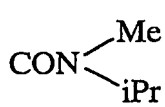 | 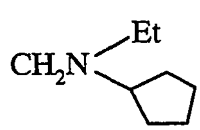 | RMN | a |
| 2.60 | 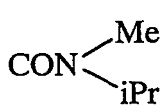 | 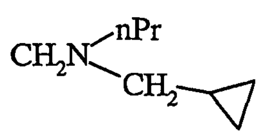 | RMN | a |

(suite)

| Préparations (stéréochimie) | $CONR_8R_9$ | $-CH_2NR_{11}R_{12}$ | F°C ou RMN | Procédé |
|---|---|---|---|---|
| 2.61 | 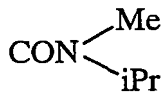 | 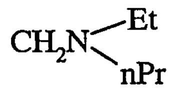 | RMN | a |
| 2.62 | 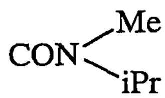 | 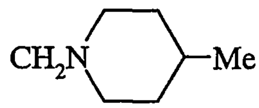 | RMN | a |
| 2.63 | 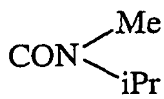 | 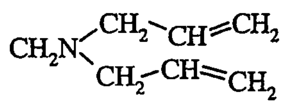 | RMN | a |
| 2.64 | 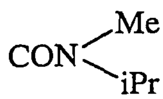 | 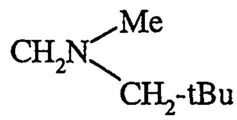 | RMN | a |
| 2.65 (R) | 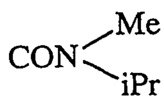 | 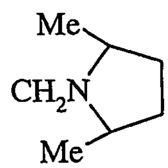 | RMN | a |
| 2.66 (R) | 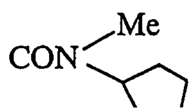 | 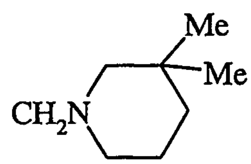 | RMN | d |
| 2.67 | 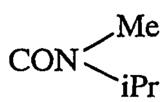 | 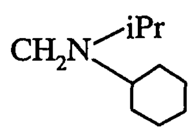 | RMN | a |
| 2.68 | 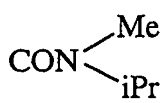 | 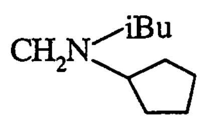 | RMN | c |
| 2.69 (R) | 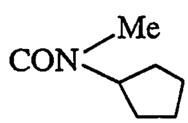 | 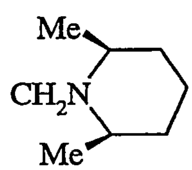 | RMN | a |
| 2.70 (R) | 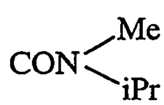 | 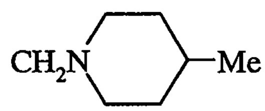 | RMN | a |
| 2.71 | 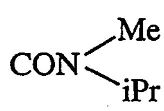 | 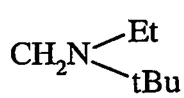 | RMN | a |

(suite)

| Préparations (stéréochimie) | $CONR_8R_9$ | $-CH_2NR_{11}R_{12}$ | F°C ou RMN | Procédé |
|---|---|---|---|---|
| 2.72 (R) | 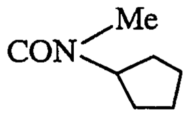 | 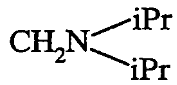 | RMN | a |
| 2.73 | 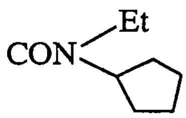 | 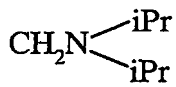 | RMN | d |
| 2.74 (R) | 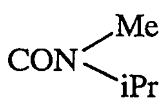 | 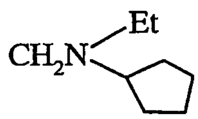 | RMN | d |
| 2.75 (R) | 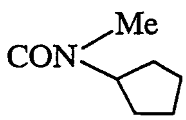 | 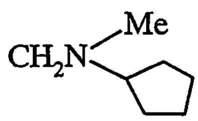 | RMN | d |
| 2.76 | 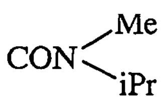 | 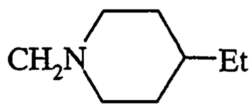 | RMN | a |
| 2.77 (R) | 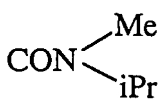 | 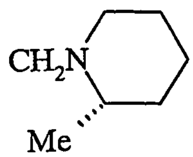 | RMN | a |
| 2.78 | 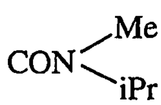 | 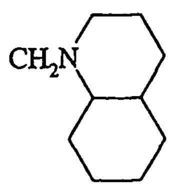 | RMN | a |
| 2.79 | 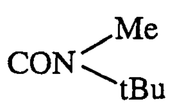 | 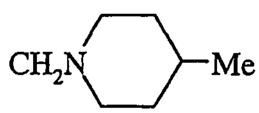 | RMN | a |
| 2.80 | 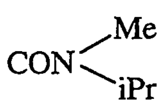 | 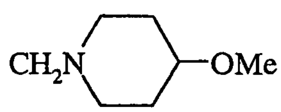 | RMN | a |
| 2.81 (R) | 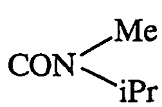 | 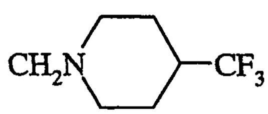 | RMN | a |
| 2.82 | 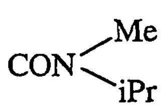 | 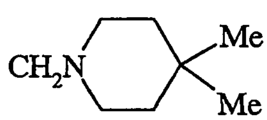 | 103°C | a |

(suite)

| Préparations (stéréochimie) | $CONR_8R_9$ | $-CH_2NR_{11}R_{12}$ | F°C ou RMN | Procédé |
|---|---|---|---|---|
| 2.83 (R) | 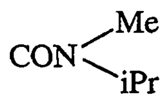 | 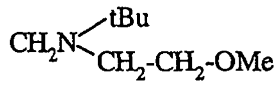 | RMN | a |
| 2.84 | 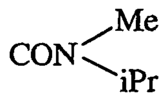 | 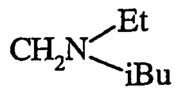 | RMN | a |
| 2.85 | 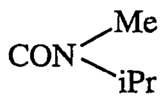 | 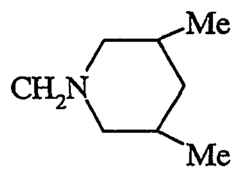 | RMN | a |
| 2.86 (R) | 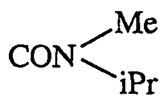 | 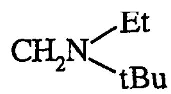 | RMN | a |
| 2.87 | 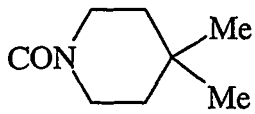 | 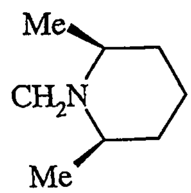 | brut | d |
| 2.88 | 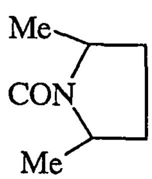 | 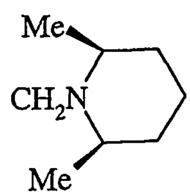 | 130°C | d |
| 2.89 (R) | 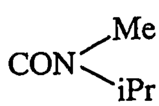 | 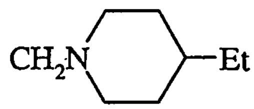 | RMN | a |
| 2.90 (R) | 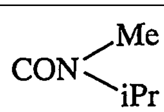 | 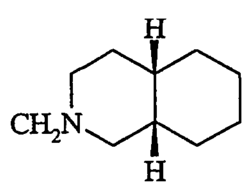 | RMN | a |
| 2.91 (R) | 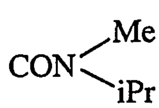 | 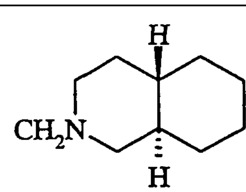 | utilisé brut | a |

(suite)

| Préparations (stéréochimie) | $CONR_8R_9$ | $-CH_2NR_{11}R_{12}$ | F°C ou RMN | Procédé |
|---|---|---|---|---|
| 2.92 (R) | 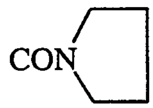 | 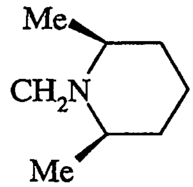 | RMN | d |
| 2.93 (R) | 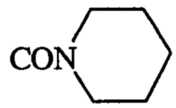 | 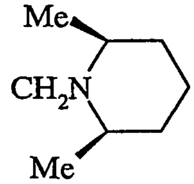 | RMN | d |
| 2.94 (R) | 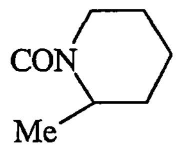 | 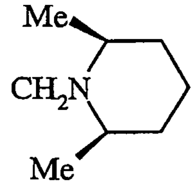 | RMN | d |
| 2.95 (R) | 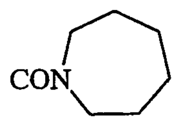 | 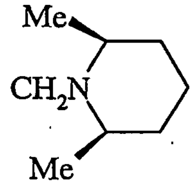 | RMN | d |
| 2.96 (R) | 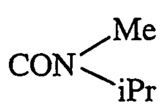 | 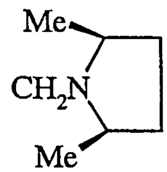 | RMN | d |
| 2.97 (R) | 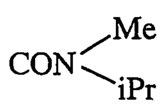 | 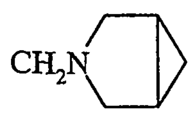 | RMN | a |
| 2.98 (R) | 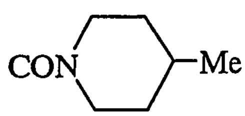 | 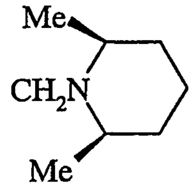 | RMN | d |
| 2.99 (R) | 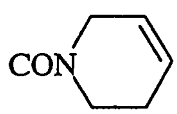 | 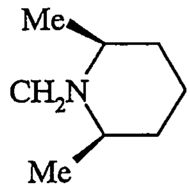 | RMN | d |

(suite)

| Préparations (stéréochimie) | $CONR_8R_9$ | $-CH_2NR_{11}R_{12}$ | F°C ou RMN | Procédé |
|---|---|---|---|---|
| 2.100 (R) | 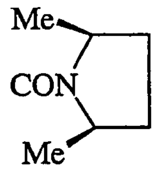 | 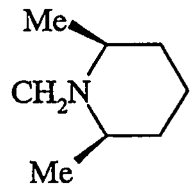 | 152°C | d |
| 2.101 (R) | 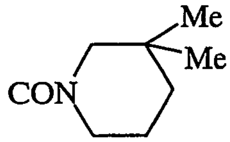 | 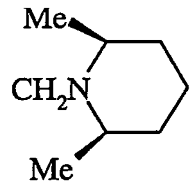 | RMN | d |
| 2.102 (R) | 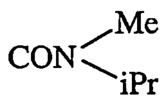 | 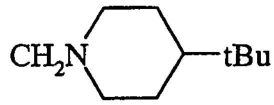 | RMN | a |
| 2.103 (R) | 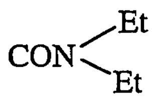 | 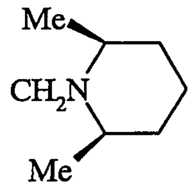 | RMN | d |
| 2.104 (R) | 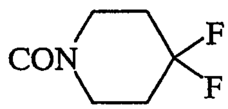 | 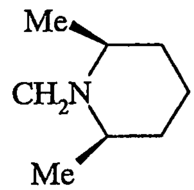 | 120°C | a |
| 2.105 (R) | 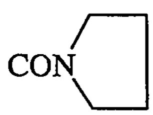 | 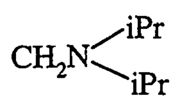 | RMN | a |
| 2.106 | 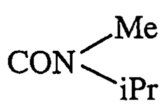 | 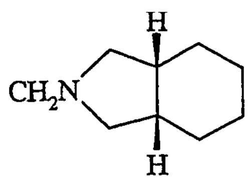 | RMN | a |
| 2.107 (R) | 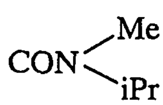 | 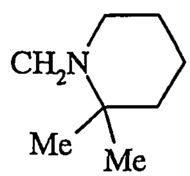 | RMN | a |

(suite)

| Préparations (stéréochimie) | $CONR_8R_9$ | $-CH_2NR_{11}R_{12}$ | F°C ou RMN | Procédé |
|---|---|---|---|---|
| 2.108 | 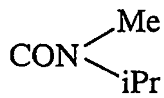 | 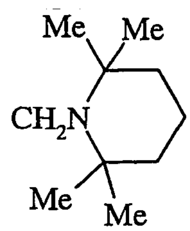 | RMN | a |
| 2.109 (R) | 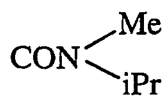 | 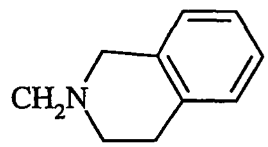 | RMN | a |
| 2.110 (R) | 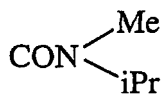 | 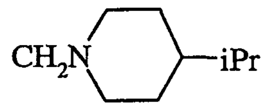 | RMN | a |
| 2.111 (R) | 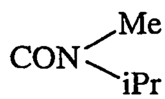 | 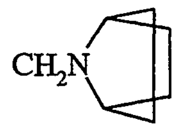 | RMN | a |
| 2.112 (R) | 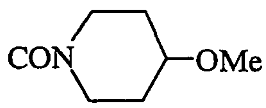 | 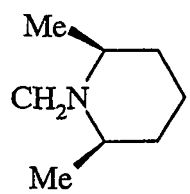 | RMN | a |
| a) Ce composé est préparé à partir d'un composé (III) dans lequel Z = CN et Y = $CONR_8R_9$, en utilisant le procédé décrit dans le Schéma 9 pour obtenir Z = $CH_2NR_{11}R_{12}$.<br>b) Ce composé est préparé à partir d'un composé (III) dans lequel Z = CN et Y = $CONR_8R_9$, en utilisant le procédé décrit dans le Schéma 9bis (étape b) pour obtenir Z=$CH_2NR_{11}R_{12}$.<br>c) Ce composé est préparé à partir d'un composé (III) dans lequel Z = CN et Y = $CONR_8R_9$, en utilisant le procédé décrit dans le Schéma 9bis (étape c) pour obtenir Z = $CH_2NR_{11}R_{12}$.<br>d) Ce composé est préparé à partir d'un composé (XXXVI) dans lequel Y = $CO_2R'$, en utilisant le procédé décrit dans le Schéma 10 pour obtenir le composé (III) avec Y = $CONR_8R_9$. | | | | |

Préparation 2.113

(R) 2-(N-Boc)amino-3-(4-(3,6-dihydro)-1(2*H*)-pyridinylméthyl)phényl)-1-(1,3-thiazol-2-yl)-1-propanone.

(III, Boc) : (R) : Y = CO 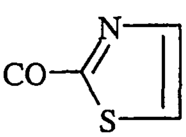 ; $R_5$ = $-CH_2-$ 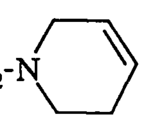

A) 2-(N-Boc)amino-3-(4-cyanophényl)-N-méthoxy-N-méthylpropanamide.

On mélange 2 g de (N-Boc)-4-cyanophénylalanine, 2,32 g de TBTU, 2,40 ml de DIPEA et 806 mg de chlorhydrate

de N,O-diméthylhydroxylamine dans 30 ml de DCM. Après 24 heures sous agitation à TA, on concentre à sec puis on extrait par AcOEt. La phase organique est lavée par une solution tampon pH = 2, une solution saturée de $NaHCO_3$ puis une solution saturée de NaCl. Le composé attendu cristallise dans un mélange d'éther isopropylique et de pentane (1,89 g).

B) 4-(2-(N-Boc)amino-3-oxo-3-(1,3-thiazol-2-yl)propyle)benzonitrile.

On prépare une solution de 1,3-thiazole dans 50 ml de THF à -78°C à laquelle on ajoute goutte à goutte, 9,57 ml de butyllithium 2,5 M dans l'hexane puis le composé de l'étape précédente dissout dans 25 ml de THF. Après 1 heure sous agitation à -78°C, on ajoute 50 ml de solution tampon à pH = 2 et on agite à nouveau 1 heure à TA. Le milieu réactionnel est extrait par AcOEt puis on lave la phase organique par un tampon pH = 2 puis une solution saturée de NaCl. On évapore à sec puis on chromatographie le résidu sur silice en éluant par un mélange heptane/acétone (8/2; v/v) pour obtenir 1,65 g du composé attendu.

C) 4-(2-(N-Boc)amino)-3-oxo-3-(1,3-thiazol-2-yl)propyl)benzaldehyde.

On place 1,65 g du composé de l'étape précédente, 3 g de Nickel dé Raney et 14 g de $NaH_2PO_2$, $H_2O$ dans 100 ml d'un mélange de pyridine/acide acétique/eau (2/1/1 ; v/v/v) et on chauffe à 55°C pendant 3 heures. On élimine le Nickel par décantation puis on concentre. Le milieu réactionnel est extrait par AcOEt puis la phase organique est lavée par un tampon pH = 2, une solution saturée de $NaHCO_3$, une solution saturée de NaCl. On évapore à sec puis le résidu est chromatographié sur silice en éluant par un mélange heptane/AcOEt (6/4; v/v) pour donner 400 mg du composé attendu.

D) (R) 2-(N-Boc)amino-3-(4-(3,6-dihydro)-1(2*H*)-pyridinylméthyl)phényl)-1-(1,3-thiazol-2-yl)-1-propanone

On place 400 mg du composé de l'étape précédente, 105 µl de 1,2,3,6-tétrahydro pyridine et 3,65 mg de NaBH $(OAc)_3$ dans 20 ml de DCE et on laisse une nuit à TA sous agitation. On concentre à sec puis on reprend le résidu par un tampon pH = 2 et on lave à l'éther. La phase acide est extraite au DCM puis lavée par une solution saturée de $NaHCO_3$. On obtient 0,283 g du composé attendu.

Préparation 2.114

2-(N-Boc)amino-N-isopropyl-N-méthyl-3-(4-(1-pipéridinylméthyl)phényl) propanethioamide.

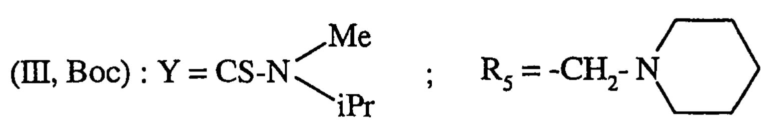

On place 1,33 g du composé de la Préparation 2.15, étape C dans 40 ml de toluène anhydre et on traite par 1,29 g de réactif de Lawesson à 80°C, sous argon, pendant 4 heures. Le milieu réactionnel est concentré puis on chromatographie sur silice en éluant par un mélange DCM/MeOH/$NH_4OH$ (100/4/0,3 ; v/v/v) pour obtenir 0,185 g du composé attendu.

Préparation 3.1

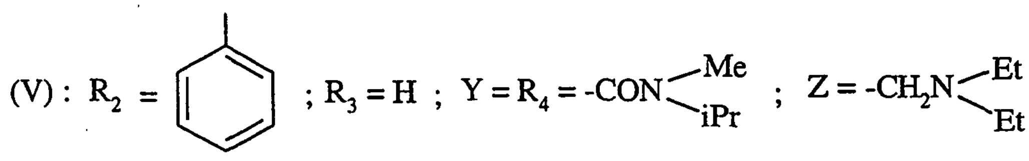

A) Acide 3-phénylaminopropionique, TFA.

On place 2 g d'ester *tert*butylique de l'acide 3-phénylaminopropionique dans 30 ml de TFA et 20 ml de $CH_2Cl_2$ et on laisse 4 heures sous agitation à TA puis on concentre à sec.

B)

A 0°C dans 30 ml de DCM, on mélange le composé obtenu à l'étape précédente avec 3,80 g de 2-amino-3-(4-((diéthylamino)méthyl)phényl)-N-isopropyl-N-méthylpropanamide et 5,02 ml de TEA, on ajoute 4 g de BOP puis on laisse sous agitation 4 heures à TA et on concentre à sec. On extrait à l'éther, lave à l'eau par une solution saturée de $NaHCO_3$ puis une solution saturée de NaCl. On sèche sur sulfate de sodium puis on chromatographie sur silice en éluant par DCM/MeOH (95/5 ; v/v) + 3 ml de $NH_4OH$ par litre d'éluant. On obtient 1,924 g du composé attendu.
RMN (DMSO + TFA) : 0,8-1,2 ppm : mt : 12H; 2,3-3,0 ppm : mt : 11H ; 3,3 ppm : m : 2H ; 4,0 ppm : mt : 1H ; 4,2 ppm : s : 2H ; 4,4 ppm : mt : 1H ; 7,0-7,4 ppm : mt : 9H.

Préparation 3.2

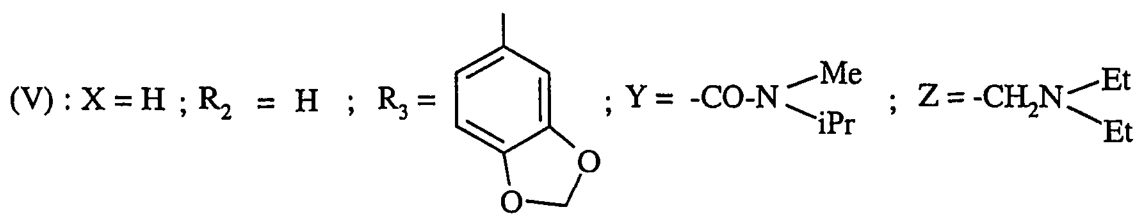

A)

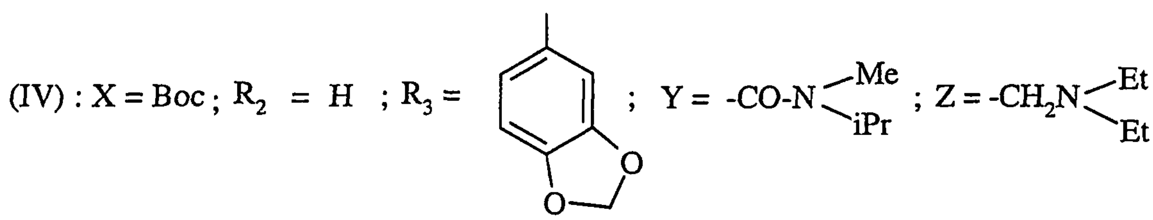

On place 1,11 g d'acide 3-(N-Boc)amino-3-(benzo[1,3]dioxol-5-ylpropionique, 1,508 g de 2-amino-3-(4-(diéthylaminoéthyl)phényl)-1-(pyrrolidin-1-yl)propan-1-one (préparation 2.8), 1,60 g de BOP et 1,50 ml de TEA dans 20 ml de DCM et on laisse sous agitation 2 heures à TA. Après concentration à sec, on reprend par AcOEt, lave à l'eau, par une solution saturée de $NaHCO_3$, puis une solution saturée de NaCl. On sèche sur sulfate de sodium et concentre. Le résidu est trituré dans le pentane et on essore le précipité formé. On chromatographie sur silice en éluant par un mélange DCM/MeOH (95/5 ; v/v) + 4 ml de $NH_4OH$ par litre d'éluant. On obtient 1,402 g du composé attendu.

B)

On place 1,4 g du composé de l'étape précédente dans 15 ml de TFA et 20 ml. de DCM et on laisse sous agitation 2 heures à TA. On concentre à sec puis reprend par du DCM, lave par une solution de NaOH 1N. On sèche sur sulfate de sodium et concentre. L'huile obtenue cristallise au réfrigérateur.
RMN : 0,8-1,1 ppm : mt : 12H; 2,2-2,6 ppm: mt : 9H ; 2,8 ppm : mt : 2H ; 3,5 ppm : s : 2H ; 4,1 ppm : t : 1H ; 4,5-5,0 ppm : mt : 2H ; 6,0 ppm : s : 2H ; 6,8-7,3 ppm : mt : 7H; 8,5 ppm : mt : 1H.
En opérant comme décrit dans les préparations 1, 2, 3 ci-dessus, on prépare les composés de formule (IV), (V) ou (VII) rassemblés ci-après :

TABLEAU VII

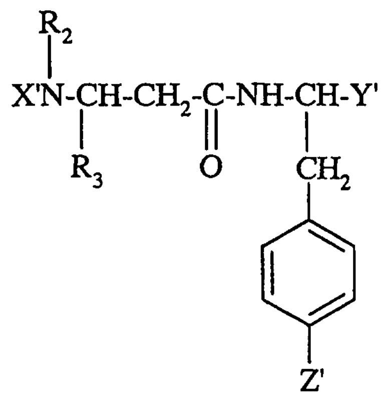

(IV) ou (V) ou (VII) ou (IX)

| Préparations | X' | $R_2$ | $R_3$ | Y' | Z' |
|---|---|---|---|---|---|
| 3.3<br>2HCl | H | H | 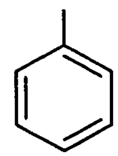 | 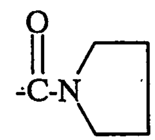 | 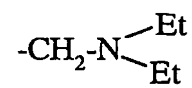 |
| 3.4 | 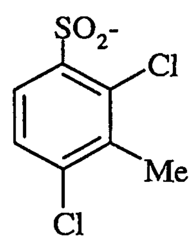 | 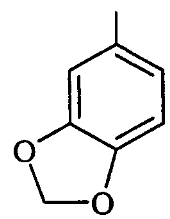 | H | 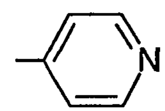 | CHO |

(suite)

| Préparations | X' | $R_2$ | $R_3$ | Y' | Z' |
|---|---|---|---|---|---|
| 3.5 | 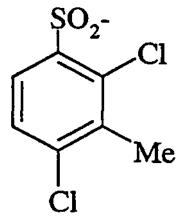 | 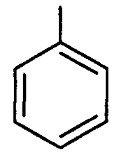 | H | 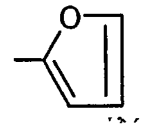 | CHO |
| 3.6 | 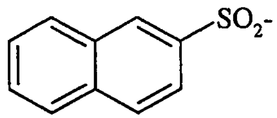 | H | 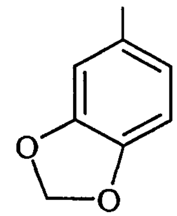 | $CO_2H$ | 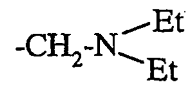 |
| 3.7 | 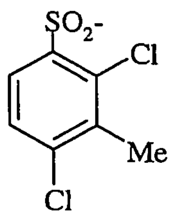 | 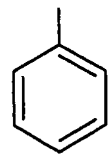 | H | 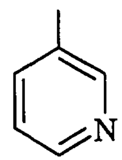 | CHO |

Préparation 3.3 : RMN (DMSO + TFA) : 1,2 ppm : mt : 6H ; 1,6 ppm : m : 4H ; 2,4-3,2 ppm : mt : 12H ; 4,2 ppm : s : 2H; 4,5 ppm : mt : 2H; 7,0-7,5 ppm: mt: 9H.
Préparation 3.4 : RMN : 2,2 ppm : t : 2H ; 2,4 ppm : s : 3H ; 2,8-3,1 : mt : 2H ; 3,7 ppm : t : 2H ; 5,0 ppm : m : 1H ; 5,9 ppm : s : 2H ; 6,4 : dd : 1H ; 6,6 ppm : d : 1H; 6,7 ppm : d : 1H ; 7,1-7,5 ppm : mt : 6H ; 7,7 ppm : d : 2H ; 8,4 ppm : mt : 3H ; 9,9 ppm : s : 1H.
Préparation 3.5 : RMN : 2,2 ppm : t : 2H ; 2,4 ppm : s : 3H ; 2,8-3,1 ppm : mt : 2H ; 3,8 ppm : t : 2H ; 5,0 ppm : mt : 1H ; 6,1 : d : 1H; 6,3 ppm : mt : 1H; 7,0-7,6 ppm : mt : 10H ; 7,7 ppm : d : 2H ; 8,3 ppm : d : 1H ; 9,9 ppm : s : 1H.
Préparation 3.7 : RMN : 2,2 ppm : t : 2H ; 2,4 ppm : s : 3H ; 2,7-3,1 ppm : mt : 2H ; 3,8 ppm : t : 2H ; 5,0 ppm : mt : 1H ; .7,0-7,8 ppm : mt : 13H ; 8,3-8,5 ppm : mt : 3H ; 9,9 ppm : s : 1H.

Préparation 3.8

(V), (R,R) : $R_2$ = H ; $R_3$ = 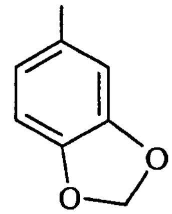 ;

Y = 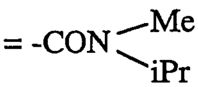 ; Z = 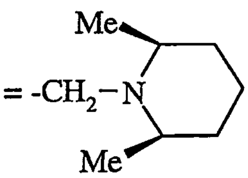

A)

(IV) (R,R) : X = Boc ; $R_2$ = H ; $R_3$ = 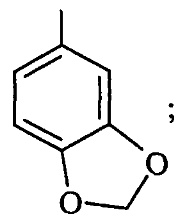 ;

Y = 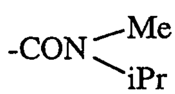 ; Z = 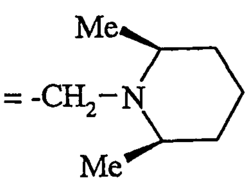

On laisse sous agitation à TA pendant 3 heures un mélange contenant 480 mg du composé de la préparation 2.41, 259 mg du composé de la préparation 1.13, 370 mg de BOP et 440 µl de DIPEA dans 10 ml de DCM. On concentre à sec le milieu puis on reprend par AcOEt, lave à l'eau, extrait au tampon pH = 2 puis basifie la phase aqueuse à pH = 8 par une solution saturée de $NaHCO_3$. On extrait au DCM puis lave par une solution saturée de NaCl pour obtenir 0,437 g du composé attendu.
RMN : 0,5 à 1,6 ppm : m : 27H ; 2,2 à 3,6 ppm : m : 10H ; 3,8 à 5 ppm : m : 4H ; 5,9 ppm : d : 2H ; 6,6 à 7,3 ppm : m : 8H ; 8,1 ppm : t : 1H.

B) On laisse sous agitation 4 heures à TA 437 mg du composé obtenu à l'étape précédente et 10 ml de TFA dans 20 ml de DCM. On concentre à sec puis on cristallise dans un mélange éther éthylique/pentane. Le résidu est repris dans un mélange DCM/solution saturée/$NaHCO_3$ ; la phase organique est séchée et concentrée sous vide à sec pour obtenir 343 mg du composé attendu.

Préparation 3.9

(IV), (R,R) : X = Boc : $R_2$ = H ; $R_3$ = 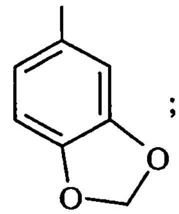 ; Y = 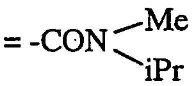 ;

Z = $-CH_2-$ 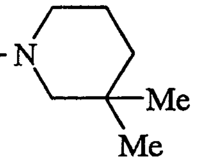

On place 163 mg du composé obtenu à la préparation 2.42 dans 5 ml de DCM avec 3 ml de TFA et on laisse 1 heure sous agitation à TA. On concentre le milieu puis on triture dans $iPr_2O$ et décante pour recueillir l'huile formée. L'huile formée est reprise dans 10 ml de DCM puis on ajoute 115 mg du composé de la préparation 1.13, 170 mg de BOP et on maintient à pH = 7 par addition de DIPEA. Après traitement habituel, on obtient 0,225 g du composé attendu, utilisé brut à l'étape suivante.

Préparation 3.10

(V), TFA (R,R) : $R_2$ = H ; $R_3$ = 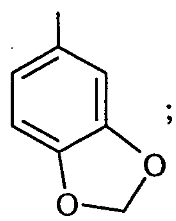 ; Y = 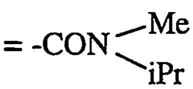 ;

Z = $-CH_2-$ 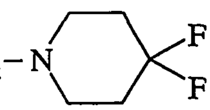

Ce composé est préparé selon les méthodes décrites ci-dessus à partir du composé de la préparation 2.43 et du composé de la préparation 1.13.

EXEMPLE 1

Chlorhydrate de N-[1-(4-aminométhylbenzyl)-2-oxo-2-(pyrrolidin-1-yl)éthyl]-3-(naphtalène-2-sulfonylamino)-3-phénylpropionamide.

(I), HCl : $R_1$ = 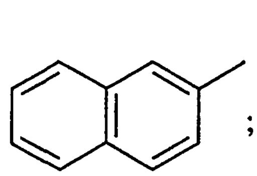 ; $R_2$ = H ; $R_3$ = 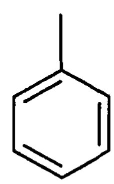 ;

$R_4$ = -CO- 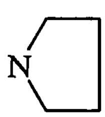 ; $R_5$ = $-CH_2NH_2$

A) N-[1-(4-cyanobenzyl)-2-oxo-2-(pyrrolidin-1-yl)éthyl]-3-(naphtalène-2-sulfonylamino)-3-phénylpropionamide

A un mélange de 0,715 g du composé de la préparation 2.1 dans 15 ml d'acétonitrile, on ajoute 0,25 ml de triéthylamine puis 0,71 g du composé de la préparation 1.1 et 0,45 g de DCC et laisse 5 heures sous agitation à TA. On concentre sous vide le mélange réactionnel, reprend le résidu dans l'acétone, filtre la DCU et concentre sous vide le filtrat. On triture le résidu dans l'éther puis décante le solvant (plusieurs fois). On obtient 0,61 g du produit attendu après séchage sous vide sur $P_2O_5$, F = 195 - 200° C.
RMN : δ (ppm) : 1,40-1,70 : m : 4H ; 2,30-3,40 : m : 8H ; 4,40-4,60 : m : et 4,60-4,70 : m : 2H ; 6,75-8,15 : m : 16H.

B) Chlorhydrate de N-[1-(4-aminométhylbenzyl)-2-oxo-2-(pyrrolidin-1-yl)éthyl]-3-(naphtalène-2-sulfonylamino)-3-phénylpropionamide.

On dissout 1 g du composé obtenu à l'étape précédente dans 4,5 ml d'ammoniaque concentrée, 20 ml de MeOH, 5 ml de dioxane et 20 ml de toluène, ajoute 0,9 g de nickel de Raney® et hydrogène à 50°C et sous une pression de 50 bars pendant 7 heures. On filtre le catalyseur sur Célite®, lave par une solution MeOH/chloroforme et concentre sous vide le filtrat. On reprend le résidu à l'éther et essore le précipité formé. On reprend le produit obtenu dans de l'éther chlorhydrique, essore le précipité formé et le sèche. On obtient 0,85 g du produit attendu.
$MH^+$ = 585

EXEMPLE 2

Chlorhydrate de N-[1-[4-(*tert*-butylaminométhyl)benzyl]-2-oxo-2-(pyrrolidin-1-yl)éthyl]-3-(napthtalène-2-sulfonylamino)-3-phénylpropionamide.

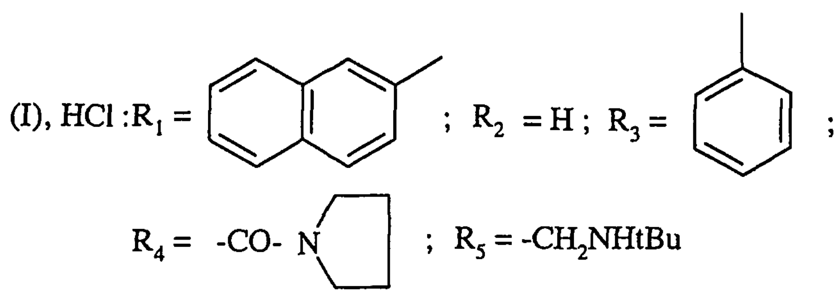

A un mélange de 1,06 g du composé obtenu à la Préparation 1.1 dans 15 ml de DMF, on ajoute 1,46 ml de DIPEA puis 1,49 g du composé obtenu à la Préparation 2.2 et 1,38 g de BOP et laisse 2 heures 30 minutes sous agitation à TA en maintenant le pH = 6 par ajout de DIPEA. On extrait le mélange réactionnel à l'AcOEt, lave la phase organique à l'eau, par NaOH 0,2N, à l'eau, par une solution saturée de NaCl, sèche sur $Na_2SO_4$ et évapore sous vide le solvant. On chromatographie le résidu sur gel de silice en éluant par le mélange chloroforme/MeOH/ $NH_4OH$ (85/15/0,2 ; v/v/v). On dissout le produit obtenu dans 10 ml de MeOH, ajoute 0,2 ml d'HCl 10N et concentre sous vide. On chromatographie le résidu sur Séphadex® LH20 en éluant par le mélange DCM/MeOH (60/40 ; v/v). On obtient 0,66 g du produit attendu.
RMN : δ (ppm) : 1,3 : s : 9H ; 1,5 - 1,9 : m : 4H ; 2,3 - 3,4 : m : 8H ; 3,95 : d : 2H ; 4,4 - 4,9 : m : 2H; 6,8 - 8,6 : m : 18H.

EXEMPLE 3

N-[1-[4-(N-propyl-N-méthylaminométhyl)benzyl]-2-oxo-2-(pyrrolidin-1-yl)éthyl]-3-(naphtalène-2-sulfonylamino)-3-phénylpropionamide.

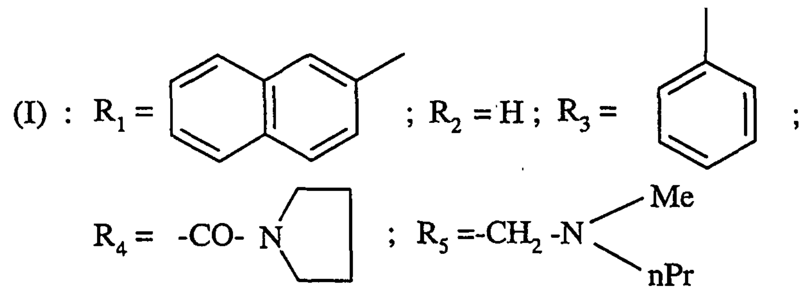

A un mélange de 0,39 g du composé obtenu à la Préparation 1.1 et 0,41 g du composé obtenu à la Préparation 2.3 dans 10 ml de DCM, on ajoute 0,458 ml de triéthylamine puis 0,533 g de BOP et laisse 4 heures sous agitation à TA. On concentre sous vide le mélange réactionnel, extrait le résidu à l'AcOEt, lave la phase organique à l'eau, par une solution saturée de NaCl, par une solution saturée de $NaHCO_3$ par une solution saturée de NaCl, sèche sur $Na_2SO_4$ et évapore sous vide le solvant. On obtient 0,3 g du produit attendu après cristallisation dans l'éther.
RMN : δ (ppm) : 0,75 t : 3H ; 1,2-1,7 : m : 6H ; 2,0 : s : 3H ; 2,15 : t : 2H ; 2,25-3,5 : m : 10H ; 4,1 à 4,8 : m : 2H ; 6,7 à 8,5 : m : 18H.

EXEMPLE 4

N-[1-[4-[N,N-bis(2-hydroxyéthyl)aminométhyl]benzyl]-2-oxo-2-(pyrrolidin-1-yl)éthyl]-3-(naphtalène-2-sulfonylamino)-3-phénylpropionamide.

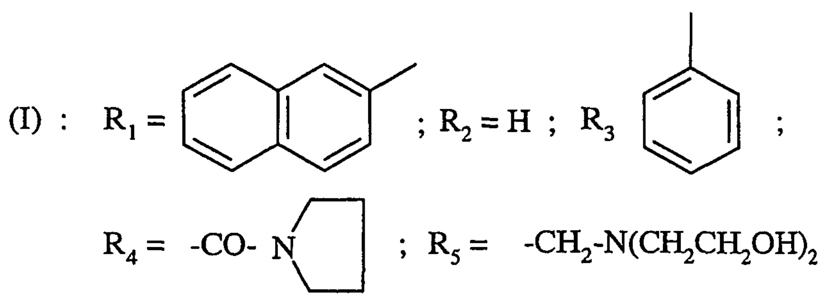

A un mélange du composé obtenu à la Préparation 2.4 dans 15 ml de DMF, on ajoute 1,3 ml de DIPEA puis 0,96 g du composé obtenu à la Préparation 1.1 et 1,33 g de BOP et laisse 2 heures sous agitation à TA en maintenant le pH = 6 par ajout de DIPEA. On extrait le mélange réactionnel à l'AcOEt, lave la phase organique à l'eau, par NaOH 0,5N, à l'eau, par une solution saturée de NaCl, sèche sur $Na_2SO_4$ et évapore sous vide le solvant. On chromatographie le résidu sur gel de silice en éluant par le mélange DCM/MeOH/ $NH_4OH$ (90/10/0,2 ; v/v/v). On chromatographie le produit obtenu sur Séphadex® LH 20 en éluant par le mélange DCM/MeOH (60/40; v/v). On obtient 0,44 g de produit attendu.
RMN : δ (ppm) : 1,3 à 1,7 : m : 4H ; 2,2 à 3,6 : m : 18H ; 4,2 à 4,8 : m : 4H ; 6,8 à 8,4 : m : 18H.

EXEMPLE 5

N-[1,2-bis[4-(diéthylaminométhyl)phényl]éthyl]-3-(naphtalène-2-sulfonylamino)-3-phénylpropionamide.

(I) : $R_1$ = 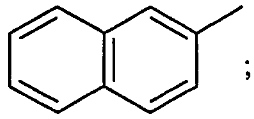 ; $R_2$ = H ; $R_3$ = 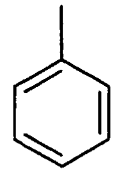 ;

$R_4$ = 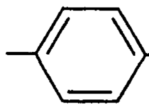 $CH_2$-N-$(Et)_2$ ; $R_5$ = $-CH_2-N(Et)_2$

A un mélange de 0,82 g du composé obtenu à la Préparation 2.5 dans 10 ml de DMF, on ajoute 0,8 ml de DIPEA puis 0,39 g du composé obtenu à la Préparation 1.1 et 0,53 g de BOP et laisse 1 heure 30 minutes sous agitation à TA en maintenant le pH = 6 par ajout de DIPEA. On extrait le mélange réactionnel à l'AcOEt, lave la phase organique à l'eau, par NaOH 0,1N, à l'eau, par une solution saturée de NaCl, sèche sur $Na_2SO_4$ et évapore sous vide le solvant. On chromatographie le résidu sur Séphadex® LH 20 en éluant par le mélange DCM/MeOH (60/40 ; v/v). On obtient 0,32 g du produit attendu.
RMN : δ (ppm) : 0,85 : t : 12H ; 2,1 à 3,6 : m : 16H ; 4,4 à 4,9 : m : 2H ; 6,6 à 8,4 : m : 22H.

EXEMPLE 6

N-[1-[4-(diéthylaminométhyl)benzyl]-2-oxo-2-(pyrrolidin-1-yl)éthyl]-3-[méthyl-(naphtalène-2-sulfonyl)amino]-3-phényl-propionamide.

(I) : $R_1$ = 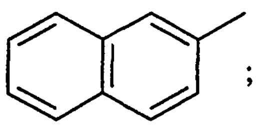 ; $R_2$ = Me ; $R_3$ = 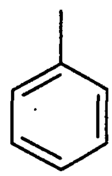 ;

$R_4$ = -CO- 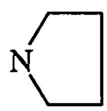 ; $R_5$ = $-CH_2-N(Et)_2$

A un mélange de 0,72 g du composé obtenu à la Préparation 2.7 dans 6 ml de DMF, on ajoute 1 ml de DIPEA, puis 0,51 g du composé obtenu à la Préparation 1.2 et 0,67 g de BOP et laisse 3 heures sous agitation à TA en maintenant le pH = 6 par ajout de DIPEA. On extrait le mélange réactionnel à l'AcOEt, lave la phase organique à l'eau, par NaOH 0,5N, à l'eau, par une solution saturée de NaCl, sèche sur $Na_2SO_4$ et évapore sous vide le solvant. On chromatographie le résidu sur gel de silice en éluant par le mélange DCM/MeOH/$NH_4OH$ (90/10/0,2 ; v/v/v). On obtient 0,27 g du produit attendu.
RMN : δ (ppm) : 0,95 : t : 6H ; 1,35 à 1,8 : m : 4H ; 2,1 à 3,6 : m : 12H ; 4,0 à 4,6 : m : 3H ; 5,55 : mt : 1H ; 6,8 à 8,6 : m : 17H.

EXEMPLE 7

Chlorhydrate de N-[2-[4-(diéthylaminométhyl)phényl)-1-(N-isopropyl-N-méthylcarbamoyl)éthyl]-3-(3,4-diméthylphényl)-3-(naphtalène-2-sulfonylamino) propionamide.

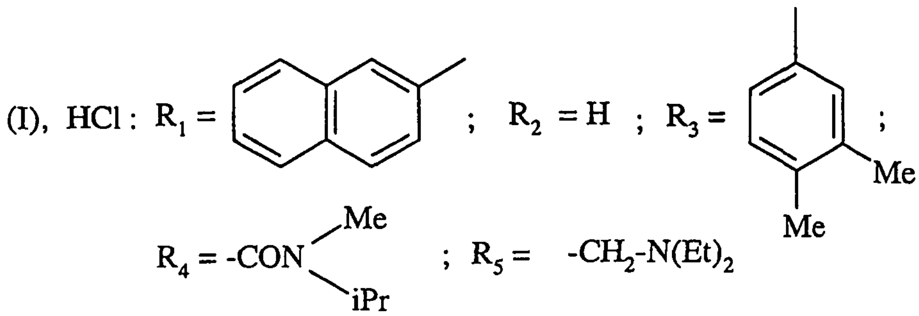

(I), HCl : $R_1$ = ; $R_2$ = H ; $R_3$ = ;

$R_4$ = -CON ; $R_5$ = $-CH_2-N(Et)_2$

A un mélange de 0,288 g du composé obtenu à la Préparation 1.4 et 0,4 g du composé obtenu à la Préparation 2.8 dans 15 ml de DCM et 3 ml de DMF, on ajoute 0,312 ml de triéthylamine puis 0,332 g de BOP et laisse 2 heures sous agitation à TA. On dilue le mélange réactionnel au DCM, lave la phase organique par NaOH 1N, à l'eau et concentre sous vide. On extrait le résidu à l'AcOEt, lave la phase organique à l'eau, par une solution saturée de $NaHCO_3$, par une solution saturée de NaCl, sèche sur $Na_2SO_4$ et évapore sous vide le solvant. On dissout le résidu dans l'acétonitrile, acidifie à pH = 1 par ajout d'éther chlorhydrique, dilue à l'éther et essore le précipité formé. On obtient 0,263 g du produit attendu.
RMN : δ (ppm): 0,6-1,3 : m : 12H ; 1,6-1,8 : m : 6H ; 2,3-3,2: m : 11H ; 3,7-4,9 : m : 5H ; 6,4-8,4 : m : 16H.

EXEMPLE 8

Chlorhydrate de N-[2-[4-(diéthylaminométhyl)phényl]-1-(N-isopropyl-N-méthylcarbamoyl)éthyl]-3-(benzo[1,3]dioxol-5-yl)-3-(naphtalène-2-sulfonylamino) propionamide.

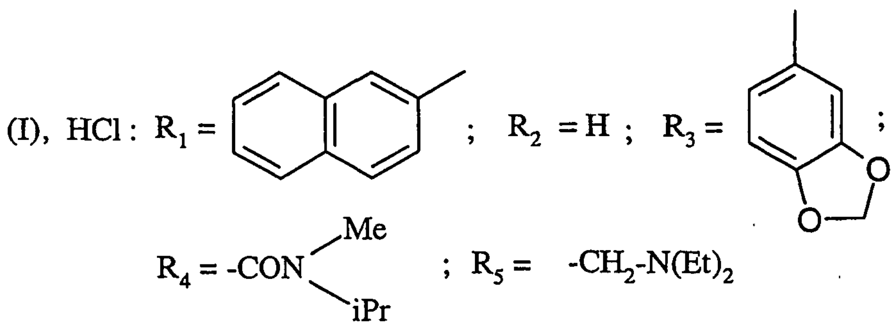

(I), HCl : $R_1$ = ; $R_2$ = H ; $R_3$ = ;

$R_4$ = -CON ; $R_5$ = $-CH_2-N(Et)_2$

A un mélange de 0,95 g du composé obtenu à la Préparation 1.7 et 1,27 g du composé obtenu à la Préparation 2.8 dans 10 ml de DCM, on ajoute 0,996 ml de triéthylamine et 1,16 g de BOP et laisse 3 heures sous agitation à TA. On concentre sous vide le mélange réactionnel, extrait le résidu à l'AcOEt, lave la phase organique à l'eau, par une solution saturée de $NaHCO_3$ par une solution saturée de NaCl, sèche sur $Na_2SO_4$ et évapore sous vide le solvant. On chromatographie le résidu sur gel de silice en éluant par le mélange DCM/MeOH (93/7 ; v/v). On dissout le produit obtenu dans AcOEt, acidifie à pH = 1 par ajout d'éther chlorhydrique et concentre sous vide. On obtient 0,78 g du produit attendu après cristallisation dans le mélange AcOEt/éther iso.
RMN : δ (ppm) : 0,6 à 1,4 : m : 12H ; 2,2 à 3,2 : m : 11H ; 3,7 à 5,0 : m : 5H ; 5,2 à 5,8 : m : 2H ; 6,2 à 8,5 : m : 16H ; 10,4 : s : 1H.

EXEMPLE 9

Chlorhydrate de N-[1-[4-(diéthylaminométhyl)benzyl]-2-oxo-2-(pyrrolidin-1-yl)éthyl]-3-(2,3-dihydrobenzo[1,4]dioxin-6-yl)-3-(naphtalène-2-sulfonylamino) propionamide.

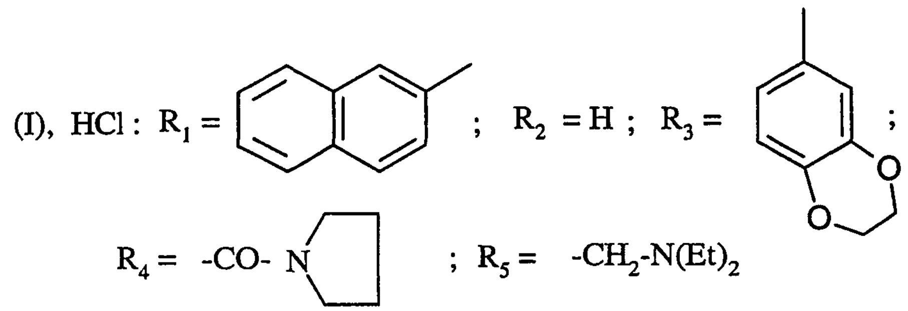

A un mélange de 0,494 g du composé obtenu à la Préparation 1.8 et 0,450 g du composé obtenu à la Préparation 2.7 dans 10 ml de DCM, on ajoute 0,497 ml de triéthylamine et 0,529 g de BOP et laisse 2 heures sous agitation à TA. On concentre sous vide le mélange réactionnel, extrait le résidu au DCM, lave la phase organique à l'eau, par NaOH 1N, à l'eau, sèche sur $Na_2SO_4$ et évapore sous vide le solvant. On dissout le produit obtenu dans un mélange propan-2-ol/acétonitrile, acidifie à pH = 1 par ajout d'éther chlorhydrique, dilue à l'éther et essore le précipité formé. On obtient 0,280 g du produit attendu.
RMN : δ (ppm) : 1,2 : mt : 6H ; 1,4 à 1,8 : m : 12H ; 3,6 à 5,0 : m : 8H ; 6,1 à 8,4 : m : 16H ; 10,5 : s : 1H.

EXEMPLE 10

Chlorhydrate de N-[2-[4-(diéthylaminométhyl)phényl]-1-(N-isopropyl-N-méthylcarbamoyl)éthyl]-3-(2,3-dihydrobenzo [1,4]dioxin-6-yl)-3-(5,6,7,8-tétrahydro naphtalène-2-sulfonylamino) propionamide.

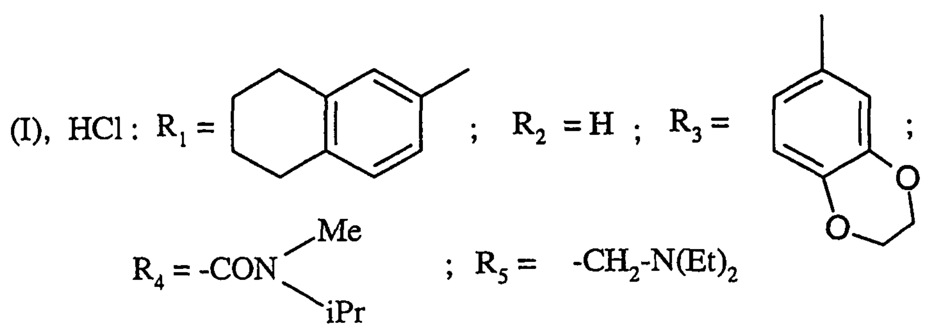

A un mélange de 0,312 g du composé obtenu à la Préparation 1.9 et 0,4 g du composé obtenu à la Préparation 2.8 dans 15 ml de DCM et 3 ml de DMF, on ajoute 0,312 ml de triéthylamine puis 0,332 g de BOP et laisse 2 heures sous agitation à TA. On concentre sous vide le mélange réactionnel, extrait le résidu à l'AcOEt, lave la phase organique par une solution tampon pH = 4, à l'eau, par une solution saturée de $NaHCO_3$, sèche sur $Na_2SO_4$ et évapore sous vide le solvant. On dissout le produit obtenu dans AcOEt, acidifie à pH = 1 par ajout d'éther chlorhydrique, dilue à l'éther et essore le précipité formé. On obtient 0,24 g du produit attendu.
RMN : δ (ppm) : 0,6-1,0 : m : 6H ; 1,05-1,35 : m : 6H ; 1,4 à 1,8 : m : 4H ; 2,2 à 3,1 : m : 15H ; 3,9-5,0 : m : 9H ; 6,2 à 8,4 : m : 12H.

EXEMPLE 11

Hexafluorophosphate de N-[1-[4-(diéthylaminométhyl)benzyl]-2-oxo-2-(pyrrolidin-1-yl)éthyl]-3-[(2,4-dichloro-3-méthyl-benzènesulfonyl)phénylamino] propionamide.

(I), $HPF_6$ : $R_1$ = 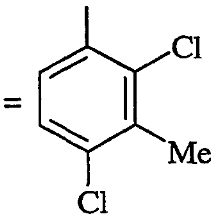 ; $R_2$ = 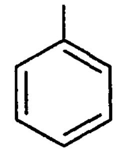 ; $R_3$ = H ;

$R_4$ = -CO- 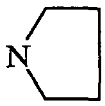 ; $R_5$ = $-CH_2-N(Et)_2$

On prépare ce composé selon le mode opératoire décrit à l'EXEMPLE 3 à partir de 0,46 g du composé obtenu à la Préparation 2.7 et 0,475 g du composé obtenu à la Préparation 1.10 dans 10 ml de DCM et 0,511 ml de triéthylamine puis 0,595 g de BOP. On obtient 0,35 g du produit attendu après cristallisation dans l'éther, F = 204-208°C.
RMN : δ (ppm) : 1,05 : t : 6H ; 1,4 à 1,8 : m : 4H ; 2,2 : t : 2H ; 2,4 : s : 3H ; 2,5 à 3,5: m: 10 H; 3,6 à 4,6: m: 5H; 6,8 à 7,7 : m: 11H; 8,3 : d: 1H ; 10,4 : se : 1H.

EXEMPLE 12

Dichlorhydrate de N-[2-[4-(diéthylaminométhyl)phényl]-1-(pyrid-3-yl)éthyl]-3-[(2,4-dichloro-3-méthylbenzènesulfonyl) phénylamino] propionamide.

(I), 2HCl : $R_1$ = 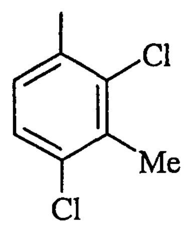 ; $R_2$ = 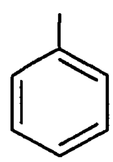 ; $R_3$ = H ;

$R_4$ = 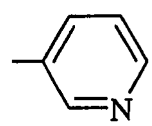 ; $R_5$ = $-CH_2-N(Et)_2$

On prépare ce composé selon le mode opératoire décrit à l'EXEMPLE 8 à partir du composé obtenu brut à la Préparation 2.10, 0,454 g du composé obtenu à la Préparation 1.10 dans 20 ml de DCM, 0,326 ml de triéthylamine et 0,518 g de BOP. On chromatographie le produit obtenu sur gel de silice en éluant par le mélange DCM/MeOH/$NH_4OH$ 2 % (95/5/0,5 ; v/v/v). On dissout le produit obtenu dans AcOEt, acidifie à pH = 1 par ajout d'éther chlorhydrique et concentre sous vide. On obtient 0,5 g du produit attendu après cristallisation dans le mélange AcOEt/éther iso, F = 155°C (décomposition).
RMN : δ (ppm) : 1,1 : t : 6H ; 2,2 : mt : 2H ; 2,4 : s : 3H ; 2,7 à 3,2 : m : 6H ; 3,8 : t : 2H ; 4,1 : de : 2H ; 5,1 : qd : 1H ; 6,8 à 9,0 : m : 16H ; 10,8 : s : 1H ;

EXEMPLE 13

N-[1-[4-(N-éthyl-N-méthylaminométhyl)benzyl]-2-oxo-2-(pyrrolidin-1-yl)éthyl]-3-(naphtalène-2-sulfonylamino)-3-phénylpropionamide.

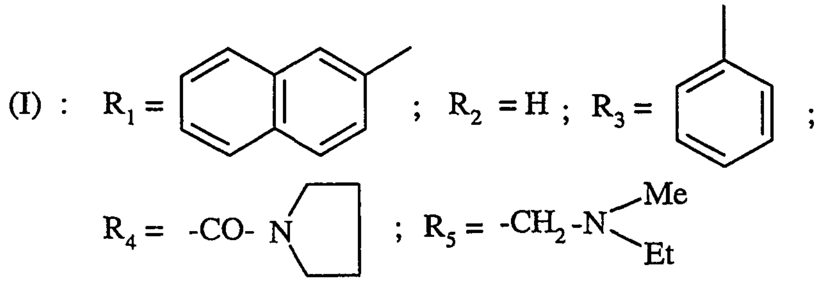

(I) : $R_1$ = ; $R_2$ = H ; $R_3$ = ;

$R_4$ = -CO- N ; $R_5$ = $-CH_2$-N Me Et

A) 3-[4-(N-éthyl-N-méthylaminométhyl)phényl]-2-[3-(naphtalène-2-sulfonylamino)-3-phénylpropionylamino]propionate d'éthyle.

A un mélange de 0,578 g du composé obtenu à la Préparation 1.1 et 0,430 g du composé obtenu à la Préparation 2.11 dans 10 ml de DCM, on ajoute 0,791 g de BOP et 0,226 ml de triéthylamine et laisse 3 heures sous agitation à TA. On concentre sous vide le mélange réactionnel, extrait le résidu à l'AcOEt, lave la phase organique à l'eau, par une solution saturée de $NaHCO_3$ par une solution saturée de NaCl, sèche sur $Na_2SO_4$ et évapore sous vide le solvant. On chromatographie le résidu sur gel de silice en éluant par le mélange DCM/MeOH (100/4 ; v/v). On obtient 0,5 g du produit attendu.

B) Acide 3-[4-(N-éthyl-N-méthylaminométhyl)phényl]-2-[3-(naphtalène-2-sulfonylamino)-3-phénylpropionylamino]propionique.

A un mélange de 0,5 g du composé obtenu à l'étape précédente dans 10 ml d'EtOH et 10 ml de dioxane, on ajoute 1,7 ml de KOH 1N et chauffe à 60°C pendant 4 heures. Après refroidissement à TA, on ajoute 1,7 ml d'HCl 1N et concentre sous vide. On reprend le résidu à l'EtOH et reconcentre sous vide. On obtient le produit attendu que l'on utilise tel quel.

C) N-[1-[4-(N-éthyl-N-méthylaminométhyl)benzyl]-2-oxo-2-(pyrrolidin-1-yl)éthyl]-3-(naphtalène-2-sulfonylamino)-3-phénylpropionamide.

A un mélange du composé obtenu à l'étape précédente et 0,07 ml de pyrrolidine dans 10 ml de DMF, on ajoute 0,403 g de BOP et 0,115 ml de triéthylamine et laisse 2 heures sous agitation à TA. On verse le mélange réactionnel dans l'eau, extrait à l'AcOEt, lave la phase organique à l'eau, par une solution saturée de $NaHCO_3$, par une solution saturée de NaCl, sèche sur $Na_2SO_4$ et évapore sous vide le solvant. On obtient 0,4 g du produit attendu après cristallisation dans l'éther iso.
RMN : δ (ppm) : 1,1 : t : 3H ; 1,3 à 1,85 : m : 4H ; 2,1 : s : 3H ; 2,2 à 3,5 : m : 12H ; 4,2 à 5,0 : m : 2H ; 6,8 à 8,6 : m : 18H.

EXEMPLE 14

N-[1-[4-(diéthylaminométhyl)benzyl]-2-oxo-2-(pyrrolidin-1-yl)éthyl]-3-(naphtalène-2-sulfonylamino)-3-phénylpropionamide.

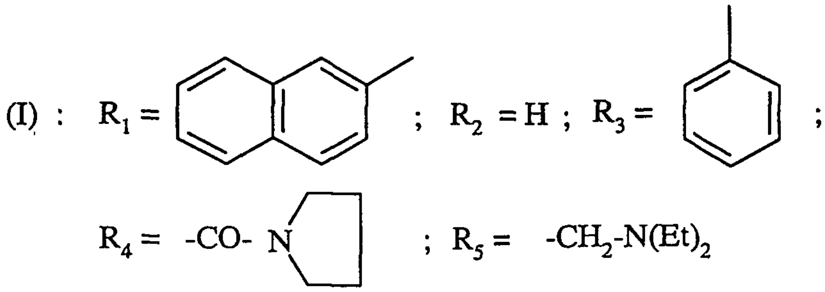

(I) : $R_1$ = ; $R_2$ = H ; $R_3$ = ;

$R_4$ = -CO- N ; $R_5$ = $-CH_2-N(Et)_2$

A) 3-[4-(Diéthylaminométhyl)phényl]-2-[3-(naphtalène-2-sulfonylamino)-3-phénylpropionylamino]propionate d'éthyle.

On laisse 4 heures sous agitation à TA un mélange de 0,599 g du composé obtenu à la Préparation 1.11, 0,368 g du composé obtenu à l'étape B de la Préparation 2.7 et 0,184 ml de triéthylamine dans 10 ml de DMF. Après concentration sous vide du mélange réactionnel, on extrait le résidu à l'AcOEt, lave la phase organique à l'eau, par une solution saturée de $NaHCO_3$ sèche sur $Na_2SO_4$ et évapore sous vide le solvant. On obtient 0,715 g du produit attendu.

B) Acide 3-[4-(diéthylaminométhyl)phényl]-2-[3-(naphtalène-2-sulfonylamino)-3-phénylpropionylamino]propionique.

A un mélange de 0,715 g du composé obtenu à l'étape précédente dans 10 ml d'EtOH et 10 ml de dioxane, on ajoute 1,75 ml de KOH 1N et chauffe une nuit à 60°C. On ajoute 1,75 ml d'HCl 1N et concentre sous vide. On obtient le produit attendu que l'on utilise tel quel.

C) N-[1-[4-(diéthylaminométhyl)benzyl]-2-oxo-2-(pyrrolidin-1-yl)éthyl]-3-(naphtalène-2-sulfonylamino)-3-phénylpropionamide.

A un mélange du composé obtenu à l'étape précédente et 0,098 ml de pyrrolidine dans 10 ml de DMF, on ajoute 0,161 ml de triéthylamine et 0,515 g de BOP et laisse 2 heures sous agitation à TA. On verse le mélange réactionnel dans l'eau, extrait à l'AcOEt, lave la phase organique par une solution saturée de $NaHCO_3$, par une solution saturée de NaCl, sèche sur $Na_2SO_4$ et évapore sous vide le solvant. On obtient 0,618 g du produit attendu.
RMN : δ (ppm) : 0,8 à 1,0 : t : 6H ; 1,3 à 1,7 : m : 4H ; 2,2 à 3,5 : m : 14H ; 4,1 à 4,8 : m : 2H ; 6,7 à 8,4 : m : 18H.

EXEMPLE 15

Chlorhydrate de N-[2-[4-(diéthylaminométhyl)phényl]-1-(N-éthyl-N-isopropyl carbamoyl)éthyl]-3-(benzo[1,3]dioxol-5-yl)-3-(naphtalène-2-sulfonylamino) propionamide.

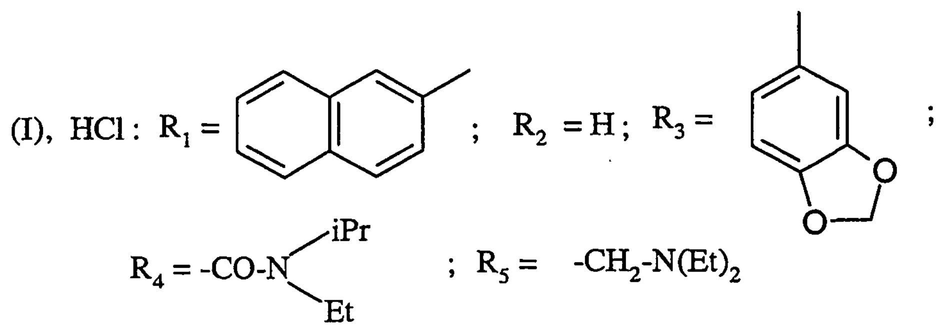

A) 3-[4-(Diéthylaminométhyl)phényl]-2-[3-(naphtalène-2-sulfonylamino)-3-(benzo [1,3]dioxol-5-yl)propionylamino]propionate d'éthyle.

A un mélange de 0,589 g du composé obtenu à la Préparation 1.7 et 0,410 g du composé obtenu à l'étape B de la Préparation 2.7 dans 10 ml de DMF, on ajoute 0,652 g de BOP et 0,205 ml de triéthylamine et laisse 2 heures sous agitation à TA. On verse le mélange réactionnel dans un mélange AcOEt/$NaHCO_3$ saturée, lave la phase organique à l'eau par une solution saturée de NaCl" sèche sur $Na_2SO_4$ et évapore sous vide le solvant. On obtient 0,839 g du produit attendu.

B) Acide 3-[4-(diéthylaminométhyl)phényl]-2-[3-(naphtalène-2-sulfonylamino)-3-(benzo[1,3]dioxol-5-yl)propionylamino] propionique.

A un mélange de 0,83 g du composé obtenu à l'étape précédente dans 7 ml de MeOH, 7 ml d'EtOH et 7 ml de dioxane, on ajoute 1,9 ml de KOH 1N et laisse 4 heures sous agitation à TA. On ajoute 1,9 ml de HCl 1N et concentre sous vide. On reprend le résidu par le mélange EtOH/MeOH (50/50 ; v/v), concentre sous vide, reprend au toluène et concentre sous vide à nouveau. On obtient 1,204 g du produit attendu.

C) Chlorhydrate de N-[2-[4-(diéthylaminométhyl)phényl]-1-(N-éthyl-N-isopropyl carbamoyl)éthyl]-3-(benzo[1,3]dioxol-5-yl)-3-(naphtalène-2-sulfonylamino) propionamide.

A un mélange de 0,6 g du composé obtenu à l'étape précédente et 0,126 ml de N-éthylisopropylamine dans 15 ml de DCM, on ajoute 0,46 g d'hexafluorophosphate de bromotripyrrolidinophosphonium et 0,33 ml de DIPEA et laisse 24 heures sous agitation à TA. On concentre sous vide, extrait le résidu à l'AcOEt, lave la phase organique à l'eau, par une solution saturée de $NaHCO_3$, par une solution saturée de NaCl, extrait la phase organique par une solution tampon pH = 4, alcalinise la phase aqueuse par ajout d'une solution saturée de $NaHCO_3$, extrait au DCM, lave la phase organique par une solution saturée de $NaHCO_3$, sèche sur $Na_2SO_4$ et évapore sous vide le solvant. On chromatographie le résidu sur gel de silice en éluant par le mélange DCM/MeOH (95/5 ; v/v). On dissout le produit obtenu dans l'AcOEt, acidifie à pH = 1 par ajout d'éther chlorhydrique, dilue à l'éther et essore le précipité formé. On obtient 0,058 g du produit attendu.

RMN : δ (ppm) : 0,6-1,4 : m : 15H ; 2,2-3,3 : m : 10H ; 3,7-4,9 : m : 5H ; 5,3-5,8 : m : 2H ; 6,3- 8,5 : m : 16H ; 10,5 : s : 1H.

EXEMPLE 16

Dichlorhydrate de N-[1-[4-(diéthylaminométhyl)benzyl]-2-oxo-2-(pyrrolidin-1-yl)éthyl]-3-phényl-3-(quinoléine-2-sulfonylamino) propionamide.

(I), 2HCl : $R_1$ = 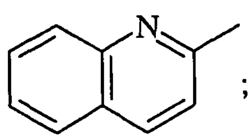 ; $R_2$ = H ; $R_3$ = 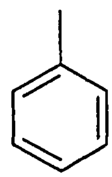 ;

$R_4$ = -CO- 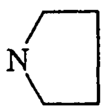 ; $R_5$ = $-CH_2-N(Et)_2$

A) 3-[4-(Diéthylaminométhyl)phényl]-2-[3-phényl-3-(quinoléine-2-sulfonyl amino)propionylamino]propionate d'éthyle.

A un mélange de 0,376 g du composé obtenu à la Préparation 1.12 et 0,421 g du composé obtenu à l'étape B de la Préparation 2.7 dans 10 ml de DMF, on ajoute 0,467 g de BOP puis 0,147 ml de triéthylamine et laisse 2 heures sous agitation à TA. On verse le mélange réactionnel dans une solution de $NaHCO_3$, extrait à l'AcOEt, lave par une solution saturée de $NaHCO_3$, extrait par HCl 1N, alcalinise la phase aqueuse acide par ajout de $NaHCO_3$ solide, extrait à l'AcOEt, lave la phase organique à l'eau, sèche sur $Na_2SO_4$ et évapore sous vide le solvant. On obtient 0,455 g du produit attendu.

B) Acide 3-[4-(diéthylaminométhyl)phényl]-2-[3-phényl-3-(quinoléine-2-sulfonyl amino)propionylamino]propionique.

A un mélange de 0,455 g du composé obtenu à l'étape précédente dans 20 ml d'EtOH et 5 ml de dioxane, on ajoute 1,63 ml de NaOH 1N et laisse une nuit sous agitation à TA. On ajoute 1,63 ml d'HCl 1N et concentre sous vide. On reprend le résidu au toluène et concentre à nouveau sous vide. On obtient le produit attendu que l'on utilise tel quel.

C) Dichlorhydrate de N-[1-[4-(diéthylaminométhyl)benzyl]-2-oxo-2-(pyrrolidin-1-yl)éthyl]-3-phényl-3-(quinoléine-2-sulfonylamino) propionamide.

A un mélange du composé obtenu à l'étape précédente et 0,063 ml de pyrrolidine dans 10 ml de DMF, on ajoute 0,103 ml de triéthylamine puis 0,327 g de BOP et laisse 2 heures sous agitation à TA. On verse le mélange réactionnel dans un mélange AcOEt/$NaHCO_3$ saturée, lave la phase organique par une solution saturée de NaCl, sèche sur $Na_2SO_4$ et évapore sous vide le solvant. On chromatographie le résidu sur gel de silice en éluant par le mélange DCM/MeOH (95/5 ; v/v) puis par le mélange DCM/MeOH/$NH_4OH$ (90/10/0,4 ; v/v/v). On dissout le produit obtenu dans l'AcOEt, acidifie à pH = 1 par ajout d'éther chlorhydrique, dilue à l'éther et essore le précipité formé. On obtient 0,086 g du produit attendu.

RMN : δ (ppm) : 1,0 à 1,9 : m : 10H ; 2,45 à 3,55 : m : 12H ; 4,0 à 5,0 : m : 4H ; 6,8 à 8,9 : m : 17H ; 10,1 : s : 1H.

EXEMPLE 17

Chlorhydrate de N-[1-[4-(éthylaminométhyl)benzyl]-2-oxo-2-(pyrrolidin-1-yl) éthyl]-3-(naphtalène-2-sulfonylamino)-3-phénylpropionamide.

(I), HCl : $R_1$ = 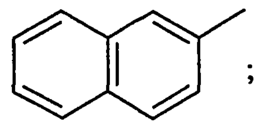 ; $R_2$ = H ; $R_3$ = 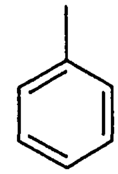 ;

$R_4$ = -CO- 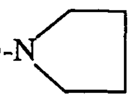 ; $R_5$ = $-CH_2-NH-Et$

A) N-[1-[4-formylbenzyl)-2-oxo-2-(pyrrolidin-1-yl)éthyl]-3-(naphtalène-2-sulfonylamino)-3-phénylpropionamide.

A un mélange de 3 g du composé obtenu à l'étape A de l'EXEMPLE 1 et 40 ml d'acide formique à 75 %, on ajoute 3 g d'alliage aluminium/nickel et chauffe à reflux pendant 2 heures. On essore à chaud un insoluble, le lave au MeOH et concentre sous vide le filtrat. On reprend le résidu au chloroforme, essore un insoluble, le lave avec le mélange MeOH/chloroforme et concentre sous vide le filtrat. On reprend le résidu au chloroforme, filtre, lave le filtrat par une solution saturée de $NaHCO_3$, par une solution saturée de NaCl, sèche sur $Na_2SO_4$ et évapore sous vide le solvant. On obtient 2,4 g du produit attendu.

B) Chlorhydrate de N-[1-[4-(éthylaminométhyl)benzyl]-2-oxo-2-(pyrrolidin-1-yl) éthyl]-3-(naphtalène-2-sulfonylamino)-3-phénylpropionamide.

A un mélange de 0,5 g du composé obtenu à l'étape précédente, 0,057 g de cyanoborohydrure de sodium, 0,077 g de chlorhydrate d'éthylamine et 0,119 ml de triéthylamine dans 15 ml de MeOH, on ajoute AcOH jusqu'à pH = 5 et laisse 2 heures sous agitation à TA. On concentre sous vide, extrait le résidu au DCM, lave la phase organique par une solution saturée de $NaHCO_3$, par une solution tampon $KHSO_4/K_2SO_4$, par une solution saturée de $NaHCO_3$, sèche sur $Na_2SO_4$ et évapore sous vide le solvant. On chromatographie le résidu sur gel de silice en éluant par le mélange DCM/MeOH (90/10 ; v/v) puis DCM/MeOH/$NH_4OH$ (90/10/0,4 ; v/v/v). On dissout le produit obtenu dans AcOEt, acidifie à pH = 1 par ajout d'éther chlorhydrique, dilue à l'éther et essore le précipité formé. On obtient 0,112 g du produit attendu après cristallisation dans le mélange MeOH/éther.
RMN : δ (ppm) : 1,0 à 1,9 : m : 7H ; 2,3 à 3,4 : m : 10H ; 4,0 : s : 2H ; 4,25 à 4,8 : m : 2H ; 6,6 à 8,2 : m : 14H.

EXEMPLE 18

N-[1-[4-(diméthylaminométhyl)benzyl]-2-oxo-2-(pyrrolidin-1-yl)éthyl]-3-(naphtalène-2-sulfonylamino)-3-phénylpropionamide.

(I) : $R_1$ = 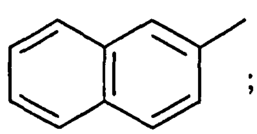 ; $R_2$ = H ; $R_3$ = 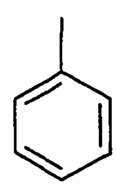 ;

$R_4$ = -CO- 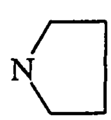 ; $R_5$ = $-CH_2-N(Me)_2$

A une solution de 0,830 g du composé obtenu à l'étape A de l'EXEMPLE 17 dans 10 ml de DCM, on ajoute 1 ml d'AcOH, 0,129 g de chlorhydrate de diméthylamine puis, par portions, 0,456 g de triacétoxyborohydrure de sodium et laisse 18 heures sous agitation à TA. On dilue le mélange réactionnel au DCM, lave la phase organique par une solution saturée de NaCl, sèche sur $Na_2SO_4$ et évapore sous vide le solvant. On chromatographie le résidu sur gel de silice en éluant par le mélange chloroforme/MeOH (95/5 ; v/v). On obtient 0,52 g du produit attendu.
$MH^+ = 613$

EXEMPLE 19

Chlorhydrate de N-[1-[4-(azétidin-1-ylméthyl)benzyl]-2-oxo-2-(pyrrolidin-1-yl) éthyl]-3-(naphtalène-2-sulfonylamino)-3-phénylpropionamide.

(I), HCl : $R_1$ = 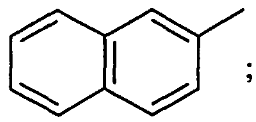 ; $R_2$ = H ; $R_3$ = 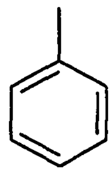 ;

$R_4$ = -CO- 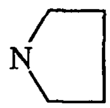 ; $R_5$ = $-CH_2-$ 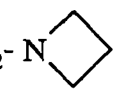

On laisse 1 heure sous agitation à TA un mélange de 0,489 g du composé obtenu à l'étape A de l'EXEMPLE 17, et 0,086 g de chlorhydrate d'azétidine et 116 µl de TEA dans 15 ml de MeOH. Puis on ajoute 0,072 ml d'AcOH, laisse 15 minutes sous agitation à TA, ajoute 0,079 g de cyanoborohydrure de sodium et laisse 2 heures sous agitation à TA. On concentre sous vide, extrait le résidu au DCM, lave la phase organique par une solution saturée de $NaHCO_3$ sèche sur $Na_2SO_4$ et évapore sous vide le solvant. On chromatographie le résidu sur gel de silice en éluant par le mélange DCM/MeOH (95/5 ; v/v) puis DCM/MeOH/$NH_4OH$ (95/5/0,5 ; v/v/v). On dissout le produit obtenu dans l'AcOEt, acidifie à pH = 1 par ajout d'éther chlorhydrique, dilue à l'éther et essore le précipité formé. On obtient 0,115 g du produit attendu.
RMN : δ (ppm) : 1,4-1,8 : m : 4H ; 2,1 à 3,25 : m : 10H ; 3,6-4,8 : m : 8H ; 6,8-8,5 : m : 18H ; 10,85 : s : 1H.

EXEMPLE 20

N-[1-[4-(dibutylaminométhyl)benzyl]-2-oxo-2-(pyrrolidin-1-yl)éthyl]-3-naphtalène-2-sulfonylamino)-3-phénylpropionamide.

(I) : $R_1$ = 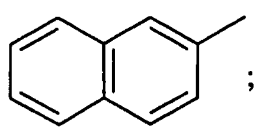 ; $R_2$ = H ; $R_3$ = 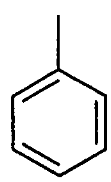 ;

$R_4$ = -CO- 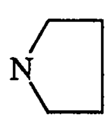 ; $R_5$ = $-CH_2-N(nBu)_2$

A) N-[1-[4-(hydroxyméthyl)benzyl]-2-oxo-2-(pyrrolidin-1-yl)éthyl]-3-(naphtalène-2-sulfonylamino)-3-phénylpropionamide.

A un mélange de 3,5 g du composé obtenu à l'EXEMPLE 1 dans 100 ml d'eau et 20 ml de dioxane, on ajoute 0,57 g de nitrite de sodium et chauffe à 110°C pendant 2 heures. On concentre sous vide le mélange réactionnel, extrait le résidu à l'AcOEt, lave la phase organique à l'eau, sèche sur $Na_2SO_4$ et évapore sous vide le solvant. On reprend le résidu à l'éther et essore le précipité formé. On obtient 2,78 g du produit attendu.

B) N-[1-[4-(chlorométhyl)benzyl]-2-oxo-2-(pyrrolidin-1-yl)éthyl]-3-(naphtalène-2-sulfonylamino)-3-phénylpropionamide et N-[1-[4-(méthylsulfonyloxyméthyl) benzyl]-2-oxo-2-(pyrrolidin-1-yl)éthyl]-3-(naphtalène-2-sulfonylamino)-3-phénylpropionamide.

A une solution de 1 g du composé obtenu à l'étape précédente dans 10 ml de DCM, on ajoute 0,3 ml de triéthylamine puis 0,167 ml de chlorure de méthanesulfonyle et laisse 30 minutes sous agitation à TA. On rajoute 0,3 ml de triéthylamine puis 0,167 ml de chlorure de méthanesulfonyle, laisse 30 minutes sous agitation et concentre sous vide le mélange réactionnel. On extrait le résidu à l'AcOEt, lave la phase organique à l'eau, sèche sur $Na_2SO_4$ et évapore sous vide le solvant. On obtient 0,68 g du mélange de produits attendus.

C) N-[1-[4-(dibutylaminométhyl)benzyl]-2-oxo-2-(pyrrolidin-1-yl)éthyl]-3-naphtalène-2-sulfonylamino)-3-phénylpropionamide.

A une solution de 0,245 g de dibutylamine dans 5 ml de THF, on ajoute 0,045 g d'hydrure de sodium à 80 % dans l'huile et laisse 30 minutes sous agitation à TA. On ajoute ensuite 1 g du composé obtenu à l'étape précédente et laisse 18 heures sous agitation à TA. On concentre sous vide, extrait le résidu au DCM, lave la phase organique à l'eau, sèche sur $Na_2SO_4$ et évapore sous vide le solvant. On chromatographie le résidu sur gel de silice en éluant par le mélange chloroforme/MeOH (95/5 ; v/v). On obtient 0,2 g du produit attendu.
$MH^+$ = 698

EXEMPLE 21

Chlorhydrate de N-[1-[4-(butylaminométhyl)benzyl]-2-oxo-2-(pyrrolidin-1-yl) éthyl]-3-(naphtalène-2-sulfonylamino)-3-phénylpropionamide.

(I), HCl : $R_1$ = 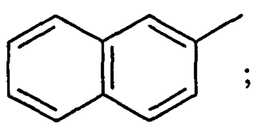 ; $R_2$ = H ; $R_3$ = 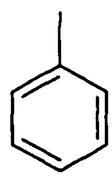 ;

$R_4$ = -CO- 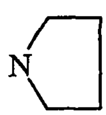 ; $R_5$ = -$CH_2$-NH-nBu

A) Chlorhydrate de N-[1-[4-(N-benzyl-N-butylaminométhyl)benzyl]-2-oxo-2-(pyrrolidin-1-yl) éthyl]-3-(naphtalène-2-sulfonylamino)-3-phénylpropionamide.

A une solution de 0,397 g de N-butylbenzylamine dans 5 ml de THF, on ajoute 0,073 g d'hydrure de sodium à 80 % dans l'huile et laisse 30 minutes sous agitation à TA. On ajoute ensuite 1,5 g du composé obtenu à l'étape B de l'EXEMPLE 20 et laisse 18 heures sous agitation à TA. On essore l'insoluble, le lave au THF et concentre sous vide le filtrat. On extrait le résidu au DCM, lave la phase organique par HCl 0,1N, par une solution saturée de NaCl, sèche sur $Na_2SO_4$ et évapore sous vide le solvant. On chromatographie le résidu sur gel de silice en éluant par le mélange chloroforme/MeOH (96/4 ; v/v). On obtient 0,265 g du produit attendu.

B) Chlorhydrate de N-[1-[4-(butylaminométhyl)benzyl]-2-oxo-2-(pyrrolidin-1-yl) éthyl]-3-(naphtalène-2-sulfonylamino)-3-phénylpropionamide.

A une solution de 0,47 g du composé obtenu à l'étape précédente dans 5 ml de MeOH, on ajoute 0,6 ml d'HCl 1N puis 0,5 g de palladium sur charbon à 10 % et on hydrogène à TA et sous une pression de 13332 Pa pendant 18 heures. On filtre le catalyseur sur Célite®, lave par un mélange MeOH/chloroforme et concentre sous vide le filtrat. On chromatographie le résidu sur gel de silice en éluant par le mélange chloroforme/MeOH (95/5 ; v/v). On obtient 0,12 g du produit attendu.

$MH^+ = 641$

En procédant selon les modes opératoires décrits dans les EXEMPLES précédents, on prépare les composés selon l'invention rassemblés dans le TABLEAU VIII ci-après.

TABLEAU VIII

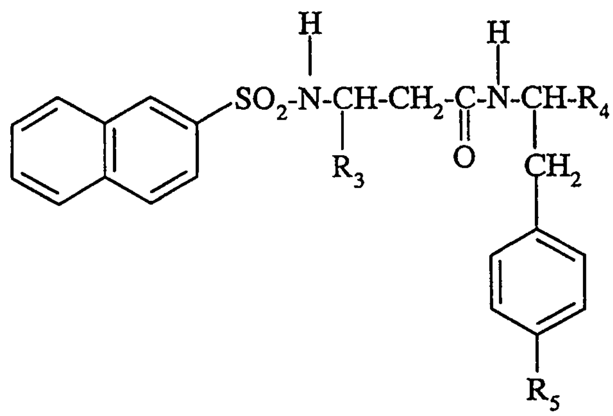

(I)

| Exemples | $R_3$ | $R_4$ | $R_5$ | Sel masse ou RMN |
|---|---|---|---|---|
| 22 (a) | 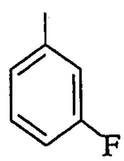 | 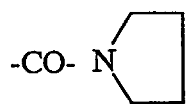 | 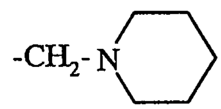 | RMN |
| 23 (b) | 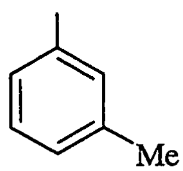 | 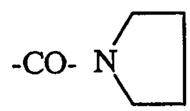 | $-CH_2-N(Et)_2$ | HCl RMN |
| 24 (c) | 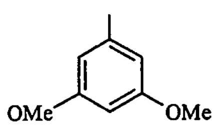 | 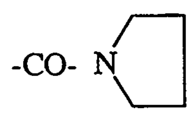 | $-CH_2-N(Et)_2$ | RMN |
| 25 (d) | 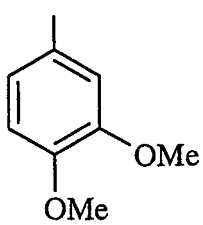 | 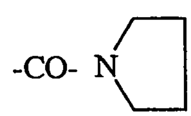 | $-CH_2-N(Et)_2$ | RMN |

(suite)

| Exemples | $R_3$ | $R_4$ | $R_5$ | Sel masse ou RMN |
|---|---|---|---|---|
| 26 (e) | 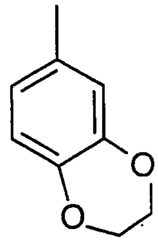 | 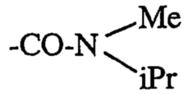 | $-CH_2-N(Et)_2$ | RMN |
| 27 (f) | 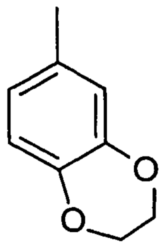 | $-CO-N(iPr)_2$ | $-CH_2-N(Et)_2$ | HCl RMN |
| 28 (g) | 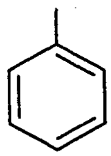 | 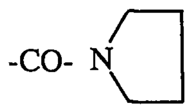 | $-CH_2-N(n\text{-}Pr)_2$ | HCl RMN |
| 29 (h) | 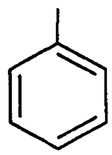 | 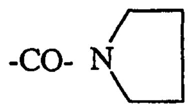 | $-CH_2-N(iPr)_2$ | HCl RMN |
| 30 (i) | 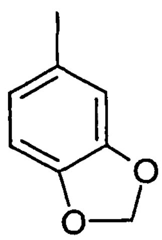 | 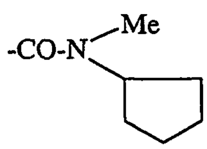 | $-CH_2-N(Et)_2$ | HCl RMN |
| 31 (j) | 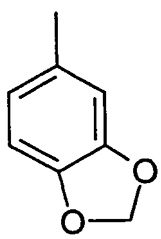 | 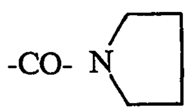 | $-CH_2-N(Et)_2$ | HCl RMN |
| 32 (k) | 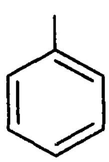 | 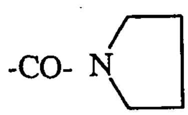 | $-CH_2-NH-CH_2CH_2OH$ | Masse |

(suite)

| Exemples | $R_3$ | $R_4$ | $R_5$ | Sel masse ou RMN |
|---|---|---|---|---|
| 33 (l) | 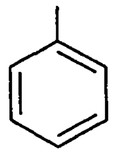 | 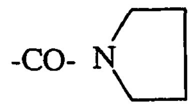 | 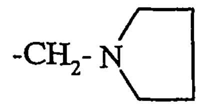 | Masse |
| 34 (m) | 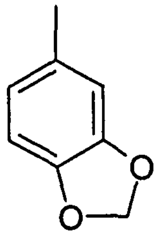 | 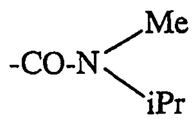 | 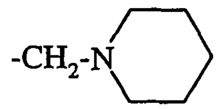 | RMN |
| 35 (n) | 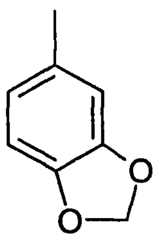 | 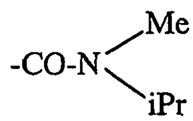 | 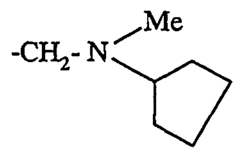 | RMN |
| 36 (o) | 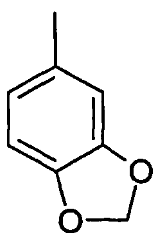 | 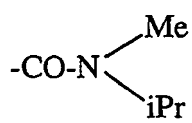 | 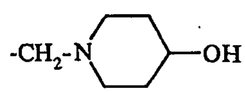 | RMN |

Masse ou Spectres de RMN du proton à 200 MHz dans DMSO-$d_6$ des composés des EXEMPLES du TABLEAU I.

EXEMPLE 22 : δ (ppm) : 1,2 - 1,9 : m : 10H ; 2,5 - 3,4 : m : 12H ; 4,2 : s : 2H ; 4,45 : t : 1H ; 4,7 : t : 1H ; 6,8 - 8,3 : m : 16H.

EXEMPLE 23 : δ (ppm) : 1,2 : t : 6H ; 1,4-1,75 : m : 4H ; 1,8 : s : 3H ; 2,2-3,2 : m : 12H ; 4,0-4,8 : m : 4H ; 6,5-8,4 : m : 17H ; 10,4 : s : 1H.

EXEMPLE 24 : δ (ppm) : 0,9 : t : 6H ; 1,3-1,6 : m : 4H ; 2,2-3,5 : m : 20H ; 4,2-4,7 : m : 2H ; 5,85 : t : 1H ; 6,15 : d : 2H ; 6,8-8,3 : m : 13H.

EXEMPLE 25 : δ (ppm) : 1,0 : t : 6H ; 1,4-1,8 : m : 4H ; 2,3-3,7 : m : 20H ; 4,3-4,8 : m : 2H ; 6,4-8,4 : m : 16H.

EXEMPLE 26 : δ (ppm) : 0,5-1,0 : m : 12H ; 2,2-2,9 : m : 11H ; 3,35-5,0 : m : 9H ; 6,2-8,4 : m : 16H.

EXEMPLE 27 : δ (ppm) : 0,5-1,4 : m : 18H ; 2,2-3,1 : m : 8H ; 3,15-4,8 : m : 10H ; 6,2-8,3 : m : 16H ; 10,35 : s : 1H.

EXEMPLE 28 : δ (ppm) : 0,6-0-9 : mt : 6H ; 1,3-1,9 : mt : 8H ; 2,2-3,65 : mt : 12H ; 4,0-4,9 : m : 4H ; 6,7-8,5 : m : 18H ; 10,5 : se : 1H.

EXEMPLE 29 : δ (ppm) : 1,0-1,8 : m : 16H ; 2,2-3,7 : m : 10H ; 4,0-4,8 : m : 4H ; 6,7-8,5 : m : 18H ; 9,2 : se : 1H.

EXEMPLE 30 : δ (ppm) : 1,3-1,8 : m : 4H ; 2,2-3,8 : m : 8H ; 3,9-5,0 : m : 6H ; 6,6-8,8 : m : 22H ; 11,35 : mt : 1H.

EXEMPLE 31 : δ (ppm) : 1,0-1,3 : mt : 6H ; 1,4-1,8 : m : 4H ; 2,2-3,2 : m : 12H ; 4,0-4,7 : m : 4H ; 5,2-5,7 : m : 2H ; 6,2-8,4 : m : 16H ; 10,2 : se : 1H.

EXEMPLE 32 : $MH^+$ = 629

EXEMPLE 33 : $MH^+$ = 639

EXEMPLE 34 : δ (ppm) : 0,5-1,0 : m : 6H ; 1,25-1,65 : m : 6H ; 2,1-3,0 : m : 11H ; 3,2-4,9 : m : 5H ; 5,2-5,8 : m : 2H ; 6,2-8,4 : m : 16H.

EXEMPLE 35 : δ (ppm) : 0,4-0,9 : m : 6H ; 1,15-2,1 : m : 11H ; 2,15-2,9 : m : 8H ; 3,2-4,8 : m : 5H ; 5,2-5,7 : mt : 2H ; 6,2-8,3 : m 16H.

(suite)

| Exemples | $R_3$ | $R_4$ | $R_5$ | Sel masse ou RMN |
|---|---|---|---|---|
| EXEMPLE 36 : δ (ppm) : 0,4-0,8 : m : 6H ; 1,05-2,65 : m : 4H ; 2,7-2,9 : m : 11H ; 3,2-4,8 : m : 7H; 5,2-5,65 : m : 2H ; 6,2-8,3 : m : 16H. | | | | |
| (a) On prépare ce composé selon le mode opératoire décrit à l'EXEMPLE 3 à partir du composé obtenu à la Préparation 1.1 et du composé obtenu à la Préparation 2.6. | | | | |
| (b) On prépare ce composé selon le mode opératoire décrit à l'EXEMPLE 3 à partir du composé obtenu à la Préparation 1.3 et du composé obtenu à la Préparation 2.7. Le produit brut obtenu est dissout dans l'AcOEt, on acidifie à pH = 1 par ajout d'éther chlorhydrique, dilue à l'éther et essore le précipité formé. | | | | |
| (c) On prépare ce composé selon le mode opératoire décrit à l'EXEMPLE 3 à partir du composé obtenu à la Préparation 1.5 et du composé obtenu à la Préparation 2.7. | | | | |
| (d) On prépare ce composé selon le mode opératoire décrit à l'EXEMPLE 3 à partir du composé obtenu à la Préparation 1.6 et du composé obtenu à la Préparation 2.7. | | | | |
| (e) On prépare ce composé selon le mode opératoire décrit à l'EXEMPLE 7 à partir du composé obtenu à la Préparation 1.8 et du composé obtenu à la Préparation 2.8, sans faire le chlorhydrate. | | | | |
| (f) On prépare ce composé selon le mode opératoire décrit à l'EXEMPLE 8 à partir du composé obtenu à la Préparation 1.8 et du composé obtenu à la Préparation 2.9. | | | | |
| (g) On prépare ce composé selon le mode opératoire décrit à l'EXEMPLE 13 étape A, à partir du composé obtenu à la Préparation 1.1 et du composé obtenu à la Préparation 2.12, puis saponification par KOH 1N selon l'étape B et le couplage avec la pyrrolidine selon l'étape C. A la fin de l'étape C formation du chlorhydrate dans l'AcOEt par ajout d'éther chlorhydrique. | | | | |
| (h) On prépare ce composé selon le mode opératoire décrit à l'EXEMPLE 13 étape A à partir du composé obtenu à la Préparation 1.1 et du composé obtenu à la Préparation 2.13, puis saponification par KOH 1N selon l'étape B et couplage avec la pyrrolidine selon l'étape C. Formation du chlorhydrate selon (g) ci-dessus. | | | | |
| (i) On prépare ce composé selon le mode opératoire décrit à l'étape C de l'EXEMPLE 13, à partir du composé obtenu à l'étape B de l'EXEMPLE 15 et de N-méthylcyclopentylamine. On chromatographie le produit obtenu sur gel de silice en éluant par le mélange DCM/MeOH/$NH_4OH$ (95/5/0,4; v/v/v) puis forme le chlorhydrate dans AcOEt/éther chlorhydrique. | | | | |
| (j) On prépare ce composé selon le mode opératoire décrit à l'étape C de l'EXEMPLE 13, à partir du composé obtenu à l'étape B de l'EXEMPLE 15 et de pyrrolidine. On chromatographie le produit obtenu sur gel de silice en éluant par le mélange DCM/MeOH/$NH_4OH$ (95/5/0,4 ; v/v/v) puis forme le chlorhydrate dans AcOEt/éther chlorhydrique et cristallise dans MeOH/éther. | | | | |
| (k) On prépare ce composé selon le mode opératoire décrit à l'EXEMPLE 17, à partir du composé obtenu à l'étape A de l'EXEMPLE 18 et de 2-aminoéthanol. | | | | |
| (l) On prépare ce composé selon le mode opératoire décrit à l'EXEMPLE 18, à partir du composé obtenu à l'étape A de l'EXEMPLE 17 et de pyrrolidine. | | | | |
| (m) On prépare ce composé selon le mode opératoire décrit à l'EXEMPLE 8, à partir du composé obtenu à la Préparation 1.7 et du composé obtenu à la Préparation 2.15, sans effectuer la chromatographie, ni la salification. | | | | |
| (n) On prépare ce composé selon le mode opératoire décrit à l'EXEMPLE 8, à partir du composé obtenu à la Préparation 1.7 et du composé obtenu à la Préparation 2.16, sans effectuer la chromatographie, ni la salification. | | | | |
| (o) On prépare ce composé selon le mode opératoire décrit à l'EXEMPLE 8, à partir du composé obtenu à la Préparation 1.7 et du composé obtenu à la Préparation 2.17, sans effectuer la chromatographie, ni la salification. | | | | |

EXEMPLE 37

(I), HCl : (R) $R_1$ = 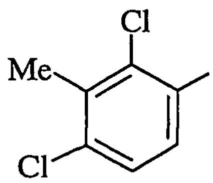 ; $R_2$ = 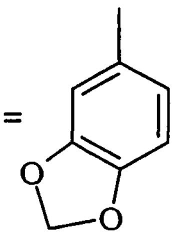 ; $R_3$ = H ;

$R_4$ = 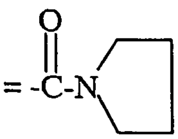 ; $R_5$ = 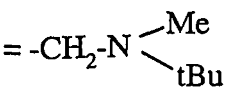

A) (R) N-[1-(4-cyanobenzyl)-2-oxo-2-(pyrrolidin-1-yl)éthyl]-3-[N-(benzo[1,3]dioxol-5-yl)amino]propionamide.

Ce composé est obtenu par action du composé de la préparation 1.17 sur le composé de la préparation 2.18.

B) (R) N-[1-(4-formylbenzyl)-2-oxo-2-(pyrrolidin-1-yl)éthyl]-3-[N-(benzo[1,3] dioxol-5-yl)amino]propionamide.

A 0°C, on place 550 mg du composé de l'étape précédente dans 16 ml d'un mélange pyridine/acide acétique/eau (2/1/1) et on ajoute 1,3 g de $NaH_2PO_2$, $H_2O$ et 260 mg de Nickel de Raney. On chauffe le milieu réactionnel 2 heures à 55°C puis on filtre sur Célite®, rince par un mélange EtOH/DCM (1/1) et concentre le filtrat. On extrait par AcOEt puis on lave successivement par de l'eau (2 fois), une solution saturée de $NaHCO_3$, de l'eau, une solution de $KHSO_4$ à 5 %, une solution de NaCl saturée. On obtient le produit attendu qui est utilisé tel quel à l'étape suivante.

126

C) On place le produit obtenu à l'étape précédente dans 10 ml de dichlorométhane et l'on traite par 160 µl de méthyl-*tert*-butylamine et 264 mg de $NaHB(OAc)_3$. Après 24 heures sous agitation à TA, le milieu est dilué par DCM puis lavé successivement par de l'eau (2 fois), une solution saturée de $NaHCO_3$, une solution saturée de NaCl. On prépare le chlorhydrate par addition de $Et_2O$ saturé en HCl gaz à une solution du composé dans AcOEt/$Et_2O$ (1/1 ; v/v). On obtient 0,36 g du composé attendu qui cristallise dans un mélange AcOEt/$Et_2O$, F = 166°C.

$$\alpha_D^{25} = -7,8 \ (c = 1 ; MeOH).$$

RMN : 1,4-2,05 ppm : m : 13H ; 2,2-4,8 ppm : m : 16H ; 6,15 ppm : s : 2H ; 6,65-7,8 ppm : m : 9H ; 8,5 ppm : d : 1H ; 9,95 ppm : se : 1H.

EXEMPLE 38

(I), HCl : (R,R) $R_1$ = 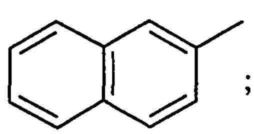 ; $R_2$ = H ; $R_3$ = 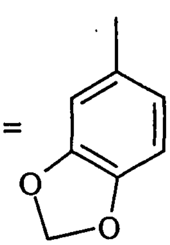 ;

$R_4$ = 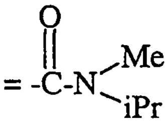 ; $R_5 = -CH_2-N(iPr)_2$

A) (R,R) 2-((3-(N-Boc)amino-3-(benzo[1,3]dioxol-5-yl)propanoyl)amino)-3-(4-(diisopropylaminométhyl)phényl)-N-isopropyl-N-méthylpropionamide.

On mélange 237 mg du composé obtenu à la préparation 1.13 et le composé obtenu à la préparation 2.19 avec 303 mg de BOP et 360 µl de DIPEA dans 10 ml de DCM. Après 3 heures sous agitation à TA, on concentre à sec. On extrait par AcOEt puis on lave successivement par de l'eau, une solution saturée de $NaHCO_3$, une solution saturée de NaCl. On obtient 420 mg du composé attendu.

B) (R,R) 2-((3-amino-3-(benzo[1,3]dioxol-5-yl)propanoyl)amino)-3-(4-(diisopropylaminométhyl)phényl)-N-isopropyl-N-méthylpropionamide, 2TFA.

On place 420 mg du composé de l'étape précédente dans 15 ml de TFA et 20 ml de DCM. Après 2 heures sous agitation à TA, on concentre à sec. On obtient 0,504 g du composé attendu qui cristallise dans $Et_2O$.

C) (R,R) 2-((3-naphtalène-2-sulfonylamino-3-(benzo[1,3]dioxol-5-yl)propanoyl)amino)-3-(4-(diisopropylaminométhyl)phényl)-N-isopropyl-N-méthylpropionamide.

On mélange 500 mg du composé de l'étape précédente avec 153 mg de chlorure de naphtalène-2-sulfonyle et 354 µl de DIPEA dans 5 ml de DCM et on laisse sous agitation 2 heures à TA. On concentre à sec puis on lave de l'eau puis par une solution saturée de $NaHCO_3$. On prépare le chlorhydrate par addition de $Et_2O$ saturé en HCl à une solution du composé dans AcOEt. On obtient 0,280 g du composé attendu.
RMN : 0,6-1,6 ppm : m : 18H; 2,2-3,1 ppm : m : 7H ; 3,4-5,8 ppm : m : 9H; 6,3-8,6 ppm : m : 16H ; 9,1 ppm : se: 1H.

$\alpha_D^{25} = +55,2°$ (c = 1 ; MeOH).

En opérant comme dans les exemples ci-dessus, on prépare les composés selon l'invention de formule (I) décrits dans le tableau IX.

TABLEAU IX

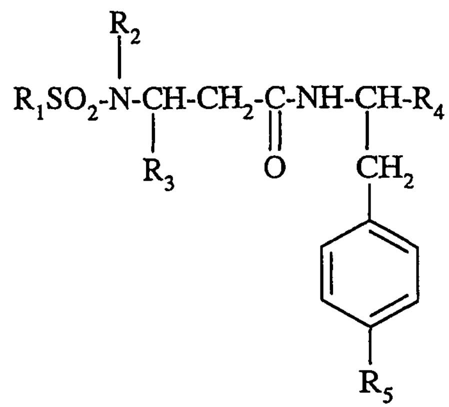

(I)

| Exemple | $R_1$ | $R_2$ | $R_3$ | $R_4$ | $R_5$ |
|---|---|---|---|---|---|
| 39<br>2HCl | 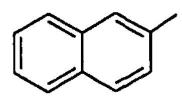 | H | 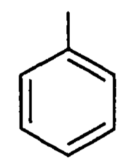 | 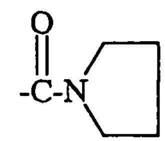 | 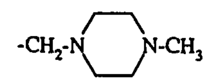 |
| 40<br>HCl | 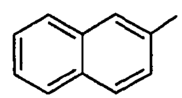 | H | 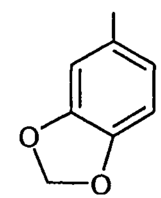 | 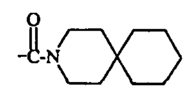 | 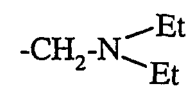 |
| 41<br>HCl | 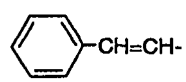 | H | 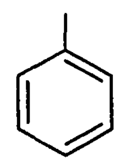 | 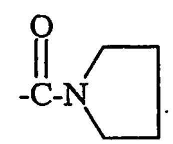 | 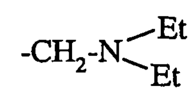 |

(suite)

| Exemple | R1 | R2 | R3 | R4 | R5 |
|---|---|---|---|---|---|
| 42<br>HCl | 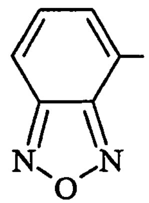 | H | 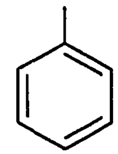 | 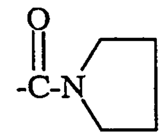 | 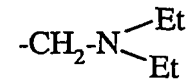 |
| 43<br>2HCl | 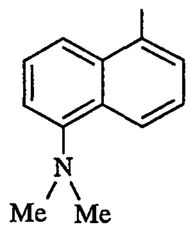 | H | 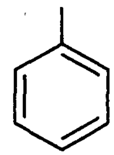 | 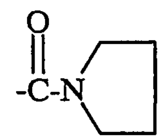 | 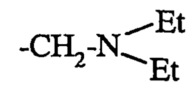 |
| 44<br>HCl | 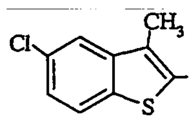 | H | 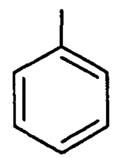 | 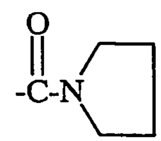 | 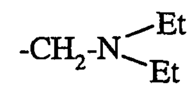 |
| 45 | 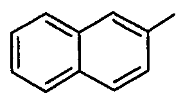 | H | 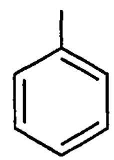 | 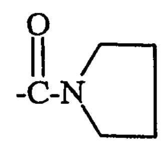 | 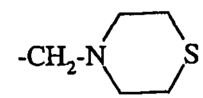 |
| 46 | 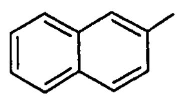 | H | 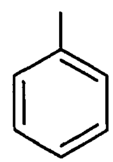 | 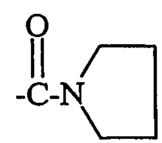 | 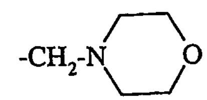 |

(suite)

| Exemple | $R_1$ | $R_2$ | $R_3$ | $R_4$ | $R_5$ |
|---|---|---|---|---|---|
| 47<br>HCl | 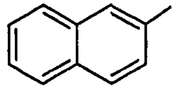 | H | 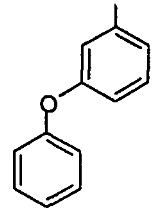 | 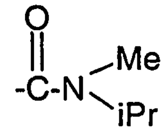 | 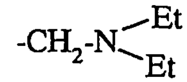 |
| 48 | 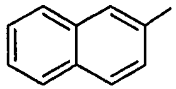 | H | 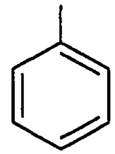 | 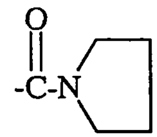 | 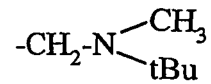 |
| 49 | 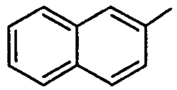 | H | 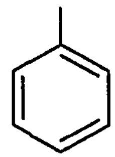 | 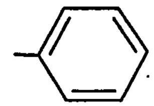 | 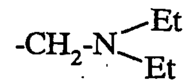 |
| 50<br>HCl | 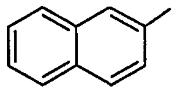 | H | 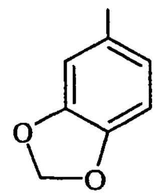 | 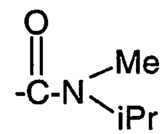 | 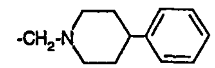 |
| 51<br>HCl | 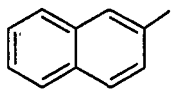 | H | 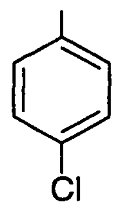 | 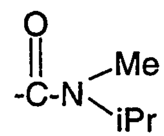 | 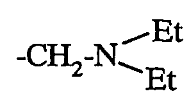 |

(suite)

| Exemple | $R_1$ | $R_2$ | $R_3$ | $R_4$ | $R_5$ |
|---|---|---|---|---|---|
| 52 HCl | 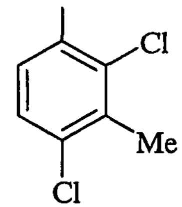 | 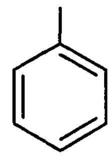 | H | 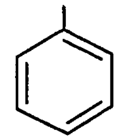 | 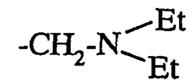 |
| 53 | 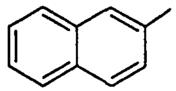 | H | 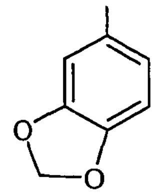 | 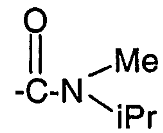 | 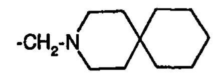 |
| 54 HCl | 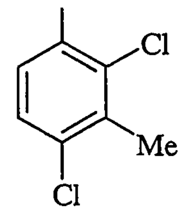 | 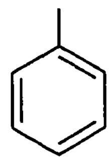 | H | 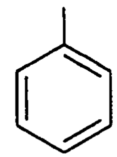 | 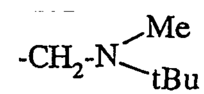 |
| 55 HCl | 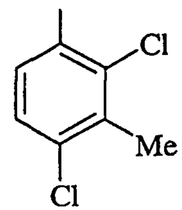 | 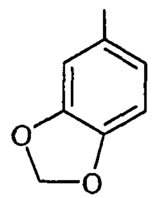 | H | 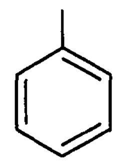 | 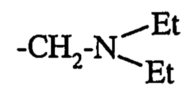 |
| 56 HCl | 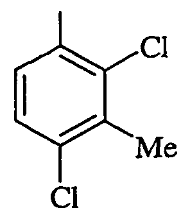 | 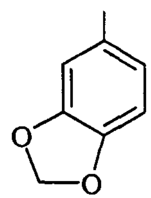 | H | 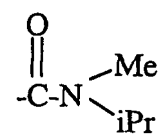 | 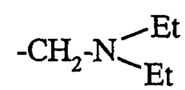 |

(suite)

| Exemple | $R_1$ | $R_2$ | $R_3$ | $R_4$ | $R_5$ |
|---|---|---|---|---|---|
| 57 | 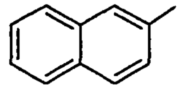 | H | 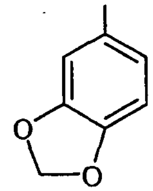 | 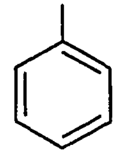 | 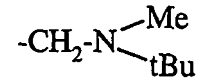 |
| 58 | 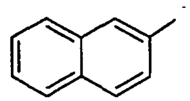 | H | 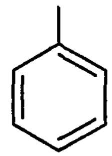 | 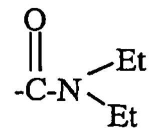 | 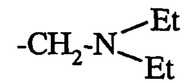 |
| 59 | 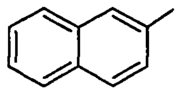 | H | 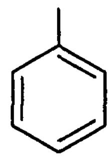 | 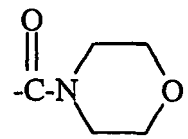 | 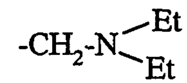 |
| 60<br>2HCl | 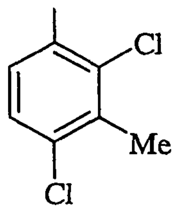 | 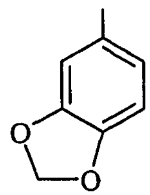 | H | 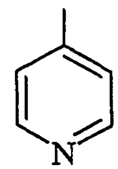 | 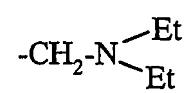 |
| 61<br>HCl | 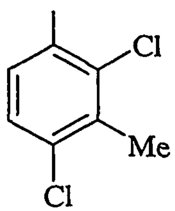 | 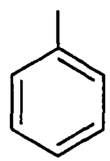 | H | 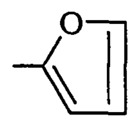 | 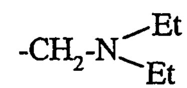 |

(suite)

| Exemple | $R_1$ | $R_2$ | $R_3$ | $R_4$ | $R_5$ |
|---|---|---|---|---|---|
| 62 HCl | 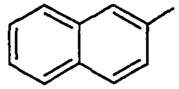 | H | 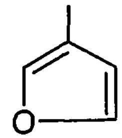 | 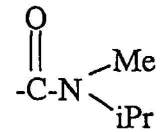 | 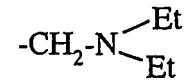 |
| 63 | 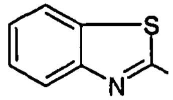 | H | 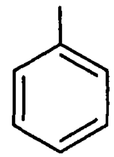 | 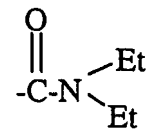 | 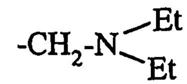 |
| 64 | 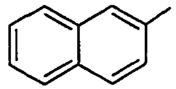 | H | 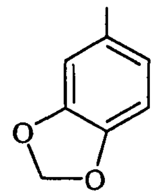 | 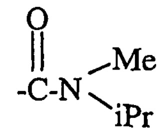 | 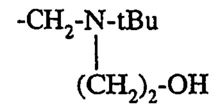 |
| 65 | 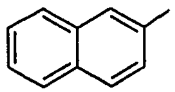 | H | 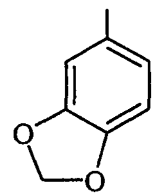 | 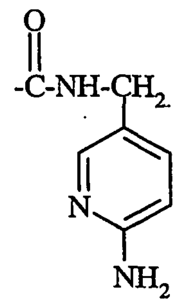 | 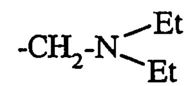 |
| 66 | 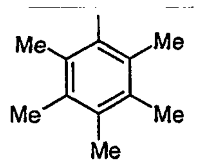 | 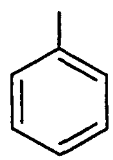 | H | 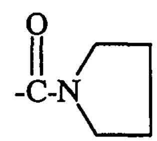 | 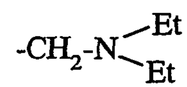 |

(suite)

| Exemple | $R_1$ | $R_2$ | $R_3$ | $R_4$ | $R_5$ |
|---|---|---|---|---|---|
| 67 | 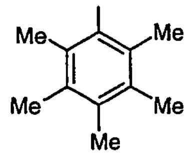 | H | 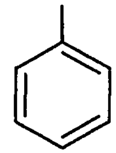 | 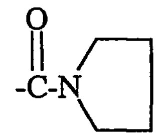 | 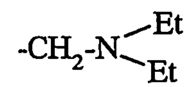 |
| 68 HCl | 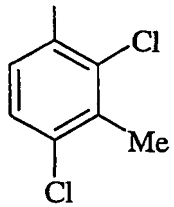 | 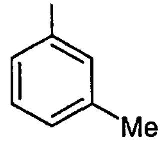 | H | 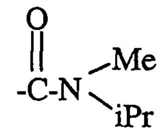 | 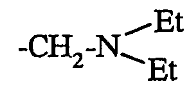 |
| 69 HCl | 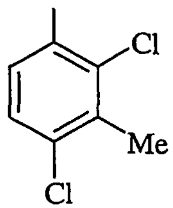 | 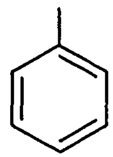 | H | 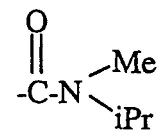 | 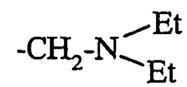 |
| 70 | 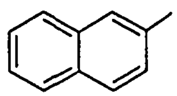 | H | 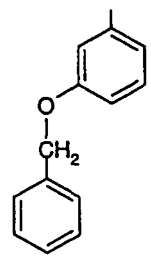 | 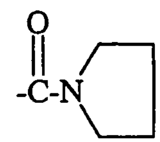 | 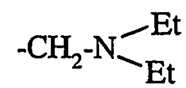 |

(suite)

| Exemple | $R_1$ | $R_2$ | $R_3$ | $R_4$ | $R_5$ |
|---|---|---|---|---|---|
| 71 2HCl | 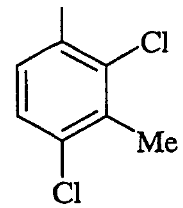 | 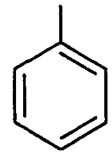 | H | 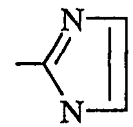 | 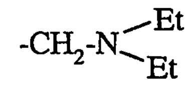 |
| 72 HCl | 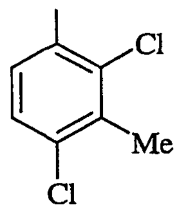 | 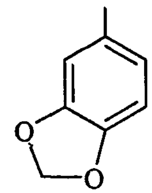 | H | 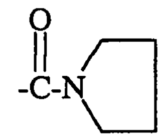 | 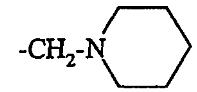 |
| 73 | 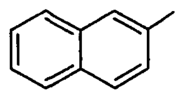 | H | 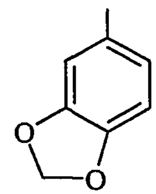 | 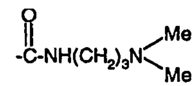 | 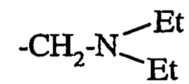 |
| 74 | 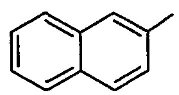 | H | 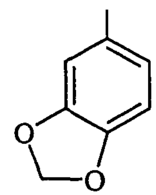 | 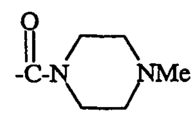 | 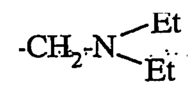 |
| 75 HCl | 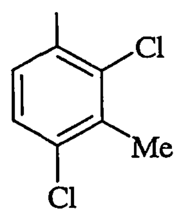 | 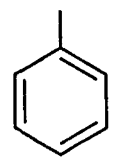 | H | 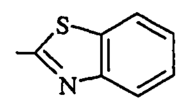 | 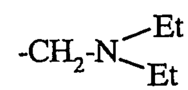 |

(suite)

| Exemple | $R_1$ | $R_2$ | $R_3$ | $R_4$ | $R_5$ |
|---|---|---|---|---|---|
| 76 | 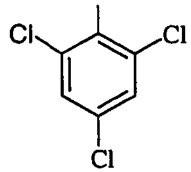 | 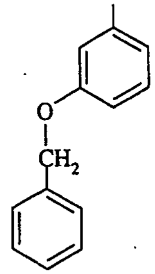 | H | 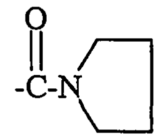 | 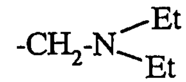 |
| 77<br>HCl | 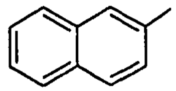 | H | 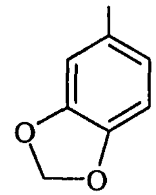 | 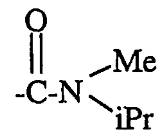 | 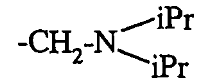 |
| 78<br>2HCl | 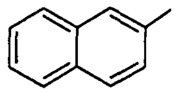 | H | 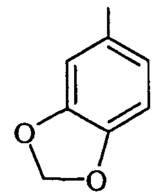 | 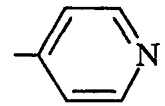 | 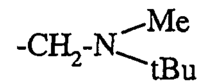 |
| 79<br>2HCl | 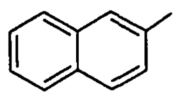 | 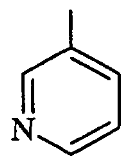 | H | 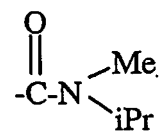 | 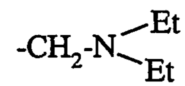 |
| 80<br>2HCl | 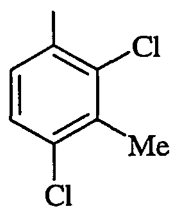 | 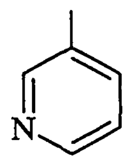 | H | 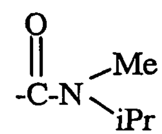 | 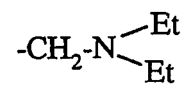 |

(suite)

| Exemple | $R_1$ | $R_2$ | $R_3$ | $R_4$ | $R_5$ |
|---|---|---|---|---|---|
| 81<br>HCl | 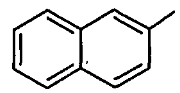 | H | 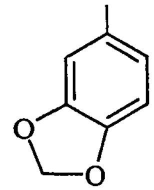 | 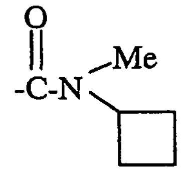 | 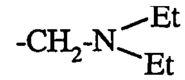 |
| 82<br>2HCl | 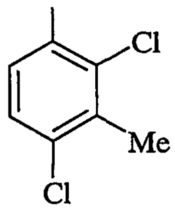 | 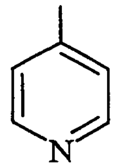 | H | 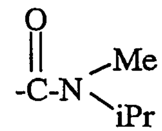 | 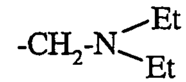 |
| 83<br>HCl | 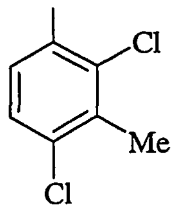 | 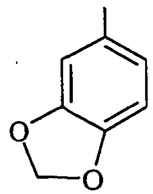 | H | 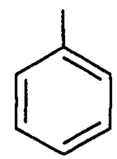 | 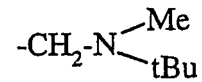 |
| 84<br>2HCl | 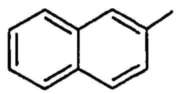 | 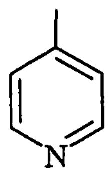 | H | 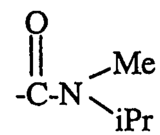 | 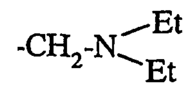 |
| 85 | 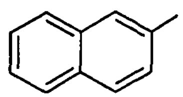 | H | 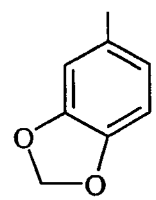 | 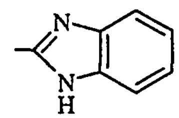 | 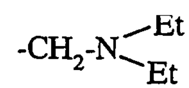 |

(suite)

| Exemple | $R_1$ | $R_2$ | $R_3$ | $R_4$ | $R_5$ |
|---|---|---|---|---|---|
| 86<br>HCl | 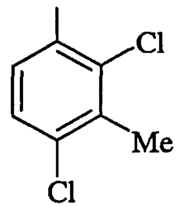 | 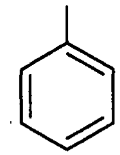 | H | 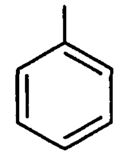 | 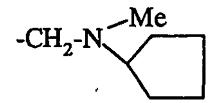 |
| 87<br>HCl | 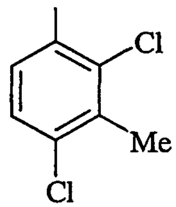 | 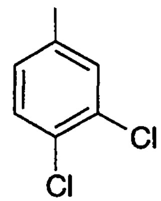 | H | 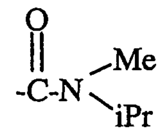 | 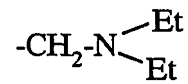 |
| 88<br>HCl | 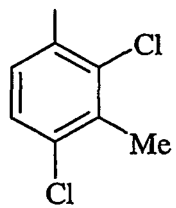 | 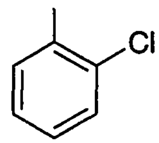 | H | 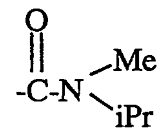 | 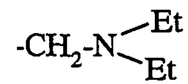 |
| 89 | 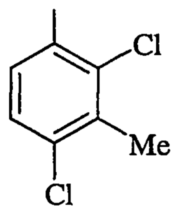 | 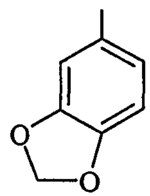 | H | 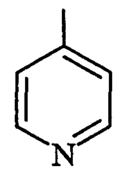 | 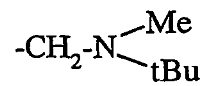 |
| 90<br>2HCl | 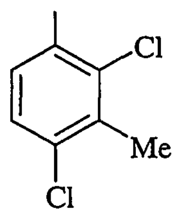 | 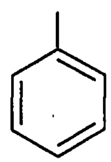 | H | 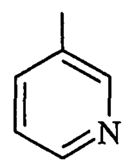 | 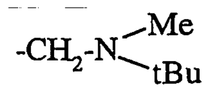 |

(suite)

| Exemple | $R_1$ | $R_2$ | $R_3$ | $R_4$ | $R_5$ |
|---|---|---|---|---|---|
| 91 HCl | 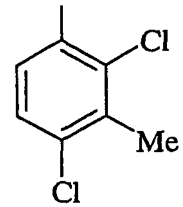 | 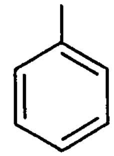 | H | 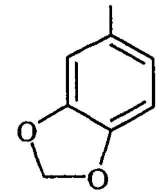 | 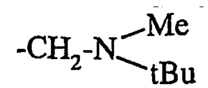 |
| 92 | 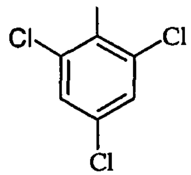 | 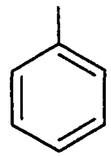 | H | 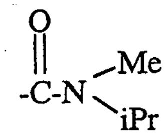 | 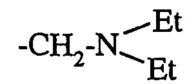 |
| 93 | 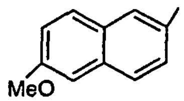 | 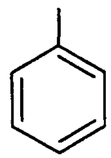 | H | 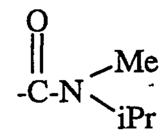 | 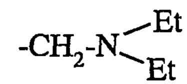 |
| 94 | 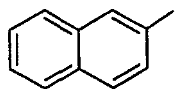 | H | 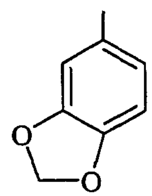 | 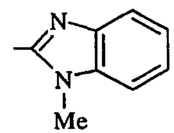 | 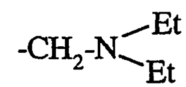 |
| 95 | 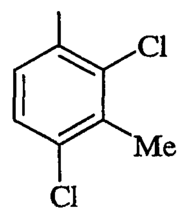 | 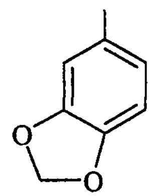 | H | 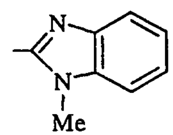 | 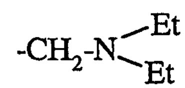 |

(suite)

| Exemple | $R_1$ | $R_2$ | $R_3$ | $R_4$ | $R_5$ |
|---|---|---|---|---|---|
| 96 HCl | 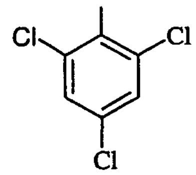 | 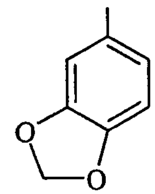 | H | 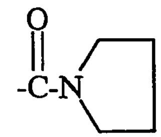 | 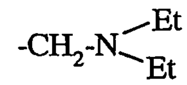 |
| 97 HCl | 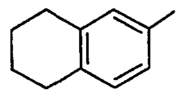 | 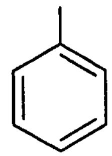 | H | 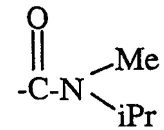 | 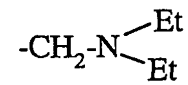 |
| 98 HCl | 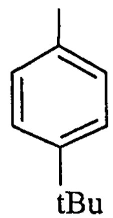 | 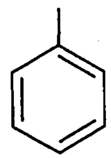 | H | 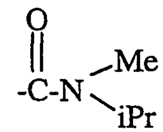 | 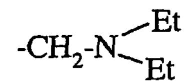 |
| 99 HCl | 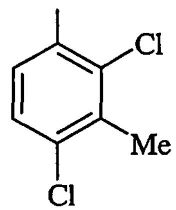 | 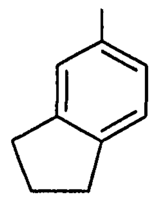 | H | 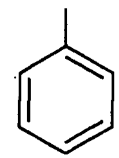 | 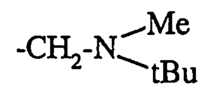 |
| 100 HCl | 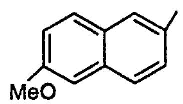 | 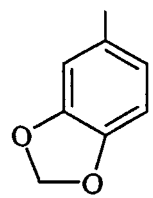 | H | 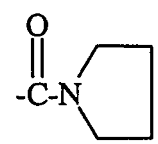 | 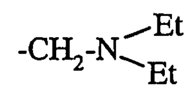 |

(suite)

| Exemple | $R_1$ | $R_2$ | $R_3$ | $R_4$ | $R_5$ |
|---|---|---|---|---|---|
| 101<br>HCl | 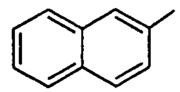 | H | 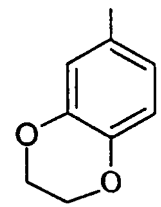 | 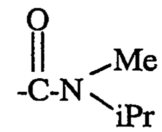 | 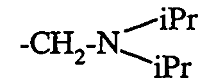 |
| 102<br>HCl<br>isomère R | 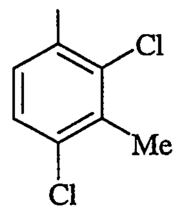 | 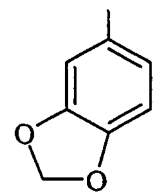 | H | 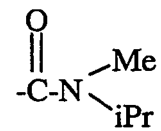 | 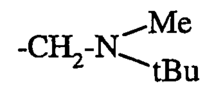 |
| 103<br>HCl | 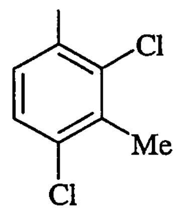 | 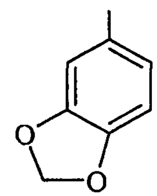 | H | 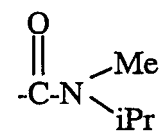 | 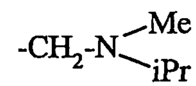 |
| 104<br>HCl<br>isomère<br>R,R | 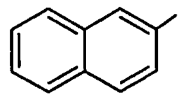 | H | 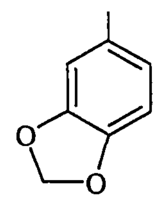 | 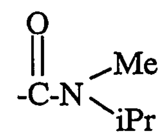 | 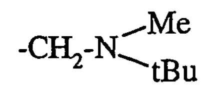 |
| 105<br>HCl<br>isomère R | 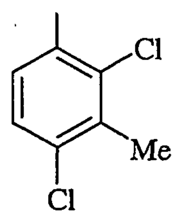 | 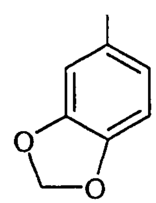 | H | 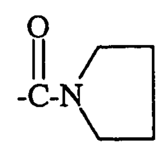 | 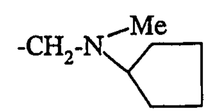 |

(suite)

| Exemple | $R_1$ | $R_2$ | $R_3$ | $R_4$ | $R_5$ |
|---|---|---|---|---|---|
| 106 | 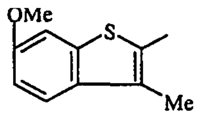 | H | 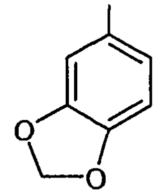 | 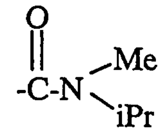 | 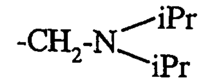 |
| 107 | 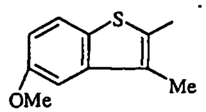 | H | 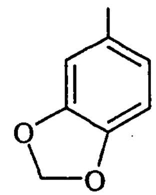 | 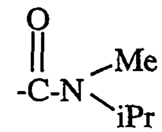 | 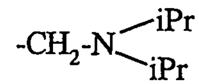 |
| 108<br>HCl<br>isomère<br>R,R | 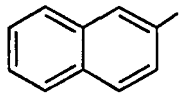 | H | 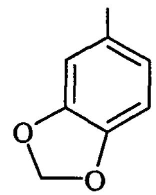 | 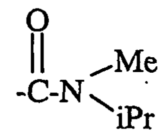 | 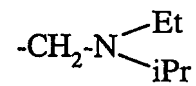 |
| 109<br>HCl<br>isomère<br>R,R | 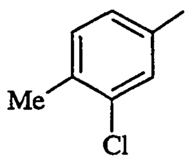 | H | 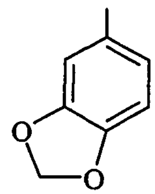 | 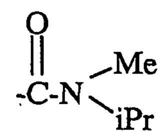 | 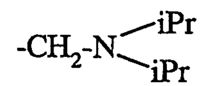 |

Exemple 39 : RMN : 1,3-1,8 ppm : m 4H ; 2,2-3,6 ppm : m : 21H ; 4,2-4,8 ppm : m : 2H; 6,8-8,4 ppm : m : 18H ; 10,2 ppm : se : 1H.

Exemple 41 : RMN : 1,1-1,8 ppm : m : 10H ; 2,4-3,4 ppm : m : 12H ; 4,2 ppm : se : 2H ; 4,4-4,8 ppm : m : 2H ; 6,5-8,5 ppm : m : 18H; 10,3 ppm : se : 1H

Exemple 42 : RMN : 1,2-2 ppm : m : 10H ; 2,4-3,3 ppm : m : 12H ; 4,2-4,9 ppm : m : 4H ; 6,9-9,1 ppm : m : 14H ; 10,2 ppm : se : 1H.

Exemple 43 : RMN : 1-1,8 ppm : m : 10H ; 2,2-3,5 ppm : m : 18H ; 4-5 ppm : m : 4H ; 6,6-8,7 ppm : m : 17H ; 10,7 ppm : se : 1H.

Exemple 44 : RMN ; 1,2 ppm : mt : 6H ; 1,4-1,8 ppm : m : 4H ; 2,2-3,3 ppm : m : 15H ; 4,1-4,9 ppm : m : 4H ; 6,8-8,1 ppm : m : 12H ; 8,35 ppm : d : 1H ; 8,9 ppm : d : 1H ; 10,25 ppm : se : 1H.

Exemple 47 : RMN : 0,5-1,3 ppm : m : 12H ; 2,1-3,1 ppm : m : 11H ; 3,6-5 ppm : m : 5H ; 6,2-8,5 ppm : m : 22H ; 10,1 ppm : se : 1H.

(suite)

| Exemple | $R_1$ | $R_2$ | $R_3$ | $R_4$ | $R_5$ |
|---|---|---|---|---|---|
| Exemple 48 : RMN : 1 ppm : s : 9H ; 1,2-1,7 ppm : m : 4H ; 1,9 ppm : s : 3H ; 2,2-3,5 ppm : m : 10H ; 4,1-4,75 ppm : m : 2H ; 6,7-8,4 ppm : m : 18H. | | | | | |
| Exemple 49 : RMN : 0,95 ppm : t : 6H ; 2,1-3,6 ppm : m : 10H ; 4,4-5,1 ppm : m : 2H ; 6,6-8,6 ppm : m : 23H. | | | | | |
| Exemple 50 : RMN : 0,5-1 ppm : m : 6H ; 1,7-3,5 ppm : m : 16H ; 4-5 ppm : m : 5H ; 5,2-5,7 ppm : m : 2H ; 6,2-8,4 ppm : m : 21H ; 10,7 ppm : se : 1H. | | | | | |
| Exemple 51 : RMN : 0,4-1,3 ppm : m : 12H ; 2,1-3,1 ppm : m : 11H ; 3,6-4,8 ppm : m : 5H ; 6,6-8,5 ppm : m : 17H ; 10 ppm : se : 1H. | | | | | |
| Exemple 52 : RMN : 1,15 ppm : t : 6H ; 2,2 ppm : t : 2H ; 2,5 ppm : s : 3H ; 2,7-3,1 ppm : m : 6H ; 3,8 ppm : t : 2H ; 4,15 ppm : d : 2H ; 4,9 ppm : qd : 1H ; 6,9-7,7 ppm : m : 16H ; 8,5 ppm : d : 1H ; 10,5 ppm : se : 1H | | | | | |
| Exemple 53 : RMN : 0,4-1,5 ppm : m : 20H ; 2-2,85 ppm : m : 11H ; 3,3-4,8 ppm : m : 5H ; 5,2-5,7 ppm : 2s : 2H ; 6,2-8,3 ppm : m : 16H. | | | | | |
| Exemple 54 : RMN : 1,4 ppm : s : 9H ; 2-2,6 ppm : m : 8H ; 2,9 ppm : d : 2H ; 3,6-4,6 ppm : m : 4H ; 4,9 ppm : qd : 1H ; 6,9-7,7 ppm : m : 16H ; 8,5 ppm : d : 1H ; 9,9 ppm : se : 1H. | | | | | |
| Exemple 55 : RMN : 1,15 ppm : t : 6H ; 2,4 ppm : s : 3H ; 2,8-3,1 ppm : m : 6H ; 3,75 ppm : t : 2H ; 4,15 ppm : d : 2H ; 4,9 ppm : qd : 1H ; 5,95 ppm : s : 2H ; 6,4-7,7 ppm : m : 14 H ; 8,4 ppm : d : 1H ; 10,1 ppm : se : 1H. | | | | | |
| Exemple 56 : RMN : 0,7-1,3 ppm : m : 12H ; 2,2 ppm : t : 2H ; 2,3-3,1 ppm : m : 12H ; 3,6-5 ppm : m : 6H ; 6 ppm : s : 2H ; 6,4-7,7 ppm : m : 9H ; 8,3 ppm : dd : 1H ; 10,2 ppm : se : 1H. | | | | | |
| Exemple 57 : RMN : 1,05 ppm : sd : 9H ; 1,85 ppm : sd : 3H ; 2,1-3,4 ppm : m : 6H ; 4,3-4,9 ppm : m : 2H ; 5,2-5,8 ppm : 2sd : 2H ; 6,1-8,4 ppm : m : 21H. | | | | | |
| Exemple 60 : RMN : 1,25 ppm : t : 6H ; 2,3 ppm : mt : 2H ; 2,5 ppm : s : 3H ; 3 ppm : mt : 6H ; 3,85 ppm : t : 2H ; 4,25 ppm : d : 2H ; 5,25 ppm : qd : 1H ; 6,1 ppm : s : 2H ; 6,4-9,2 ppm : m : 14H ; 10,7 ppm : se : 1H. | | | | | |
| Exemple 61 : RMN : 1,2 ppm : t : 6H ; 2,3 ppm : mt : 2H ; 2,5 ppm : s : 3H ; 2,7-3,3 ppm : m : 6H ; 3,9 ppm : t : 2H ; 4,2 ppm : d : 2H ; 5,1 ppm : qd : 1H ; 6,1-7,8 ppm : m : 14H ; 8,45 ppm : d : 1H ; 10,6 ppm : se : 1H. | | | | | |
| Exemple 62 : RMN : 0,5-1,4 ppm : m : 12H ; 2-5 ppm : m : 16H ; 5,95-8,5 ppm : m : 16H. | | | | | |
| Exemple 64 : RMN : 0,6-1,3 ppm : m : 12H ; 2-3,1 ppm : m : 17H ; 3,6-5 ppm : m : 6H ; 6,7-7,7 ppm : m : 10H ; 8,3 ppm : dd: 19H; 9,9 ppm : se : 1H. | | | | | |
| Exemple 65 : RMN : 0,85 ppm : td : 6H ; 2,1-2,9 ppm : m : 8H ; 3,2-4,7 ppm : m : 6H ; 5,2-5,8 ppm : m : 3H ; 6,1-8,3 ppm : m : 19H. | | | | | |
| Exemple 68 : RMN : 0,6-1,3 ppm : m : 12H ; 2-3,1 ppm : m : 17H ; 3,6-5 ppm : m : 6H ; 6,7-7,7 ppm : m : 10H ; 8,3 ppm : dd : 1H ; 9,9 ppm : se : 1H. | | | | | |
| Exemple 69: RMN: 0,65-1,3 ppm : m : 12H ; 2,15 ppm : t : 2H ; 2,25 ppm : s : 3H ; 2,3-3,1 ppm : m : 9H ; 3,7-5 ppm : m: 6H ; 7-7,7 ppm : m : 11H ; 8,4 ppm : dd : 1H ; 9,9 ppm : se : 1H. | | | | | |
| Exemple 71 : RMN : 1,2 ppm : t : 6H ; 2,1-2,6 ppm : m : 5H ; 2,8-3,5 ppm : m : 6H ; 3,9 ppm : t : 2H ; 4,2 ppm : de : 2H ; 5,2 ppm : qd : 1H ; 7-7,8 ppm : m : 13H ; 8,9 ppm : d : 1H ; 10,4 ppm : se : 1H ; 14,4 ppm : se : 2H. | | | | | |
| Exemple 72 : RMN : 1-1,9 ppm : m : 10H ; 2,2 ppm : t : 2H ; 2,5 ppm : s : 3H ; 2,6-3,5 ppm : m : 10H ; 3,75 ppm : t : 2H ; 4,15 ppm : d : 2H ; 4,5 ppm : qd : 1H ; 6 ppm : s : 2H ; 6,3-7,7 ppm : m : 9H ; 8,3 ppm : d : 1H ; 10,2 ppm : s : 1H. | | | | | |
| Exemple 73 : RMN : 0,9 ppm : mt : 6H ; 1,3 ppm : mt : 2H ; 1,9-2,2 ppm : m : 5H ; 2,25-3,6 ppm : m : 12H ; 4,2 ppm : mt : 1H; 4,55 ppm : mt : 1H ; 5,2-5,7 ppm : m : 2H; 6,2-8,4 ppm : m : 17H. | | | | | |
| Exemple 74 : RMN: 0,9 ppm : mt : 6H ; 1,3-3,5 ppm : m : 19H ; 4,3-4,8 ppm : m : 2H ; 5,2-5,7 ppm : m : 2H ; 6,2-8,4 ppm : m : 16H. | | | | | |
| Exemple 77 : RMN : 0,5-1,4 ppm : m : 18H ; 2,1-3 ppm : m : 8H ; 3,3-4,8 ppm : m : 7H ; 5,2-5,65 ppm : m : 2H ; 6,2-8,4 ppm : m : 16H ; 9,15 ppm : s : 1H. | | | | | |
| Exemple 78 : RMN : 1,4 ppm : s : 9H ; 2,2-2,75 ppm : m : 5H ; 2,9 ppm : t : 2H ; 3,8 ppm : mt : 1H ; 4,5 ppm : mt : 2H ; 5 ppm : mt : 1H ; 5,1-5,7 ppm : 2sd : 2H ; 6,2-9,1 ppm : m : 20H ; 9,6 ppm : s : 1H. | | | | | |
| Exemple 79 : RMN : 0,7-1,3 ppm : m : 12H ; 2,1-3,1 ppm : m : 11H ; 3,7-5 ppm : m : 6H ; 7,1-8,2 ppm : m : 16H ; 10,85 ppm : s : 1H. | | | | | |

(suite)

| Exemple | $R_1$ | $R_2$ | $R_3$ | $R_4$ | $R_5$ |
|---|---|---|---|---|---|
| Exemple 80 : RMN : 0,7-1,4 ppm : m : 12H ; 2,2-3,2 ppm : m : 14H ; 3,7-5 ppm : m : 6H ; 7-8,8 ppm: m: 11H ; 10,8 ppm: s : 1H. | | | | | |
| Exemple 82 : RMN : 0,7-1,5 ppm : m : 12H ; 2,4-3,3 ppm : m : 14H ; 4-5,1 ppm : m : 6H ; 7,2-9 ppm : m : 11H ; 10,8 ppm : s : 1H. | | | | | |
| Exemple 83 : RMN : 1,4 ppm : s : 9H ; 2-3 ppm : m : 10H ; 3,6-4,6 ppm : m: 4H ; 4,9 ppm : mt : 1H ; 5,95 ppm : s : 2H ; 6,3-7,7 ppm : m : 14H ; 8,4 ppm : d : 1H ; 9,65 ppm : se : 1H. | | | | | |
| Exemple 84 : RMN : 0,6-1,4 ppm : m : 12H ; 2,4-3,2 ppm : m : 11H ; 3,8-5,1 ppm : m : 6H ; 7-8,9 ppm : m : 15H ; 10,9 ppm : se : 2H. | | | | | |
| Exemple 85 : RMN : 1 ppm : t : 6H ; 2,3-3,7 ppm : m : 10H ; 4,7 ppm : mt : 1H ; 5 ppm : mt : 1H ; 5,1-5,8 ppm : 2sd : 2H ; 6,2-8,8 ppm : m : 20H ; 12,15 ppm : sd : 1H. | | | | | |
| Exemple 86 : RMN : 1,2-2,55 ppm : m : 16 H ; 2,85 ppm : mt : 2H ; 3,1-4,4 ppm : m : 5H ; 4,9 ppm : mt : 1H ; 6,8-7,6 ppm : m : 16H ; 8,4 ppm : d : 1H ; 10,6 ppm : se : 1H. | | | | | |
| Exemple 87 : RMN : 0,6-1,3 ppm : m : 12H ; 2,2 ppm : t : 2H ; 2,35-3,1 ppm : m : 12H ; 3,7-5 ppm : m : 6H ; 6,9-7,8 ppm : m : 9H ; 8,3 ppm : dd : 1H ; 10,1 ppm : se : 1H. | | | | | |
| Exemple 88 : RMN : 0,7-1,3 ppm : m : 12H ; 2,3 ppm : t : 2H ; 2,4-3,1 ppm : m : 12H ; 3,5-5 ppm : m : 6H ; 7-7,7 ppm : m : 10H ; 8,4 ppm : dd : 1H; 10 ppm : se : 1H. | | | | | |
| Exemple 89 : RMN : 1,05 ppm : s : 9H ; 1,85 ppm : s : 3H ; 2,2 ppm : t : 2H ; 2,6 ppm : s : 3H ; 2,8 ppm : mt : 2H ; 3,4 ppm : s : 2H ; 3,7 ppm : s : 2H ; 4,9 ppm : mt : 1H ; 5,95 ppm : s : 2H ; 6,3-8,6 ppm : m : 14H. | | | | | |
| Exemple 90 : RMN : 1,5 ppm : s : 9H ; 2,2-2,6 ppm : m : 8H ; 3 ppm : m : 2H; 3,8-4,6 ppm : 2m : 4H ; 5,2 ppm : m : 1H ; 7-9,2 ppm : m : 17H ; 10 ppm : se : 1H. | | | | | |
| Exemple 91 : RMN : 1,5 ppm : s : 9H ; 2,3 ppm : t : 2H ; 2,4-2,6 ppm : m : 6H ; 3 ppm : de : 2H ; 3,8-4,1 ppm : m : 3H ; 4,4-5 ppm : m : 2H ; 6 ppm : s : 2H ; 6,6-7,8 ppm : m : 14H ; 8,5 ppm : d : 1H ; 10,4 ppm : se : 1H. | | | | | |
| Exemple 94 : RMN : 0,9 ppm : t : 6H ; 2,1-3,7 ppm : m : 14H ; 4,50 ppm : mt : 1H ; 5-5,7 ppm : m : 3H ; 6-8,6 ppm : m : 20H. | | | | | |
| Exemple 95 : RMN : 0,95 ppm : t : 6H ; 2,1-2,7 ppm : m : 9H ; 3-4 ppm : m : 9H ; 5,4 ppm : qd : 1H ; 6,05 ppm : s : 2H ; 6,4-7,8 ppm : m : 13H ; 8,7 ppm : d : 1H. | | | | | |
| Exemple 99 : RMN : 1,5 ppm : s : 9H ; 1,9-2,55 ppm : m : 10H ; 2,6-3,1 ppm : m : 6H : 3,75-4,8 ppm : m : 4H ; 5,05 ppm : mt : 1H ; 6,7-7,8 ppm : m : 14H ; 8,55 ppm : d : 1H ; 9,4 ppm : se : 1H. | | | | | |
| Exemple 101 : RMN : 0,5-1,4 ppm : m : 18H ; 2,1-3 ppm : m : 7H ; 3,3-4,9 ppm : m : 11H ; 6,1-8,4 ppm : m : 16H ; 9,1 ppm : se : 1H. | | | | | |
| Exemple 102 : RMN : 0,8-1,2 ppm : m : 6H ; 1,5 ppm : s : 9H ; 2,2-3,1 ppm : m : 13H ; 3,7-5,1 ppm : m : 6H : 6,1 ppm : s : 1H ; 6,5-7,8 ppm : m : 9H ; 8,3-8,6 ppm : m : 1H ; 9,5 ppm : se : 1H. | | | | | |
| Exemple 103 : RMN : 0,8-1,5 ppm : m : 12H ; 2,35 ppm : t : 2H ; 2,5 ppm : s : 6H ; 2,6-3,1 ppm : m : 5H ; 3,45 ppm : mt : 1H ; 3,8-5,1 ppm : m : 6H ; 6,15 ppm : s : 2H ; 6,5-7,8 ppm : m : 9H ; 8,3-8,6 ppm : m : 1H ; 10,5 ppm : se : 1H. | | | | | |
| Exemple 104 : RMN : 0,7 ppm : mt : 6H ; 1,4 ppm : s : 9H ; 2,2-3 ppm : m : 10H ; 3,8 ppm : mt : 1H ; 4,3-4,9 ppm : m : 4H ; 5,2-5,7 ppm : 2s : 2H ; 6,2-8,4 ppm : m : 16H ; 9,5 ppm : s : 1H. | | | | | |
| Exemple 105 : RMN : 1,4-2,4 ppm : m : 14H ; 2,5 ppm : s : 6H ; 2,65 à 5 ppm : m : 12H ; 6,1 ppm : s : 2H ; 6,5-7,8 ppm : m : 9H ; 8,45 ppm : d : 1H ; 10,8 ppm : se : 1H. | | | | | |
| Exemple 106 : $MH^+$ : 765 | | | | | |
| Exemple 107 : $MH^+$ : 765 | | | | | |
| Exemple 108 : RMN : 0,6 à 1,4 ppm : m : 15H ; 2,1 à 3,5 ppm : m : 11H ; 3,6 à 4,9 ppm : m : 5H ; 5,2 à 5,7 ppm : 2s : 2H ; 6,2 à 6,5 ppm : m : 3H ; 7 à 8,4 ppm : m : 13H ; 9,8 ppm : se : 1H. | | | | | |
| Exemple 109 : RMN : 0,5 à 1,5 ppm : m : 18H ; 2,1 à 3 ppm : m : 10H ; 3,4 à 4,9 ppm : m : 7H ; 5,75 à 5,80 ppm : 2s : 2H ; 6,2 à 6,65 ppm : m : 3H ; 7 à 7,6 ppm : m : 7H ; 8,1 à 8,4 ppm : m : 2H ; 8,8 ppm : se : 1H. | | | | | |

EXEMPLE 110

(I), HCl : (R,R) $R_1$ = 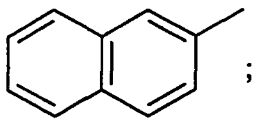 ; $R_2$ = H ; $R_3$ = 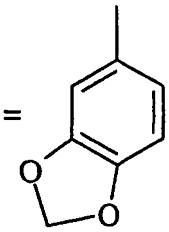 ;

$R_4$ = 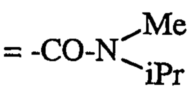 ; $R_5$ = 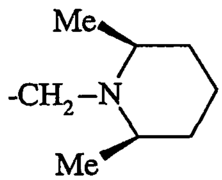

On laisse une nuit sous agitation à TA un mélange contenant 250 mg du composé de la préparation 3.8, 74 mg de chlorure de naphtalène-2-sulfonyle et 171 µl de DIPEA dans 10 ml de DCM. On concentre à sec le milieu puis on reprend par AcOEt, lave à l'eau et par une solution saturée de $NaHCO_3$. On extrait par un tampon pH = 2 puis on basifie à pH = 8 par une solution saturée de $NaHCO_3$. On extrait au DCM puis lave par une solution saturée de NaCl. On reprend dans un mélange AcOEt/$Et_2O$ et on prépare le chlorhydrate par addition d'éther chlorhydrique. On obtient 140 mg du composé attendu.
RMN : 0,7 à 2,1 ppm : m : 18H ; 2,3 à 3,2 ppm : m : 9H ; 3,3 ppm : mt : 1H ; 3,8 à 5 ppm : m : 4H ; 5,4 à 5,8 ppm : 2s : 2H ; 6,4 à 6,8 ppm : m : 3H ; 7,1 à 8,2 ppm : m : 11H ; 8,2 à 8,5 ppm : mt : 2H ; 9,4 à 10,3 ppm : 2se : 1H.

$$\alpha_D^{25} = +50° \text{ (c = 1 ; MeOH)}$$

$MH^+$ : 727

EXEMPLE 111

(I), HCl : (R,R) $R_1$ = 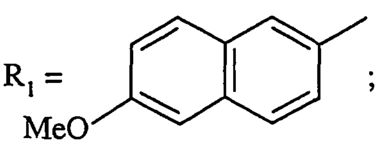 ; $R_2$ = H ; $R_3$ = 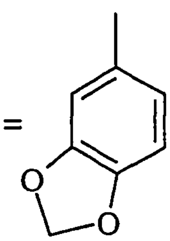 ;

$R_4$ = 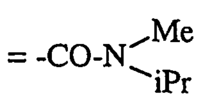 ; $R_5$ = 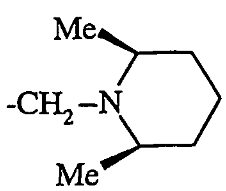

Ce composé est préparé en procédant selon l'exemple décrit ci-dessus, à partir du composé de la préparation 3.8 et du chlorure de 6-méthoxynaphtalène-2-sulfonyle obtenu selon J. Med. Chem., 1999, 42, 3557-3571.

RMN : 0,6 à 2 ppm : m : 18H ; 2,2 à 3,6 ppm : m : 10H ; 3,8 à 5 ppm : m : 7H ; 5,4 à 5,8 ppm : 2s : 2H ; 6,3 à 6,6 ppm : m : 3H ; 7,1 à 8,1 ppm : m : 10H ; 8,1 à 8,5 ppm : m : 2H ; 9,4 à 10,2 ppm : 2se : 1H.

$\alpha_D^{25}$ = +32° (c = 1 ; MeOH)

$MH^+$ : 757

EXEMPLE 111 bis

(I) : (R,R) $R_1$ = ; $R_2$ = H ; $R_3$ = ; $R_4$ = ; $R_5$ =

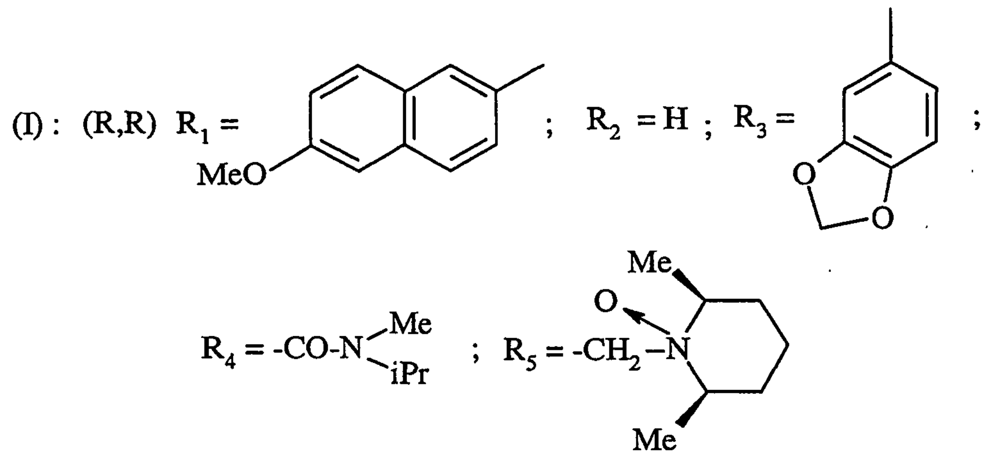

On place 152 mg du composé de l'Exemple 111 dans un mélange de 50 ml de DCM et 20 ml de solution saturée de $NaHCO_3$. La phase organique obtenue est séchée puis concentrée pour obtenir le composé sous forme de base. Le milieu est repris par 5 ml de DCM, on traite par 75 mg d'acide métachloroperbenzoïque à 60 % pendant 1 heure à TA. On extrait au DCM puis la phase organique et lavée par une solution saturée de NaCl. On obtient 0,14 g du composé attendu qui cristallise dans le méthyl-*tert*-butyléther.

MH+ : 773

RMN : 0,6 à 1,8 ppm : m : 18H ; 2,2 à 3,4 ppm : m : 10H ; 3,8 ppm : s : 3H ; 4,3 à 4,9 ppm : m : 4H ; 5,4 à 5,6 ppm : d : 2H ; 6,3 ppm : mt : 3H ; 7,1 à 7,8 ppm : m : 10H.

EXEMPLE 112

(I), HCl : (R,R) $R_1$ = ; $R_2$ = H ; $R_3$ = ; $R_4$ = ; $R_5$ =

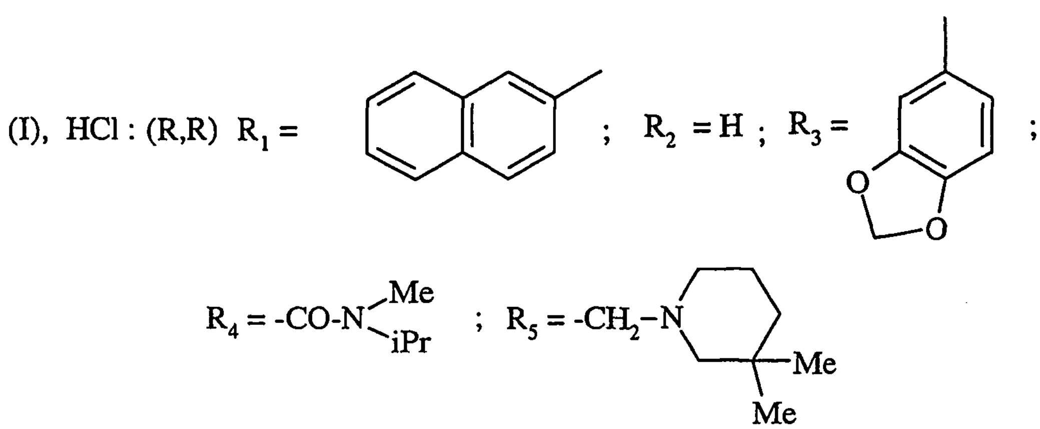

On place 225 mg du produit obtenu à la préparation 3.9 dans 3 ml de TFA et 10 ml de DCM et on laisse sous agitation 30 minutes à TA. On concentre le milieu puis on triture dans $iPr_2O$. On décante l'huile formée. L'huile formée

est reprise dans 5 ml de DCM et on traite par 100 µl de TEA puis 82 mg de chlorure de naphtalène-2-sulfonyle en maintenant à pH = 7 par addition de TEA. On laisse sous agitation une nuit à TA puis on concentre le milieu. On extrait par AcOEt, lave à l'eau (3 fois), par une solution saturée de NaCl. On prépare le chlorhydrate qui cristallise du mélange $AcOEt/Et_2O$.
RMN : 0,5 à 2 ppm : m : 16H ; 2,2 à 3 ppm : m : 11H ; 3,2 à 4,8 ppm : m : 5H ; 5,3 et 5,6 ppm : 2s : 2H ; 6,3 à 6,6 ppm : m : 3H ; 7 à 7,3 ppm : m : 2H ; 7,4 à 7,8 ppm : m : 6H ; 7,9 à 8,1 ppm : m : 5H ; 8,3 ppm : mt : 1H.

$$\alpha_D^{25} = +42,1° \ (c = 1 \ ; MeOH)$$

$MH^+$ : 727

EXEMPLE 113

(I), HCl : (R,R) $R_1$ = 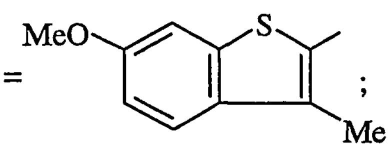 ; $R_2$ = H ; $R_3$ = 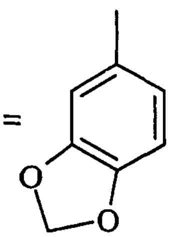 ;

$R_4$ = 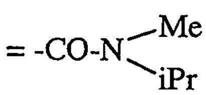 ; $R_5$ = $-CH_2-$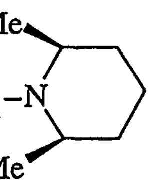

Ce composé est préparé selon les exemples décrits ci-dessus à partir du composé de la préparation 3.8 et du composé de la préparation 1.33, étape A.
$MH^+$ : 777

EXEMPLE 114

(I), HCl : (R,R) $R_1$ = 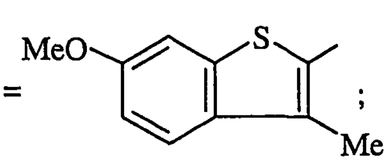 ; $R_2$ = H ; $R_3$ = 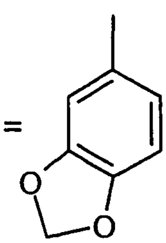 ;

$R_4$ = 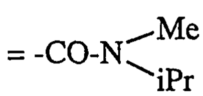 ; $R_5$ = $-CH_2-$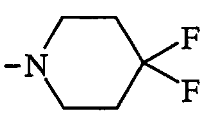

Ce composé est préparé selon les exemples décrits ci-dessus à partir du composé de la préparation 3.10 et du chlorure de sulfonyle décrit à la préparation 1.33, étape A.
$MH^+$ : 785

D'autres composés de formule (I) selon l'invention ont été préparés en utilisant les méthodes décrites ci-dessus et identifiés par leur spectre de masse et leur spectre RMN. Ils sont décrits dans les 2 Tableaux suivants :

TABLEAU X

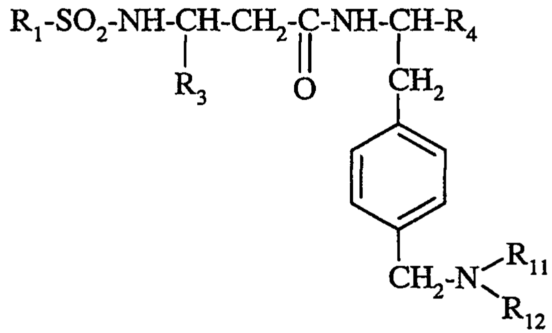

(I)

| Exemples | $R_1$ | $R_3$ | $R_4$ | 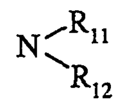 | Sel |
|---|---|---|---|---|---|
| 115 | 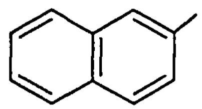 | 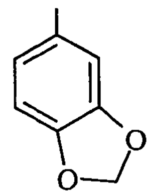 | 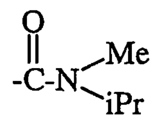 | 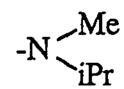 | - |
| 116 (R,R) | 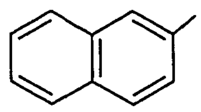 | 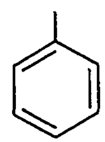 | 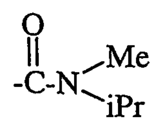 | 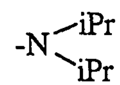 | HCl |
| 117 | 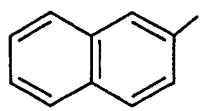 | 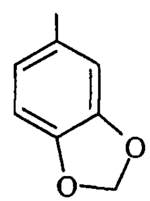 | 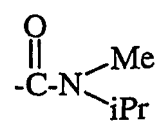 | 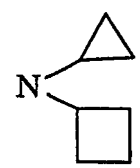 | HCl |

(suite)

| Exemples | $R_1$ | $R_3$ | $R_4$ | 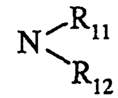 | Sel |
|---|---|---|---|---|---|
| 118 | 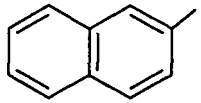 | 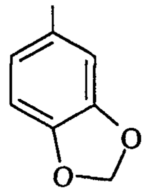 | 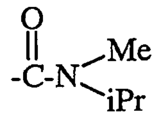 | 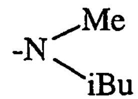 | HCl |
| 119 (R,R) | 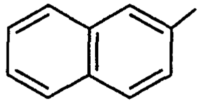 | 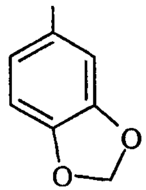 | 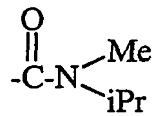 | 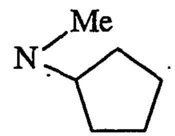 | HCl |
| 120 | 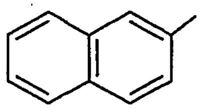 | 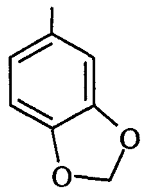 | 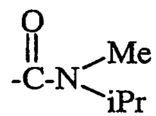 | 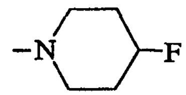 | HCl |
| 121 | 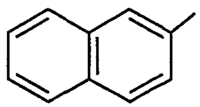 | 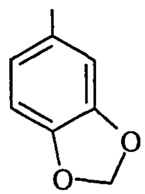 | 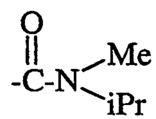 | 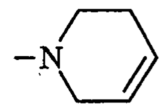 | HCl |
| 122(R,R) | 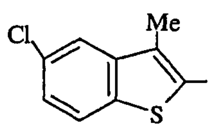 | 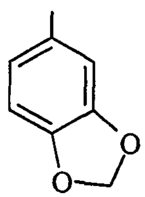 | 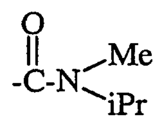 | 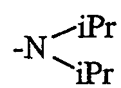 | HCl |

(suite)

| Exemples | $R_1$ | $R_3$ | $R_4$ | 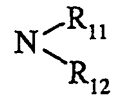 | Sel |
|---|---|---|---|---|---|
| 123 (R,R) | 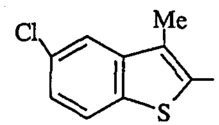 | 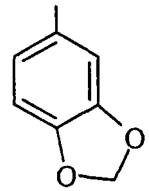 | 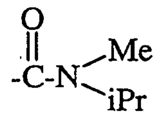 | 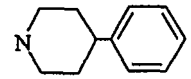 | HCl |
| 124 (R,R) | 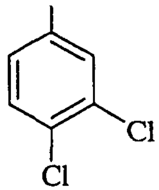 | 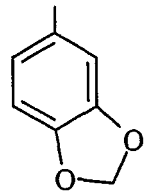 | 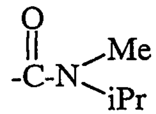 | 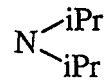 | HCl |
| 125 (R,R) | 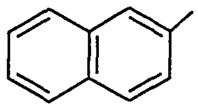 | 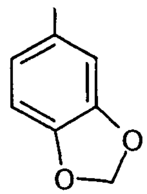 | 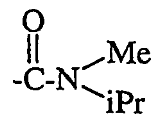 | 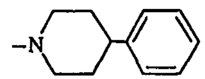 | HCl |
| 126 | 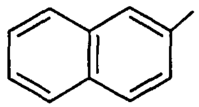 | 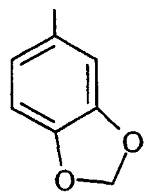 | 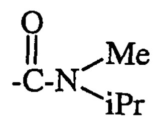 | 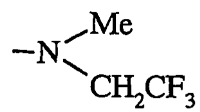 | - |
| 127 | 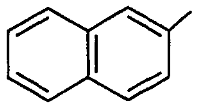 | 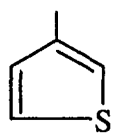 | 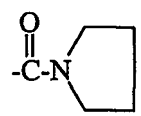 | 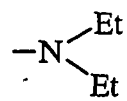 | HCl |

(suite)

| Exemples | $R_1$ | $R_3$ | $R_4$ | 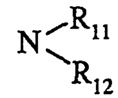 | Sel |
|---|---|---|---|---|---|
| 128 | 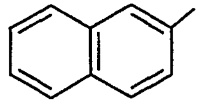 | 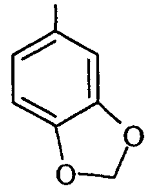 | 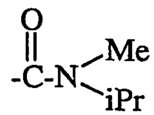 | 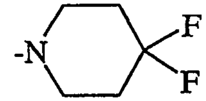 | - |
| 129 - | 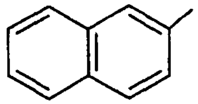 | 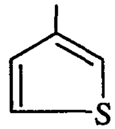 | 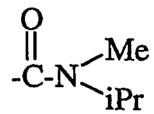 | 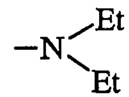 | HCl |
| 130 - | 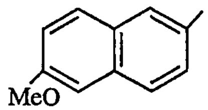 | 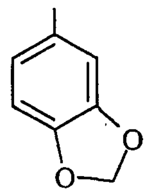 | 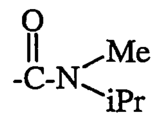 | 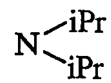 | HCl |
| 131 - | 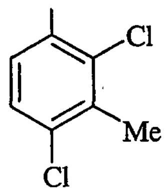 | 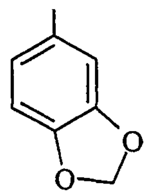 | 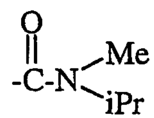 | 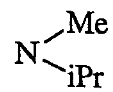 | HCl |
| 132 - | 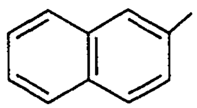 | 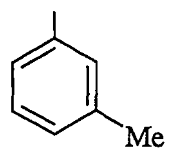 | 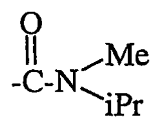 | 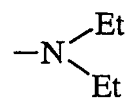 | HCl |

(suite)

| Exemples | $R_1$ | $R_3$ | $R_4$ | 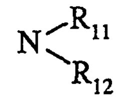 | Sel |
|---|---|---|---|---|---|
| 133 | 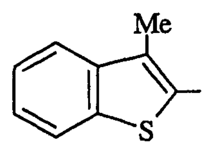 | 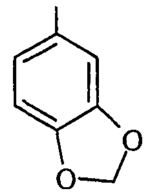 | 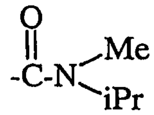 | 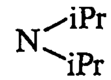 | HCl |
| 134 (R,R) | 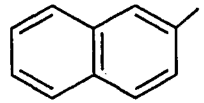 | 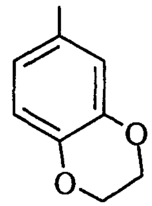 | 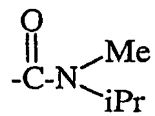 | 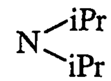 | HCl |
| 135 (R,R) | 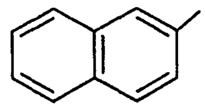 | 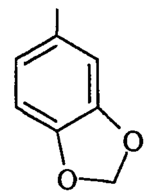 | 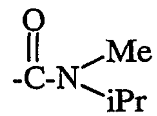 | 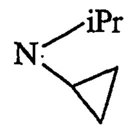 | HCl |
| 136 - | 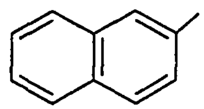 | 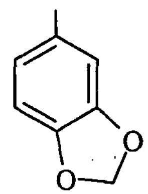 | 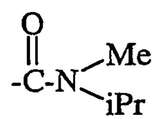 | 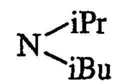 | HCl |
| 137 - | 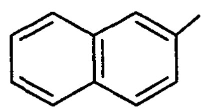 | 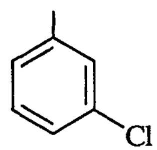 | 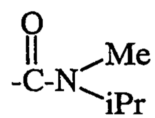 | 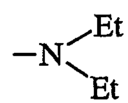 | HCl |

(suite)

| Exemples | $R_1$ | $R_3$ | $R_4$ | 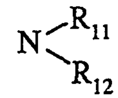 | Sel |
|---|---|---|---|---|---|
| 138<br>- | 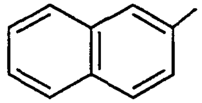 | 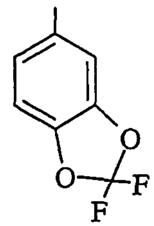 | 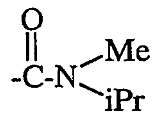 | 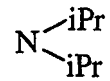 | HCl |
| 139<br>- | 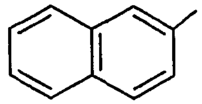 | 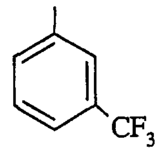 | 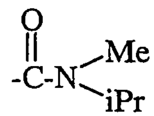 | 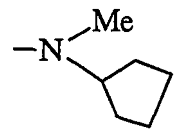 | HCl |
| 140<br>- | 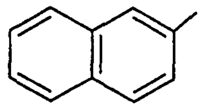 | 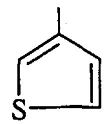 | 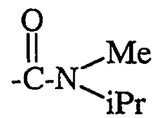 | 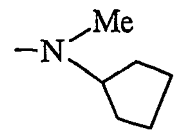 | HCl |
| 141<br>(R,R) | 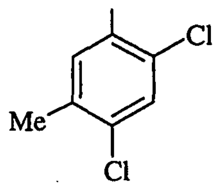 | 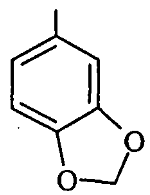 | 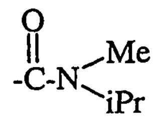 | 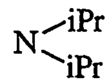 | HCl |
| 142<br>(R,R) | 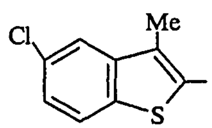 | 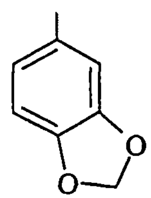 | 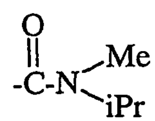 | 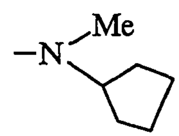 | HCl |

(suite)

| Exemples | $R_1$ | $R_3$ | $R_4$ | 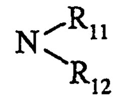 | Sel |
|---|---|---|---|---|---|
| 143 - | 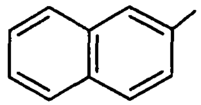 | 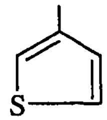 | 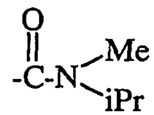 | 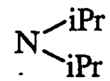 | HCl |
| 144 (R,R) | 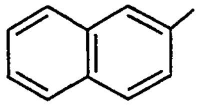 | 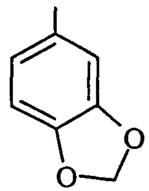 | 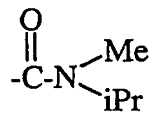 | 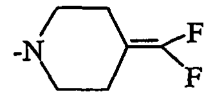 | HCl |
| 145 - | 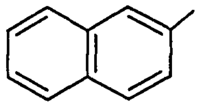 | 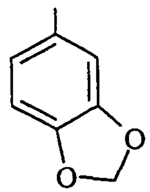 | 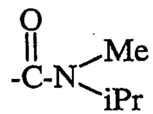 | 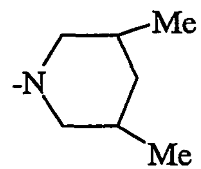 | HCl |
| 146 - | 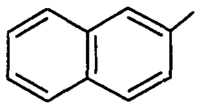 | 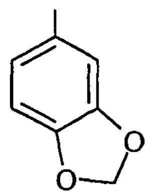 | 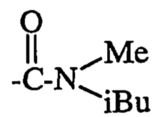 | 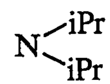 | HCl |
| 147 - | 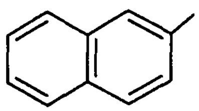 | 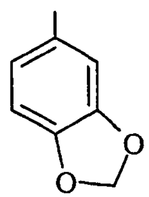 | 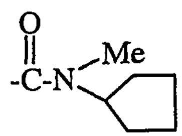 | 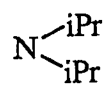 | HCl |

(suite)

| Exemples | $R_1$ | $R_3$ | $R_4$ | 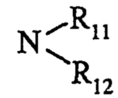 | Sel |
|---|---|---|---|---|---|
| 148 (R,R) | 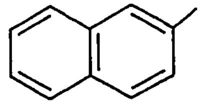 | 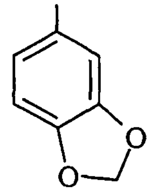 | 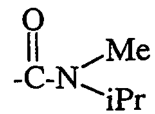 | 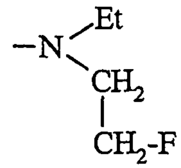 | HCl |
| 149 | 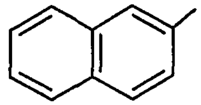 | 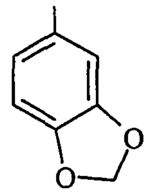 | 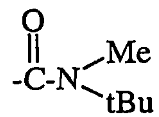 | 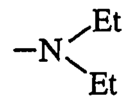 | - |
| 150 | 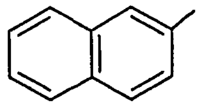 | 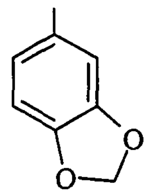 | 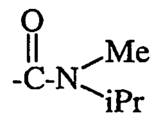 | 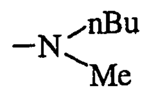 | HCl |
| 151 | 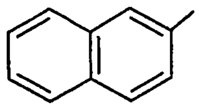 | 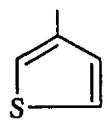 | 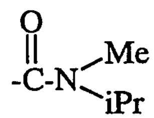 | 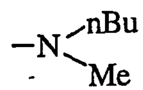 | HCl |
| 152 | 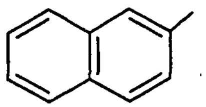 | 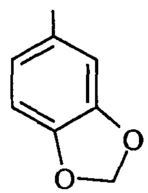 | 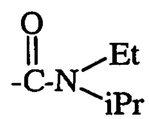 | 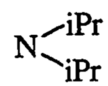 | HCl |

(suite)

| Exemples | $R_1$ | $R_3$ | $R_4$ | 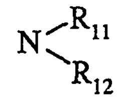 | Sel |
|---|---|---|---|---|---|
| 153 | 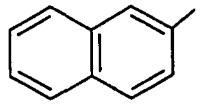 | 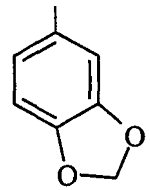 | 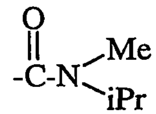 | 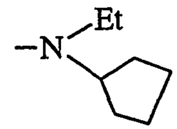 | HCl |
| 154 | 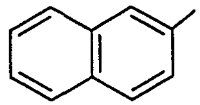 | 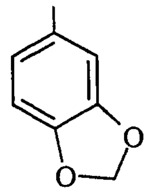 | 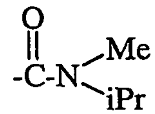 | 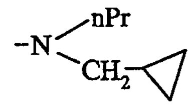 | HCl |
| 155 | 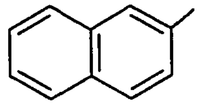 | 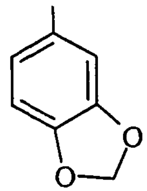 | 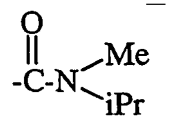 | 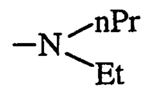 | HCl |
| 156 | 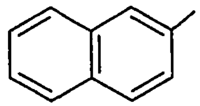 | 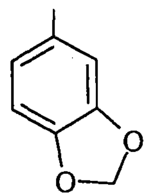 | 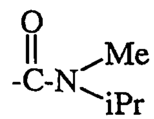 | 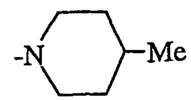 | |
| 157 | 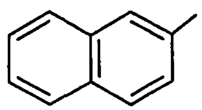 | 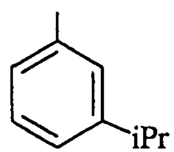 | 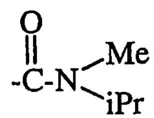 | 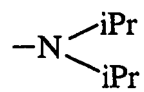 | HCl |

(suite)

| Exemples | R1 | R3 | R4 | 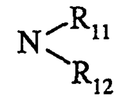 | Sel |
|---|---|---|---|---|---|
| 158 | 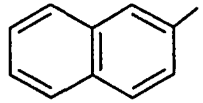 | 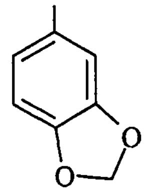 | 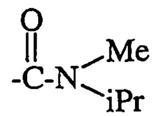 | 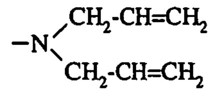 | HCl |
| 159 | 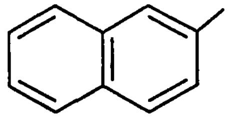 | 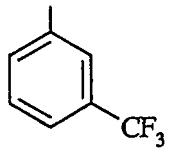 | 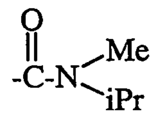 | 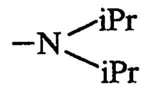 | HCl |
| 160 | 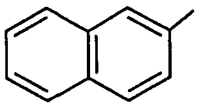 | 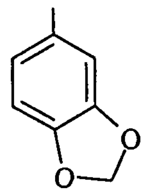 | 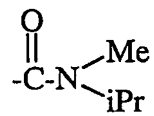 | 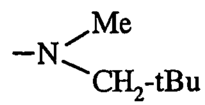 | HCl |
| 161 (R,R) | 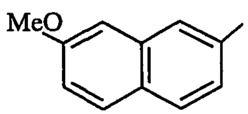 | 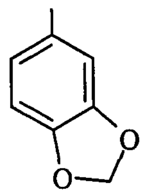 | 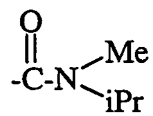 | 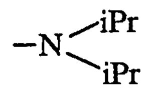 | HCl |
| 162 (R,R) | 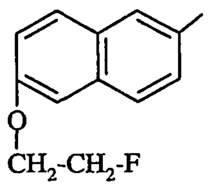 | 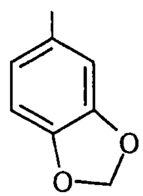 | 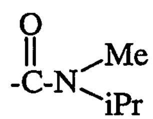 | 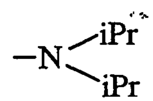 | HCl |

(suite)

| Exemples | $R_1$ | $R_3$ | $R_4$ | 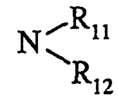 | Sel |
|---|---|---|---|---|---|
| 163 (R,R) | 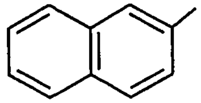 | 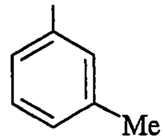 | 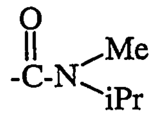 | 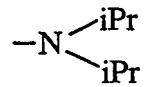 | HCl |
| 164 (R,R) | 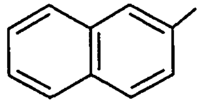 | 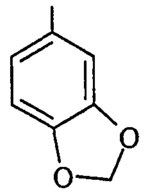 | 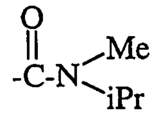 | 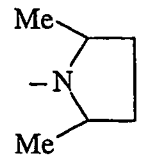 | HCl |
| 165 (R,R) a) | 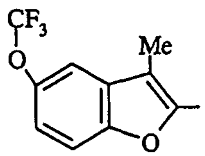 | 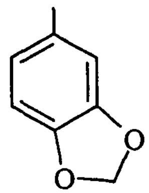 | 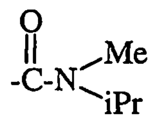 | 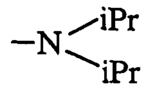 | HCl |
| 166 (R,R) | 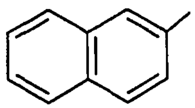 | 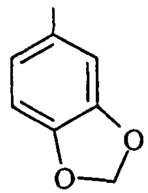 | 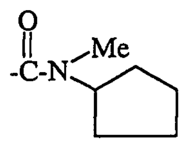 | 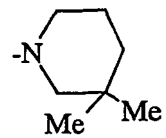 | HCl |
| 167 | 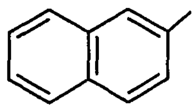 | 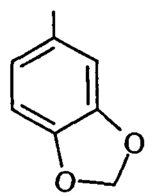 | 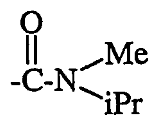 | 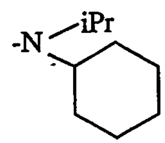 | HCl |

(suite)

| Exemples | $R_1$ | $R_3$ | $R_4$ | 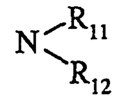 | Sel |
|---|---|---|---|---|---|
| 168 | 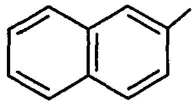 | 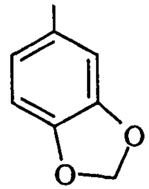 | 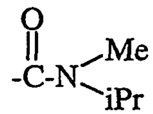 | 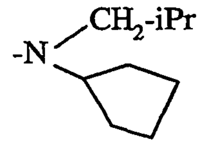 | HCl |
| 169 (R,R) | 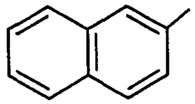 | 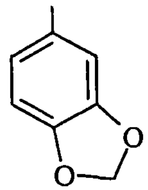 | 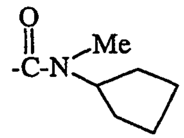 | 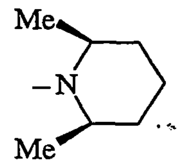 | HCl |
| 170 (R,R) | 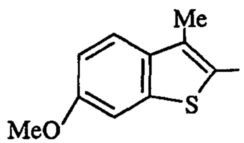 | 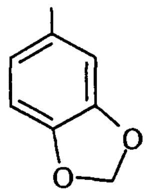 | 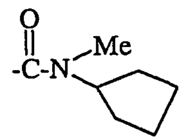 | 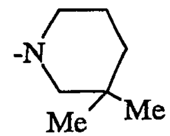 | HCl |
| 171 (R,R) a) | 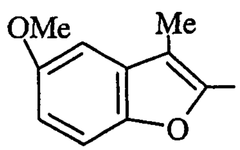 | 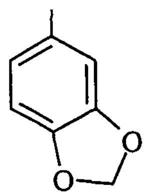 | 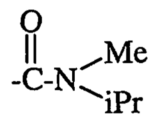 | 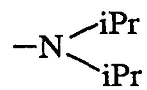 | HCl |

(suite)

| Exemples | $R_1$ | $R_3$ | $R_4$ | 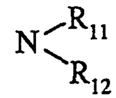 | Sel |
|---|---|---|---|---|---|
| 172 (R,R) | 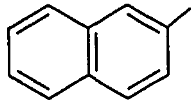 | 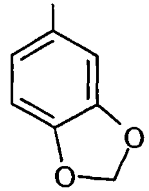 | 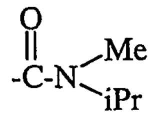 | 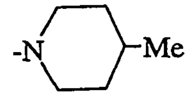 | HCl |
| 173 (R,R) | 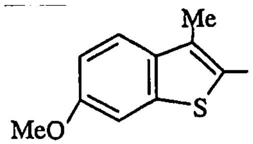 | 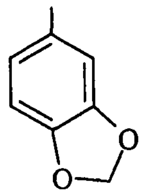 | 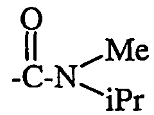 | 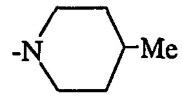 | HCl |
| 174 (R,R) | 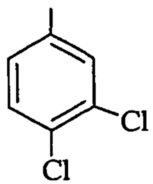 | 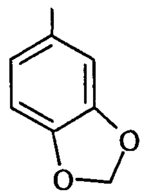 | 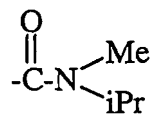 | 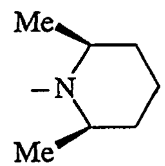 | HCl |
| 175 | 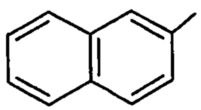 | 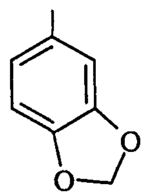 | 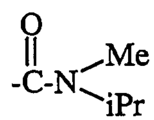 | 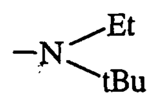 | HCl |
| 176 (R,R) | 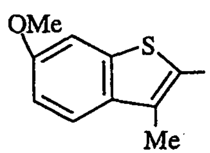 | 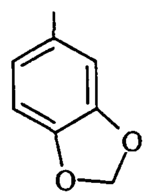 | 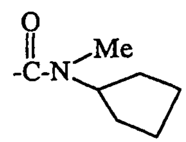 | 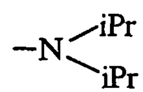 | HCl |

(suite)

| Exemples | $R_1$ | $R_3$ | $R_4$ | 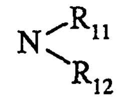 | Sel |
|---|---|---|---|---|---|
| 177 - | 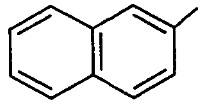 | 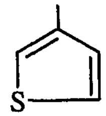 | 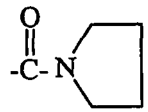 | 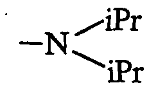 | HCl |
| 178 (R,R) | 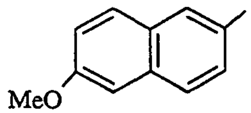 | 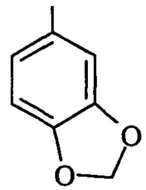 | 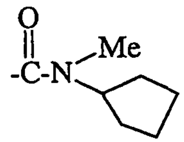 | 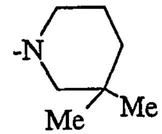 | HCl |
| 179 R,(R,S) | 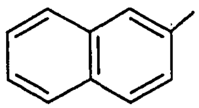 | 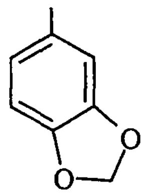 | 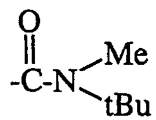 | 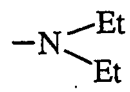 | HCl |
| 180 | 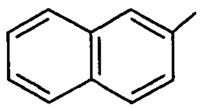 | 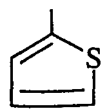 | 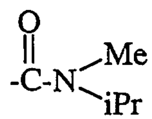 | 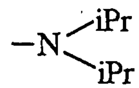 | HCl |
| 181 (R,R) | 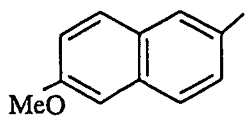 | 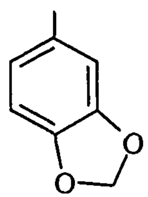 | 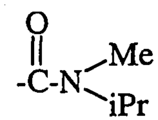 | 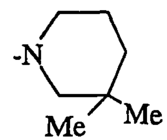 | HCl |

(suite)

| Exemples | $R_1$ | $R_3$ | $R_4$ | 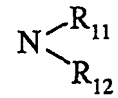 | Sel |
|---|---|---|---|---|---|
| 182 | 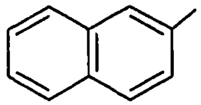 | 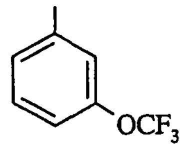 | 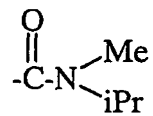 | 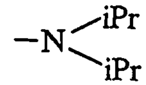 | HCl |
| 183 | 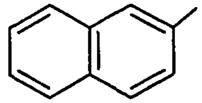 | 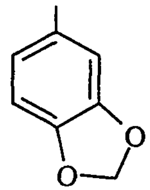 | 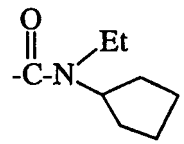 | 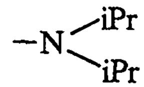 | HCl |
| 184 (R,R) | 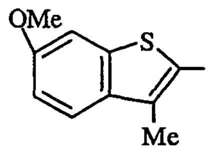 | 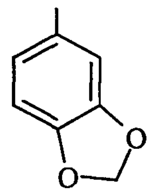 | 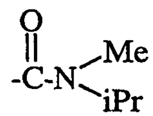 | 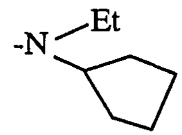 | HCl |
| 185 (R,R) | 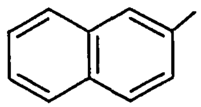 | 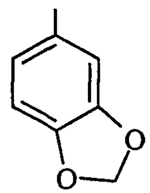 | 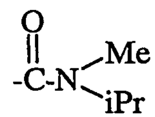 | 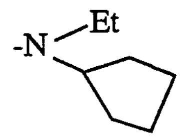 | HCl |
| 186 (R,R) | 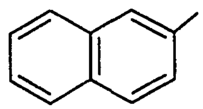 | 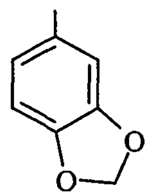 | 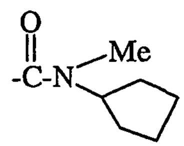 | 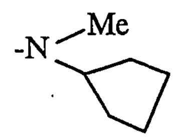 | HCl |

(suite)

| Exemples | $R_1$ | $R_3$ | $R_4$ | 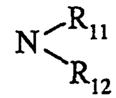 | Sel |
|---|---|---|---|---|---|
| 187 (R,R) | 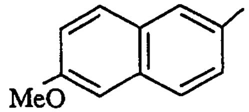 | 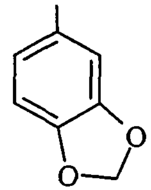 | 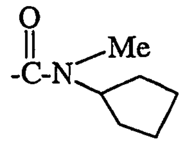 | 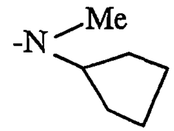 | HCl |
| 188 - | 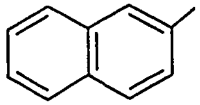 | 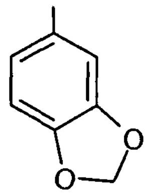 | 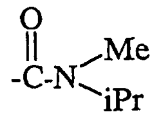 | 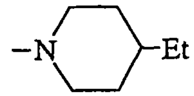 | HCl |
| 189 (R,R) | 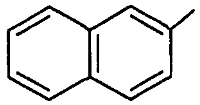 | 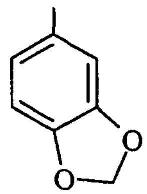 | 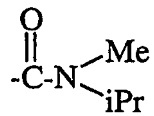 | 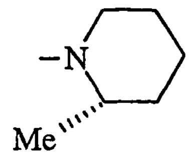 | HCl |
| 190 - | 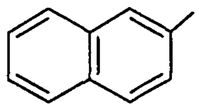 | 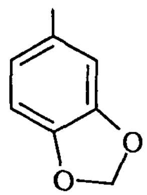 | 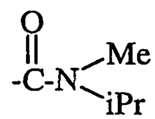 | 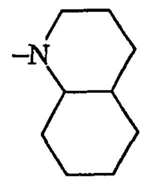 | HCl |
| 191 (R,(R,S)) | 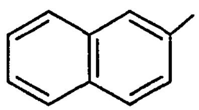 | 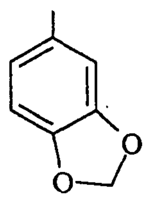 | 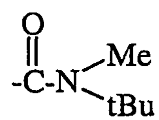 | 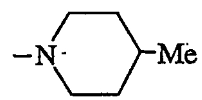 | HCl |

(suite)

| Exemples | $R_1$ | $R_3$ | $R_4$ | 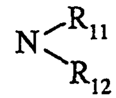 | Sel |
|---|---|---|---|---|---|
| 192 (R,R) | 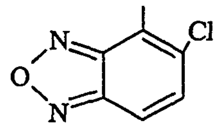 | 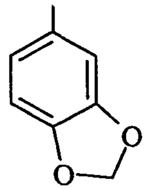 | 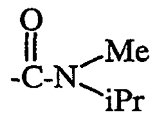 | 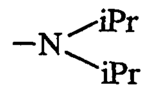 | HCl |
| 193 | 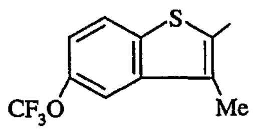 | 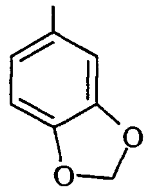 | 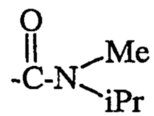 | 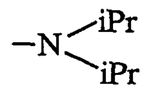 | HCl |
| 194 (R,R) | 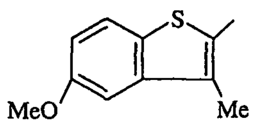 | 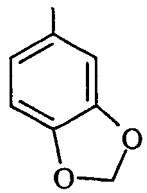 | 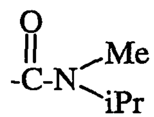 | 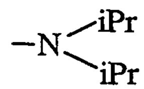 | HCl |
| 195 | 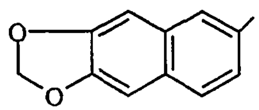 | 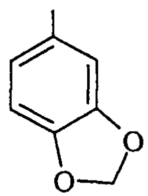 | 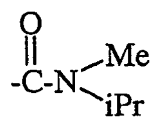 | 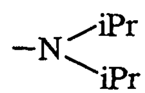 | HCl |
| 196 (R,R) | 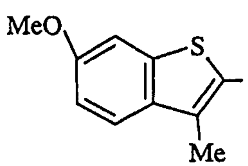 | 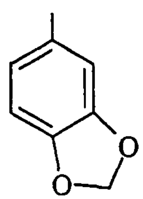 | 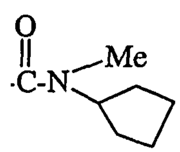 | 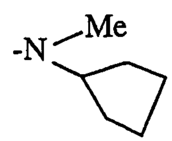 | HCl |

(suite)

| Exemples | $R_1$ | $R_3$ | $R_4$ | 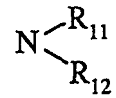 | Sel |
|---|---|---|---|---|---|
| 197 - | 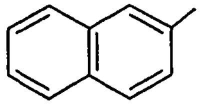 | 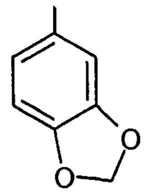 | 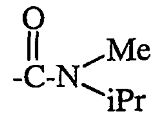 | 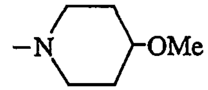 | $HPF_6$ |
| 198 (R,R) | 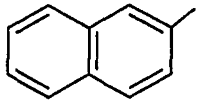 | 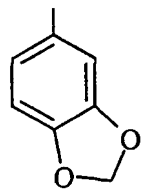 | 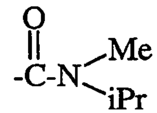 | 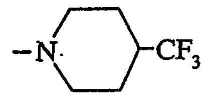 | HCl |
| 199 | 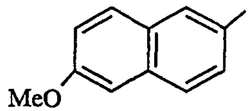 | 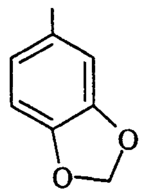 | 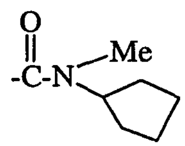 | 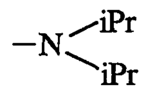 | HCl |
| 200 (R,R) | 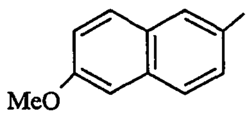 | 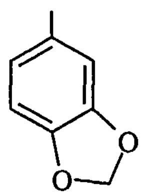 | 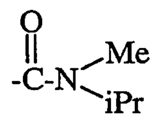 | 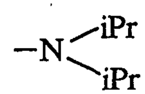 | HCl |
| 201 - | 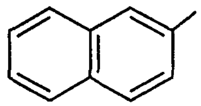 | 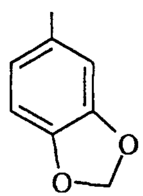 | 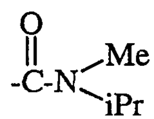 | 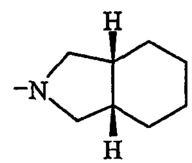 | HCl |

(suite)

| Exemples | $R_1$ | $R_3$ | $R_4$ | 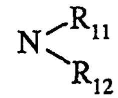 | Sel |
|---|---|---|---|---|---|
| 202 (R,R) | 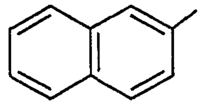 | 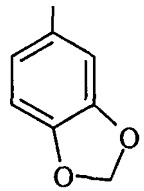 | 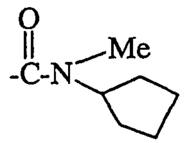 | 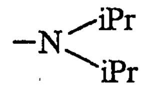 | HCl |
| 203 | 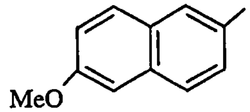 | 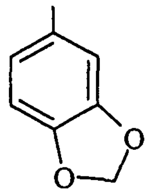 | 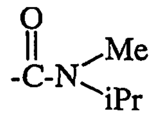 | 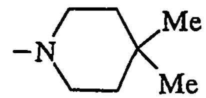 | HCl |
| 204 (R,R) | 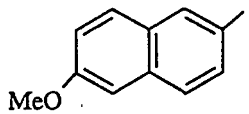 | 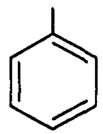 | 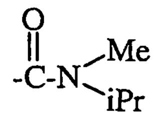 | 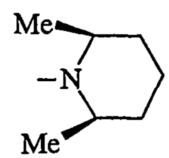 | HCl |
| 205 (R,R) | 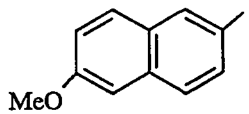 | 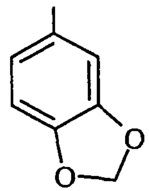 | 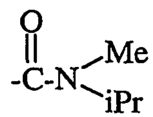 | 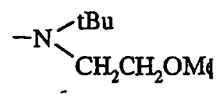 | HCl |
| 206 - | 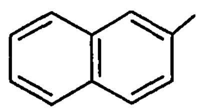 | 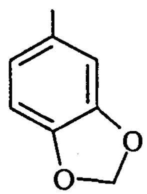 | 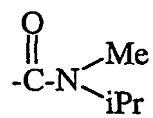 | 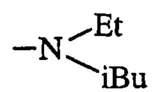 | HCl |

(suite)

| Exemples | $R_1$ | $R_3$ | $R_4$ | 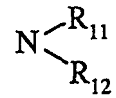 | Sel |
|---|---|---|---|---|---|
| 207 (R,R) | 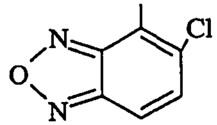 | 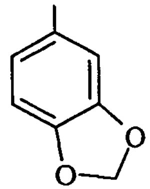 | 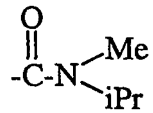 | 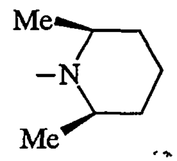 | HCl |
| 208 | 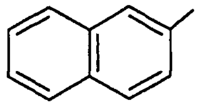 | 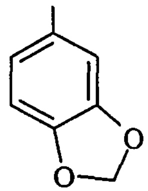 | 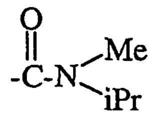 | 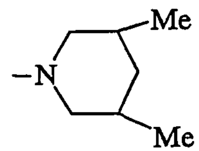 | HCl |
| 209 (R,R) | 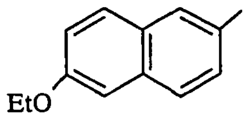 | 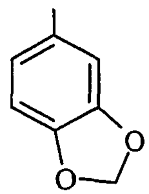 | 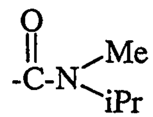 | 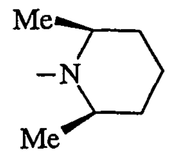 | HCl |
| 210 (R,R) | 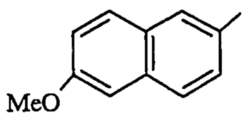 | 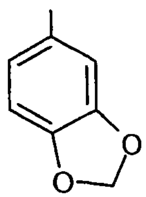 | 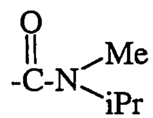 | 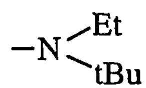 | - |

(suite)

| Exemples | $R_1$ | $R_3$ | $R_4$ | 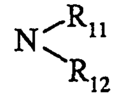 | Sel |
|---|---|---|---|---|---|
| 211 | 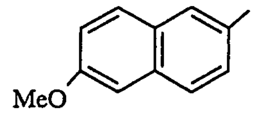 | 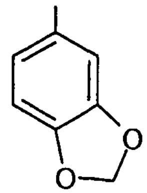 | 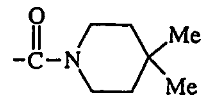 | 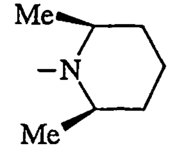 | HCl |
| 212<br>- | 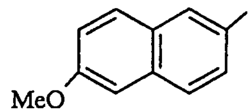 | 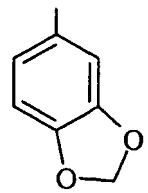 | 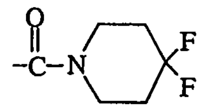 | 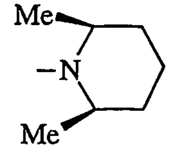 | HCl |
| 213<br>- | 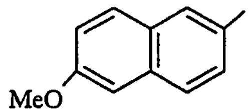 | 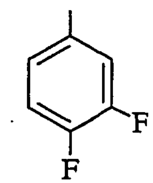 | 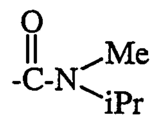 | 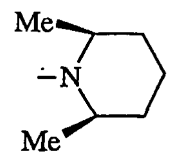 | HCl |
| 214<br>(R,R) | 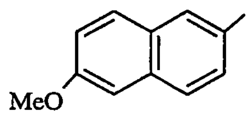 | 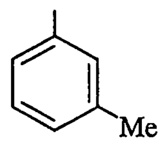 | 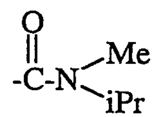 | 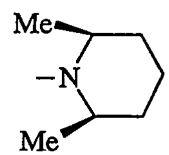 | HCl |
| 215<br>(R,R) | 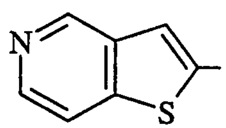 | 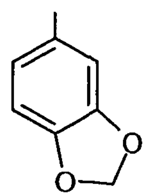 | 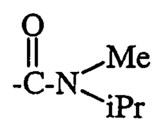 | 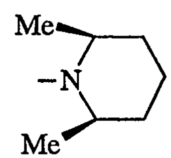 | HCl |

(suite)

| Exemples | $R_1$ | $R_3$ | $R_4$ | 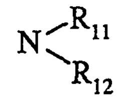 | Sel |
|---|---|---|---|---|---|
| 216 (R,R) | 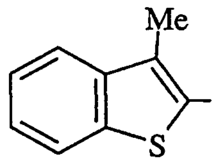 | 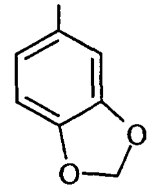 | 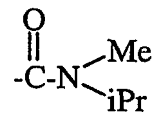 | 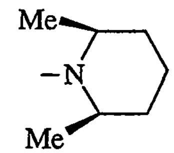 | HCl |
| 217 (R,R) | 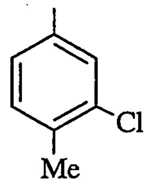 | 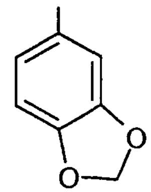 | 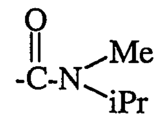 | 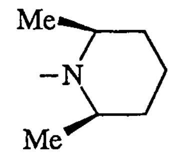 | HCl |
| 218 (R,R) | 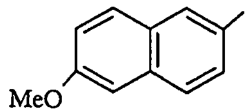 | 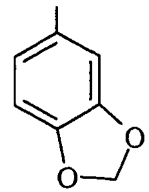 | 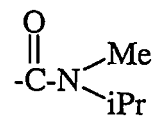 | 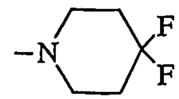 | HCl |
| 219 | 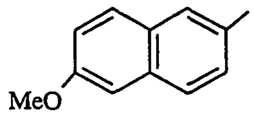 | 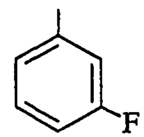 | 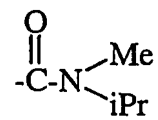 | 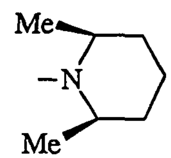 | HCl |
| 220 - | 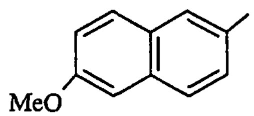 | 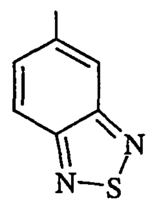 | 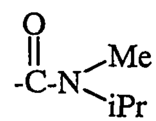 | 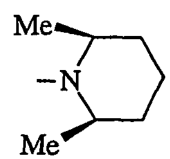 | HCl |

(suite)

| Exemples | $R_1$ | $R_3$ | $R_4$ | 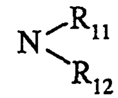 | Sel |
|---|---|---|---|---|---|
| 221<br>- | 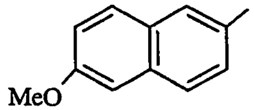 | 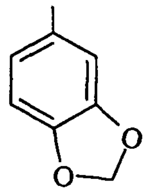 | 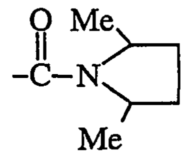 | 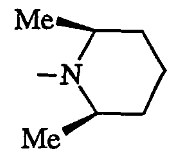 | HCl |
| 222<br>(R,R) | 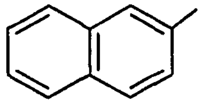 | 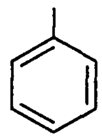 | 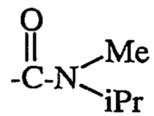 | 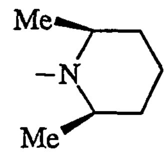 | HCl |
| 223<br>(R,R) | 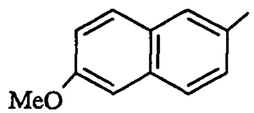 | 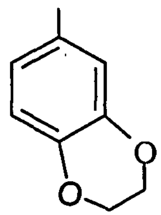 | 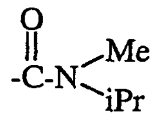 | 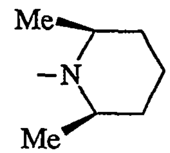 | HCl |
| 224<br>(R,R) | 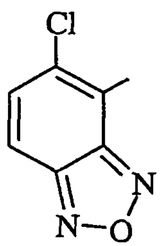 | 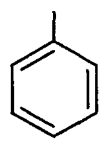 | 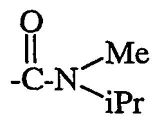 | 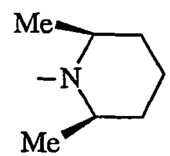 | HCl |

(suite)

| Exemples | $R_1$ | $R_3$ | $R_4$ | 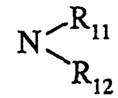 | Sel |
|---|---|---|---|---|---|
| 225 (R,R) | 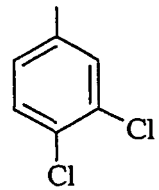 | 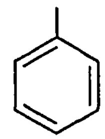 | 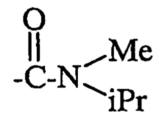 | 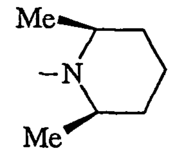 | HCl |
| 226 (R,R) | 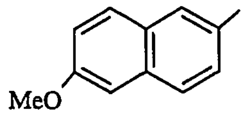 | 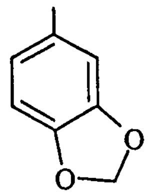 | 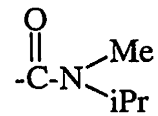 | 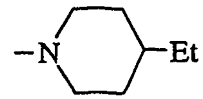 | HCl |
| 227 (R,R) | 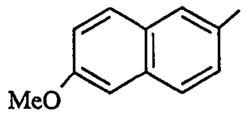 | 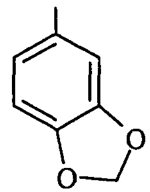 | 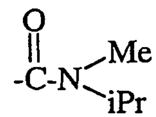 | 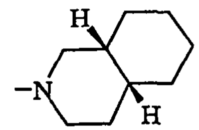 | HCl |
| 228 | 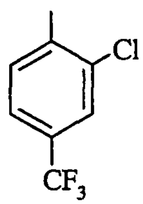 | 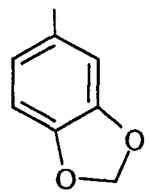 | 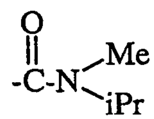 | 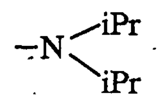 | HCl |
| 229 (R,R) | 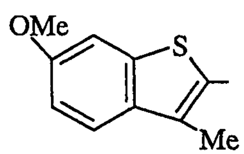 | 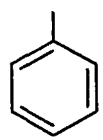 | 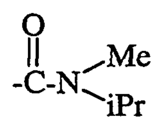 | 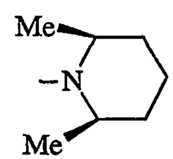 | HCl |

(suite)

| Exemples | $R_1$ | $R_3$ | $R_4$ | 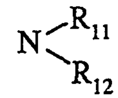 | Sel |
|---|---|---|---|---|---|
| 230 (R,R) | 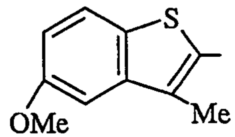 | 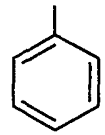 | 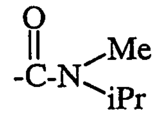 | 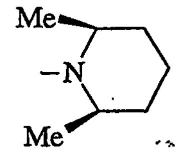 | HCl |
| 231 (R,R) | 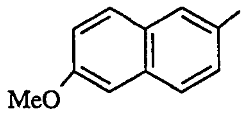 | 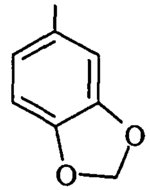 | 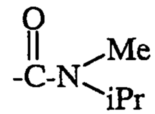 | 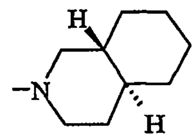 | HCl |
| 232 (R,R) | 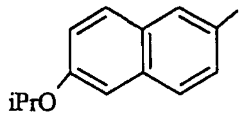 | 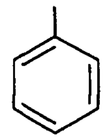 | 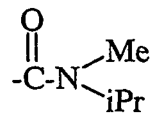 | 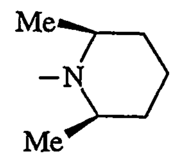 | HCl |
| 233 (R,R) | 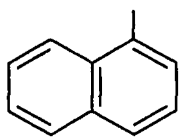 | 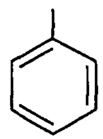 | 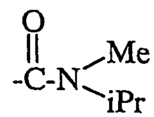 | 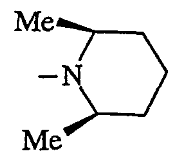 | HCl |
| 234 - | 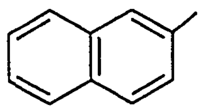 | 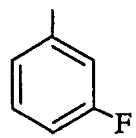 | 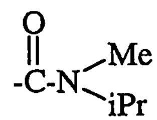 | 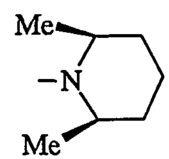 | HCl |

(suite)

| Exemples | $R_1$ | $R_3$ | $R_4$ | 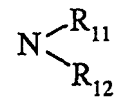 | Sel |
|---|---|---|---|---|---|
| 235<br>- | 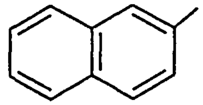 | 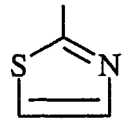 | 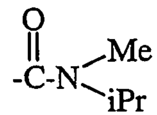 | 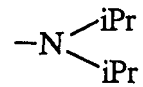 | HCl |
| 236<br>(R,R) | 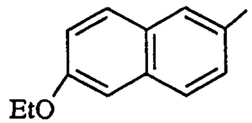 | 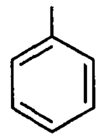 | 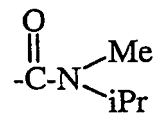 | 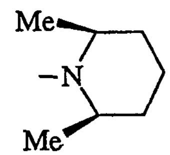 | HCl |
| 237<br>((R,S),R) | 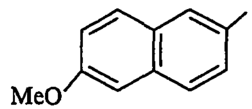 | 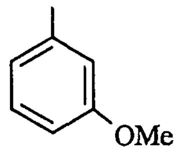 | 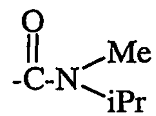 | 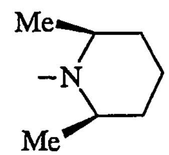 | HCl |
| 238<br>((R,S),R) | 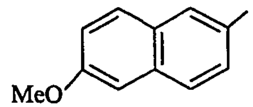 | 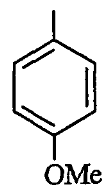 | 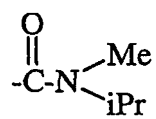 | 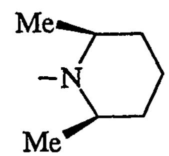 | HCl |
| 239<br>(R,R) | 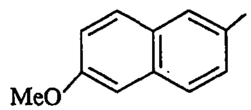 | 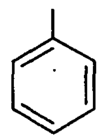 | 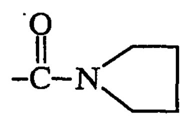 | 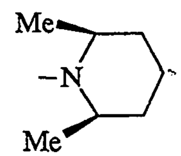 | HCl |

(suite)

| Exemples | $R_1$ | $R_3$ | $R_4$ | 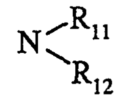 | Sel |
|---|---|---|---|---|---|
| 240 (R,R) | 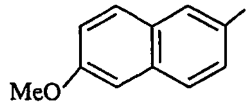 | 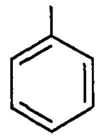 | 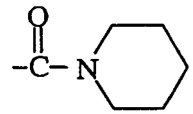 | 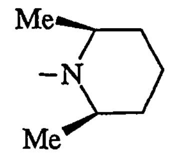 | HCl |
| 241 (R,R) | 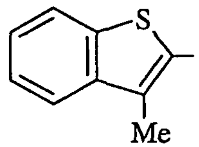 | 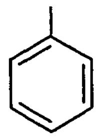 | 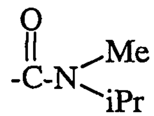 | 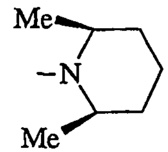 | HCl |
| 242 | 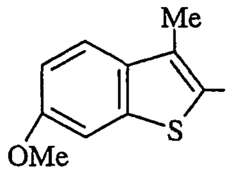 | 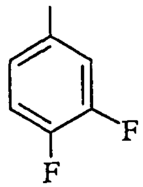 | 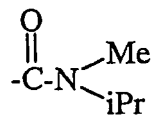 | 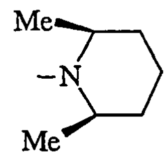 | HCl |
| 243 | 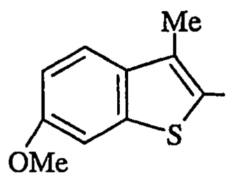 | 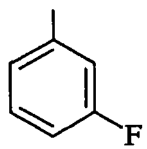 | 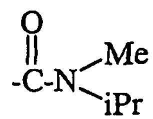 | 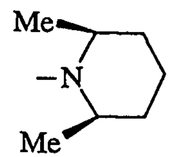 | HCl |
| 244 | 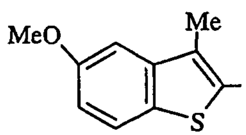 | 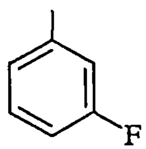 | 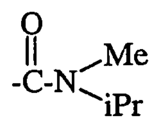 | 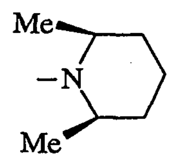 | HCl |

(suite)

| Exemples | $R_1$ | $R_3$ | $R_4$ | 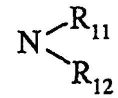 | Sel |
|---|---|---|---|---|---|
| 245 (R,R) | 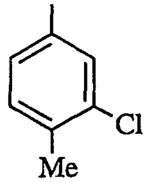 | 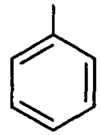 | 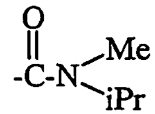 | 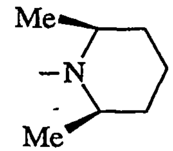 | HCl |
| 246 (R,R) | 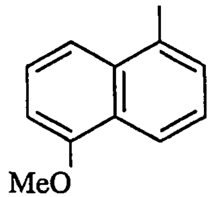 | 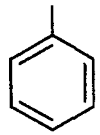 | 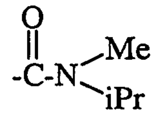 | 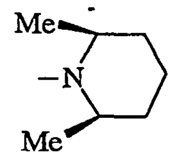 | HCl |
| 247 (R,R) | 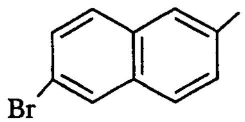 | 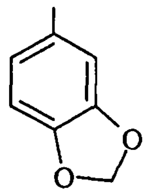 | 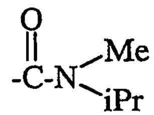 | 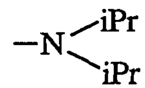 | HCl |
| 248 (R,R) | 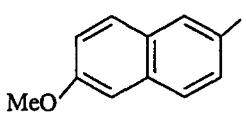 | 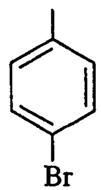 | 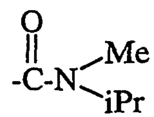 | 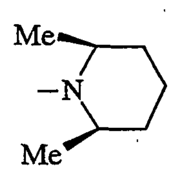 | HCl |
| 249 ((R),R,S)) | 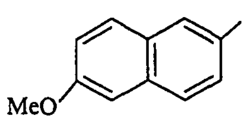 | 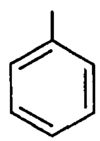 | 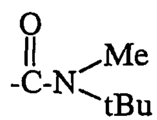 | 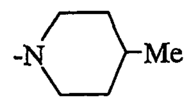 | HCl |

(suite)

| Exemples | $R_1$ | $R_3$ | $R_4$ | 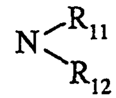 | Sel |
|---|---|---|---|---|---|
| 250 (R,R) | 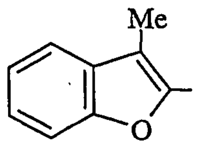 | 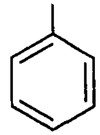 | 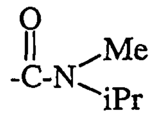 | 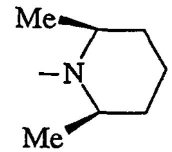 | HCl |
| 251 (R,R) | 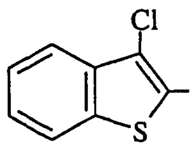 | 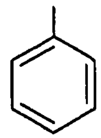 | 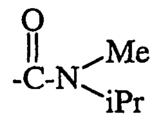 | 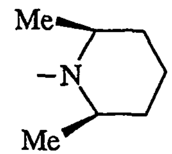 | HCl |
| 252 (R,R) | 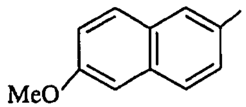 | 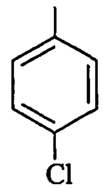 | 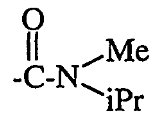 | 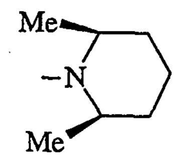 | HCl |
| 253 (R,R) | 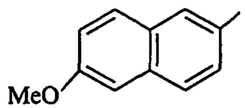 | 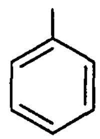 | 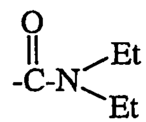 | 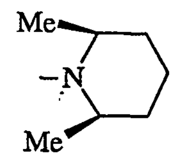 | HCl |
| 254 (R,R) | 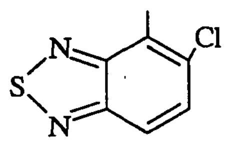 | 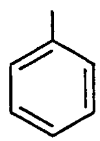 | 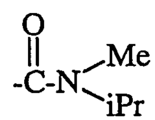 | 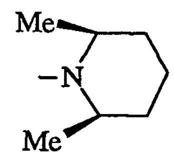 | HCl |

(suite)

| Exemples | $R_1$ | $R_3$ | $R_4$ | 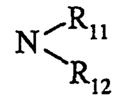 | Sel |
|---|---|---|---|---|---|
| 255 (R,R) | 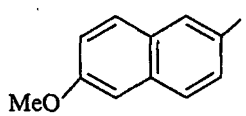 | 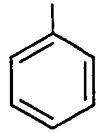 | 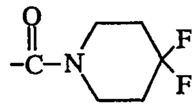 | 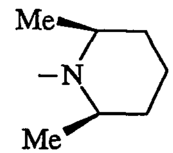 | HCl |
| 256 (R,R) | 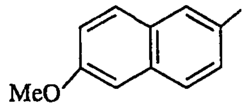 | 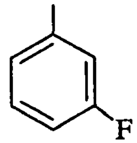 | 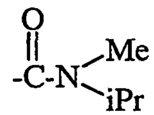 | 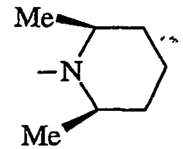 | HCl |
| 257 | 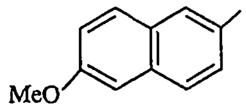 | 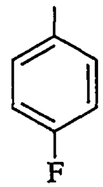 | 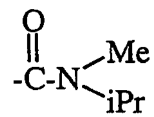 | 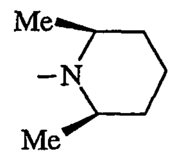 | HCl |
| 258 ((R,S),R) | 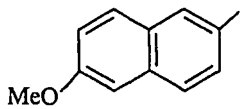 | 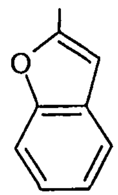 | 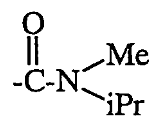 | 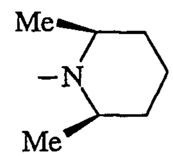 | HCl |
| 259 (R,R) | 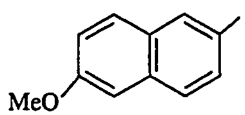 | 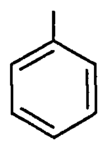 | 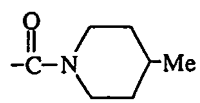 | 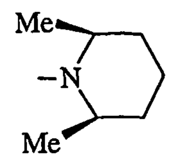 | HCl |

(suite)

| Exemples | $R_1$ | $R_3$ | $R_4$ | 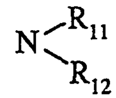 | Sel |
|---|---|---|---|---|---|
| 260 (R,R) | 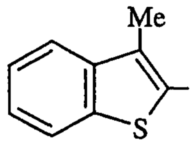 | 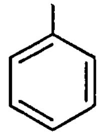 | 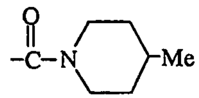 | 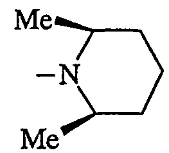 | HCl |
| 261 (R,R) | 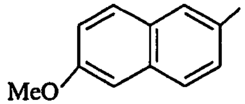 | 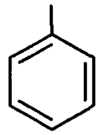 | 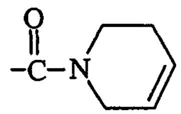 | 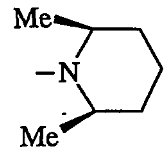 | HCl |
| 262 ((R,S),R) | 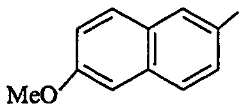 | 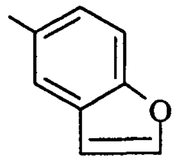 | 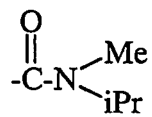 | 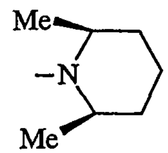 | HCl |
| 263 (R,R) | 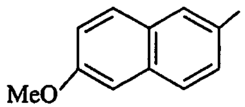 | 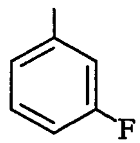 | 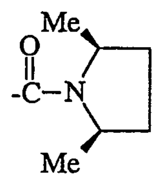 | 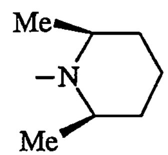 | HCl |
| 264 | 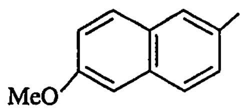 | 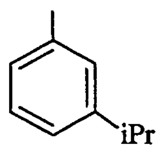 | 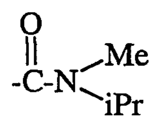 | 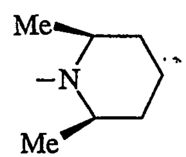 | HCl |

(suite)

| Exemples | $R_1$ | $R_3$ | $R_4$ | 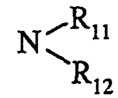 | Sel |
|---|---|---|---|---|---|
| 265 | 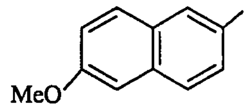 | 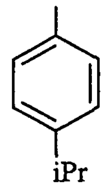 | 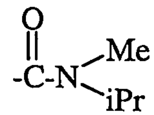 | 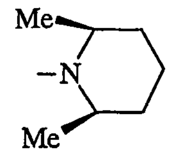 | HCl |
| 266 (R,R) | 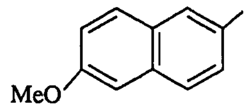 | 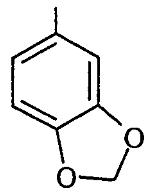 | 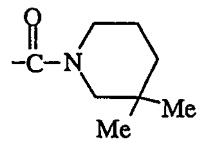 | 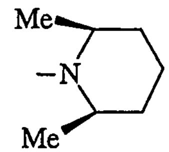 | HCl |
| 267 (R,R) | 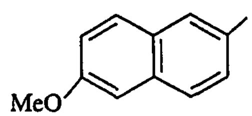 | 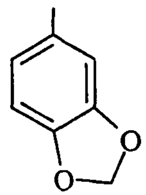 | 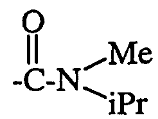 | 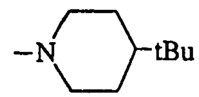 | HCl |
| 268 (R,R) | 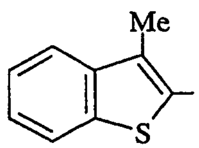 | 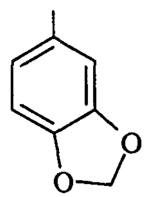 | 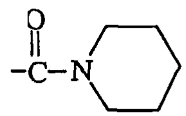 | 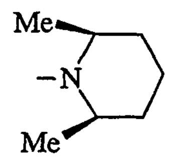 | HCl |
| 269 (R,R) | 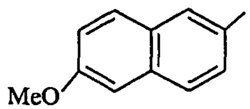 | 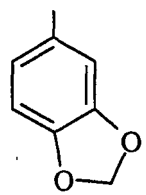 | 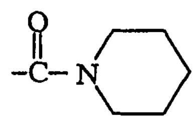 | 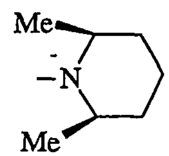 | HCl |

(suite)

| Exemples | $R_1$ | $R_3$ | $R_4$ | 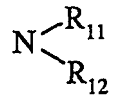 | Sel |
|---|---|---|---|---|---|
| 270 (R,R) | 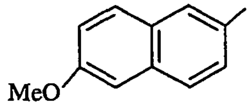 | 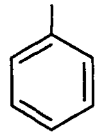 | 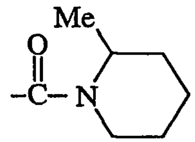 | 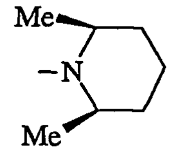 | HCl |
| 271 (R,R) | 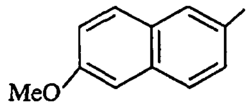 | 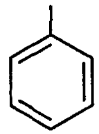 | 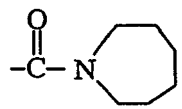 | 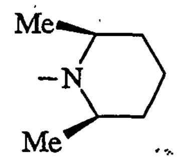 | HCl |
| 272 ((R,S),R) | 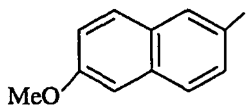 | 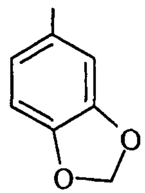 | 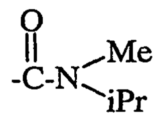 | 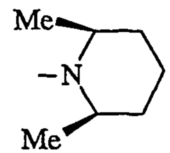 | HCl |
| 273 (R,R) | 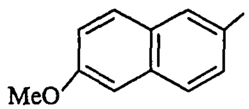 | 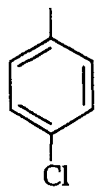 | 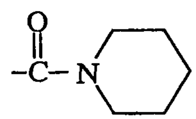 | 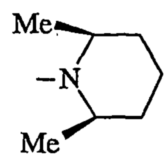 | HCl |
| 274 (R,R) | 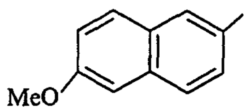 | 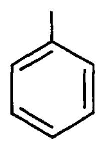 | 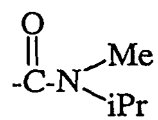 | 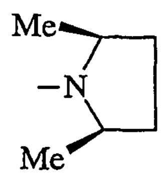 | HCl |

(suite)

| Exemples | $R_1$ | $R_3$ | $R_4$ | 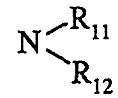 | Sel |
|---|---|---|---|---|---|
| 275 (R,R) | 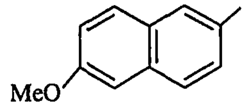 | 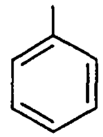 | 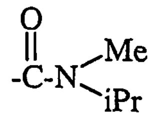 | 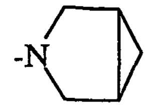 | HCl |
| 276 (R,R) | 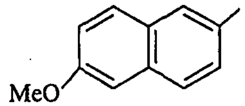 | 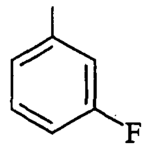 | 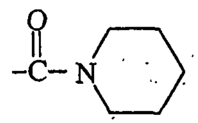 | 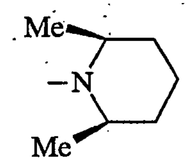 | HCl |
| 277 | 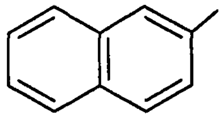 | 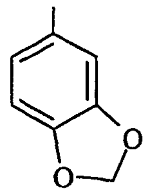 | 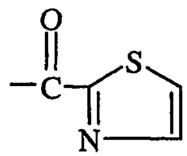 | 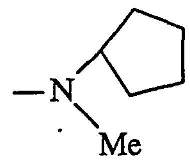 | HCl |
| 278 (R,R) | 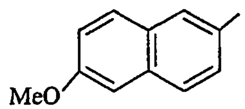 | 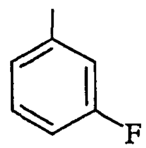 | 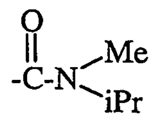 | 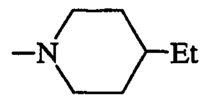 | HCl |
| 279 (R,R) | 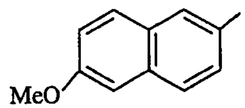 | 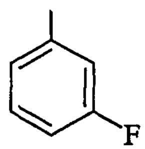 | 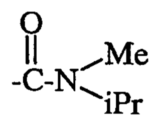 | 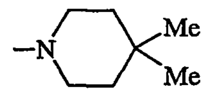 | HCl |

(suite)

| Exemples | $R_1$ | $R_3$ | $R_4$ | 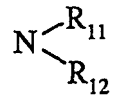 | Sel |
|---|---|---|---|---|---|
| 280 (R,R) | 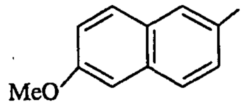 | 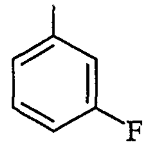 | 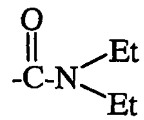 | 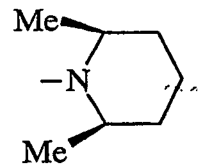 | HCl |
| 281 (R,R) | 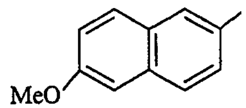 | 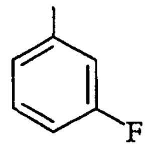 | 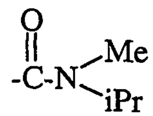 | 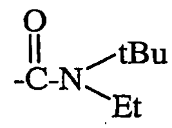 | HCl |
| 282 ((R,S),R) | 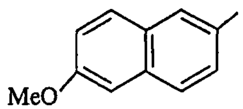 | 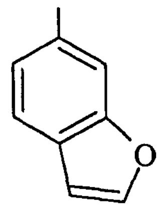 | 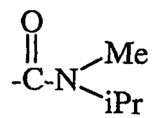 | 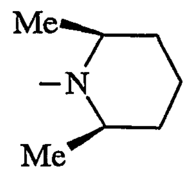 | HCl |
| 283 ((R,S),R) | 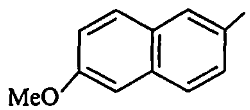 | 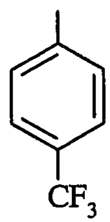 | 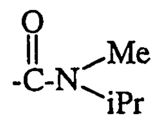 | 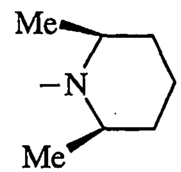 | HCl |

(suite)

| Exemples | $R_1$ | $R_3$ | $R_4$ | 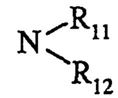 | Sel |
|---|---|---|---|---|---|
| 284 (R,R) | 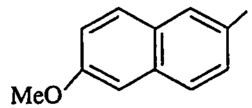 | 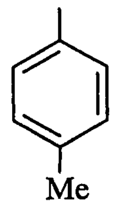 | 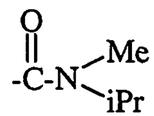 | 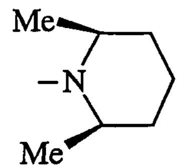 | HCl |
| 285 (R,R) | 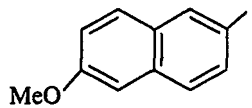 | 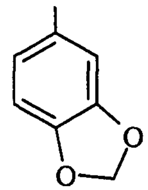 | 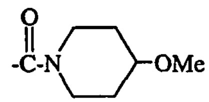 | 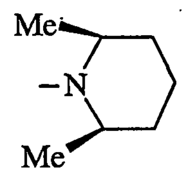 | HCl |
| 286 | 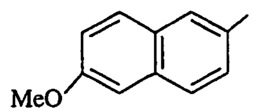 | 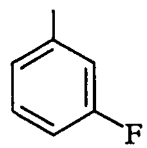 | 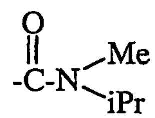 | 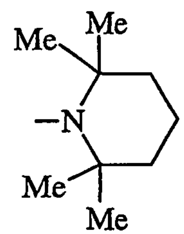 | HCl |
| 287 (R,R) | 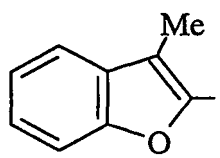 | 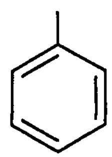 | 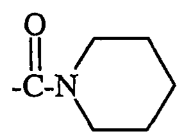 | 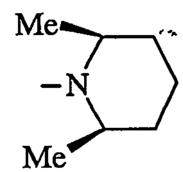 | HCl |

(suite)

| Exemples | $R_1$ | $R_3$ | $R_4$ | 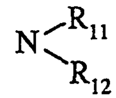 | Sel |
|---|---|---|---|---|---|
| 288 (R,R) | 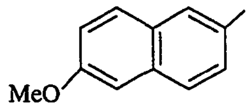 | 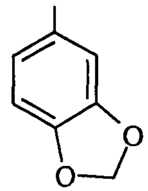 | 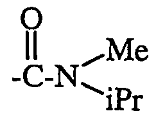 | 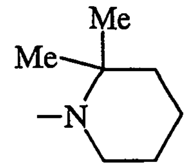 | HCl |
| 289 ((R,S),R) | 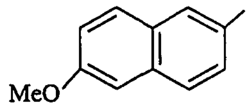 | 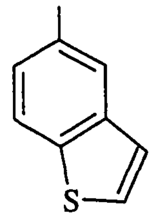 | 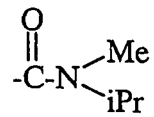 | 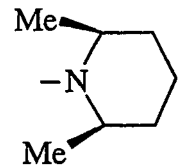 | HCl |
| 290 (R,R) | 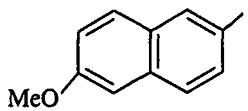 | 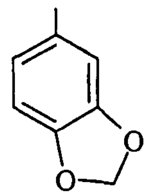 | 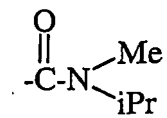 | 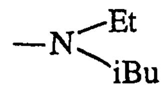 | HCl |
| 291 ((R,S),R) | 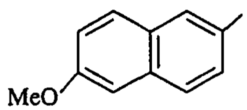 | 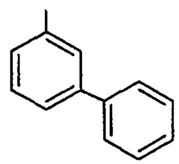 | 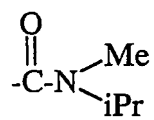 | 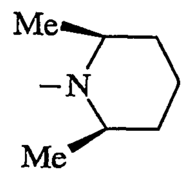 | HCl |

(suite)

| Exemples | $R_1$ | $R_3$ | $R_4$ | 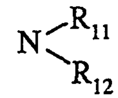 | Sel |
|---|---|---|---|---|---|
| 292 (R,R) | 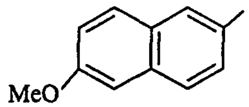 | 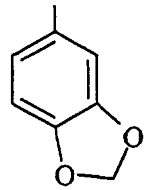 | 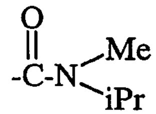 | 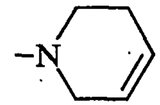 | HCl |
| 293 ((R,S),R) | 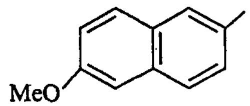 | 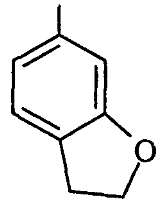 | 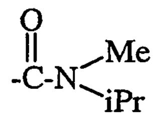 | 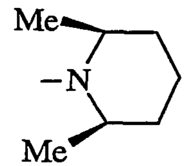 | HCl |
| 294 (R,R) | 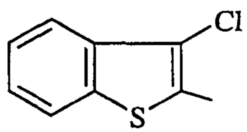 | 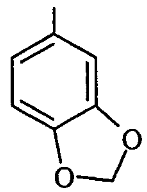 | 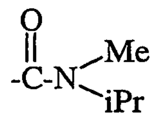 | 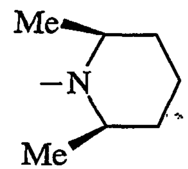 | HCl |
| 295 ((R,S),R) | 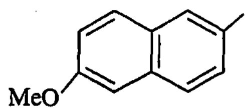 | 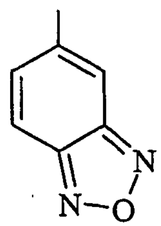 | 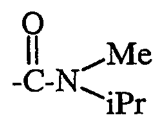 | 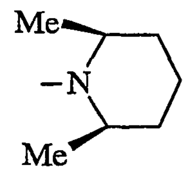 | HCl |

(suite)

| Exemples | $R_1$ | $R_3$ | $R_4$ | 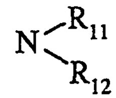 | Sel |
|---|---|---|---|---|---|
| 296 (R,R) | 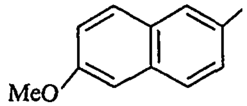 | 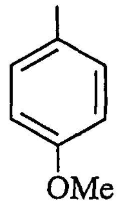 | 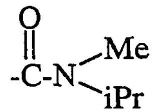 | 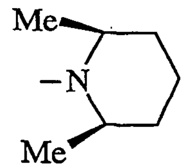 | HCl |
| 297 (R,R) | 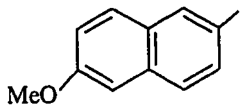 | 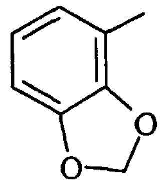 | 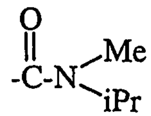 | 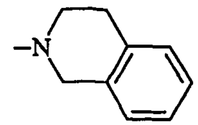 | HCl |
| 298 ((R,S),R) | 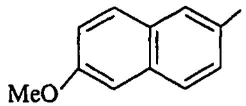 | 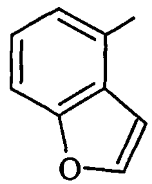 | 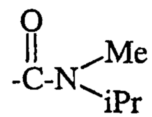 | 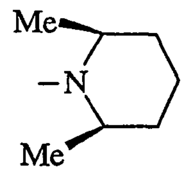 | HCl |
| 299 (R,R) | 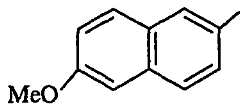 | 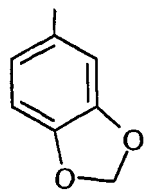 | 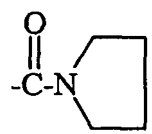 | 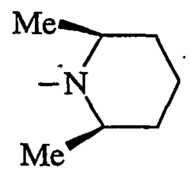 | HCl |
| 300 (R,R) | 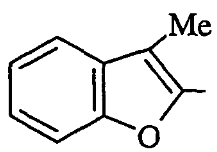 | 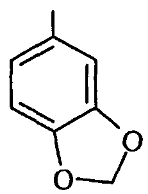 | 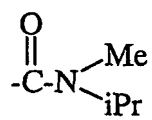 | 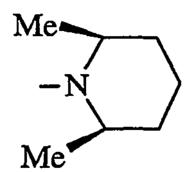 | HCl |

(suite)

| Exemples | $R_1$ | $R_3$ | $R_4$ | 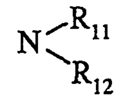 | Sel |
|---|---|---|---|---|---|
| 301 (R,R) | 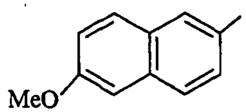 | 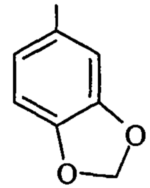 | 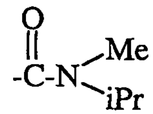 | 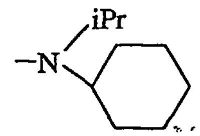 | HCl |
| 302 (R,R) | 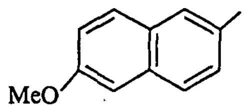 | 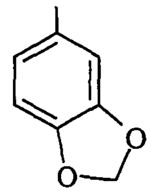 | 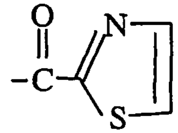 | 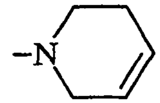 | HCl |
| 303 | 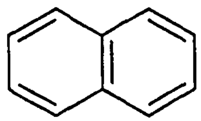 | 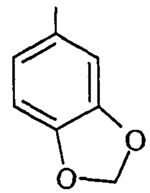 | 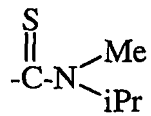 | 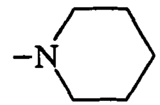 | HCl |
| 304 (S,R) | 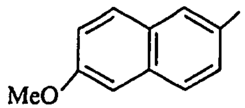 | 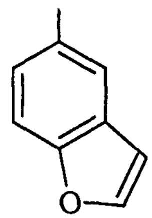 | 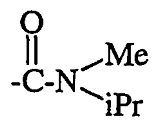 | 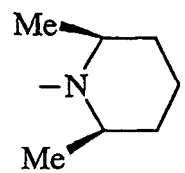 | $(CF_3CO_2H)$ |

(suite)

| Exemples | $R_1$ | $R_3$ | $R_4$ | 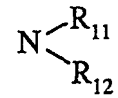 | Sel |
|---|---|---|---|---|---|
| 305<br>242142A<br>(R,R) | 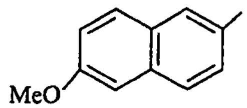 | 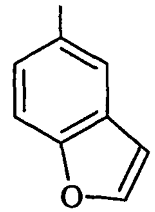 | 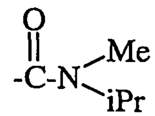 | 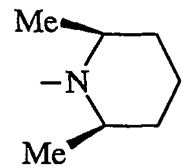 | $(CF_3CO_2H)$ |
| 306<br>(R,R) | 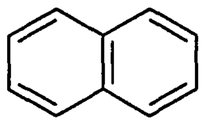 | 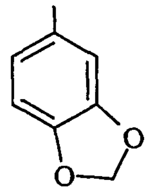 | 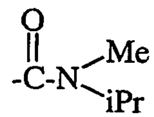 | 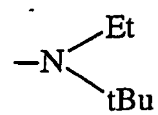 | HCl |
| 307<br>((R,S),R) | 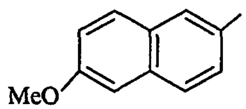 | 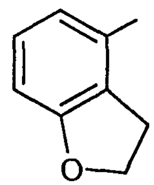 | 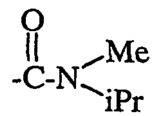 | 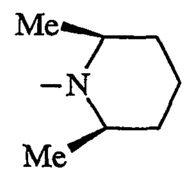 | HCl |
| 308<br>(S,R) | 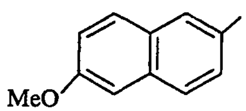 | 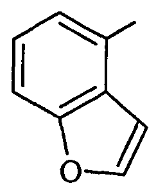 | 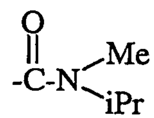 | 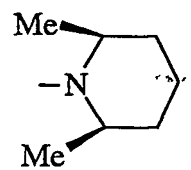 | $(CF_3CO_2H)$ |
| 309<br>(R,R) | 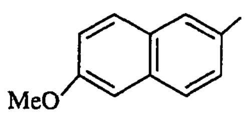 | 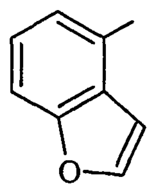 | 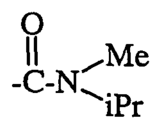 | 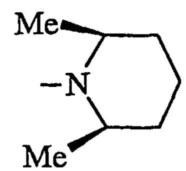 | $(CF_3CO_2H)$ |

(suite)

| Exemples | $R_1$ | $R_3$ | $R_4$ | 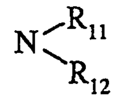 | Sel |
|---|---|---|---|---|---|
| 310 (R,R) | 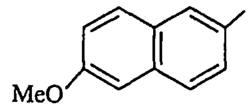 | 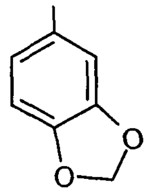 | 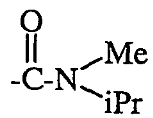 | 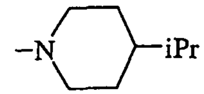 | HCl |
| 311 ((R,S),R) | 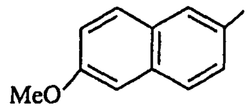 | 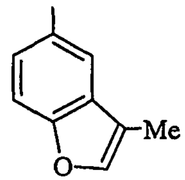 | 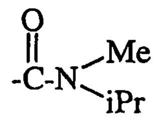 | 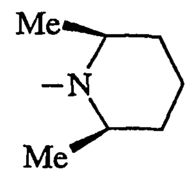 | HCl |
| 312 (R,R) | 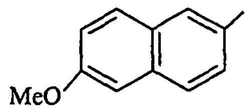 | 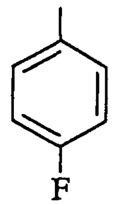 | 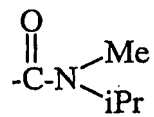 | 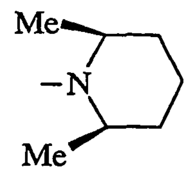 | HCl |
| 313 (S,S) | 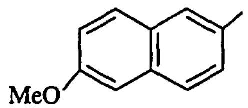 | 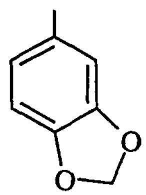 | 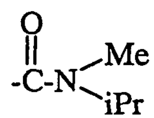 | 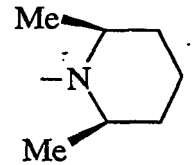 | HCl |
| 314 (R,R) | 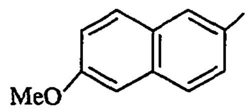 | 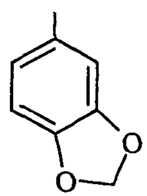 | 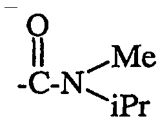 | 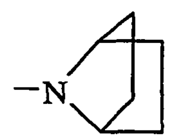 | HCl |

a) Pour ces composés le chlorure de sulfonyle intermédiaire (VI) est obtenu selon la Préparation 1.32.

TABLEAU XI

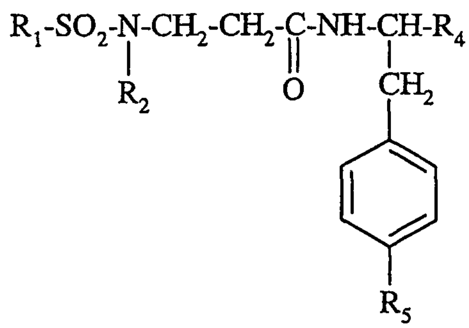

(I)

| Exemples | $R_1$ | $R_2$ | $R_4$ | $R_5$ | Sel |
|---|---|---|---|---|---|
| 315 (R) | 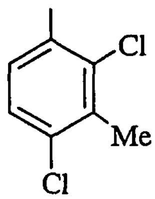 | 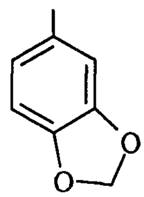 | 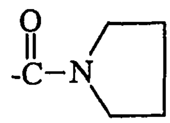 | 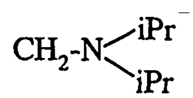 | HCl |
| 316 (R) | 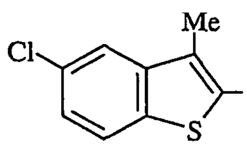 | 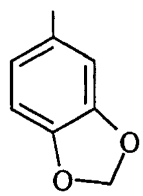 | 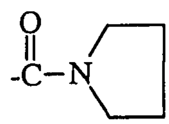 | 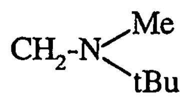 | HCl |
| 317 | 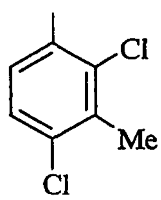 | 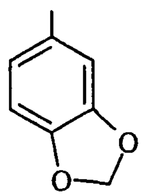 | 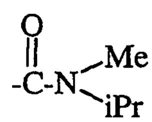 | 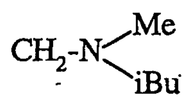 | HCl |
| 318 | 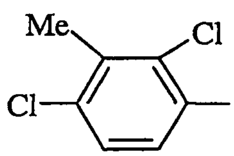 | 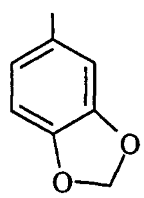 | 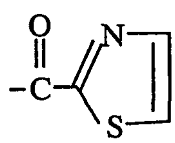 | 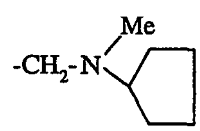 | HCl |

Par ailleurs, plusieurs composés selon l'invention ont été préparés par une méthode dite "synthèse parallèle" en faisant agir différents halogénures de sulfonyle de formule $R_1SO_2Hal$ sur un composé de formule (V) dans laquelle Y représente $CONR_8R_9$ et Z représente $CH_2NR_{11}R_{12}$.

EXEMPLES 319 à 335

A) On prépare une solution de 0,98 g du composé de la préparation 3.2, dans un mélange de 40 ml de $CH_3CN$ et 10 ml de DCM.
B) On prépare des tubes contenant chacun :

- 1,014 ml de la solution préparée en A, soit 40 µmoles ;
- 1,2 équivalent de chlorure de sulfonyle de formule $R_1SO_2Cl$, soit 48 µmoles ;
- 1,5 équivalent de résine morpholinométhylpolystyrène à 3,64 mmole/g, soit 16,5 mg de résine.

C) Les tubes sont placés sous agitation pendant 24 heures à TA puis on traite chacun par 0,6 équivalent de résine tris-(2-aminométhyl)amine soit 6 mg.

Pour chaque tube, on filtre, concentre puis reprend le milieu par DMSO pour obtenir une solution contenant 1µmole par litre du composé attendu. La nature du composé formé et sa pureté sont controlées par la mesure du temps de rétention (tR en minutes) HPLC et du spectre de masse.
Les solutions des composés selon l'invention obtenues sont utilisées telles quelles pour mesurer l'affinité desdits composés pour les récepteurs $B_1$ de la bradykinine.
Dans les exemples ci-après, les chlorures de sulfonyle $R_1SO_2Cl$ utilisés sont commerciaux.

TABLEAU XII

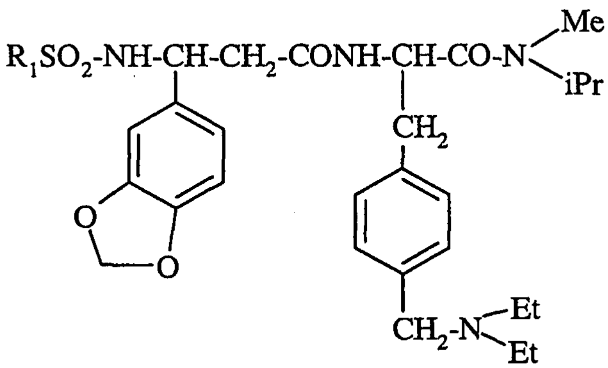

(I)

| Exemples | $R_1$ | masse | tR (minutes) |
|---|---|---|---|
| 319 | 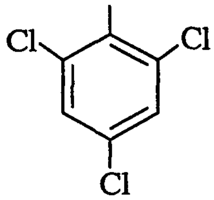 | 738 | 4,19 |
| 320 | 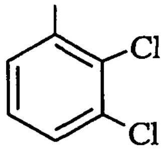 | 704 | 4,02 |
| 321 | 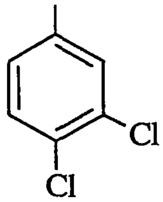 | 704 | 4,13 |
| 322 | 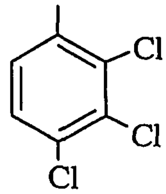 | 738 | 4,2 |

(suite)

| Exemples | $R_1$ | masse | tR (minutes) |
|---|---|---|---|
| 323 | 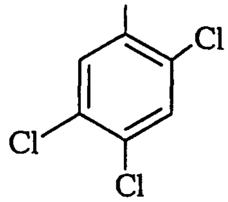 | 738 | 4,25 |
| 324 | 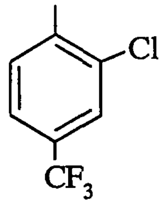 | 738 | 4,19 |
| 325 | 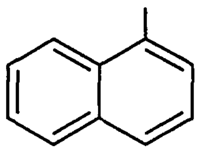 | 686 | 3,97 |
| 326 | 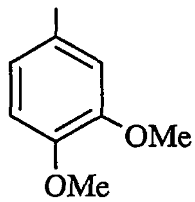 | 696 | 3,56 |
| 327 | 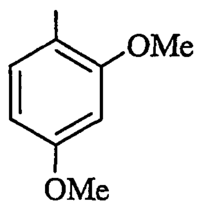 | 696 | 3,7 |
| 328 | 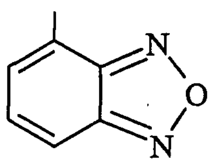 | 678 | 3,72 |
| 329 | 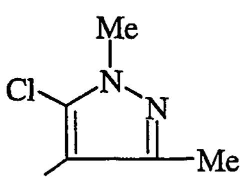 | 688 | 3,41 |
| 330 | 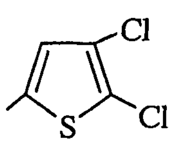 | 710 | 4,17 |

(suite)

| Exemples | $R_1$ | masse | tR (minutes) |
|---|---|---|---|
| 331 | 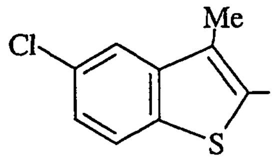 | 740 | 4,31 |
| 332 | 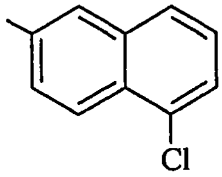 | 720 | 4,2 |
| 333 | 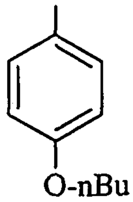 | 708 | 4,26 |
| 334 | 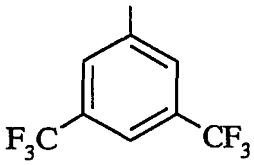 | 772 | 4,33 |
| 335 | 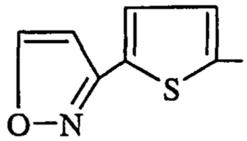 | 709 | 3,82 |

EXEMPLES 336 à 338

A) On prépare une solution de 1,924 g du composé de la préparation 3.1 dans 100 ml de $CH_3CN$.
B) On prépare des tubes contenant chacun :

- 941 µl de la solution préparée en A, soit 40 µmoles ;
- 1,2 équivalent de chlorure de sulfonyle de formule $R_1SO_2Cl$ soit 48 µmoles ;
- 1,5 équivalent de résine morpholinométhylpolystyrène à 3,64 mmole/g soit 16,5 mg de résine;
- 0,2 équivalent de résine DMAP/polystyrène, utile comme activateur.

C) Les tubes sont placés sous agitation pendant 24 heures à TA puis on traite chacun par 0,6 équivalent de résine tris-(2-aminoéthyl)amine, soit 6 mg. Pour chaque tube, on filtre, concentre, puis on reprend par DMSO pour obtenir une solution contenant 1 µmole par litre du composé attendu. La nature du composé formé et sa pureté sont controlées par la mesure du temps de rétention (tR minutes) HPLC et du spectre de masse.

Les solutions des composés selon l'invention obtenues sont utilisées telles quelles pour mesurer l'affinité desdits composés pour les récepteurs $B_1$ de la bradykinine.

TABLEAU XIII

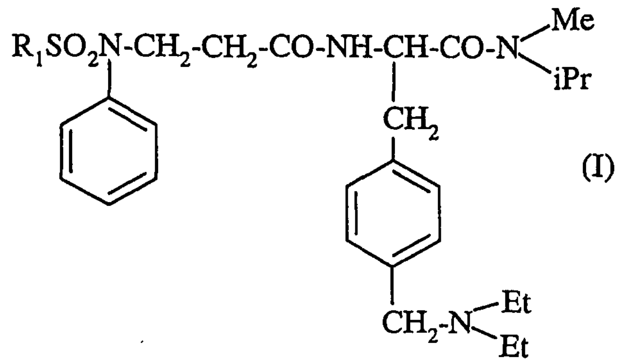

(I)

| Exemples | $R_1$ | masse | tR (minutes) |
|---|---|---|---|
| 336 | 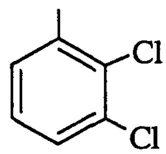 | 660 | 4,49 |
| 337 | 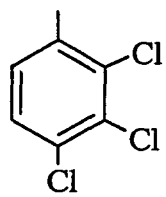 | 694 | 4,9 |
| 338 | 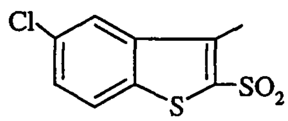 | 696 | 5,1 |

EXEMPLES 339 à 353

Préparation

(R,R)-2-(((3-amino-3-benzo[1,3]dioxol-5-yl)propanoyl)amino)-3-(4-(diisopropyl aminométhyl)phényl)-N-isopropyl-N-méthylpropionamide.

(IV) (R,R) : X = H, $R_2$ = H, $R_3$ = 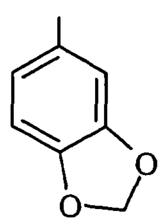 , $R_4$ = 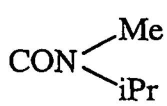 , $R_5$ = 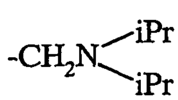

A) On dissout 8,6 g du trifluoroacétate obtenu à l'Exemple 38, étape B dans DCM et on lave par une solution de $NaHCO_3$ saturée puis NaCl saturée. Après séchage et concentration, on obtient 5,43 g du composé attendu sous forme de mousse sèche.

B) On prépare une solution de 524,7 mg du composé de la Préparation A dans 25 ml de $CH_3CN$.

C) On prépare des tubes contenant chacun :

- 1 ml de la solution préparée en B, soit 40 µmoles ;
- 1,2 équivalent de chlorure de sulfonyle de formule $R_1SO_2Cl$ soit 48 µmoles ;
- 1,5 équivalent de résine morpholinométhylpolystyrène à 3,64 µmole/g soit 16,5 mg de résine :

D) Les tubes sont placés sous agitation pendant une nuit à TA puis on traite chacun par 0,6 équivalent de résine tris-(2-aminométhyl)amine soit 6 mg. Pour chaque tube, on filtre, concentre puis reprend par DMSO pour obtenir une solution contenant 1 µmole par litre du composé attendu. La nature du composé et sa pureté sont controlées par la mesure du temps de rétension (tR minutes) HPLC et du spectre de masse.

Les solutions des composés selon l'invention obtenues sont utilisées telles quelles pour mesurer l'affinité desdits composés pour les récepteurs $B_1$ de la bradykinine.

TABLEAU XIV

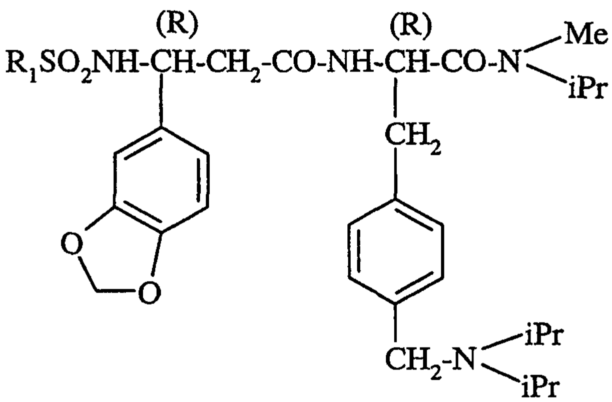

(I)

| Exemples | $R_1$ | Masse | tR minutes |
|---|---|---|---|
| 339 | 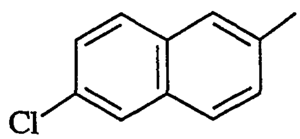 | 748 | 5,22 |
| 340 | 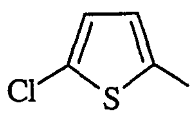 | 704 | 4,79 |
| 341 | 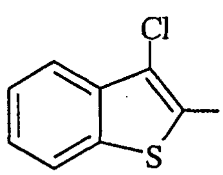 | 754 | 5,08 |
| 342 | 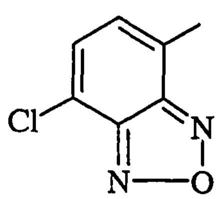 | 740 | 4,78 |

(suite)

| Exemples | $R_1$ | Masse | tR minutes |
|---|---|---|---|
| 343 | 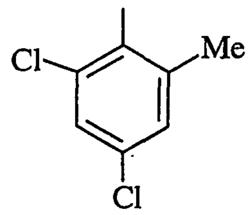 | 746 | 5,17 |
| 344 | 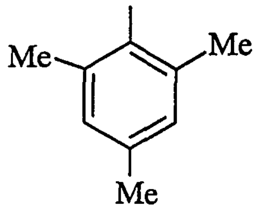 | 706 | 5,06 |
| 345 | 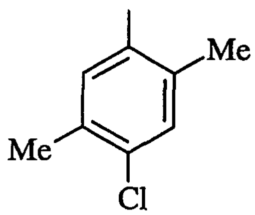 | 726 | 5,22 |
| 346 | 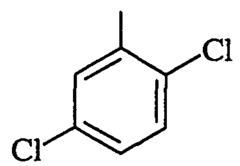 | 732 | 4,94 |
| 347 | 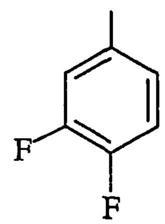 | 700 | 4,73 |
| 348 | 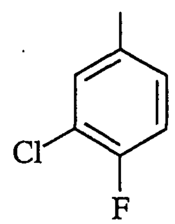 | 716 | 4,9 |
| 349 | 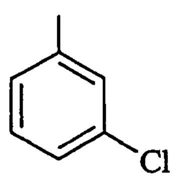 | 698 | 4,77 |

(suite)

| Exemples | $R_1$ | Masse | tR minutes |
|---|---|---|---|
| 350 | 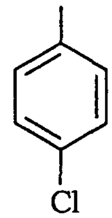 | 698 | 4,8 |
| 351 | 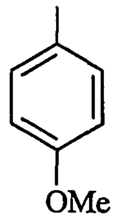 | 694 | 4,47 |
| 352 | 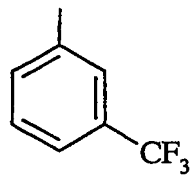 | 732 | 4,96 |
| 353 | 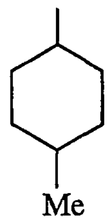 | 678 | 4,64 |

EXEMPLES 354 à 363

Préparation

(R,R)-2-(((3-amino-3-benzo[1,3]dioxol-5-yl)propanoyl)amino)-3-(4-(N-méthyl-N-cyclopentylaminométhyl)phényl)-N-isopropyl-N,N-disopropylpropionamide.

(IV) (R,R) : X = H, $R_2$ = H, $R_3$ = 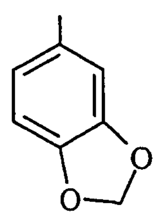 , $R_4$ = 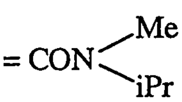 , $R_5$ = 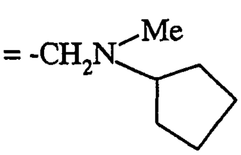

Ce composé est préparé à partir des composés obtenus aux Préparations 2.47 et 1.13 : ces 2 composés sont mis en réaction après déprotection nécessaire et l'on obtient après déprotection le composé attendu sous forme d'un ditrifluoroacétate.

A) On dissout 210 mg de ce ditrifluoroacétate dans DCM et on lave par une solution de $NaHCO_3$ saturée puis NaCl saturée. Après séchage et concentration, on obtient 141 mg du composé attendu sous forme d'huile.

B) On prépare une solution de 141 mg du composé de l'étape A dans dans 20 ml de $CH_3CN$.

C) On prépare des tubes contenant chacun :

- 1,48 ml de la solution préparée, soit 20 μmoles ;
- 1,2 équivalent de chlorure de sulfonyle de formule $R_1SO_2Cl$ soit 24 μmoles ;
- 1,5 équivalent de résine morpholinométhylpolystyrène à 3,64 μmole/g soit 8,24 mg de résine.

D) Les tubes sont placés sous agitation pendant une nuit à TA puis on traite chacun par 0,6 équivalent de résine tris-(2-aminométhyl)amine soit 3 mg. Pour chaque tube, on filtre, concentre puis reprend par DMSO pour obtenir une solution contenant 1 μmole par litre du composé attendu. La nature du composé et sa pureté sont controlées par la mesure du temps de rétension (tR minutes) HPLC et du spectre de masse.

TABLEAU XV

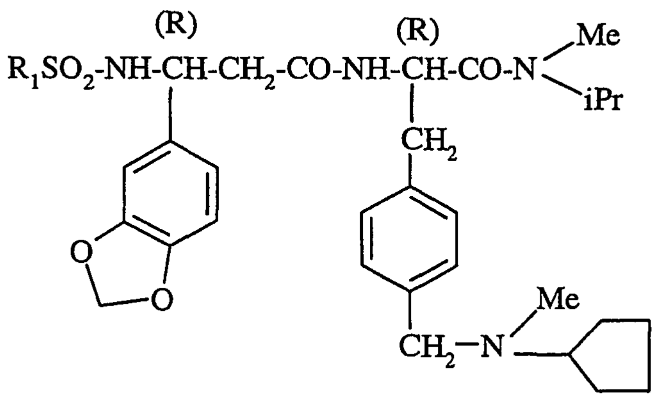

(I)

| Exemples | $R_1$ | Masse | tR minutes |
|---|---|---|---|
| 354 | 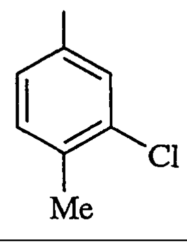 | 710 | 4,62 |
| 355 | 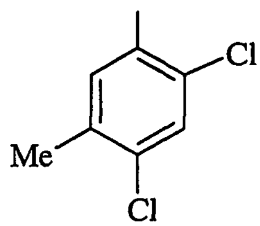 | 744 | 4,87 |
| 356 | 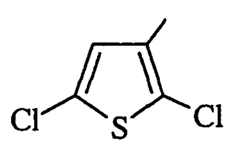 | 736 | 4,7 |
| 357 | 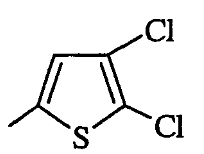 | 736 | 4,83 |

(suite)

| Exemples | $R_1$ | Masse | tR minutes |
|---|---|---|---|
| 358 | 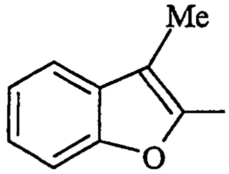 | 702 | 4,51 |
| 359 | 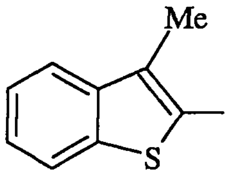 | 732 | 4,73 |
| 360 | 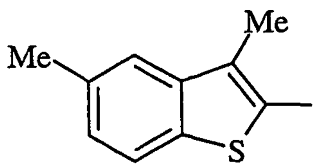 | 746 | 4,92 |
| 361 | 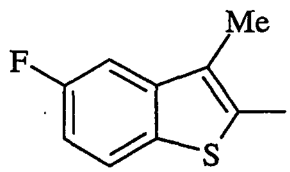 | 750 | 4,79 |
| 362 | 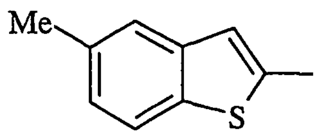 | 732 | 4,81 |
| 363 | 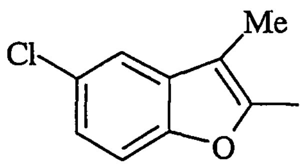 | 750 | 4,95 |

## Revendications

**1.** Composé de formule :

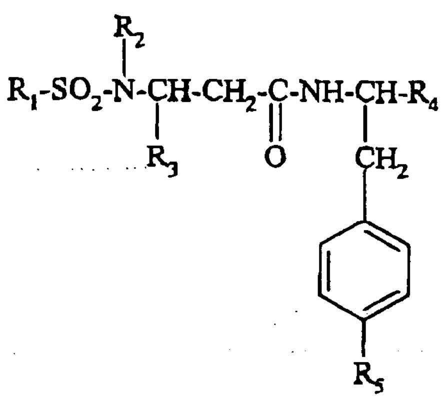

(I)

dans laquelle :

- $R_1$ représente un groupe phénylvinyle ; phényle non substitué ou substitué une ou plusieurs fois par $R_6$, identiques ou différents ; naphtyle non substitué ou substitué une ou plusieurs fois par $R_6$, identiques ou différents ; tétrahydronaphtyle ; naphto[2,3-d][1,3]dioxol-6-yle ; un radical hétérocyclique choisi parmi quinolyle, isoquinolyle, 1-benzofuran-2-yle, 2,1,3-benzoxadiazol-4-yle, 2,1,3-benzothiadiazol-4-yle, 1,3-benzothiazol-2-yle, 1-benzothiophèn-2-yle, 1*H*-pyrazol-4-yle, thièn-2-yle, 5-isoxazolthièn-2-yle, benzothièn-2-yle, thiéno[3,2-c]pyridin-2-yle ; lesdits radicaux hétérocycliques étant non substitués ou substitués une ou plusieurs fois par $R_6$ identiques ou différents ;
- $R_2$ représente l'hydrogène ou un groupe $(C_1\text{-}C_4)$alkyle et $R_3$ représente un groupe phényle non substitué ou substitué une ou plusieurs fois par $R_7$, identiques ou différents ; un radical hétérocyclique choisi parmi benzo [1,3]dioxol-5-yle, 2,1,3-benzothiadiazol-5-yle, 2,1,3-benzoxadiazol-5-yle, benzothiophèn-5-yle, 2,3-dihydrobenzo[1,4]dioxin-6-yle, benzofuranyle, dihydrobenzofuranyle, 1,3-thiazol-2-yle, furyle, thiènyle, ledit radical hétérocyclique étant non substitué ou substitué une ou plusieurs fois par un atome d'halogène ou par un groupe $(C_1\text{-}C_4)$alkyle ;
- ou bien $R_2$ représente un groupe phényle non substitué ou substitué une ou plusieurs fois par $R_6$, identiques ou différents ; un radical hétérocyclique choisi parmi benzo[1,3]dioxol-5-yle, pyridyle, indanyle, et $R_3$ représente l'hydrogène ;
- $R_4$ représente un groupe $-CONR_8R_9$ ; un groupe $-CSNR_8R_9$ ; un groupe-$COR_{13}$ ; un groupe phényle non substitué ou substitué une ou plusieurs fois par $R_{10}$ ; un radical hétérocyclique choisi parmi pyridyle, imidazolyle, furyle, benzimidazolyle, benzothiazol-2-yle, benzo[1,3]dioxol-5-yle, lesdits radicaux étant non substitués ou substitués par un ou plusieurs groupes méthyle ou atomes d'halogène ;
- $R_5$ représente un groupe $-CH_2NR_{11}R_{12}$ ou $-CH_2N(O)R_{11}R_{12}$ ;
- $R_6$ représente un atome d'halogène; un groupe $(C_1\text{-}C_4)$alkyle ; trifluorométhyle ; $(C_1\text{-}C_4)$ alcoxy ; 2-fluoroéthoxy ; trifluorométhoxy, méthylènedioxy, difluorométhylènedioxy ;
- $R_7$ représente un atome d'halogène ; un groupe $(C_1\text{-}C_4)$alkyle ; phényle ; trifluorométhyle ; $(C_1\text{-}C_4)$alcoxy ; benzyloxy ; trifluorométhoxy ;
- $R_8$ et $R_9$ représentent chacun indépendamment l'hydrogène ; un groupe $(C_1\text{-}C_4)$alkyle ; $(C_3\text{-}C_7)$cycloalkyle; $(C_3\text{-}C_7)$cycloalkyle$(C_1\text{-}C_4)$alkyle ; $\omega$-$(C_1\text{-}C_4)$ dialkylamino$(C_2\text{-}C_4)$alkyle ;
- ou bien $R_8$ et $R_9$ ensemble avec l'atome d'azote auquel ils sont liés constituent un radical hétérocyclique choisi parmi pyrrolidinyle, morpholin-4-yle, thiomorpholin-4-yle, azépin-1-yle, pipéridinyle non substitué ou substitué par un ou plusieurs atomes d'halogène ou un ou plusieurs groupes $(C_1\text{-}C_4)$alkyle ou $(C_1\text{-}C_4)$alcoxy ou trifluorométhyle, 3,4-dihydropipéridin-1-yle, cyclohexylspiro-4-pipéridin-1-yle, pipérazinyle non substitué ou substitué par un ou plusieurs groupes $(C_1\text{-}C_4)$alkyle ;
- $R_{10}$ représente un atome halogène ; un groupe $(C_1\text{-}C_4)$alkyle ; hydroxy ; $(C_1\text{-}C_6)$alcoxy ; $R_{10}$ peut de plus représenter un groupe $-CH_2NR_{11}R_{12}$ lorsque $R_5$ représente un groupe-$CH_2NR_{11}R_{12}$, lesdits groupes étant alors identiques ;
- $R_{11}$ et $R_{12}$ représentent chacun indépendamment l'hydrogène ; un groupe $(C_1\text{-}C_6)$alkyle ; $(C_2\text{-}C_4)$alcènyle ; $(C_3\text{-}C_7)$cycloalkyle ; $(C_3\text{-}C_7)$cycloalkyle$(C_1\text{-}C_4)$alkyle ; $\omega$-hydroxy$(C_2\text{-}C_4)$alkylène ; $\omega$-méthoxy$(C_2\text{-}C_4)$alkylène ; $\omega$-trifluorométhyl$(C_2\text{-}C_4)$alkylène ; $\omega$-halogéno$(C_2\text{-}C_4)$alkylène,
- ou bien $R_{11}$ et $R_{12}$ ensemble avec l'atome d'azote auquel ils sont liés constituent un radical hétérocyclique, mono ou bicyclique, choisi parmi azétidinyle, pyrrolidinyle, morpholin-4-yle, thiomorpholin-4-yle, pipéridin-1-yle, pipérazin-1-yle, 1,2,3,6-tétrahydropyrid-1-yle, 2,3,4,5-tétrahydropyridinium, decahydroquinolyle, decahydroisoquinolyle, tétrahydroisoquinolyle, octahydro-1*H*-isoindolyle, $(C_4\text{-}C_6)$cycloalkylspiropipéridinyle, 3-azabicyclo[3.1.0]hexyle, 7-azabicyclo[2.2.1]heptan-7-yle, non substitués ou substitués une ou plusieurs fois par un atome d'halogène, un groupe $(C_1\text{-}C_4)$alkyle, hydroxy, $(C_1\text{-}C_4)$alcoxy, trifluorométhyle, difluorométhylène, phényle ;
- $R_{13}$ représente un groupe phényle, thiazol-2-yle ou pyridyle ;

ainsi que ses sels avec des acides minéraux ou organiques et/ou solvats et/ou hydrates.

**2.** Sel d'un composé de formule (I) selon la revendication 1 choisi parmi le chlorhydrate, le bromhydrate, le sulfate, l'hydrogénosulfate, le dihydrogénophosphate, le méthanesulfonate, l'oxalate, le maléate, le succinate, le fumarate, le naphtalène-2-sulfonate, le benzènesulfonate, le paratoluènesulfonate.

**3.** Composé de formule selon la revendication 1 :

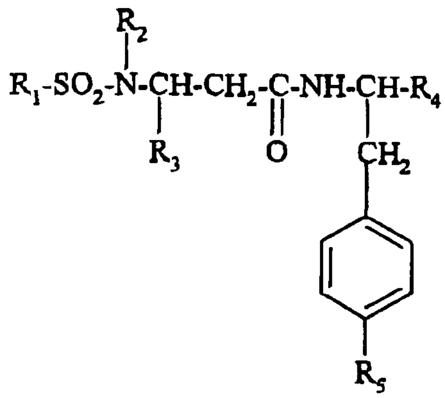

(Ibis)

dans laquelle :

- $R_1$ représente un groupe phénylvinyle ; phényle non substitué ou substitué une ou plusieurs fois par $R_6$, identiques ou différents; naphtyle non substitué ou substitué une ou plusieurs fois par $R_6$, identiques ou différents ; tétrahydronaphtyle ; un radical hétérocyclique choisi parmi quinolyle, 1-benzofuran-2-yle, 2,1,3-benzoxadiazol-4-yle, 2,1,3-benzothiadiazol-4-yle, 1,3-benzothiazol-2-yle, 1-benzothiophèn-2-yle, 1*H*-pyrazol-4-yle, thièn-2-yle, 5-isoxazolthièn-2-yle, benzothièn-2-yle, thiéno[3,2-c]pyridin-2-yle ; naphto[2,3-d] [1,3] dioxol-6-yle; lesdits radicaux hétérocycliques étant non substitués ou substitués une ou plusieurs fois par $R_6$ identiques ou différents ;
- $R_2$ représente l'hydrogène ou un groupe $(C_1-C_4)$alkyle et $R_3$ représente un groupe phényle non substitué ou substitué une ou plusieurs fois par $R_7$, identiques ou différents ; un radical hétérocyclique choisi parmi benzo [1,3]dioxol-5-yle non substitué ou substitué en -2 par deux atomes de fluor ; 2,1,3-benzothiadiazol-5-yle ; 2,3-dihydro-benzo[1,4]dioxin-6-yle ; 1,3-thiazol-2-yle ; 1-benzofuran-2-yle ; 1-benzofuran-5-yle ; furyle ; thièn-2-yle ; thièn-3-yle ;
- ou bien $R_2$ représente un groupe phényle non substitué ou substitué une ou plusieurs fois par $R_6$, identiques ou différents ; un radical hétérocyclique choisi parmi benzo[1,3]dioxol-5-yle ; pyridyle ; indanyle ; et $R_3$ représente l'hydrogène;
- $R_4$ représente un groupe $-CONR_8R_9$ ; un groupe phényle non substitué ou substitué une ou plusieurs fois par $R_{10}$ ; radical hétérocyclique choisi parmi pyridyle, imidazolyle, furyle, benzimidazolyle, benzothiazol-2-yle, benzo [1,3]dioxol-5-yle, lesdits radicaux étant non substitués ou substitués par un méthyle ;
- $R_5$ représente un groupe $-CH_2NR_{11}R_{12}$ ;
- $R_6$ représente un atome d'halogène ; un groupe $(C_1-C_4)$alkyle ; trifluorométhyle ; $(C_1-C_4)$ alcoxy ; 2-fluoroéthoxy ; trifluorométhoxy, méthylènedioxy, difluorométhylènedioxy ;
- $R_7$ représente un atome d'halogène; un groupe $(C_1-C_4)$alkyle; trifluorométhyle ; $(C_1-C_4)$alcoxy ; benzyloxy ; trifluorométhoxy ;
- $R_8$ et $R_9$ représentent chacun indépendamment l'hydrogène ; un groupe $(C_1-C_4)$alkyle ; $(C_3-C_7)$cycloalkyle ; $(C_3-C_7)$cycloalkyle$(C_1-C_4)$alkyle : ω-$(C_1-C_4)$ dialkylamino$(C_2-C_4)$alkyle ;
- ou bien $R_8$ et $R_9$ ensemble avec l'atome d'azote auquel ils sont liés constituent un radical hétérocyclique choisi parmi pyrrolidinyle, pipéridinyle, morpholin-4-yle, thiomorpholin-4-yle, pipérazin-1-yle, 4-méthylpipérazin-1-yle, 4-méthylpipéridin-1-yle, 2-méthylpipéridin-1-yle, 4,4-diméthylpipéridin-1-yle, 4,4-difluoropipéridin-1-yle, 4-trifluorométhylpipéridin-1-yle, 3,4-dihydropipéridin-1-yle, azépin-1-yle, cyclohexylspiro-4-pipéridin-1-yle ;
- $R_{10}$ représente un atome d'halogène ; un groupe $(C_1-C_4)$alkyle ; hydroxy ; $(C_1-C_6)$alcoxy ; $R_{10}$ peut de plus représenter un groupe $-CH_2NR_{11}R_{12}$ lorsque $R_5$ représente un groupe-$CH_2NR_{11}$ $R_{12}$, lesdits groupes étant alors identiques ;
- $R_{11}$ et $R_{12}$ représentent chacun indépendamment l'hydrogène ; un groupe $(C_1-C_6)$alkyle; $(C_2-C_4)$alcènyle ; $(C_3-C_7)$cycloalkyle ; $(C_3-C_7)$cycloalkyle$(C_1-C_4)$alkyle ; ω-hydroxy$(C_2-C_4)$alkylène ; ω-méthoxy$(C_2-C_4)$alkylène ; ω-trifluorométhyl$(C_2-C_4)$alkylène ; ω-halogéno$(C_2-C_4)$alkylène ;
- ou bien $R_{11}$ et $R_{12}$ ensemble avec l'atome d'azote auquel ils sont liés constituent un radical hétérocyclique, mono ou bicyclique, choisi parmi azétidinyle, pyrrolidinyle, morpholin-4-yle, thiomorpholin-4-yle, pipéridin-1-yle, pipérazin-1-yle, 1,2,3,6-tétrahydropyrid-1-yle, decahydroquinolyle, decahydro isoquinolyle, octahydro-1*H*-isoindolyle, $(C_4-C_6)$cycloalkylspiropipéridinyle, 3-azabicyclo[3.1.0]hexyle, non substitués ou substitués une ou plusieurs fois par un atome d'halogène, un groupe $(C_1-C_4)$alkyle, hydroxy, $(C_1-C_4)$alcoxy, trifluorométhyle, difluorométhylène, phényle ;

ainsi que ses sels avec des acides minéraux ou organiques et/ou solvats ou hydrates.

**4.** Composé de formule (I) selon la revendication 1 ou la revendication 2 dans laquelle $R_1$ représente un groupe 2,4-dichloro-3-méthylphényle, naphtyle, 6-méthoxynapht-2-yle, 3-méthylbenzothiophèn-2-yle, 3-méthyl-5-chlorobenzothiophèn-2-yle, 3-méthyl-5-méthoxybenzothiophèn-2-yle, 3-méthyl-6-méthoxybenzothiophèn-2-yle ou 3-méthyl-1-benzofuran-2-yle.

**5.** Composé de formule (I) selon la revendication 1 ou la revendication 2 dans laquelle $R_2$ représente l'hydrogène et $R_3$ représente un groupe benzo[1,3]dioxol-5-yle ou phényle non substitué ou substitué par un halogène.

**6.** Composé de formule (I) selon la revendication 1 ou la revendication 2 dans laquelle $R_4$ représente un groupe $-CONR_8R_9$ et $-NR_8R_9$ représente un radical di($C_1$-$C_4$)alkylamino, pyrrolidinyle ou pipéridinyle non substitué ou substitué une ou deux fois par un méthyle ou un halogène.

**7.** Composé de formule (I) selon la revendication 1 ou la revendication 2 dans laquelle $R_5$ représente un groupe $-CH_2NR_{11}R_{12}$ dans lequel $-NR_{11}R_{12}$ représente un radical éthylisobutylamino, éthylisopropylamino, éthyl*tert*butylamino, diisopropylamino, cyclopentylméthylamino, cyclopentyl éthylamino ou un radical pipéridinyle non substitué ou substitué une ou plusieurs fois par un méthyle ou un halogène.

**8.** Composé selon la revendication 1 ou la revendication 2 de formule :

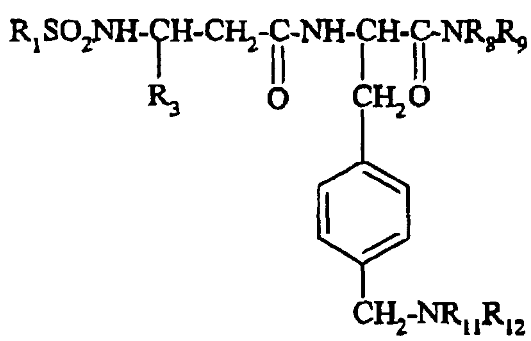

(Ia)

dans laquelle:

- $R_1$ représente un groupe 2,4-dichloro-3-méthylphényle, naphtyle, 6-méthoxynapht-2-yle, 3-méthylbenzothiophèn-2-yle, 3-méthyl-5-chlorobenzothiophèn-2-yle, 3-méthyl-5-méthoxybenzothiophèn-2-yle, 3-méthyl-6-méthoxybenzothiophèn-2-yle ou 3-méthyl-1-benzofuran-2-yle ;
- $R_3$ représente un groupe benzo[1,3]dioxot-5-yle ou phényle non substitué ou substitué par un halogène ;
- $R_8$ et $R_9$ ensemble avec l'atome d'azote auxquels ils sont liés, constituent un radical di($C_1$-$C_4$)alkylamino, pyrrolidinyle ou pipéridinyle non substitué ou substitué une ou deux fois par un méthyle ou un halogène ;
- $R_1$ et $R_{12}$ ensemble avec l'atome d'azote auxquels ils sont liés, constituent un radical éthylisobutylamino, éthylisopropylamino, éthyl*tert*butylamino, diisopropylamino, cyclopentylméthylamino, cyclopentyléthylamino ou un radical pipéridinyle non substitué ou substitué une ou plusieurs fois par un méthyle ou un halogène ;

ainsi que ses sels avec des acides minéraux ou organiques et/ou leurs solvats ou leurs hydrates.

**9.** Composé selon la revendication 1 choisi parmi :

- (R,R) 2-((3-(1,3-benzodioxol-5-yl)-3-(((6-méthoxy-2-naphtyl)sulfonyl)amino) propanoyl)amino)-3-(4-((2,6-cis-diméthyl-1-pipéndinyl)methyl)phényl)-N-isopropyl-N-méthylpropanamide ;
- (R,R) 2-((3-(1,3-benzodioxol-5-yl)-3-((2-naphtylsulfonyl)amino)propanoyl) amino)-3-(4-((cyclopentyl(éthyl)amino)méthyl)phényl)-N-isopropyl-N-méthylpropanamide ;
- (R,R) 3-(4-((2,6-cis-diméthyl-1-pipénidinyl)méthyl)phényl)-2-((3-(((6-méthoxy-2-naphtyl)sulfonyl)amino)-3-phénylpropanoyl)amino)N-isopropyl-N-méthyl propanamide ;
- (R,R) 2-((3-(1,3-benzydioxol-5-yl)-3-(((6-méthoxy-2-naphtyl)sulfonyl)amino) propanoyl)amino)3-(4-((*tert*-butyl (éthyl)amino)méthyl)phényl)-N-isopropyl-N-méthylpropanamide ;

- (R,R) 2-((3-(1,3-benzodioxol-5-yl)-3-(((3-méthyl-1-benzothiophèn-2-yl)sulfonyl)amino)propanoyl)amino)-3-(4-((2,6-cis-diméthyl-1-pipéridinyl)méthyl) phényl)N-isopropyl-N-méthylpropanamide ;
- (R,R) 3-(4-((2,6-cis-diméthyl-1-pipéridinyl)méthyl)phényl)-2-((3-(((5-méthoxy-3-méthyl-1-benzothiophèn-2-yl) sulfonyl)amino)-3-phénylpropanoyl)amino)-N-isopropyl-N-méthylpropanamide ;
- (R,R) N-(4-((2,6-cis-diméthyl-1-pipéridinyl)méthyl)benzyl)-3-(((6-méthoxy-2-naphtyl)sulfonyl)amino)-3-phényl-N-(1-pipéridinylcarbonyl)propanamide ;
- (R,R) 2-((3-(4-chlorophényl)-3-(((6-méthoxy-2-naphtyl)sulfonyl) amino)propanoyl)amino)-3-(4-((2,6-cis-diméthyl-1-pipéridinyl)méthyl)phényl)-N-isopropyl-N-méthylpropanamide ;
- (R,R) 3-(4-((2,6-cis-diméthyl-1-pipéridinyl)méthyl)phényl)-2-((3-(((6-méthoxy-2-naphtyl)sulfonyl)amino)-3-phénylpropanoyl)amino)-N,N-diéthylpropanamide ;
- (R,R) 2-((3-(3-fluorophényl)-3-(((6-méthoxy-2-naphtyl)sulfonyl)amino) propanoyl)amino)-3-(4-((2,6-cis-diméthyl-1-pipéridinyl)méthyl)phényl)-N-isopropyl-N-méthylpropanamide ;
- (R,R) 2-((3-(1,3-benzodioxol-5-yl)-3-(((3-méthyl-1-benzofuran-2-yl)sulfonyl) amino)propanoyl)amino)-3-(4-((2,6-cis-diméthyl-1-pipéridinyl)méthyl)phényl)-N-isopropyl-N-méthylpropanamide ;
- (R,R) 2-((3-(1,3-benzodioxol-5-yl)-3-((2-naphtylsulfonyl)amino)propanoyl) amino)-3-(3-((*tert*-butyl(éthyl)amino)méthyl)phényl)-N-isopropyl-N-méthyl propanamide ;
- (R,R) 3-(4-(7-azabicyclo[2.2.1]hept-7-ylméthyl)phényl)-2-((3-(1,3-benzodioxol-5-yl)-3-(((6-méthoxynaphtyl) sulfonyl)amino)propanoyl)amino)-N-isopropyl-N-méthyl propanamide ;

ainsi que ses sels et/ou solvats et/ou hydrates.

**10.** Le (R,R) 2-((3-(1,3-benzodioxol-5-yl)-3-(((6-méthoxy-2-naphtyl)sulfonyl)amino) propanoyl)amino)-3-(4-((2,6-cis-diméthyl-1-pipéridinyl)méthyl)phényl)-N-isopropyl-N-méthylpropanamide ;
ainsi que ses sels avec des acides minéraux ou organiques et/ou solvats ou leurs hydrates.

**11.** Procédé pour la préparation d'un composé de formule (I), de ses sels et/ou solvats ou hydrates, **caractérisé en ce que** :

on fait réagir un acide ou un dérivé fonctionnel de cet acide de formule :

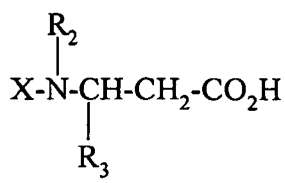

(II)

dans laquelle $R_2$ et $R_3$ sont tels que définis pour un composé de formule (I) dans la revendication 1, X représente soit l'hydrogène, soit un groupe $R_1SO_2$- dans lequel $R_1$ est tel que défini pour un composé de formule (I) dans la revendication 1, soit un groupe N-protecteur, avec un composé de formule :

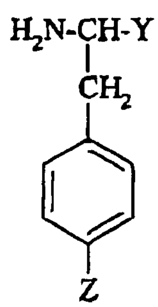

(III)

dans laquelle Y représente soit $R_4$ tel que défini pour un composé de formule (I) dans la revendication 1, soit un $(C_1\text{-}C_4)$alcoxycarbonyle, et Z représente soit $R_5$ tel que défini pour un composé de formule (I) dans la revendication 1, soit un groupe -CN, à la condition que lorsque Y représente $R_4$ qui représente un phényle substitué par un groupe $-CH_2NR_{11}R_{12}$, Z représente $R_5$ qui représente un groupe $-CH_2NR_{11}R_{12}$, $R_{11}$ et $R_{12}$ étant tels que définis pour un

composé de formule (I) dans la revendication 1 ; et,

- lorsque X = $R_1$ $SO_2$-, Y = $R_4$ et Z = $R_5$, on obtient le composé de formule (I) attendu ;
- ou lorsque X ≠ $R_1SO_2$ et/ou Y ≠ $R_4$ et/ou Z ≠ $R_5$, c'est à dire lorsqu'au moins un des groupes X, Y, Z représente respectivement X = H ou groupe N-protecteur, Y =($C_1$-$C_4$)alcoxycarbonyle, Z = -CN, on soumet le composé ainsi obtenu de formule :

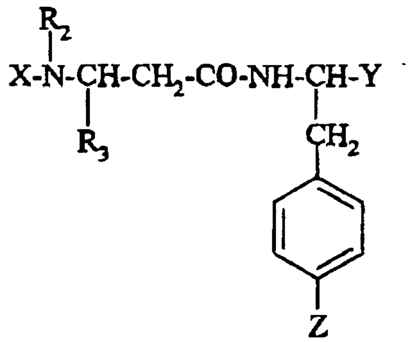

(IV)

à une ou plusieurs des étapes suivantes :

- lorsque X représente un groupe N- protecteur, on élimine ce groupe et on fait réagir le composé ainsi obtenu de formule :

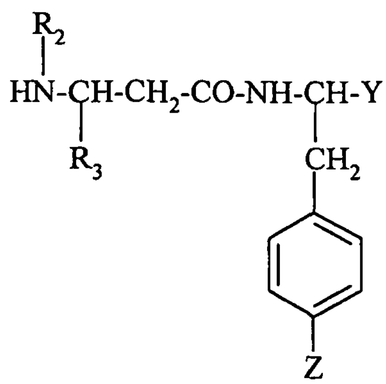

(V)

avec un halogénure de sulfonyle de formule :

$R_1SO_2$-Hal (VI)

dans laquelle Hal représente un halogène ;

- lorsque Y représente un ($C_1$-$C_4$)alcoxycarbonyle, on l'hydrolyse et on fait réagir l'acide ainsi obtenu ou un dérivé fonctionnel de cet acide de formule :

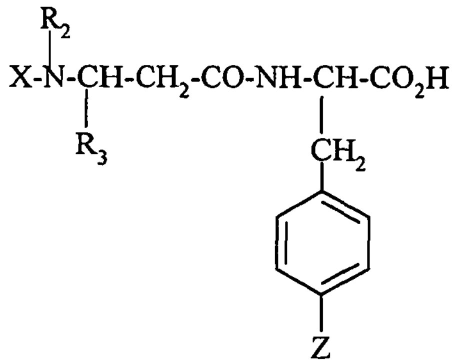

(VII)

avec un composé de formule :

$HNR_8R_9$ (VIII) ;

dans laquelle $R_8$ et $R_9$ sont tels que définis pour un composé de formule (I) à la revendication 1;

- lorsque Z représente un groupe -CN, on transforme ce groupe en $R_5$.

**12.** Composé de formule :

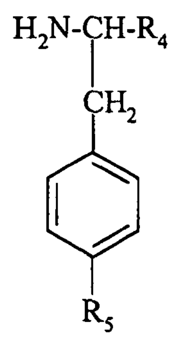

(III')

dans laquelle :

- $R_4$ représente un groupe $-CONR_8R_9$; un groupe $CSNR_8R_9$ ; un groupe $COR_{13}$ ; un phényle non substitué ou substitué une ou plusieurs fois par $R_{10}$ ; un radical hétérocyclique choisi parmi pyridyle, imidazolyle, furyle, benzimidazolyle, benzothiazol-2-yle, benzo[1,3]dioxol-5-yle, lesdits radicaux étant non substitués ou substitués par un méthyle ;
- $R_5$ représente un groupe $-CH_2NR_{11}R_{12}$ ou-$CH_2N(O)R_{11}R_{12}$ ;
- $R_8$ et $R_9$ représentent chacun indépendamment l'hydrogène ; un groupe $(C_1-C_4)$alkyle; $(C_3-C_7)$cycloalkyle; $(C_3-C_7)$cylcoalkyle$(C_1-C_4)$alkyle ; ω-$(C_1-C_4)$dialkylamino$(C_2-C_4)$alkyle ;
- ou bien $R_8$ et $R_9$ ensemble avec l'atome d'azote auquel ils sont liés constituent un radical hétérocyclique choisi parmi pyrrolidinyle, pipéridinyle, morpholin-4-yle, thiomorpholin-4-yle, pipérazin-1-yle, 4-méthylpipérazin-1-yle, 4-méthylpipéridin-1-yle, 2-méthylpipéridin-1-yle, 4,4-diméthylpipéridin-1-yle, 4,4-difluoropipéridin-1-yle, 4-trifluorométhylpipéridin-1-yle, 4-méthoxypipéridin-1-yle, 3,4-dihydropipéridin-1-yle, azépin-1-yle, cyclohexylspiro-4-pipéridin-1-yle ;
- $R_{10}$ représente un atome d'halogène ; un groupe $(C_1-C_4)$alkyle ; hydroxy ; $(C_1-C_6)$alcoxy ; $R_{10}$ peut de plus représenter un groupe $-CH_2NR_{11}R_{12}$ lorsque $R_5$ représente un groupe-$CH_2NR_{11}R_{12}$, lesdits groupes étant alors identiques ;
- $R_{11}$ et $R_{12}$ représentent chacun indépendamment l'hydrogène ; un groupe $(C_1-C_6)$alkyle; $(C_2-C_4)$alcènyle ; $(C_3-C_7)$cycloalkyle ; $(C_3-C_7)$cycloalkyle$(C_1-C_4)$alkyle ; ω-hydroxy$(C_2-C_4)$alkylène ; ω-méthoxy$(C_2-C_4)$alkylène ; ω-trifluorométhyl$(C_2-C_4)$alkylène ; ω-halogéno$(C_2-C_4)$alkylène ;
- ou bien $R_{11}$ et $R_{12}$ ensemble avec l'atome d'azote auquel ils sont liés constituent un radical hétérocyclique,

mono ou bicyclique, choisi parmi azétidinyle, pyrrolidinyle, morpholin-4-yle, thiomorpholin-4-yle, pipéridin-1-yle, pipérazin-1-yle, 1,2,3,6-tétrahydropyrid-1-yle, 2,3,4,5-tétrahydropyridinium, decahydroquinolyle, decahydroisoquinolyle, tétrahydroisoquinolyle, octahydro-1*H*-isoindolyle, $(C_4-C_6)$cycloalkylspiropipéridinyle, 3-azabicyclo[3.1.0]hexyle, 7-azabicyclo[2.2.1]heptan-7-yle, non substitué ou substitué une ou plusieurs fois par un atome d'halogène, un groupe $(C_1-C_4)$alkyle, hydroxy, $(C_1-C_4)$alcoxy, trifluorométhyle, difluorométhylène ;
- $R_{13}$ représente un groupe phényle, thiazol-2-yle ou pyridyle ;

ainsi que ses sels avec des acides minéraux ou organiques, sous forme de racémiques ou d'énantiomères purs.

**13.** Composition pharmaceutique contenant en tant que principe actif un composé selon l'une des revendications 1 à 8 ou un de ses sels et/ou solvats et/ou hydrates pharmaceutiquement acceptables.

**14.** Médicament **caractérisé en ce qu'**il est constitué d'un composé selon l'une quelconque des revendications 1 à 8.

**15.** Utilisation d'un composé selon l'une des revendications 1 à 8 ou de l'un de ses sels et/ou hydrates et/ou solvats pharmaceutiquement acceptables pour la préparation de médicaments destinés à traiter toute pathologie bradykinine dépendante.

**16.** Utilisation selon la revendication 15 pour la préparation de médicaments destinés à traiter les pathologies de l'inflammation, les maladies inflammatoires persistantes ou chroniques.

## Claims

**1.** Compound of formula:

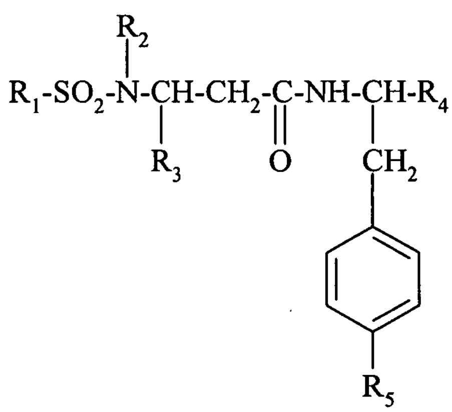

(I)

in which:

- $R_1$ represents a phenylvinyl group; a phenyl group which is unsubstituted or substituted one or more times with $R_6$, which may be identical or different; a naphthyl group which is unsubstituted or substituted one or more times with $R_6$, which may be identical or different; a tetrahydronaphthyl group; a naphtho[2,3-d][1,3]dioxol-6-yl group; a heterocyclic radical chosen from quinolyl, isoquinolyl, 1-benzofur-2-yl, 2,1,3-benzoxadiazol-4-yl, 2,1,3-benzothiadiazol-4-yl, 1,3-benzothiazol-2-yl, 1-benzothiophen-2-yl, 1*H*-pyrazol-4-yl, thien-2-yl, 5-isoxazolthien-2-yl, benzothien-2-yl, thieno[3,2-c]pyrid-2-yl; the said heterocyclic radicals being unsubstituted or substituted one or more times with $R_6$, which may be identical or different;
- $R_2$ represents hydrogen or a $(C_1-C_4)$alkyl group and $R_3$ represents a phenyl group which is unsubstituted or substituted one or more times with $R_7$, which may be identical or different; a heterocyclic radical chosen from benzo[1,3]dioxol-5-yl, 2,1,3-benzothiadiazol-5-yl, 2,1,3-benzoxadiazol-5-yl, benzothiophen-5-yl, 2,3-dihydrobenzo[1,4]dioxin-6-yl, benzofuryl, dihydrobenzofuryl, 1,3-thiazol-2-yl, furyl and thienyl, the said heterocyclic radical being unsubstituted or substituted one or more times with a halogen atom or with a $(C_1-C_4)$alkyl group;
- or alternatively $R_2$ represents a phenyl group which is unsubstituted or substituted one or more times with $R_6$, which may be identical or different; a heterocyclic radical chosen from benzo [1,3]dioxol-5-yl, pyridyl and indanyl, and $R_3$ represents hydrogen;

- $R_4$ represents a group $-CONR_8R_9$; a group $-CSNR_8R_9$; a group $-COR_{13}$; a phenyl group which is unsubstituted or substituted one or more times with $R_{10}$; a heterocyclic radical chosen from pyridyl, imidazolyl, furyl, benzimidazolyl, benzothiazol-2-yl and benzo[1,3]dioxol-5-yl, the said radicals being unsubstituted or substituted with one or more methyl groups or halogen atoms;
- $R_5$ represents a group $-CH_2NR_{11}R_{12}$ or-$CH_2N(O)NR_{11}R_{12}$;
- $R_6$ represents a halogen atom; a ($C_1$-$C_4$)alkyl group; a trifluoromethyl group; a ($C_1$-$C_4$)alkoxy group; a 2-fluoroethoxy group; a trifluoromethoxy, methylenedioxy or difluoromethylenedioxy group;
- $R_7$ represents a halogen atom; a ($C_1$-$C_4$)alkyl group; a phenyl group; a trifluoromethyl group; a ($C_1$-$C_4$)alkoxy group; a benzyloxy group; a trifluoromethoxy group;
- $R_8$ and $R_9$ each independently represent hydrogen; a ($C_1$-$C_4$)alkyl group; a ($C_3$-$C_7$)cycloalkyl group; a ($C_3$-$C_7$)cycloalkyl($C_1$-$C_4$)alkyl group; an ω-($C_1$-$C_4$)dialkylamino($C_2$-$C_4$)alkyl group;
- or alternatively $R_8$ and $R_9$, together with the nitrogen atom to which they are attached, constitute a heterocyclic radical chosen from pyrrolidinyl, morpholin-4-yl, thiomorpholin-4-yl, azepin-1-yl, piperidyl which is unsubstituted or substituted with one or more halogen atoms or one or more ($C_1$-$C_4$)alkyl or ($C_1$-$C_4$)alkoxy or trifluoromethyl groups, 3,4-dihydropiperid-1-yl, cyclohexyl-spiro-4-piperid-1-yl, and piperazinyl which is unsubstituted or substituted with one or more ($C_1$-$C_4$)alkyl groups;
- $R_{10}$ represents a halogen atom; a ($C_1$-$C_4$)alkyl group; a hydroxy group; a ($C_1$-$C_6$) alkoxy group; $R_{10}$ can also represent a group $-CH_2NR_{11}R_{12}$ when $R_5$ represents a group $-CH_2NR_{11}R_{12}$, the said groups then being identical;
- $R_{11}$ and $R_{12}$ each independently represent hydrogen; a ($C_1$-$C_6$)alkyl group; a ($C_2$-$C_4$)alkenyl group; a ($C_3$-$C_7$) cycloalkyl group; a ($C_3$-$C_7$) cycloalkyl ($C_1$-$C_4$)alkyl group; an ω-hydroxy($C_2$-$C_4$)alkylene group; an ω-methoxy ($C_2$-$C_4$)alkylene group; an ω-trifluoromethyl($C_2$-$C_4$)alkylene group; an ω-halo($C_2$-$C_4$)alkylene group,
- or alternatively $R_{11}$ and $R_{12}$, together with the nitrogen atom to which they are attached, constitute a monocyclic, bicyclic or heterocyclic radical chosen from azetidinyl, pyrrolidinyl, morpholin-4-yl, thiomorpholin-4-yl, piperid-1-yl, piperazin-1-yl, 1,2,3,6-tetrahydropyrid-1-yl, 2,3,4,5-tetrahydropyridinium, decahydroquinolyl, decahydroisoquinolyl, tetrahydroisoquinolyl, octahydro-1*H*-isoindolyl, ($C_4$-$C_6$)cycloalkyl-spiro-piperidyl, 3-azabicyclo [3.1.0]hexyl and 7-azabicyclo[2.2.1]heptan-7-yl, which may be unsubstituted or substituted one or more times with a halogen atom or a ($C_1$-$C_4$)alkyl, hydroxyl, ($C_1$-$C_4$)alkoxy, trifluoromethyl, difluoromethylene or phenyl group;
- $R_{13}$ represents a phenyl, thiazol-2-yl or pyridyl group;

and also the salts thereof with mineral or organic acids and/or solvates thereof and/or hydrates thereof.

**2.** Salt of a compound of formula (I) according to Claim 1, chosen from the hydrochloride, the hydrobromide, the sulfate, the hydrogen sulfate, the dihydrogen phosphate, the methanesulfonate, the oxalate, the maleate, the succinate, the fumarate, the 2-naphthalenesulfonate, the benzenesulfonate and the para-toluenesulfonate.

**3.** Compound of formula according to Claim 1:

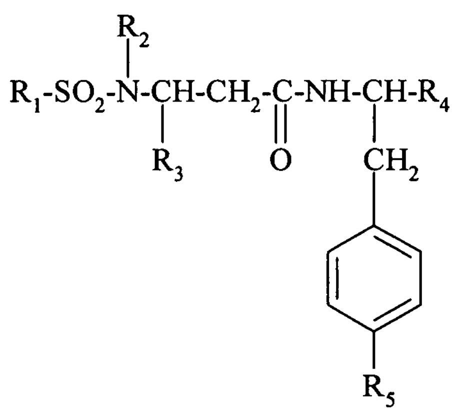

(Ia)

in which:

- $R_1$ represents a phenylvinyl group; a phenyl group which is unsubstituted or substituted one or more times with $R_6$, which may be identical or different; a naphthyl group which is unsubstituted or substituted one or more times with $R_6$, which may be identical or different; a tetrahydronaphthyl group; a heterocyclic radical chosen

from quinolyl, 1-benzofur-2-yl, 2,1,3-benzoxadiazol-4-yl, 2,1,3-benzothiadiazol-4-yl, 1,3-benzothiazol-2-yl, 1-benzothiophen-2-yl, 1*H*-pyrazol-4-yl, thien-2-yl, 5-isoxazolethien-2-yl, benzothien-2-yl, thieno[3,2-c]pyrid-2-yl; naphtho[2,3-d][1,3]dioxol-6-yl; the said heterocyclic radicals being unsubstituted or substituted one or more times with $R_6$, which may be identical or different;

- $R_2$ represents hydrogen or a ($C_1$-$C_4$)alkyl group and $R_3$ represents a phenyl group which is unsubstituted or substituted one or more times with $R_7$, which may be identical or different; a heterocyclic radical chosen from benzo[1,3]dioxol-5-yl which is unsubstituted or substituted in the
- 2 position with two fluorine atoms; 2,1,3-benzothiadiazol-5-yl; 2,3-dihydrobenzo[1,4]dioxin-6-yl; 1,3-thiazol-2-yl; 1-benzofur-2-yl; 1-benzofur-5-yl; furyl; thien-2-yl; thien-3-yl;
- or $R_2$ represents a phenyl group which is unsubstituted or substituted one or more times with $R_6$, which may be identical or different; a heterocyclic radical chosen from benzo[1,3]dioxol-5-yl; pyridyl; indanyl; and $R_3$ represents hydrogen;
- $R_4$ represents a group -$CONR_8R_9$; a phenyl group which is unsubstituted or substituted one or more times with $R_{10}$; a heterocyclic radical chosen from pyridyl, imidazolyl, furyl, benzimidazolyl, benzothiazol-2-yl and benzo[1,3]dioxol-5-yl, the said radicals being unsubstituted or substituted with a methyl;
- $R_5$ represents a group -$CH_2NR_{11}R_{12}$;
- $R_6$ represents a halogen atom; a ($C_1$-$C_4$)alkyl group; a trifluoromethyl group; a ($C_1$-$C_4$)alkoxy group; a 2-fluoroethoxy group; a trifluoromethoxy, methylenedioxy or difluoromethylenedioxy group;
- $R_7$ represents a halogen atom; a ($C_1$-$C_4$)alkyl group; a trifluoromethyl group; a ($C_1$-$C_4$)alkoxy group; a benzyloxy group; a trifluoromethoxy group;
- $R_8$ and $R_9$ each independently represent hydrogen; a ($C_1$-$C_4$)alkyl group; a ($C_3$-$C_7$)cycloalkyl group; a ($C_3$-$C_7$) cycloalkyl ($C_1$-$C_4$)alkyl group; an ω-($C_1$-$C_4$)dialkylamino($C_2$-$C_4$)alkyl group;
- or $R_8$ and $R_9$, together with the nitrogen atom to which they are attached, constitute a heterocyclic radical chosen from pyrrolidinyl, piperidyl, morpholin-4-yl, thiomorpholin-4-yl, piperazin-1-yl, 4-methylpiperazin-1-yl, 4-methylpiperid-1-yl, 2-methylpiperid-1-yl, 4,4-dimethylpiperid-1-yl, 4,4-difluoropiperid-1-yl, 4-trifluoromethylpiperid-1-yl, 3,4-dihydropiperid-1-yl, azepin-1-yl and cyclohexyl-spiro-4-piperid-1-yl;
- $R_{10}$ represents a halogen atom; a ($C_1$-$C_4$)alkyl group; a hydroxyl group; a ($C_1$-$C_6$)alkoxy group; $R_{10}$ can also represent a group -$CH_2NR_{11}R_{12}$ when $R_5$ represents a group -$CH_2NR_{11}R_{12}$, the said groups then being identical;
- $R_{11}$ and $R_{12}$ each independently represent hydrogen; a ($C_1$-$C_6$)alkyl group; a ($C_2$-$C_4$)alkenyl group; a ($C_3$-$C_7$) cycloalkyl group; a ($C_3$-$C_7$)cycloalkyl($C_1$-$C_4$)alkyl group; an ω-hydroxy($C_2$-$C_4$)alkylene group; an ω-methoxy ($C_2$-$C_4$)alkylene group; an ω-trifluoromethyl($C_2$-$C_4$)alkylene group; an ω-halo($C_2$-$C_4$)alkylene group;
- or $R_{11}$ and $R_{12}$, together with the nitrogen atom to which they are attached, constitute a monocyclic, bicyclic or heterocyclic radical chosen from azetidinyl, pyrrolidinyl, morpholin-4-yl, thiomorpholin-4-yl, piperid-1-yl, piperazin-1-yl, 1,2,3,6-tetrahydropyrid-1-yl, decahydroquinolyl, decahydroisoquinolyl, octahydro-1*H*-isoindolyl, ($C_4$-$C_6$)cycloalkyl-spiropiperidyl and 3-azabicyclo[3.1.0]hexyl, which may be unsubstituted or substituted one or more times with a halogen atom or a ($C_1$-$C_4$)alkyl, hydroxyl, ($C_1$-$C_4$)alkoxy, trifluoromethyl, difluoromethylene or phenyl group;

and also the salts thereof with mineral or organic acids and/or solvates thereof or hydrates thereof.

**4.** Compound of formula (I) according to Claim 1 or Claim 2, in which $R_1$ represents a 2,4-dichloro-3-methylphenyl, naphthyl, 6-methoxynaphth-2-yl, 3-methylbenzothiophen-2-yl, 3-methyl-5-chlorobenzothiophen-2-yl, 3-methyl-5-methoxybenzothiophen-2-yl, 3-methyl-6-methoxybenzothiophen-2-yl or 3-methyl-1-benzofur-2-yl group.

**5.** Compound of formula (I) according to Claim 1 or Claim 2, in which $R_2$ represents hydrogen and $R_3$ represents a benzo[1,3]dioxol-5-yl or phenyl group which is unsubstituted or substituted with a halogen.

**6.** Compound of formula (I) according to Claim 1 or Claim 2, in which $R_4$ represents a group -$CONR_8R_9$ and -$NR_8R_9$ represents a di($C_1$-$C_4$)alkylamino radical, a pyrrolidinyl or piperidyl group which is unsubstituted or substituted one or two times with a methyl or a halogen.

**7.** Compound of formula (I) according to Claim 1 or Claim 2, in which $R_5$ represents a group-$CH_2NR_{11}R_{12}$ in which -$NR_{11}R_{12}$ represents an ethylisobutylamino, ethylisopropylamino, ethyl-tert-butylamino, diisopropylamino, cyclopentylmethylamino or cyclopentylethylamino radical or a piperidyl radical which is unsubstituted or substituted one or more times with a methyl or a halogen.

**8.** Compound according to Claim 1 or Claim 2 of formula:

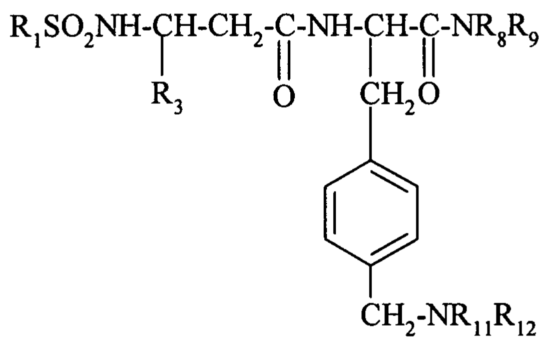

(Ia)

in which:

- $R_1$ represents a 2,4-dichloro-3-methylphenyl, naphthyl, 6-methoxynaphth-2-yl, 3-methylbenzothiophen-2-yl, 3-methyl-5-chlorobenzothiophen-2-yl, 3-methyl-5-methoxybenzothiophen-2-yl, 3-methyl-6-methoxybenzothiophen-2-yl or 3-methyl-1-benzofur-2-yl group;
- $R_3$ represents a benzo[1,3]dioxol-5-yl or phenyl group which is unsubstituted or substituted with a halogen;
- $R_8$ and $R_9$, together with the nitrogen atom to which they are attached, constitute a di($C_1$-$C_4$)alkylamino, pyrrolidinyl or piperidyl radical which is unsubstituted or substituted one or two times with a methyl or a halogen;
- $R_{11}$ and $R_{12}$, together with the nitrogen atom to which they are attached, constitute an ethylisobutylamino, ethylisopropylamino, ethyl-tert-butylamino, diisopropylamino, cyclopentylmethylamino or cyclopentylethylamino radical or a piperidyl radical which is unsubstituted or substituted one or more times with a methyl or a halogen;

and also the salts thereof with mineral or organic acids and/or solvates thereof or hydrates thereof.

**9.** Compound according to Claim 1, chosen from:

- (R,R) 2-((3-(1,3-benzodioxol-5-yl)-3-(((6-methoxy-2-naphthyl)sulphonyl)amino)propanoyl)amino)-3-(4-((2,6-cis-dimethyl-1-piperidyl)methyl)phenyl)-N-isopropyl-N-methylpropanamide;
- (R,R) 2-((3-(1,3-benzodioxol-5-yl)-3-((2-naphthylsulphonyl)amino)propanoyl)amino)-3-(4-((cyclopentyl(ethyl)amino)methyl)phenyl)-N-isopropyl-N-methylpropanamide;
- (R,R) 3-(4-((2,6-cis-dimethyl-1-piperidyl)methyl)phenyl)-2-((3-(((6-methoxy-2-naphthyl)sulphonyl)amino)-3-phenylpropanoyl)amino)-N-isopropyl-N-methyl propanamide;
- (R,R) 2-((3-(1,3-benzodioxol-5-yl)-3-(((6-methoxy-2-naphthyl)sulphonyl)amino)propanoyl)amino)-3-(4-((*tert*-butyl(ethyl)amino)methyl)phenyl)-N-isopropyl-N-methylpropanamide;
- (R,R) 2-((3-(1,3-benzodioxol-5-yl)-3-(((3-methyl-1-benzothiophen-2-yl)sulphonyl)amino)propanoyl)amino)-3-(4-((2,6-cis-dimethyl-1-piperidyl)methyl)phenyl)-N-isopropyl-N-methylpropanamide;
- (R,R) 3-(4-((2,6-cis-dimethyl-1-piperidyl)methyl)phenyl)-2-((3-(((5-methoxy-3-methyl-1-benzothiophen-2-yl)sulphonyl)amino)-3-phenylpropanoyl)amino)-N-isopropyl-N-methylpropanamide;
- (R,R) N-(4-((2,6-cis-dimethyl-1-piperidyl)methyl)benzyl)-3-(((6-methoxy-2-naphthyl)sulphonyl)amino)-3-phenyl-N-(1-piperidylcarbonyl)propanamide;
- (R,R) 2-((3-(4-chlorophenyl)-3-(((6-methoxy-2-naphthyl)sulphonyl)amino)propanoyl)amino)-3-(4-((2,6-cis-dimethyl-1-piperidyl)methyl)phenyl)-N-isopropyl-N-methylpropanamide;
- (R,R) 3-(4-((2,6-cis-dimethyl-1-piperidyl)methyl)phenyl)-2-((3-(((6-methoxy-2-naphthyl)sulphonyl)amino)-3-phenylpropanoyl)amino)-N,N-diethylpropanamide;
- (R,R) 2-((3-(3-fluorophenyl)-3-(((6-methoxy-2-naphthyl)sulphonyl)amino)propanoyl)amino)-3-(4-((2,6-cis-dimethyl-1-piperidyl)methyl)phenyl)-N-isopropyl-N-methylpropanamide;
- (R,R) 2-((3-(1,3-benzodioxol-5-yl)-3-(((3-methyl-1-benzofur-2-yl)sulphonyl)amino)propanoyl)amino)-3-(4-((2,6-cis-dimethyl-1-piperidyl)methyl)phenyl)-N-isopropyl-N-methylpropanamide;
- (R,R) 2-((3-(1,3-benzodioxol-5-yl)-3-((2-naphthylsulphonyl)amino)propanoyl)amino)-3-(3-((*tert*-butyl(ethyl)amino)methyl)phenyl)-N-isopropyl-N-methylpropanamide;
- (R,R) 3-(4-(7-azabicyclo[2.2.1]hept-7-ylmethyl)phenyl)-2-((3-(1,3-benzodioxol-5-yl)-3-(((6-methoxynaphthyl)sulphonyl)amino)propanoyl)amino)-N-isopropyl-N-methyl propanamide;

and also the salts and/or solvates and/or hydrates thereof.

**10.** (R,R)-2-((3-(1,3-Benzodioxol-5-yl)-3-(((6-methoxy-2-naphthyl)sulfonyl)amino)propanoyl)amino)-3-(4-((2,6-cis-dimethyl-1-piperidinyl)methyl)phenyl)-N-isopropyl-N-methylpropanamide;
and also the salts thereof with mineral or organic acids and/or the solvates or hydrates thereof.

**11.** Process for preparing a compound of formula (I), salts thereof and/or solvates thereof or hydrates thereof, **characterized in that**:

an acid or a functional derivative of this acid of formula:

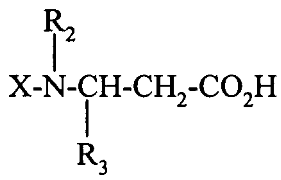

(II)

in which $R_2$ and $R_3$ are as defined for a compound of formula (I) in Claim 1, X represents either hydrogen or a group $R_1SO_2$- in which $R_1$ is as defined for a compound of formula (I) in Claim 1, or an N-protecting group, is reacted with a compound of formula:

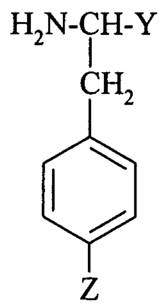

(III)

in which Y represents either $R_4$ as defined for a compound of formula (I) in Claim 1, or a $(C_1-C_4)$ alkoxycarbonyl, and Z represents either $R_5$ as defined for a compound of formula (I) in Claim 1, or a -CN group, on the condition that, when Y represents $R_4$ which represents a phenyl substituted with a group $-CH_2NR_{11}R_{12}$, Z represents $R_5$ which represents a group $-CH_2NR_{11}R_{12}$, $R_{11}$ and $R_{12}$ being as defined for a compound of formula (I) in Claim 1; and

- when X = $R_1SO_2$-, Y = $R_4$ and Z = $R_5$, the expected compound of formula (I) is obtained;
- or when X ≠ $R_1SO_2$ and/or Y ≠ $R_4$ and/or Z ≠ $R_5$, that is to say when at least one of the X, Y and Z groups represents, respectively, X = H or an N-protecting group, Y =$(C_1-C_4)$alkoxycarbonyl, Z = -CN, the compound thus obtained of formula:

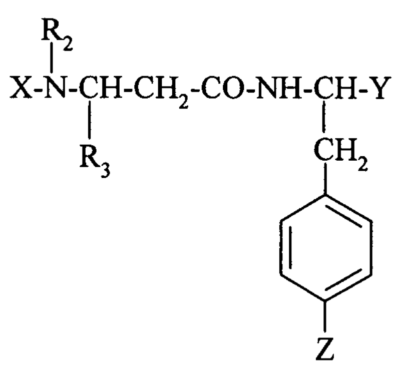

(IV)

is subjected to one or more of the following steps:

- when X represents an N- protecting group, this group is removed and the compound thus obtained of formula:

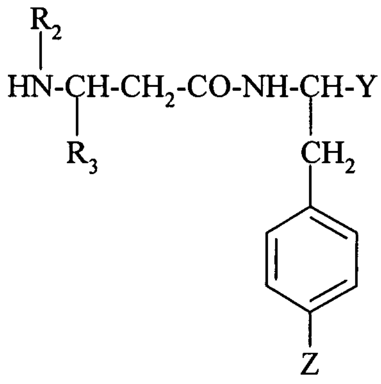

(V)

is reacted with a sulphonyl halide of formula:

$R_1SO_2$-Hal (VI)

in which Hal represents a halogen;

- when Y represents a ($C_1$-$C_4$)alkoxycarbonyl, it is hydrolyzed and the acid thus obtained or a functional derivative of this acid of formula:

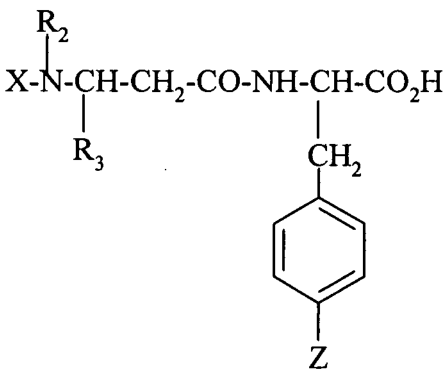

(VII)

is reacted with a compound of formula:

$HNR_8R_9$ (VIII);

in which $R_8$ and $R_9$ are as defined for a compound of formula (I) from Claim 1;

- when Z represents a -CN group, this group is converted into $R_5$.

**12.** Compound of formula:

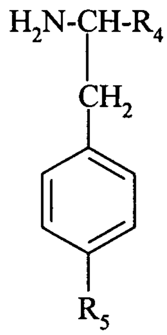

(III')

in which:

- $R_4$ represents a group $-CONR_8R_9$; a group-$CSNR_8R_9$ ; a group $-COR_{13}$; a phenyl group which is unsubstituted or substituted one or more times with $R_{10}$; a heterocyclic radical chosen from pyridyl, imidazolyl, furyl, benzimidazolyl, benzothiazol-2-yl and benzo[1,3]dioxol-5-yl, the said radicals being unsubstituted or substituted with a methyl;
- $R_5$ represents a group $-CH_2NR_{11}R_{12}$ or-$CH_2N(O)NR_{11}R_{12}$ ;
- $R_8$ and $R_9$ each independently represent hydrogen; a $(C_1-C_4)$alkyl group; a $(C_3-C_7)$cycloalkyl group; a $(C_3-C_7)$cycloalkyl$(C_1-C_4)$alkyl group; an ω-$(C_1-C_4)$dialkylamino$(C_2-C_4)$alkyl group;
- or alternatively $R_8$ and $R_9$, together with the nitrogen atom to which they are attached, constitute a heterocyclic radical chosen from pyrrolidinyl, piperidyl, morpholin-4-yl, thiomorpholin-4-yl, piperazin-1-yl, 4-methylpiperazin-1-yl, 4-methylpiperid-1-yl, 2-methylpiperid-1-yl, 4,4-dimethylpiperid-1-yl, 4,4-difluoropiperid-1-yl, 4-trifluoromethylpiperid-1-yl, 4-methoxypiperid-1-yl, 3,4-dihydropiperid-1-yl, azepin-1-yl and cyclohexyl-spiro-4-piperid-1-yl;
- $R_{10}$ represents a halogen atom; a $(C_1-C_4)$alkyl group; a hydroxy group; a $(C_1-C_6)$alkoxy group; $R_{10}$ can also represent a group $-CH_2NR_{11}R_{12}$ when $R_5$ represents a group $-CH_2NR_{11}R_{12}$, the said groups then being identical;
- $R_{11}$ and $R_{12}$ each independently represent hydrogen; a $(C_1-C_6)$ alkyl group; a $(C_2-C_4)$alkenyl group; a $(C_3-C_7)$cycloalkyl group; a $(C_3-C_7)$cycloalkyl$(C_1-C_4)$alkyl group; an ω-hydroxy $(C_2-C_4)$alkylene group; an ω-methoxy $(C_2-C_4)$alkylene group; an ω-trifluoromethyl$(C_2-C_4)$alkylene group; an ω)-halo$(C_2-C_4)$alkylene group;
- or alternatively $R_{11}$ and $R_{12}$, together with the nitrogen atom to which they are attached, constitute a monocyclic, bicyclic or heterocyclic radical chosen from azetidinyl, pyrrolidinyl, morpholin-4-yl, thiomorpholin-4-yl, piperid-1-yl, piperazin-1-yl, 1,2,3,6-tetrahydropyrid-1-yl, 2,3,4,5-tetrahydropyridinium, decahydroquinolyl, decahydroisoquinolyl, tetrahydroisoquinolyl, octahydro-1*H*-isoindolyl, $(C_4-C_6)$cycloalkyl-spiro-piperidyl, 3-azabicyclo [3.1.0]hexyl and 7-azabicyclo[2.2.1]heptan-7-yl, which may be unsubstituted or substituted one or more times with a halogen atom or a $(C_1-C_4)$alkyl, hydroxyl, $(C_1-C_4)$alkoxy, trifluoromethyl or difluoromethylene group;
- $R_{13}$ represents a phenyl, thiazol-2-yl or pyridyl group;

and also the salts thereof with mineral or organic acids, in the form of racemic mixtures or of pure enantiomers.

**13.** Pharmaceutical composition containing, as active principle, a compound according to one of Claims 1 to 8 or one of the pharmaceutically acceptable salts and/or solvates and/or hydrates thereof.

**14.** Medicinal product, **characterized in that** it constitutes a compound according to any one of Claims 1 to 8.

**15.** Use of a compound according to one of Claims 1 to 8 or of one of the pharmaceutically acceptable salts and/or hydrates and/or solvates thereof for the preparation of medicinal products intended for treating any bradykinin-dependent pathology.

**16.** Use according to Claim 15, for the preparation of medicinal products intended for treating inflammation pathologies and persistent or chronic inflammatory diseases.

**Patentansprüche**

**1.** Verbindung der Formel:

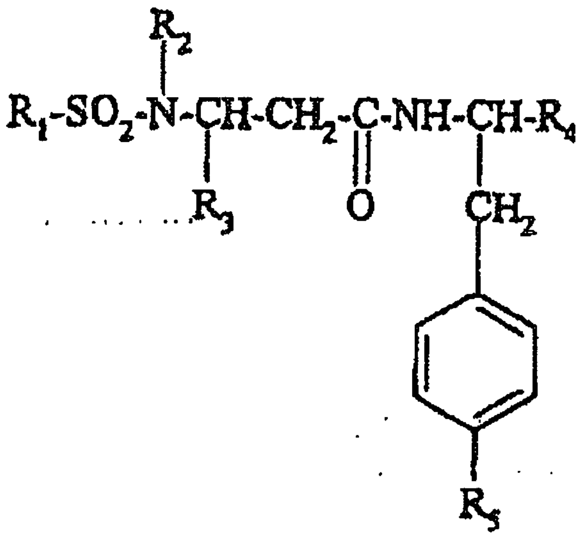

(I)

worin:

- $R_1$ für eine Phenylvinylgruppe; eine unsubstituierte oder ein- oder mehrfach durch gleiche oder verschiedene Gruppen $R_6$ substituierte Phenylgruppe; eine unsubstituierte oder ein- oder mehrfach durch gleiche oder verschiedene Gruppen $R_6$ substituierte Naphthylgruppe; eine Tetrahydronaphthylgruppe; eine Naphtho[2,3-d][1,3]dioxol-6-ylgruppe oder einen unter Chinolyl, Isochinolyl, 1-Benzofuran-2-yl, 2,1,3-Benzoxadiazol-4-yl, 2,1,3-Benzothiadiazol-4-yl, 1,3-Benzothiazol-2-yl, 1-Benzothiophen-2-yl, 1*H*-Pyrazol-4-yl, Thien-2-yl, 5-Isoxazolthien-2-yl, Benzothien-2-yl und Thieno-[3,2-c]pyridin-2-yl ausgewählten heterocyclischen Rest steht, wobei die heterocyclischen Reste unsubstituiert oder ein- oder mehrfach durch gleiche oder verschiedene Gruppen $R_6$ substituiert sind;
- $R_2$ für Wasserstoff oder eine $(C_1-C_4)$-Alkylgruppe steht und $R_3$ für eine unsubstituierte oder ein- oder mehrfach durch gleiche oder verschiedene Gruppen $R_7$ substituierte Phenylgruppe oder einen unter Benzo[1,3]dioxol-5-yl, 2,1,3-Benzothiadiazol-5-yl, 2,1,3-Benzoxadiazol-5-yl, Benzothiophen-5-yl, 2,3-Dihydrobenzo[1,4]dioxin-6-yl, Benzofuranyl, Dihydrobenzofuranyl, 1,3-Thiazol-2-yl, Furyl und Thienyl ausgewählten heterocyclischen Rest, der unsubstituiert oder ein- oder mehrfach durch ein Halogenatom oder eine $(C_1-C_4)$-Alkylgruppe substituiert ist; steht;
- oder $R_2$ für eine unsubstituierte oder ein- oder mehrfach durch gleiche oder verschiedene Gruppen $R_6$ substituierte Phenylgruppe oder einen unter Benzo[1,3]dioxol-5-yl, Pyridyl und Indanyl ausgewählten heterocyclischen Rest steht und $R_3$ für Wasserstoff steht;
- $R_4$ für eine Gruppe $-CONR_8R_9$; eine Gruppe $-CSNR_8R_9$; eine Gruppe $-COR_{13}$; eine unsubstituierte oder ein- oder mehrfach durch $R_{10}$ substituierte Phenylgruppe oder einen unter Pyridyl, Imidazolyl, Furyl, Benzimidazolyl, Benzothiazol-2-yl und Benzo[1,3]dioxol-5-yl ausgewählten heterocyclischen Rest steht, wobei die Reste unsubstituiert oder durch eine oder mehrere Methylgruppen oder Halogenatome substituiert sind ;
- $R_5$ für eine Gruppe $-CH_2NR_{11}R_{12}$ oder $-CH_2N(O)R_{11}R_{12}$ steht;
- $R_6$ für ein Halogenatom; eine $(C_1-C_4)$-Alkylgruppe; eine Trifluormethylgruppe; eine $(C_1-C_4)$-Alkoxygruppe; eine 2-Fluorethoxygruppe; eine Trifluormethoxygruppe; eine Methylendioxygruppe oder eine Difluormethylendioxygruppe steht;
- $R_7$ für ein Halogenatom; eine $(C_1-C_4)$-Alkylgruppe; eine Phenylgruppe; eine Trifluormethylgruppe; eine $(C_1-C_4)$-Alkoxygruppe; eine Benzyloxygruppe oder eine Trifluormethoxygruppe steht;
- $R_8$ und $R_9$ jeweils unabhängig voneinander für Wasserstoff; eine $(C_1-C_4)$-Alkylgruppe; eine $(C_3-C_7)$-Cycloalkylgruppe; eine $(C_3-C_7)$-Cycloalkyl-$(C_1-C_4)$-alkylgruppe oder eine $\omega$-$(C_1-C_4)$-Dialkylamino-$(C_2-C_4)$-alkylgruppe stehen;
- oder $R_8$ und $R_9$ gemeinsam mit dem Stickstoffatom, an das sie gebunden sind, einen unter Pyrrolidinyl, Morpholin-4-yl, Thiomorpholin-4-yl, Azepin-1-yl, unsubstituiertem oder durch ein oder mehrere Halogenatome oder eine oder mehrere $(C_1-C_4)$-Alkylgruppen, $(C_1-C_4)$-Alkoxygruppen oder Trifluormethylgruppen substituiertem Piperidinyl, 3,4-Dihydropiperidin-1-yl, Cyclohexylspiro-4-piperidin-1-yl und unsubstituiertem oder durch eine oder mehrere $(C_1-C_4)$-Alkylgruppen substituiertem Piperazinyl ausgewählten heterocyclischen Rest bilden;
- $R_{10}$ für ein Wasserstoffatom; eine $(C_1-C_4)$-Alkylgruppe; eine Hydroxygruppe oder eine $(C_1-C_6)$-Alkoxygruppe

steht und außerdem für eine Gruppe $-CH_2NR_{11}R_{12}$ stehen kann, wenn $R_5$ für eine Gruppe $-CH_2NR_{11}R_{12}$ steht, wobei die Gruppen dann identisch sind;

- $R_{11}$ und $R_{12}$ jeweils unabhängig voneinander für Wasserstoff; eine $(C_1-C_6)$ -Alkylgruppe; eine $(C_2-C_4)$-Alkenylgruppe; eine $(C_3-C_7)$-Cycloalkylgruppe; eine $(C_3-C_7)$ -Cycloalkyl- $(C_1-C_4)$ -alkylgruppe; eine ω-Hydroxy-$(C_2-C_4)$-alkylengruppe; eine ω-Methoxy-$(C_2-C_4)$-alkylengruppe; eine ω-Trifluormethyl-$(C_2-C_4)$-alkylengruppe oder eine ω-Halogen- $(C_2-C_4)$-alkylengruppe stehen;
- oder $R_{11}$ und $R_{12}$ gemeinsam mit dem Stickstoffatom, an das sie gebunden sind, einen unter Azetidinyl, Pyrrolidinyl, Morpholin-4-yl, Thiomorpholin-4-yl, Piperidin-1-yl, Piperazin-1-yl, 1,2,3,6-Tetrahydropyrid-1-yl, 2,3,4,5-Tetrahydropyridinium, Decahydrochinolyl, Decahydroisochinolyl, Tetrahydroisochinolyl, Octahydro-1*H*-isoindolyl, $(C_4-C_6)$-Cycloalkylspiropiperidinyl, 3-Azabicyclo[3.1.0]hexyl und 7-Azabicyclo[2.2.1]-heptan-7-yl ausgewählten mono- oder bicyclischen heterocyclischen Rest bilden, der unsubstituiert oder ein- oder mehrfach durch ein Halogenatom oder eine $(C_1-C_4)$-Alkylgruppe, eine Hydroxygruppe, eine $(C_1-C_4)$ -Alkoxygruppe, eine Trifluormethylgruppe, eine Difluormethylengruppe oder eine Phenylgruppe substituiert ist;
- $R_{13}$ für eine Phenylgruppe, eine Thiazol-2-ylgruppe oder eine Pyridylgruppe steht;

sowie Salze davon mit anorganischen oder organischen Säuren und/oder Solvate davon und/oder Hydrate davon.

**2.** Salz einer Verbindung der Formel (I) nach Anspruch 1, ausgewählt unter dem Hydrochlorid, dem Hydrobromid, dem Sulfat, dem Hydrogensulfat, dem Dihydrogenphosphat, dem Methansulfonat, dem Oxalat, dem Maleat, dem Succinat, dem Fumarat, dem Naphthalin-2-sulfonat, dem Benzolsulfonat und dem para-Toluolsulfonat.

**3.** Verbindung der Formel nach Anspruch 1:

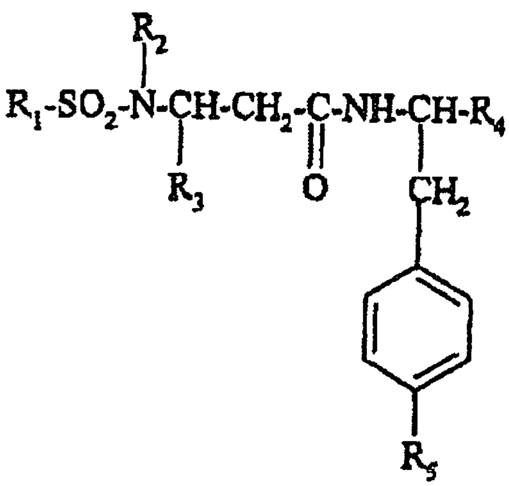

(Ibis)

worin:

- $R_1$ für eine Phenylvinylgruppe; eine unsubstituierte oder ein- oder mehrfach durch gleiche oder verschiedene Gruppen $R_6$ substituierte Phenylgruppe; eine unsubstituierte oder ein- oder mehrfach durch gleiche oder verschiedene Gruppen $R_6$ substituierte Naphthylgruppe; eine Tetrahydronaphthylgruppe oder einen unter Chinolyl, 1-Benzofuran-2-yl, 2,1,3-Benzoxadiazol-4-yl, 2,1,3-Benzothiadiazol-4-yl, 1,3-Benzothiazol-2-yl, 1-Benzothiophen-2-yl, 1*H*-Pyrazol-4-yl, Thien-2-yl, 5-Isoxazolthien-2-yl, Benzothien-2-yl; Thieno[3,2-c]pyridin-2-yl ausgewählten heterocyclischen Rest oder Naphtho[2,3-d][1,3]dioxol-6-ylgruppe steht, wobei die heterocyclischen Reste unsubstituiert oder ein- oder mehrfach durch gleiche oder verschiedene Gruppen $R_6$ substituiert sind;
- $R_2$ für Wasserstoff oder eine $(C_1-C_4)$ -Alkylgruppe steht und $R_3$ für eine unsubstituierte oder ein- oder mehrfach durch gleiche oder verschiedene Gruppen $R_7$ substituierte Phenylgruppe oder einen unter unsubstituiertem oder in 2-Stellung durch zwei Fluoratome substituiertem Benzo[1,3]dioxol-5-yl; 2,1,3-Benzothiadiazol-5-yl; 2,3-Dihydrobenzo-[1,4]dioxin-6-yl; 1,3-Thiazol-2-yl; 1-Benzofuran-2-yl; 1-Benzofuran-5-yl; Furyl; Thien-2-yl und Thien-3-yl ausgewählten heterocyclischen Rest steht;
- oder $R_2$ für eine unsubstituierte oder ein- oder mehrfach durch gleiche oder verschiedene Gruppen $R_6$ substituierte Phenylgruppe oder einen unter Benzo[1,3]dioxol-5-yl, Pyridyl und Indanyl ausgewählten heterocyclischen Rest steht und $R_3$ für Wasserstoff steht;

- $R_4$ für eine Gruppe $-CONR_8R_9$; eine unsubstituierte oder ein- oder mehrfach durch $R_{10}$ substituierte Phenylgruppe oder einen unter Pyridyl, Imidazolyl, Furyl, Benzimidazolyl, Benzothiazol-2-yl und Benzo[1,3]dioxol-5-yl ausgewählten heterocyclischen Rest steht, wobei die Reste unsubstituiert oder durch eine Methylgruppe substituiert sind;
- $R_5$ für eine Gruppe $-CH_2NR_{11}R_{12}$ steht;
- $R_6$ für ein Halogenatom; eine $(C_1\text{-}C_4)$-Alkylgruppe; eine Trifluormethylgruppe; eine $(C_1\text{-}C_4)$-Alkoxygruppe; eine 2-Fluorethoxygruppe; eine Trifluormethoxygruppe; eine Methylendioxygruppe oder eine Difluormethylendioxygruppe steht;
- $R_7$ für ein Halogenatom; eine $(C_1\text{-}C_4)$-Alkylgruppe; eine Trifluormethylgruppe; eine $(C_1\text{-}C_4)$-Alkoxygruppe; eine Benzyloxygruppe oder eine Trifluormethoxygruppe steht;
- $R_8$ und $R_9$ jeweils unabhängig voneinander für Wasserstoff; eine $(C_1\text{-}C_4)$-Alkylgruppe; eine $(C_3\text{-}C_7)$-Cycloalkylgruppe; eine $(C_3\text{-}C_7)$-Cycloalkyl-$(C_1\text{-}C_4)$-alkylgruppe oder eine ω-$(C_1\text{-}C_4)$-Dialkylamino-$(C_2\text{-}C_4)$-alkylgruppe stehen;
- oder $R_8$ und $R_9$ gemeinsam mit dem Stickstoffatom, an das sie gebunden sind, einen unter Pyrrolidinyl, Piperidinyl, Morpholin-4-yl, Thiomorpholin-4-yl, Piperazin-1-yl, 4-Methylpiperazin-1-yl, 4-Methylpiperidin-1-yl, 2-Methylpiperidin-1-yl, 4,4-Dimethylpiperidin-1-yl, 4,4-Difluorpiperidin-1-yl, 4-Trifluormethylpiperidin-1-yl, 3,4-Dihydropiperidin-1-yl, Azepin-1-yl und Cyclohexylspiro-4-piperidin-1-yl ausgewählten heterocyclischen Rest bilden;
- $R_{10}$ für ein Wasserstoffatom; eine $(C_1\text{-}C_4)$-Alkylgruppe; eine Hydroxygruppe oder eine $(C_1\text{-}C_6)$-Alkoxygruppe steht und außerdem für eine Gruppe $-CH_2NR_{11}R_{12}$ stehen kann, wenn $R_5$ für eine Gruppe $-CH_2NR_{11}R_{12}$ steht, wobei die Gruppen dann identisch sind;
- $R_{11}$ und $R_{12}$ jeweils unabhängig voneinander für Wasserstoff; eine $(C_1\text{-}C_6)$ -Alkylgruppe; eine $(C_2\text{-}C_4)$-Alkenylgruppe; eine $(C_3\text{-}C_7)$-Cycloalkylgruppe; eine $(C_3\text{-}C_7)$-Cycloalkyl-$(C_1\text{-}C_4)$-alkylgruppe; eine ω-Hydroxy-$(C_2\text{-}C_4)$-alkylengruppe; eine ω-Methoxy-$(C_2\text{-}C_4)$-alkylengruppe; eine ω-Trifluormethyl-$(C_2\text{-}C_4)$-alkylengruppe oder eine ω-Halogen-$(C_2\text{-}C_4)$-alkylengruppe stehen;
- oder $R_{11}$ und $R_{12}$ gemeinsam mit dem Stickstoffatom, an das sie gebunden sind, einen unter Azetidinyl, Pyrrolidinyl, Morpholin-4-yl, Thiomorpholin-4-yl, Piperidin-1-yl, Piperazin-1-yl, 1,2,3,6-Tetrahydropyrid-1-yl, Decahydrochinolyl, Decahydroisochinolyl, Octahydro-1*H*-isoindolyl, $(C_4\text{-}C_6)$-Cycloalkylspiropiperidinyl und 3-Azabicyclo-[3.1.0]hexyl ausgewählten mono- oder bicyclischen heterocyclischen Rest bilden, der unsubstituiert oder ein- oder mehrfach durch ein Halogenatom oder eine $(C_1\text{-}C_4)$-Alkylgruppe, eine Hydroxygruppe, eine $(C_1\text{-}C_4)$ -Alkoxygruppe, eine Trifluormethylgruppe, eine Difluormethylengruppe oder eine Phenylgruppe substituiert ist;

sowie Salze davon mit anorganischen oder organischen Säuren und/oder Solvate davon und/oder Hydrate davon.

**4.** Verbindung der Formel (I) nach Anspruch 1 oder Anspruch 2, in der $R_1$ für eine 2,4-Dichlor-3-methylphenylgruppe, eine Naphthylgruppe, eine 6-Methoxynaphth-2-ylgruppe, eine 3-Methylbenzothiophen-2-ylgruppe, eine 3-Methyl-5-chlorbenzothiophen-2-ylgruppe, eine 3-Methyl-5-methoxybenzothiophen-2-ylgruppe, eine 3-Methyl-6-methoxybenzothiophen-2-ylgruppe oder eine 3-Methyl-1-benzofur-2-ylgruppe steht.

**5.** Verbindung der Formel (I) nach Anspruch 1 oder Anspruch 2, in der $R_2$ für Wasserstoff steht und $R_3$ für eine Benzo [1,3]dioxol-5-ylgruppe oder eine Phenylgruppe, die unsubstituiert oder durch ein Halogen substituiert ist, steht.

**6.** Verbindung der Formel (I) nach Anspruch 1 oder Anspruch 2, in der $R_4$ für eine Gruppe $-CONR_8R_9$ steht und $-NR_8R_9$ für einen Di-$(C_1\text{-}C_4)$-alkylaminorest, einen Pyrrolidinylrest oder einen Piperidinylrest, der unsubstituiert oder ein- oder zweifach durch Methyl oder Halogen substituiert ist, steht.

**7.** Verbindung der Formel (I) nach Anspruch 1 oder Anspruch 2, in der $R_5$ für eine Gruppe $-CH_2NR_{11}R_{12}$ steht, worin $-NR_{11}R_{12}$ für einen Ethylisobutylaminorest, einen Ethylisopropylaminorest, einen Ethyl-*tert*-butylaminorest, einen Diisopropylaminorest, einen Cyclopentylmethylaminorest, einen Cyclopentylethylaminorest oder einen Piperidinylrest, der unsubstituiert oder ein- oder mehrfach durch Methyl oder Halogen substituiert ist, steht.

**8.** Verbindung nach Anspruch 1 oder Anspruch 2 der Formel:

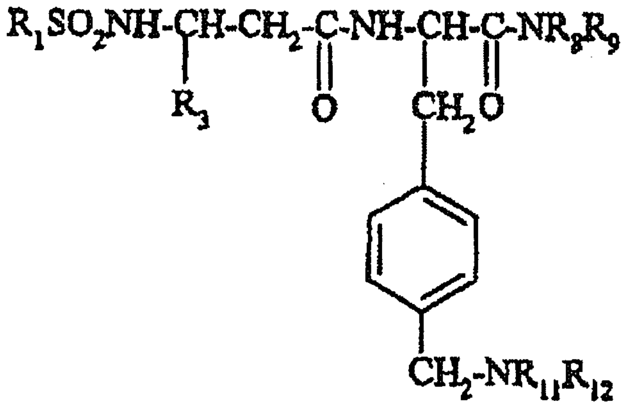

(Ia)

worin:

- $R_1$ für eine 2,4-Dichlor-3-methylphenylgruppe, eine Naphthylgruppe, eine 6-Methoxynaphth-2-ylgruppe, eine 3-Methylbenzothiophen-2-ylgruppe, eine 3-Methyl-5-chlorbenzothiophen-2-ylgruppe, eine 3-Methyl-5-methoxybenzothiophen-2-ylgruppe, eine 3-Methyl-6-methoxybenzothiophen-2-ylgruppe oder eine 3-Methyl-1-benzofur-2-ylgruppe steht;
- $R_3$ für eine Benzo[1,3]dioxol-5-ylgruppe oder eine Phenylgruppe, die unsubstituiert oder durch ein Halogen substituiert ist, steht;
- $R_8$ und $R_9$ gemeinsam mit dem Stickstoffatom, an das sie gebunden sind, einen Di-($C_1$-$C_4$)-alkylaminorest, einen Pyrrolidinylrest oder einen Piperidinylrest, der unsubstituiert oder ein- oder zweifach durch Methyl oder Halogen substituiert ist, bilden;
- $R_{11}$ und $R_{12}$ gemeinsam mit dem Stickstoffatom, an das sie gebunden sind, einen Ethylisobutylaminorest, einen Ethylisopropylaminorest, einen Ethyl-tert-butylaminorest, einen Diisopropylaminorest, einen Cyclopentylmethylaminorest, einen Cyclopentylethylaminorest oder einen Piperidinylrest, der unsubstituiert oder ein- oder mehrfach durch Methyl oder Halogen substituiert ist, bilden;

sowie Salze davon mit anorganischen oder organischen Säuren und/oder Solvate davon und/oder Hydrate davon.

**9.** Verbindung nach Anspruch 1, ausgewählt unter:

- (R,R)-2-((3-(1,3-Benzodioxol-5-yl)-3-(((6-methoxy-2-naphthyl)sulfonyl)amino)propanoyl)amino)-3-(4-((2,6-cis-dimethyl-1-piperidinyl)-methyl)phenyl)-N-isopropyl-N-methylpropanamid;
- (R,R)-2-((3-(1,3-Benzodioxol-5-yl)-3-((2-naphthylsulfonyl)amino)propanoyl)amino)-3-(4-((cyclopentyl(ethyl)amino)methyl)phenyl)-N-isopropyl-N-methylpropanamid;
- (R,R)-3-(4-((2,6-cis-Dimethyl-1-piperidinyl)methyl)phenyl)-2-((3-(((6-methoxy-2-naphthyl)sulfonyl)amino)-3-phenylpropanoyl)amino)-N-isopropyl-N-methyl propanamid;
- (R,R)-2-((3-(1,3-Benzodioxol-5-yl)-3-(((6-methoxy-2-naphthyl)sulfonyl)amino)propanoyl)amino)-3-(4-((*tert*-butyl(ethyl)amino)methyl)phenyl)-N-isopropyl-N-methylpropanamid;
- (R,R)-2-((3-(1,3-Benzodioxol-5-yl)-3-(((3-methyl-1-benzothiophen-2-yl)sulfonyl)amino)propanoyl)amino)-3-(4-((2,6-cis-dimethyl-1-piperidinyl)methyl)phenyl)-N-isopropyl-N-methylpropanamid;
- (R,R)-3-(4-((2,6-cis-Dimethyl-1-piperidinyl)methyl)phenyl)-2-((3-(((5-methoxy-3-methyl-1-benzothiophen-2-yl)sulfonyl)amino)-3-phenylpropanoyl)amino)-N-isopropyl-N-methylpropanamid;
- (R,R)-N-(4-((2,6-cis-Dimethyl-1-piperidinyl)methyl)benzyl)-3-(((6-methoxy-2-naphthyl)sulfonyl)amino)-3-phenyl-N-(1-piperidinylcarbonyl)propanamid;
- (R,R)-2-((3-(4-Chlorphenyl)-3-(((6-methoxy-2-naphthyl)sulfonyl)amino)propanoyl)amino)-3-(4-((2,6-cis-dimethyl-1-piperidinyl) methyl) phenyl)-N-isopropyl-N-methylpropanamid;
- (R,R)-3-(4-((2,6-cis-Dimethyl-1-piperidinyl)methyl)phenyl)-2-((3-(((6-methoxy-2-naphthyl)sulfonyl)amino)-3-phenylpropanoyl)amino)-N,N-diethylpropanamid;
- (R,R)-2-((3-(3-Fluorphenyl)-3-(((6-methoxy-2-naphthyl)sulfonyl)amino)propanoyl)amino)-3-(4-((2,6-cis-dimethyl-1-piperidinyl)methyl)phenyl)-N-isopropyl-N-methylpropanamid;
- (R,R)-2-((3-(1,3-Benzodioxol-5-yl)-3-(((3-methyl-1-benzofuran-2-yl)sulfonyl)amino)propanoyl)amino)-3-(4-((2,6-cis-dimethyl-1-piperidinyl)methyl)phenyl)-N-isopropyl-N-methylpropanamid;

- (R,R)-2-((3-(1,3-Benzodioxol-5-yl)-3-((2-naphthylsulfonyl)amino)propanoyl)amino)-3-(3-((*tert*-butyl(ethyl)amino)methyl)phenyl)-N-isopropyl-N-methylpropanamid;
- (R,R)-3-(4-(7-Azabicyclo[2.2.1]hept-7-ylmethyl)phenyl)-2-((3-(1,3-benzodioxol-5-yl)-3-(((6-methoxynaphthyl)sulfonyl)amino)propanoyl)amino)-N-isopropyl-N-methylpropanamid;

sowie Salze davon und/oder Solvate davon und/oder Hydrate davon.

**10.** (R,R)-2-((3-(1,3-Benzodioxol-5-yl)-3-(((6-methoxy-2-naphthyl)sulfonyl)amino)propanoyl)amino)-3-(4-((2,6-cis-dimethyl-1-piperidinyl)methyl)phenyl)-N-isopropyl-N-methylpropanamid;
sowie Salze davon mit anorganischen oder organischen Säuren und/oder Solvate davon und/oder Hydrate davon.

**11.** Verfahren zur Herstellung einer Verbindung der Formel (I), Salzen davon und/oder Solvaten davon oder Hydraten davon, **dadurch gekennzeichnet, daß** man:

eine Säure oder ein funktionelles Derivat dieser Säure der Formel:

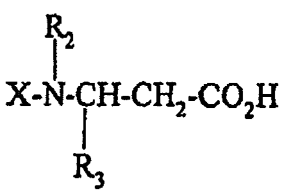

(II)

worin $R_2$ und $R_3$ die für eine Verbindung der Formel (I) in Anspruch 1 angegebene Bedeutung besitzen, X für Wasserstoff, eine Gruppe $R_1SO_2$-, worin $R_1$ die für eine Verbindung der Formel (I) in Anspruch 1 angegebene Bedeutung besitzt, oder eine N-Schutzgruppe steht, mit einer Verbindung der Formel:

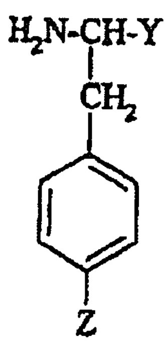

(III)

worin Y für $R_4$ mit der für eine Verbindung der Formel (I) in Anspruch 1 angegebenen Bedeutung oder ($C_1$-$C_4$)-Alkoxycarbonyl steht und Z für $R_5$ mit der für eine Verbindung der Formel (I) in Anspruch 1 angegebenen Bedeutung oder eine -CN-Gruppe steht, mit der Maßgabe, daß dann, wenn Y für $R_4$ steht, das für durch eine Gruppe $-CH_2NR_{11}R_{12}$ substituiertes Phenyl steht, Z für $R_5$ steht, das für eine Gruppe $-CH_2NR_{11}R_{12}$ steht, wobei $R_{11}$ und $R_{12}$ die für eine Verbindung der Formel (I) in Anspruch 1 angegebene Bedeutung besitzen, umsetzt und

- dann, wenn $X = R_1SO_2$-, $Y = R_4$ und $Z = R_5$, die erwartete Verbindung der Formel (I) erhält:

- oder dann, wenn $X \neq R_1SO_2$- und/oder $Y \neq R_4$ und/oder $Z \neq R_5$, d. h. wenn mindestens eine der Gruppen X, Y und Z für X = H oder eine N-Schutzgruppe, Y = ($C_1$-$C_4$)-Alkoxycarbonyl bzw. Z = -CN steht, die so erhaltene Verbindung der Formel:

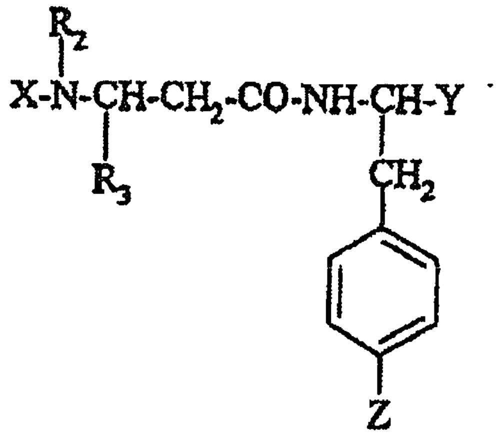

(IV)

einem oder mehreren der folgenden Schritte unterwirft:

- wenn X für eine N- Schutzgruppe steht, spaltet man diese Gruppe ab und setzt die so erhaltene Verbindung der Formel:

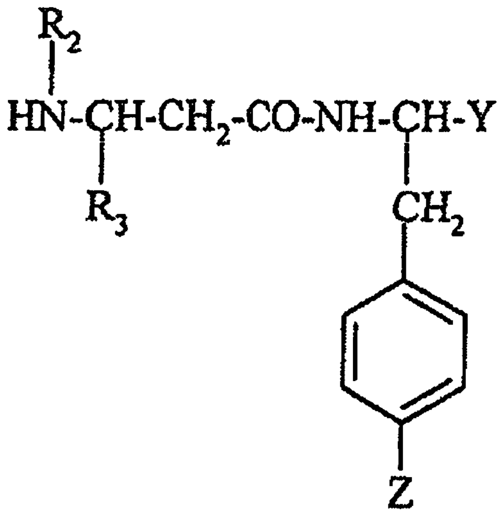

(V)

mit einem Sulfonylhalogenid der Formel:

$R_1SO_2$-Hal (VI)

worin Hal für ein Halogen steht, um;

- wenn Y für ($C_1$-$C_4$)-Alkoxycarbonyl steht, hydrolysiert man dieses und setzt die so erhaltene Säure oder ein funktionelles Derivat dieser Säure der Formel:

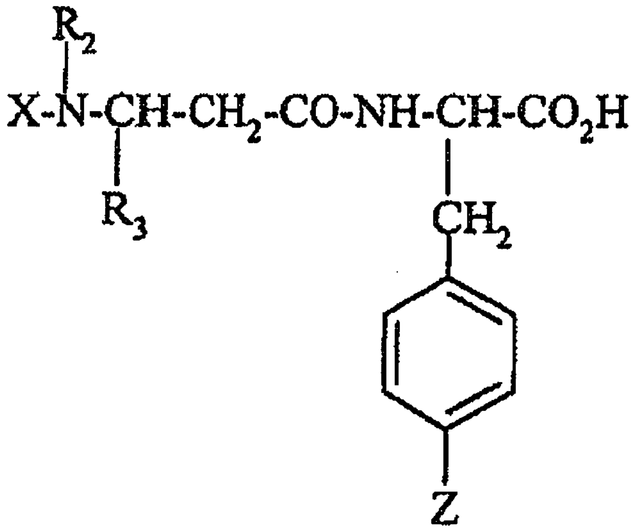

(VII)

mit einer Verbindung der Formel:

$HNR_8R_9$ (VIII)

worin $R_8$ und $R_9$ die für eine Verbindung der Formel (I) gemäß Anspruch 1 angegebene Bedeutung besitzen, um;

- wenn Z für eine -CN-Gruppe steht, wandelt man diese Gruppe in $R_5$ um.

**12.** Verbindung der Formel:

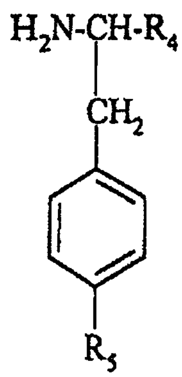

(III')

worin:

- $R_4$ für eine Gruppe $-CONR_8R_9$; eine Gruppe $-CSNR_8R_9$; eine Gruppe $-COR_{13}$; eine unsubstituierte oder ein- oder mehrfach durch $R_{10}$ substituierte Phenylgruppe oder einen unter Pyridyl, Imidazolyl, Furyl, Benzimidazolyl, Benzothiazol-2-yl und Benzo[1,3]dioxol-5-yl ausgewählten heterocyclischen Rest steht, wobei die Reste unsubstituiert oder durch eine Methylgruppe substituiert sind;
- $R_5$ für eine Gruppe $-CH_2NR_{11}R_{12}$ oder $-CH_2N(O)R_{11}R_{12}$ steht;
- $R_8$ und $R_9$ jeweils unabhängig voneinander für Wasserstoff; eine $(C_1\text{-}C_4)$-Alkylgruppe; eine $(C_3\text{-}C_7)$-Cycloalkylgruppe; eine $(C_3\text{-}C_7)$-Cycloalkyl-$(C_1\text{-}C_4)$-alkylgruppe oder eine $\omega$-$(C_1\text{-}C_4)$-Dialkylamino-$(C_2\text{-}C_4)$-alkylgruppe stehen;
- oder $R_8$ und $R_9$ gemeinsam mit dem Stickstoffatom, an das sie gebunden sind, einen unter Pyrrolidinyl, Piperidinyl, Morpholin-4-yl, Thiomorpholin-4-yl, Piperazin-1-yl, 4-Methylpiperazin-1-yl, 4-Methylpiperidin-1-yl, 2-Methylpiperidin-1-yl, 4,4-Dimethylpiperidin-1-yl, 4,4-Difluorpiperidin-1-yl, 4-Trifluormethylpiperidin-1-yl, 4-Methoxypiperidin-1-yl, 3,4-Dihydropiperidin-1-yl, Azepin-1-yl und Cyclohexylspiro-4-piperidin-1-yl ausgewählten heterocyclischen Rest bilden;

- $R_{10}$ für ein Halogenatom; eine $(C_1-C_4)$-Alkylgruppe; eine Hydroxygruppe oder eine $(C_1-C_6)$-Alkoxygruppe steht und außerdem für eine Gruppe $-CH_2NR_{11}R_{12}$ stehen kann, wenn $R_5$ für eine Gruppe $-CH_2NR_{11}R_{12}$ steht, wobei die Gruppen dann identisch sind;
- $R_{11}$ und $R_{12}$ jeweils unabhängig voneinander für Wasserstoff; eine $(C_1-C_6)$ -Alkylgruppe; eine $(C_2-C_4)$-Alkenylgruppe; eine $(C_3-C_7)$-Cycloalkylgruppe; eine $(C_3-C_7)$-Cycloalkyl-$(C_1-C_4)$-alkylgruppe; eine ω-Hydroxy-$(C_2-C_4)$-alkylengruppe; eine ω-Methoxy-$(C_2-C_4)$-alkylengruppe; eine ω-Trifluormethyl-$(C_2-C_4)$-alkylengruppe oder eine ω-Halogen-$(C_2-C_4)$-alkylengruppe stehen;
- oder $R_{11}$ und $R_{12}$ gemeinsam mit dem Stickstoffatom, an das sie gebunden sind, einen unter Azetidinyl, Pyrrolidinyl, Morpholin-4-yl, Thiomorpholin-4-yl, Piperidin-1-yl, Piperazin-1-yl, 1,2,3,6-Tetrahydropyrid-1-yl, 2, 3, 4, 5-Tetrahydropyridinium, Decahydrochinolyl, Decahydroisochinolyl, Tetrahydroisochinolyl, Octahydro-1*H*-isoindolyl, $(C_4-C_6)$-Cycloalkylspiropiperidinyl, 3-Azabicyclo[3.1.0]hexyl und 7-Azabicyclo[2.2.1]heptan-7-yl ausgewählten mono- oder bicyclischen heterocyclischen Rest bilden, der unsubstituiert oder ein- oder mehrfach durch ein Halogenatom oder eine $(C_1-C_4)$-Alkylgruppe, eine Hydroxygruppe, eine $(C_1-C_4)$-Alkoxygruppe, eine Trifluormethylgruppe oder eine Difluormethylengruppe substituiert ist;
- $R_{13}$ für eine Phenylgruppe, eine Thiazol-2-ylgruppe oder eine Pyridylgruppe steht;

sowie Salze davon mit anorganischen oder organischen Säuren in Form von racemischen Gemischen oder reinen Enantiomeren.

**13.** Pharmazeutische Zusammensetzung, enthaltend als Wirkstoff eine Verbindung nach einem der Ansprüche 1 bis 8 oder ein pharmazeutisch unbedenkliches Salz und/oder Solvat und/oder Hydrat davon.

**14.** Arzneimittel, **dadurch gekennzeichnet, daß** es aus einer Verbindung nach einem der Ansprüche 1 bis 8 besteht.

**15.** Verwendung einer Verbindung nach einem der Ansprüche 1 bis 8 oder eines pharmazeutisch unbedenklichen Salzes und/oder Solvats und/oder Hydrats davon zur Herstellung von Arzneimitteln zur Behandlung von bradykininabhängigen Pathologien.

**16.** Verwendung nach Anspruch 15 zur Herstellung von Arzneimitteln zur Behandlung von Entzündungspathologien und persistenten oder chronischen Entzündungskrankheiten.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9725315 A
- EP 236163 A
- WO 9708145 A
- WO 9840055 A
- EP 0614911 A
- EP 0236164 A
- WO 9822443 A
- US 2424063 A
- WO 9522547 A
- WO 9403437 A
- EP 540041 A


### Littérature non-brevet citée dans la description


- **D. REGOLI et al.** *Pharmacol. Rev.,* 1980, vol. 32, 1-46
- **J.G. MENKE et al.** *J. Biol. Chem.,* 1994, vol. 269 (34), 21583-21586
- **J.F. HESS.** *Biochem. Biophys. Res. Commun.,* 1992, vol. 184, 260-268
- Protective Groups in Organic Chemistry. Plenum Press, 1973
- Protective Groups in Organic Synthesis. John Wiley et Sons, 1991
- The Peptides. Academic Press, 1979, vol. 1, 65-104
- *Ann. Rev. Biochem.,* 1986, vol. 55, 855-878
- *Synth. Commun.,* 1990, vol. 20 (3), 459-467
- *Farmaco, Ed. Sci.,* 1988, vol. 43 (7/8), 597-612
- *J. Am. Chem. Soc.,* 1940, vol. 62, 511-512
- *J. Org. Chem.,* 1992, vol. 57, 2631-2641
- *J. Het. Chem.,* 1988, vol. 25, 639-641
- *Eur. J. Med. Chem. Chim. Therap.,* 1992, vol. 27 (9), 961-965
- *J. Med. Chem.,* 1997, vol. 40 (26), 4308-4318
- *Bull. Soc. Chim. Fr.,* 1987, vol. 6, 1079-1083
- *Biorg. Med. Chem.,* 1994, vol. 2 (9), 881
- *Tetrahedron Lett.,* 1991, vol. 32 (44), 6327-6328
- *Zh. Obshch. Khim.,* 1958, vol. 28, 213-215
- *Chem. Heterocycl. Compd., (Engl. Transl.,* 1969, vol. 5, 453-456
- *J. American. Chem. Soc.,* 1957, vol. 79, 4524-4527
- *Tetrahedron Lett.,* 2000, vol. 41 (31), 5803-5806
- *Tetrahedron Lett.,* 2000, vol. 41, 5803-5806
- *Bull. Soc. Chim. Fr.,* 1973, 2985 2987 2988
- *J. Am. Chem. Soc.,* 1982, vol. 104 (3), 730
- *Tetrahedron Lett.,* 1996, 1137
- *Tetrahedron Asymmetry,* 1992, vol. 3 (5), 637-650
- *J. Pharm. Sci.,* 1963, vol. 52, 1191
- *J. Org. Chem.,* 1979, vol. 44 (5), 771-7
- *Chem. Pharm. Bull.,* 1993, vol. 41 (11), 1971-1986
- *J. Org. Chem.,* 1970, vol. 35 (11), 3663-3666
- *Synth. Commun.,* 1999, vol. 29 (10), 1747-1756
- *J. Chem. Soc. Perkin Trans.,* 1984, vol. 2, 737-744
- *J. Org. Chem. USSR (Engl. Trans 1,* 1992, vol. 28 (3.1), 374-380
- *Tetrahedron,* 1999, vol. 55 (31), 9439-9454
- *J. Amer. Chem. Soc.,* 1955, vol. 77, 4100-4102
- *Chem. Ber.,* 1991, 791-801
- *Tetrahedron,* 1985, vol. 41 (22), 5061-5087
- *Tetrahedron,* 1995, vol. 51/46, 12731-12744
- *Synthesis,* 1996, vol. 8, 991-996
- **K.H. SCHNECK et al.** *Eur. J. Pharmacol.,* 1994, vol. 266, 277-282
- **D.G. SAWUTZ et al.** *Eur. J. Pharmacol.,* 1992, vol. 227, 309-315
- **L. LEVESQUE et al.** *Br. J. Pharmacol.,* 1993, vol. 109, 1254-1262
- **F. OURY DONAT et al.** *J. Neurochem.,* 1994, vol. 62 (4), 1399-1407
- **T. LINDMARK et al.** *J. Pharmacol. Exp. Therap.,* 1995, vol. 275 (2), 958-964
- *Pain,* 2000, vol. 86, 265-271
- *Pain,* 1987, 203-114
- *Brit. Med. J.,* 1993, vol. 110, 1441-1444
- *Drug News and Perspectives,* 1994, vol. 10 (7), 603-611
- *Brit. J. Pharmacol.,* 1993, vol. 110, 193-198
- *Pain,* 1993, vol. 53, 191-197

- *Brit. J. Pharmacol.,* 1995, vol. 114, 1005-1013
- *Brit. J. Pharmacol.,* 1993, vol. 109, 14-17
- *Abst. 14th Intern. Symp. on Kinins,* 10 Septembre 1995, vol. C49
- *Tetrahedron : Asymmetry,* 1992, vol. 3 (5), 637-650
- *J. Med. Chem.,* 1999, vol. 42, 3557-3571